# EUROPEAN PATENT SPECIFICATION

(11) **EP 4 408 845 B1**
(45) Date of publication and mention of the grant of the patent: **18.11.2026**
(21) Application number: 22786081.4
(22) Date of filing: 30.09.2022
(51) Int. Cl.: C07D 471/12, A61P 3/04, A61P 3/10, A61P 7/00, A61P 29/00, A61P 35/00, A61P 37/00, A61K 31/5025

(54) **NOVEL COMPOUNDS**
NEUARTIGE VERBINDUNGEN
NOUVEAUX COMPOSÉS

(30) Priority: 30.09.2021 EP 21200374
(43) Date of publication of application: 07.08.2024
(73) Proprietor: Sitryx Therapeutics Limited, Magdalen Centre, The Oxford Science Park Oxford OX4 4GA (GB)
(72) Inventor: COUSIN, David, Nottingham Nottinghamshire NG1 1GR (GB); FYFE, Matthew Colin Thor, Oxford OX4 4GA (GB); BARBA, Oscar, Oxford OX4 4GA (GB)
(74) Representative: Sagittarius IP
(86) International application number: PCT/GB2022/052482
(87) International publication number: WO 2023/052783

(56) References cited:
- WO-A1-2012/151450
- WO-A1-2014/160440
- WO-A1-2019/035865
- WO-A1-2020/167976
- WO-A2-2010/042867
- JIAN-KANG JIANG ET AL: "Evaluation of thieno[3,2-b]pyrrole[3,2-d]pyridazinones as activators of the tumor cell specific M2 isoform of pyruvate kinase", BIOORGANIC & MEDICINAL CHEMISTRY LETTERS, vol. 20, no. 11, 11 April 2010 (2010-04-11), pages 3387 - 3393, XP055039623, ISSN: 0960-894X, DOI: 10.1016/j.bmcl.2010.04.015
- BRUEL AMÉLIE ET AL: "Synthesis and biological evaluation of new 5-benzylated 4-oxo-3,4-dihydro-5H-pyridazino[4,5-b]indoles as PI3K[alpha] inhibitors", EUROPEAN JOURNAL OF MEDICINAL CHEMISTRY, vol. 57, 10 September 2012 (2012-09-10), AMSTERDAM, NL, pages 225 - 233, XP055866863, ISSN: 0223-5234, DOI: 10.1016/j.ejmech.2012.09.001
- PANATHUR NAVEEN ET AL: "Synthesis of novel 5-[(1,2,3-triazol-4-yl)methyl]-1-methyl-3H-pyridazino[4,5-b]indol-4-one derivatives by click reaction and exploration of their anticancer activity", MEDICINAL CHEMISTRY RESEARCH, BIRKHAEUSER, BOSTON, US, vol. 25, no. 1, 5 October 2015 (2015-10-05), pages 135 - 148, XP035802895, ISSN: 1054-2523, [retrieved on 20151005], DOI: 10.1007/S00044-015-1473-Y

## Description

### Field of the invention

The present invention relates to compounds and their use in treating or preventing an inflammatory disease, a disease associated with an undesirable immune response, cancer, obesity, a diabetic disease or a blood disorder, and to related compositions and intermediate compounds.

### Background of the invention

Pyruvate kinase (PK) is the enzyme responsible for the final rate-limiting step of glycolysis, catalyzing phosphoenolpyruvic acid (PEP) and ADP to pyruvate and ATP. Four PK isoforms exist in mammals from two separate genes (Alves-Filho *et al.,* 2016). PKL and PKR, products of the Pklr gene, are expressed in the liver and red blood cells, respectively. PKM1 and 2 are alternatively spliced products of the Pkm gene. PKM1 is expressed in tissues with high energy demands such as heart, muscle, and brain, and PKM2 is expressed in embryonic tissues, cancer and normal proliferating cells such as lymphocytes and intestinal epithelial cells. Whereas PKM1 is a constitutively active enzyme, PKM2 is a low-activity enzyme that relies on allosteric activation by multiple endogenous regulators, for example, the upstream glycolytic intermediate, fructose-1,6-bisphosphate (FBP). Binding of these allosteric regulators induces conformational changes that promote tetramerization of PKM2 leading to an increase in the last rate-limiting step of glycolysis. Pyruvate will enter the TCA cycle in the mitochondria where it is used to generate ATP through oxidative phosphorylation. Without allosteric activation PKM2 takes on a dimeric or monomeric form with low enzymatic activity, leading to accumulation of glycolytic intermediates which meet the requirements for biosynthetic precursors of the activated or proliferating cell. Dimeric PKM2 can also translocate to the nucleus where it can further promote aerobic glycolysis and regulate transcriptional activity, acting as a protein kinase to target transcription factors and histones.

Cancer cells primarily use glycolysis to generate cellular energy and biosynthesis intermediates, termed the Warburg effect and PKM2 plays a dominant role in glycolysis to achieve the nutrient demands of cancer cell proliferation (Chhipa *et al.,* 2018). PKM2 is overexpressed in almost all cancers and has been shown to promote proliferation and metastasis of tumour cells. In addition to controlling glycolytic flux, the non-metabolic role of PKM2 as a coactivator and protein kinase contribute to tumorigenesis (Dong *et al.,* 2016). PKM2 binds directly to and phosphorylates histone H3 leading to expression of c-Myc and Cyclin D1 and the proliferation of cancer cells. Activation of PKM2 tetramer by small molecules could be an attractive therapy in cancer to contain tumour growth by preventing the non-metabolic functions of dimeric PKM2.

Following activation or an inflammatory stimulus, PKM2 is upregulated in many immune cells including macrophages and T cells (Pålsson-McDermott *et al.,* 2020). The non-metabolic roles of dimeric PKM2 have been shown to regulate immune responses: PKM2 acts as a transcriptional coactivator of Hif-1α, b-catenin and STAT3 leading to expression of pro-inflammatory cytokines such as IL-1β and TNFα. Activation of PKM2 by small molecules to prevent nuclear translocation could have therapeutic benefit in a range of inflammatory and auto-immune conditions, such as rheumatoid arthritis, inflammatory bowel diseases, inflammatory skin pathologies, coronary artery disease and multiple sclerosis.

In diabetes, PKM2 regulates glucose responsive pancreatic beta-cell function and protects from metabolic stress (Abulizi *et al.,* 2020; Lewandowski *et al.,* 2020). Dimeric PKM2 plays a role in aberrant glycolysis by promoting the accumulation of HIF-1α, and in diabetic nephropathy PKM2 is associated with a pathogenic role in glomerular injury and epithelial-to-mesenchymal transition leading to fibrosis (Liu *et al.,* 2020). PKM2 activation has been shown to amplify insulin release and improve insulin sensitivity and protect against progression of diabetic glomerular pathology and kidney fibrosis (Liu *et al.,* 2020; Abulizi *et al.,* 2020; Lewandowski *et al.,* 2020; Qi *et al.,* 2017).

Obesity is defined as abnormal or excessive fat accumulation that presents a risk to health, and is linked to a higher incidence of type 2 diabetes and cardiovascular disease. This metabolic disorder is strongly associated with insulin resistance and the adverse impact on glucose metabolism and disposal in obese subjects (Barazzoni *et al.,* 2018). Studies on 3T3-L1 adipocytes exposed to varying levels of insulin resulted in significant increases in PKM2 mRNA levels, independent of the levels of glucose in the media (Puckett *et al.,* 2021). Work on the impact of altered PKM2 phosphorylation status and resulting decreased catalytic activity, has identified PKM2 as a potential contributor to insulin resistance in the adipose tissue and made an association with metabolic status in humans (Bettaieb *et al.,* 2013). Restoring PKM2 activity with a small molecule allosteric activator has been shown to improve insulin sensitivity (Abulizi *et al.* 2020; Lewandowski *et al.* 2020) and warrants further investigation as a novel target for pharmacological intervention in obesity.

Pyruvate kinase deficiency (PKD) is one of the most common enzyme defects in erythrocytes, that presents as hemolytic anemia, the accelerated destruction of red blood cells (Bianchi *et al.,* 2020). Mature red blood cells depend entirely on glycolysis for maintaining cell integrity and function, and so pyruvate kinase plays a crucial role in erythrocyte metabolism and survival. The inherited mutations in PKR enzymes lead to dysregulation of its catalytic activity and cause a deficit in cellular energy within the red blood cell, as evidenced by lower pyruvate kinase enzyme activity, a decline in ATP levels and a build-up of upstream metabolites. PKR decreased activity has also been linked to changes in the erythrocytes morphology and cell membrane surface suggesting a wider involvement of this enzyme in the entire lifespan of these cells (Cangado *et* al., 2018). PK-deficient erythrocytes are prematurely removed from the circulation by the spleen through accelerated hemolysis leading to iron accumulation. Increase and/or restoration of PKR activity to quasi-basal levels is thought to have potential to treat the PK deficiency-related complications. The current standard of care for PKD is supportive, including blood transfusions, splenectomy, chelation therapy to address iron overload and/or interventions for other treatment- and disease-related morbidities. There is no approved therapy to treat the underlying cause of PK deficiency. Activation of the PKR enzyme with a small molecule allosteric activator increases PK enzyme activity and enhanced glycolysis in erythrocytes from patients with PK deficiency (Kung *et al.,* 2017). The most advanced PK activator being studied in clinical settings is Mitapivat (AG-348); patients treated with this agent reported an increase in basal hemoglobin levels (Grace *et al.,* 2019) thus reinforcing the potential to treat blood disorders such as PK-deficiency with a small-molecule PKR modulator.

Pharmacological intervention by using small molecules agonists such as TEPP-46 and DASA-58 have been utilised extensively *in vitro* and *in vivo* biological settings to demonstrate the several potential benefits provided by augmenting PK activity through allosteric modulation (Yi *et al.,* 2021). Although these compounds show a good level of *in vitro* activity, their ADME and pharmacokinetic/pharmacodynamic profiles have prevented them from being developed for the treatment of human disease. The structure of TEPP-46 is as follows:

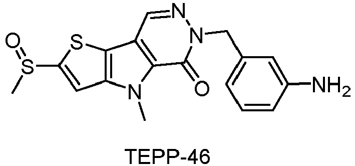

WO2020/167976A1 (Agios Pharmaceuticals, Inc.) describes compounds that are said to regulate PK activity, for the treatment of cancer, obesity and diabetes related disorders. WO2019/035865A1 (Agios Pharmaceuticals, Inc.) describes methods of modulating PKM2 in a subject by administering certain compounds and the treatment of disorders including cancer, obesity and diabetes related disorders. WO2012/151450A1 (Agios Pharmaceuticals, Inc.) describes compounds that are said to activate PK, for use in a method for increasing lifetime of the red blood cells. WO2010/042867A2 (US Health) describes compounds that are said to activate PKM2, for the treatment of disorders such as cancer and anaemia. Further compounds that are said to be PKM2 activators are described by Jiang *et al.* 2010.

There remains a need to identify and develop new disease modifying PK modulators to meet several unmet medical needs linked to PK disfunction, in particular the need to develop compounds that demonstrate suitable activity while also having favourable physical-chemical parameters. The compounds herein described as PK modulators, in particular PKM2 and/or PKLR modulators, in particular PKM2 and/or PKLR activators, address the aforementioned unmet needs by exhibiting suitable affinity and functional activity for PK enzymes, in particular PKM2 and/or PKLR, while having better overall physical/chemical properties with improved ADME and PK profiles making them suitable for the treatment of human diseases linked to an altered function of pyruvate kinase enzymes expression and/or activity.

### Summary of the invention

The present invention provides a compound of formula (Ia): 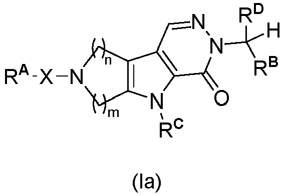
R^{A} is C₁₋₁₀ alkyl, C₂₋₁₀ alkenyl, C₂₋₁₀ alkynyl, C₃₋₁₀ cycloalkyl, phenyl or 5-10 membered heteroaryl; wherein R^{A} is optionally substituted on an available atom by one or more R^{1A}, wherein each R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2A}R^{3A} , CO₂H, CONR^{2A}R^{3A} and C₃₋₆ cycloalkyl;
   R^{2A} and R^{3A} are independently selected from the group consisting of H and C₁₋₆ alkyl; or R^{2A} and R^{3A} together with the N atom to which they are attached combine to form a 5-7 membered heterocyclic ring which is optionally substituted on an available atom by one or more groups selected from C₁₋₂ alkyl and oxo;
R^{B} is phenyl, phenyl fused to a 5-7 membered heterocyclic ring, 5-10 membered heteroaryl, or 4-7 membered heterocyclyl; wherein R^{B} is optionally substituted on an available atom by one or more R^{1B}, wherein each R^{1B} is independently selected from the group consisting of halo, C₁₋₆ alkyl, C₁₋₆ hydroxyalkyl, C₁₋₆ aminoalkyl, C₁₋₆ haloalkyl, hydroxy, OC₁₋₆ alkyl, OC₁₋₆ haloalkyl, cyano, NR^{2B}R^{3B}, CO₂H, CONR^{2B}R^{3B}, S(O)₂NR^{2B}R^{3B}, S(O)₂C₁₋₆ alkyl, C₃₋₆ cycloalkyl and oxo;
   R^{2B} and R^{3B} are independently selected from the group consisting of H and C₁₋₆ alkyl; or R^{2B} and R^{3B} together with the N atom to which they are attached combine to form a 5-7 membered heterocyclic ring which is optionally substituted on an available atom by one or more groups selected from C₁₋₂ alkyl and oxo;
R^{C} is H or C₁₋₂ alkyl;
R^{D} is H, C₁₋₂ alkyl, C₁₋₂ hydroxyalkyl or C₁₋₂ methoxyalkyl;
X is C=O, S(=O)₁₋₂, -CH₂-S(=O)₁₋₂-, S(=O)(=NH) or -NH-S(=O)₂-;
n is 0 to 4 and m is 0 to 4, wherein n + m is 2, 3 or 4;
or a pharmaceutically acceptable salt and/or solvate thereof.

Compounds of formula (la) are useful in therapy, in particular in the treatment of PK-mediated diseases, disorders and conditions. Compounds of formula (Ia) may be activators of the PK family of proteins, especially activators of PKM2 and/or PKLR.

The present invention provides a pharmaceutical composition comprising a compound of formula (la) or a pharmaceutically acceptable salt and/or solvate thereof.

The present invention provides a compound of formula (Ia) or a pharmaceutically acceptable salt and/or solvate thereof for use as a medicament.

The present invention provides a compound of formula (Ia) or a pharmaceutically acceptable salt and/or solvate thereof for use in treating or preventing a disease, disorder or condition associated with the function of PK, in particular PKM2 and/or PKLR.

The present invention provides a compound of formula (Ia) or a pharmaceutically acceptable salt and/or solvate thereof for use in treating or preventing an inflammatory disease, a disease associated with an undesirable immune response, cancer, obesity, a diabetic disease or a blood disorder.

Also provided are intermediate compounds for use in the preparation of compounds of formula (I).

### Detailed description of the invention

### Compounds of formula (la)

In one embodiment, the compound of formula (la) is a compound of formula (I): 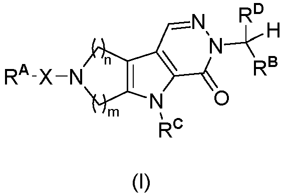 wherein,
R^{A} is C₁₋₁₀ alkyl, C₂₋₁₀ alkenyl, C₂₋₁₀ alkynyl, C₃₋₁₀ cycloalkyl, phenyl or 5-10 membered heteroaryl; wherein R^{A} is optionally substituted on an available carbon atom by one or more R^{1A}, wherein R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2A}R^{3A}, CO₂H, CONR^{2A}R^{3A} and C₃₋₆ cycloalkyl;
   R^{2A} and R^{3A} are independently selected from the group consisting of H and C₁₋₆ alkyl; or R^{2A} and R^{3A} together with the N atom to which they are attached combine to form a 5-7 membered heterocyclic ring which is optionally substituted on an available atom by one or more groups selected from C₁₋₂ alkyl and oxo;
R^{B} is phenyl, phenyl fused to a 5-7 membered heterocyclic ring, 5-10 membered heteroaryl, or 4-7 membered heterocyclyl; wherein R^{B} is optionally substituted on an available carbon atom by one or more R^{1B}, wherein R^{1B} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2B}R^{3B}, CO₂H, CONR^{2B}R^{3B}, C₃₋₆ cycloalkyl and oxo;
   R^{2B} and R^{3B} are independently selected from the group consisting of H and C₁₋₆ alkyl; or R^{2B} and R^{3B} together with the N atom to which they are attached combine to form a 5-7 membered heterocyclic ring which is optionally substituted on an available atom by one or more groups selected from C₁₋₂ alkyl and oxo;
R^{C} is H or C₁₋₂ alkyl;
R^{D} is H, C₁₋₂ alkyl, C₁₋₂ hydroxyalkyl or C₁₋₂ methoxyalkyl;
X is C=O, S(=O)₁₋₂, -CH₂-S(=O)₁₋₂-, S(=O)(=NH) or -NH-S(=O)₂-;
n is 0 to 4 and m is 0 to 4, wherein n + m is 2, 3 or 4;
or a pharmaceutically acceptable salt and/or solvate thereof.

In one embodiment, the compound of formula (la) is a compound of formula (Ib): 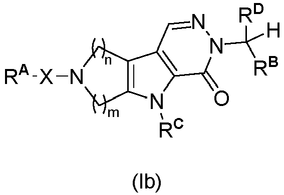 wherein,
R^{A} is C₁₋₁₀ alkyl, C₂₋₁₀ alkenyl, C₂₋₁₀ alkynyl, C₃₋₁₀ cycloalkyl, phenyl or 5-10 membered heteroaryl; wherein R^{A} is optionally substituted on an available carbon or nitrogen atom by one or more R^{1A}, wherein each R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2A}R^{3A}, CO₂H, CONR^{2A}R^{3A} and C₃₋₆ cycloalkyl;
   R^{2A} and R^{3A} are independently selected from the group consisting of H and C₁₋₆ alkyl; or R^{2A} and R^{3A} together with the N atom to which they are attached combine to form a 5-7 membered heterocyclic ring which is optionally substituted on an available atom by one or more groups selected from C₁₋₂ alkyl and oxo;
R^{B} is phenyl, phenyl fused to a 5-7 membered heterocyclic ring, 5-10 membered heteroaryl, or 4-7 membered heterocyclyl; wherein R^{B} is optionally substituted on an available carbon or nitrogen atom by one or more R^{1B}, wherein each R^{1B} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2B}R^{3B}, CO₂H, CONR^{2B}R^{3B}, C₃₋₆ cycloalkyl and oxo;
   R^{2B} and R^{3B} are independently selected from the group consisting of H and C₁₋₆ alkyl; or R^{2B} and R^{3B} together with the N atom to which they are attached combine to form a 5-7 membered heterocyclic ring which is optionally substituted on an available atom by one or more groups selected from C₁₋₂ alkyl and oxo;
R^{C} is H or C₁₋₂ alkyl;
R^{D} is H, C₁₋₂ alkyl, C₁₋₂ hydroxyalkyl or C₁₋₂ methoxyalkyl;
X is C=O, S(=O)₁₋₂, -CH₂-S(=O)₁₋₂-, S(=O)(=NH) or -NH-S(=O)₂-;
n is 0 to 4 and m is 0 to 4, wherein n + m is 2, 3 or 4;
or a pharmaceutically acceptable salt and/or solvate thereof.

Embodiments and preferences set out herein with respect to the compound of formula (la) apply equally to the pharmaceutical composition, compound for use and process aspects of the invention, and apply equally to compounds of formula (I) and (Ib).

The term "C₁₋₁₀ alkyl" refers to a straight or branched fully saturated hydrocarbon group having from 1 to 10 carbon atoms. The term encompasses methyl, ethyl, n-propyl, isopropyl, n-butyl, isobutyl, sec-butyl, tert-butyl, n-pentyl, n-heptyl, n-hexyl and n-octyl. Other branched variants such as heptyl-CH(CH₃)- and hexyl-CH(CH₃)- are also included. C₁₋₉ alkyl, C₁₋₈ alkyl, C₁₋₇ alkyl, C₁₋₆ alkyl, C₁₋₅ alkyl, C₁₋₄ alkyl, C₁₋₃ alkyl, C₁₋₂ alkyl, C₂₋₁₀ alkyl, C₂₋₉ alkyl, C₂₋₈ alkyl, C₂₋₇ alkyl, C₂₋₆ alkyl, C₂₋₅ alkyl, C₂₋₄ alkyl, C₂₋₃ alkyl, C₃₋₁₀ alkyl, C₃₋₉ alkyl, C₃₋₈ alkyl, C₃₋₇ alkyl, C₃₋₆ alkyl, C₃₋₅ alkyl, C₃₋₄ alkyl, C₄₋₁₀ alkyl, C₄₋₉ alkyl, C₄₋₈ alkyl, C₄₋₇ alkyl, C₄₋₆ alkyl, C₄₋₅ alkyl, C₅₋₁₀ alkyl, C₅₋₉ alkyl, C₅₋₈ alkyl, C₅₋₇ alkyl, C₅₋₆ alkyl, C₆₋₁₀ alkyl, C₆₋₉ alkyl, C₆₋₈ alkyl, C₆₋₇ alkyl, C₇₋₁₀ alkyl, C₇₋₉ alkyl, C₇₋₈ alkyl, C₈₋₁₀ alkyl, C₈₋₉ alkyl and C₉₋₁₀ alkyl are as defined above but contain different numbers of carbon atoms. The term "C₁₋₁₀ alkyl" also encompasses "C₁₋₁₀ alkylene" which is a bifunctional straight or branched fully saturated hydrocarbon group having from 1 to 10 carbon atoms. Example "C₁₋₁₀ alkylene" groups include methylene, ethylene, n-propylene, n-butylene, n-heptylene, n-hexylene and n-octylene.

The term "C₂₋₁₀ alkenyl" refers to a straight or branched hydrocarbon group having from 2 to 10 carbon atoms and at least one carbon-carbon double bond. The term encompasses, CH=CH₂, CH₂CH=CH₂, CH=CHCH₃, CH₂CH₂CH=CH₂, CH=CHCH₂CH₃, CH₂CH=CHCH₃, CH₂CH₂CH₂CH=CH₂, CH=CHCH₂CH₂CH₃, CH₂CH=CHCH₂CH₃, CH₂CH₂CH=CHCH₃, CH=CHCH=CHCH₃ and CH₂CH=CHCH=CH₂. Branched variants such as CH(CH₃)CH=CH₂ and CH=C(CH₃)₂ are also included. Other alkenyl groups, for example C₂₋₉ alkenyl, C₂₋₈ alkenyl, C₂₋₇ alkenyl, C₂₋₆ alkenyl, C₂₋₅ alkenyl, C₂₋₄alkenyl, C₂₋₃ alkenyl, C₃₋₁₀ alkenyl, C₃₋₉ alkenyl, C₃₋₈ alkenyl, C₃₋₇ alkenyl, C₃₋₆ alkenyl, C₃₋₅ alkenyl, C₃₋₄ alkenyl, C₄₋₁₀ alkenyl, C₄₋₉ alkenyl, C₄₋₈ alkenyl, C₄₋₇ alkenyl, C₄₋₆ alkenyl, C₄₋₅ alkenyl, C₅₋₁₀ alkenyl, C₅₋₉ alkenyl, C₅₋₈ alkenyl, C₅₋₇ alkenyl, C₅₋₆ alkenyl, C₆₋₁₀ alkenyl, C₆₋₉ alkenyl, C₆₋₈ alkenyl, C₆₋₇ alkenyl, C₇₋₁₀ alkenyl, C₇₋₉ alkenyl, C₇₋₈ alkenyl, C₃₋₁₀ alkenyl, C₈₋₉ alkenyl and C₉₋₁₀ alkenyl are as defined above but contain different numbers of carbon atoms. The term "C₂₋₁₀ alkenyl" also encompasses "C₂₋₁₀ alkenylene" which is a bifunctional straight or branched hydrocarbon group having from 2 to 10 carbon atoms and at least one carbon-carbon double bond. Example "C₂₋₁₀ alkenylene" groups include ethenylene, n-propenylene, n-butenylene, n-heptenylene, n-hexenylene and n-octenylene.

The term "C₂₋₁₀ alkynyl" refers to a straight or branched hydrocarbon group having from 2 to 10 carbon atoms and at least one carbon-carbon triple bond. The term encompasses, C≡CH, CH₂C≡CH, C≡CCH₃, CH₂CH₂C≡CH, C≡CCH₂CH₃, CH₂C≡CCH₃, CH₂CH₂CH₂C≡CH, C≡CCH₂CH₂CH₃, CH₂C≡CCH₂CH₃, CH₂CH₂C≡CCH₃, C≡CC≡CCH₃ and CH₂C≡CC≡CH. Branched variants such as CH(CH₃)C≡CH are also included. Other alkynyl groups, for example C₂₋₉ alkynyl, C₂₋₈ alkynyl, C₂₋₇ alkynyl, C₂₋₆ alkynyl, C₂₋₅ alkynyl, C₂₋₄ alkynyl, C₂₋₃ alkynyl, C₃₋₁₀ alkynyl, C₃₋₉ alkynyl, C₃₋₈ alkynyl, C₃₋₇ alkynyl, C₃₋₆ alkynyl, C₃₋₅ alkynyl, C₃₋₄ alkynyl, C₄₋₁₀ alkynyl, C₄₋₉ alkynyl, C₄₋₈ alkynyl, C₄₋₇ alkynyl, C₄₋₆ alkynyl, C₄₋₅ alkynyl, C₅₋₁₀ alkynyl, C₅₋₉ alkynyl, C₅₋₈ alkynyl, C₅₋₇ alkynyl, C₅₋₆ alkynyl, C₆₋₁₀ alkynyl, C₆₋₉ alkynyl, C₆₋₈ alkynyl, C₆₋₇ alkynyl, C₇₋₁₀ alkynyl, C₇₋₉ alkynyl, C₇₋₈ alkynyl, C₃₋₁₀ alkynyl, C₈₋₉ alkynyl and C₉₋₁₀ alkynyl are as defined above but contain different numbers of carbon atoms. The term "C₂₋₁₀ alkynyl" also encompasses "C₂₋₁₀ alkynylene" which is a bifunctional straight or branched hydrocarbon group having from 2 to 10 carbon atoms and at least one carbon-carbon triple bond. Example "C₂₋₁₀ alkynylene" groups include ethynylene, n-propynylene, n-butynylene, n-heptynylene, n-hexynylene and n-octynylene.

The term "C₁₋₆ haloalkyl" (e.g. C₁₋₅ haloalkyl, C₁₋₄ haloalkyl, C₁₋₃ haloalkyl, C₁₋₂ haloalkyl or C₁ haloalkyl) refers to a straight or a branched fully saturated hydrocarbon group containing the specified number of carbon atoms and at least one halogen atom, such as fluoro or chloro, especially fluoro. An example of haloalkyl is CF₃. Further examples of haloalkyl are CHF₂ and CH₂CF₃.

The term "C₃₋₁₀ cycloalkyl" (such as C₃₋₄ cycloalkyl, C₃₋₅ cycloalkyl, C₃₋₆ cycloalkyl, C₃₋₇ cycloalkyl, C₃₋₈ cycloalkyl, C₃₋₉ cycloalkyl, C₄₋₅ cycloalkyl, C₄₋₆ cycloalkyl, C₄₋₇ cycloalkyl, C₄₋₈ cycloalkyl, C₄₋₉ cycloalkyl, C₄₋₁₀ cycloalkyl, C₅₋₆ cycloalkyl, C₅₋₇ cycloalkyl, C₅₋₈ cycloalkyl, C₅₋₉ cycloalkyl, C₅₋₁₀ cycloalkyl, C₆₋₇ cycloalkyl, C₆₋₈ cycloalkyl, C₆₋₉ cycloalkyl, C₆₋₁₀ cycloalkyl, C₇₋₈ cycloalkyl, C₇₋₉ cycloalkyl, C₇₋₁₀ cycloalkyl, C₈₋₉ cycloalkyl, C₈₋₁₀ cycloalkyl and C₉₋₁₀ cycloalkyl) refers to a fully saturated cyclic hydrocarbon group having from 3 to 10 carbon atoms. The term encompasses cyclopropyl, cyclobutyl, cyclopentyl, cyclohexyl, cycloheptyl and cyclooctyl as well as bridged systems such as bicyclo[1.1.1]pentyl.

The term "4-7 membered heterocyclic ring" refers to a non-aromatic cyclic group having 4 to 7 ring atoms, at least one of which is a heteroatom selected from N, O, S and B. The term "heterocyclic ring" is interchangeable with "heterocyclyl". The term encompasses pyrrolidinyl, tetrahydrofuranyl, tetrahydrothienyl, tetrahydropyranyl, piperidinyl, piperazinyl, morpholinyl, thiomorpholinyl. Other heterocyclyl groups, for example, 5-7 membered heterocyclyl, 6-7 membered heterocyclyl, 5-6 membered heterocyclyl, 4-6 membered heterocyclyl, 4 membered heterocyclyl, 5 membered heterocyclyl, 6 membered heterocyclyl and 7 membered heterocyclyl are as defined above but contain different numbers of ring atoms. Bicyclic heterocyclic compounds are also encompassed, such as the following:

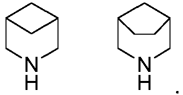

In some cases, heterocyclic rings may be substituted on one or more ring carbon atoms by oxo. Examples of rings of this type include pyridone and pyridazinone. However, it should be noted that although pyridone and pyridazinone are defined herein as a "heterocyclic ring", any aromatic tautomers of pyridone and pyridazinone are also encompassed, as shown below:

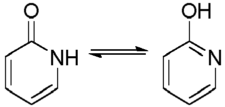

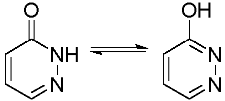

The term "5-10 membered heteroaryl" refers to a cyclic group with aromatic character having 5-10 ring atoms, at least one of which is a heteroatom independently selected from N, O and S. The term encompasses pyrrolyl, furanyl, thienyl, imidazolyl, pyrazolyl, thiazolyl, isothiazolyl, oxadiazolyl, thiadiazolyl, triazolyl, oxazolyl, isoxazolyl, tetrazolyl, pyridinyl, pyrimidinyl, pyradizinyl and pyrazinyl. Bicyclic heteroaryl compounds are also encompassed such as furo[3,2-b]pyridinyl, pyrazolo[1,5-a]pyridinyl and imidazo[1,2-a]pyridinyl. Where a heteroaryl group contains more than one ring, not all rings must contain a heteroatom, and not all rings must be aromatic in character. Other heteroaryl groups, for example, 5-9 membered heteroaryl, 5-8 membered heteroaryl, 5-7 membered heteroaryl, 5-6 membered heteroaryl, 6-10 membered heteroaryl, 6-9 membered heteroaryl, 6-8 membered heteroaryl, 6-7 membered heteroaryl, 5 membered heteroaryl, 6 membered heteroaryl, 7 membered heteroaryl, 8 membered heteroaryl, 9 membered heteroaryl and 10 membered heteroaryl are as defined above but contain different numbers of ring atoms. Further examples of heteroaryl include indolyl, indazolyl, benzofuranyl, benzimidazolyl, benzothiazolyl, benzothiophenyl, quinolinyl, isoquinolinyl and quinazolinyl.

The term "hydroxy" (which may also be referred to as "hydroxyl") refers to an -OH group.

The term "C₁₋₆ hydroxyalkyl" (e.g. C₁₋₅ hydroxyalkyl, C₁₋₄ hydroxyalkyl, C₁₋₃ hydroxyalkyl, C₁₋₂ hydroxyalkyl or C₁ hydroxyalkyl) refers to a straight or a branched fully saturated hydrocarbon group containing the specified number of carbon atoms and at least one -OH group. Examples include -CH₂C(H)OH-, -C(H)OHCH₃, -C(H)OH-, -CH₂OH and -CH₂CH₂OH.

The term "C₁₋₆ amino alkyl" (e.g. C₁₋₅ aminoalkyl, C₁₋₄ aminoalkyl, C₁₋₃ aminoalkyl, C₁₋₂ aminoalkyl or C₁ aminoalkyl) refers to a straight or a branched fully saturated hydrocarbon group containing the specified number of carbon atoms and at least one -NH₂ group. Examples include -CH(NH₂)CH₃, -CH₂NH₂, -C(H)NH₂- and -CH₂CH₂NH₂.

The term "C₁₋₂ methoxyalkyl" refers to a straight or a branched fully saturated hydrocarbon group containing the specified number of carbon atoms and at least one methoxy (OCH₃) group, for example -CH₂OCH₃, -CH₂CH₂OCH₃ or -C(H)(OCH₃)CH₃.

The term "oxo" refers to a =O substituent, whereby an oxygen atom is doubly bonded to carbon (e.g. C=O) or another element (e.g. S=O, S(=O)₂). The carbon or other element is suitably an atom of an alkyl, cycloalkyl or heterocyclyl group.

The term "halo" refers to fluorine, chlorine, bromine or iodine. Particular examples of halo are fluorine and bromine, especially fluorine.

Where substituents are indicated as being *optionally substituted* in formulae (I), (Ia) and (Ib) in the embodiments and preferences set out below, the optional substituent is attached to an available atom (e.g. carbon or nitrogen atom), which typically means an atom which is attached to a hydrogen atom e.g. for a carbon atom, a C-H or CH₂ group or for a nitrogen atom, an N-H group. The optional substituent replaces the hydrogen atom attached to the atom e.g. the carbon atom or nitrogen atom.

In one embodiment, R^{A} is C₁₋₁₀ alkyl optionally substituted on an available carbon atom by one or more R^{1A}. Suitably, R^{A} is C₁₋₅ alkyl, for example methyl, ethyl or n-propyl, and in particular is methyl or ethyl, especially methyl. In alternative suitable compounds, R^{A} is C₁₋₅ alkyl, for example methyl, ethyl or n-propyl substituted by one or more R^{1A}, as defined below. For example, R^{A} is C₁₋₅ alkyl substituted by OC₁₋₄ alkyl, such as methoxy. In one embodiment, R^{A} is methoxyethyl.

In some embodiments, R^{A} is C₃₋₁₀ cycloalkyl optionally substituted on an available carbon atom by one or more R^{1A}. Suitably, R^{A} is C₃₋₆ cycloalkyl, for example cyclopropyl, cyclobutyl or cyclopentyl, and in particular is cyclopropyl.

In some embodiments, R^{A} is phenyl optionally substituted on an available carbon atom by one or more R^{1A}.

In some embodiments R^{A} is unsubstituted phenyl.

In some embodiments R^{A} is phenyl substituted by one or more R^{1A}, where each R^{1A} is independently as defined above. For example, in one embodiment R^{A} is phenyl substituted by OC₁₋₆ alkyl, e.g. 4-methoxyphenyl.

In some embodiments, R^{A} is 5-10 membered heteroaryl, and is optionally substituted on an available atom (e.g. a carbon or nitrogen atom) by one or more R^{1A}. Suitably, R^{A} is selected from the group consisting of pyrrolyl, furanyl, thienyl, imidazolyl, pyrazolyl, thiazolyl, isothiazolyl, oxadiazolyl, thiadiazolyl, triazolyl, oxazolyl, isoxazolyl, tetrazolyl, pyridinyl, pyrimidinyl, pyradizinyl, pyrazinyl, furo[3,2-b]pyridinyl, pyrazolo[1,5-a]pyridinyl, imidazo[1,2-a]pyridinyl, indolyl, indazolyl, benzofuranyl, benzimidazolyl, benzothiazolyl, benzothiophenyl, quinolinyl, isoquinolinyl and quinazolinyl, and in particular is thiazolyl. In one embodiment, R^{A} is thiazolyl substituted by R^{1A}, wherein R^{1A} is methyl.

In some embodiments, R^{A} is C₁₋₁₀ alkyl, C₃₋₁₀ cycloalkyl, phenyl or 5-10 membered heteroaryl, and is optionally substituted on an available atom (e.g. a carbon or nitrogen atom) by one or more R^{1A}. In one embodiment, R^{A} is C₁₋₅ alkyl, C₃₋₅ cycloalkyl, phenyl or 5-10 membered heteroaryl, and is optionally substituted on an available atom by one or more R^{1A}.

In some embodiments, R^{A} is unsubstituted. In another embodiment, R^{A} is substituted by one or more (such as one, two or three e.g. one) R^{1A}. Suitably, R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano and NR^{2A}R^{3A}; and in particular is independently selected from halo, C₁₋₄ alkyl, C₁₋₄ hydroxyalkyl, C₁₋₄ haloalkyl, hydroxy, OC₁₋₄ alkyl, OC₁₋₄ haloalkyl, cyano and NR^{2A}R^{3A}; e.g. independently selected from F, Cl, methyl, ethyl, C₁₋₂ hydroxyalkyl, C₁₋₂ haloalkyl, hydroxy, OC₁₋₂ alkyl, OC₁₋₂ haloalkyl and NH₂. In one embodiment, R^{1A} is independently selected from methyl and OCH₃.

In some embodiments, when R^{A} is C₁₋₁₀ alkyl, C₂₋₁₀ alkenyl or C₂₋₁₀ alkynyl, R^{A} is optionally substituted on an available carbon atom by one or more R^{1A}, wherein each R^{1A} is independently selected from the group consisting of halo, hydroxy, OC₁₋₆ alkyl, OC₁₋₆ haloalkyl, cyano, NR^{2A}R^{3A}, CO₂H, CONR^{2A}R^{3A} and C₃₋₆ cycloalkyl; and when R^{A} is C₃₋₁₀ cycloalkyl, phenyl or 5-10 membered heteroaryl, R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2A}R^{3A}, CO₂H, COR^{2A}R^{3A} and C₃₋₆ cycloalkyl.

In some embodiments, R^{2A} and R^{3A} are independently selected from the group consisting of H and C₁₋₆ alkyl, e.g. C₁₋₄ alkyl. Suitably, R^{2A} and R^{3A} are independently selected from the group consisting of H, methyl and ethyl. In one embodiment, R^{2A} and R^{3A} are both H. In one embodiment, R^{2A} and R^{3A} are both methyl.

In some embodiments, R^{2A} and R^{3A} together with the N atom to which they are attached combine to form a 5-7 membered heterocyclic ring which is optionally substituted on an available atom by one or more groups selected from C₁₋₂ alkyl and oxo. In one embodiment, R^{2A} and R^{3A} together with the N atom to which they are attached combine to form a 5 membered heterocyclic ring which is optionally substituted on an available atom by one or more groups selected from C₁₋₂ alkyl and oxo. In one embodiment, R^{2A} and R^{3A} together with the N atom to which they are attached combine to form a 6 membered heterocyclic ring which is optionally substituted on an available atom by one or more groups selected from C₁₋₂ alkyl and oxo. In one embodiment, R^{2A} and R^{3A} together with the N atom to which they are attached combine to form a 7 membered heterocyclic ring which is optionally substituted on an available atom by one or more groups selected from C₁₋₂ alkyl and oxo. In one embodiment, R^{2A} and R^{3A} together with the N atom to which they are attached combine to form pyrrolidin-1-yl, piperidin-1-yl, morpholin-4-yl, thiomorpholin-4-yl, 1-oxo-thiomorpholin-4-yl, 1,1-dioxo-thiomorpholin-4-yl, 1,4-piperazin-1-yl or N-methyl-1,4-piperazin-1-yl. In one embodiment, R^{2A} and R^{3A} together with the N atom to which they are attached combine to form pyrrolidin-1-yl or piperidin-1-yl. In one embodiment, R^{2A} and R^{3A} together with the N atom to which they are attached combine to form pyrrolidin-1-yl. In one embodiment, R^{2A} and R^{3A} together with the N atom to which they are attached combine to form piperidin-1-yl.

In some embodiments, R^{B} is phenyl optionally substituted on an available carbon atom by one or more R^{1B}.

In some embodiments, R^{B} is phenyl fused to a 5-7 membered heterocyclic ring, and is optionally substituted on an available atom (e.g. a carbon or nitrogen atom) by one or more R^{1B}. Suitably, R^{B} is phenyl fused to a 5-6 membered heterocyclic ring, for example R^{B} is selected from the group consisting of 2,3-dihydrobenzofuranyl, indolinyl, 1,2,3,4-tetrahydroquinolinyl, benzo-1,4-dioxanyl, 1,3-benzodiazole and 3,4-dihydro-2H-1,4-benzoxazine; and in particular is 2,3-dihydrobenzofuranyl, benzo-1,4-dioxanyl or 3,4-dihydro-2H-1,4-benzoxazine.

In some embodiments, R^{B} is 5-10 membered heteroaryl optionally substituted on an available atom by one or more R^{1B}. Suitably, R^{B} is selected from the group consisting of pyrrolyl, furanyl, thienyl, imidazolyl, pyrazolyl, thiazolyl, isothiazolyl, oxadiazolyl, thiadiazolyl, triazolyl, oxazolyl, isoxazolyl, tetrazolyl, pyridinyl, pyrimidinyl, pyradizinyl, pyrazinyl, furo[3,2-b]pyridinyl, pyrazolo[1,5-a]pyridinyl, pyrrolo[2,3-b]pyridinyl, pyrrolo[2,3-c]pyridinyl, imidazo[1,2-a]pyridinyl, thieno[3,2-c]pyridinyl, indolyl, indazolyl, benzofuranyl, benzimidazolyl, benzothiazolyl, benzothiophenyl, quinolinyl, isoquinolinyl and quinazolinyl, and in particular is selected from the group consisting of furanyl, thienyl, pyrrolyl, imidazolyl, pyrazolyl, pyridinyl and pyrimidinyl. In one embodiment, R^{B} is selected from the group consisting of furanyl, thienyl, pyrazolyl and pyridinyl.

In some embodiments, R^{B} is 4-7 membered heterocyclyl (such as 5-7 membered heterocyclyl) optionally substituted on an available atom (e.g. a carbon or nitrogen atom) by one or more R^{1B}. Suitably, R^{B} is selected from the group consisting of pyrrolidinyl, tetrahydrofuranyl, tetrahydrothienyl, tetrahydropyranyl, piperidinyl, piperazinyl, morpholinyl, thiomorpholinyl, pyridone and pyridazinone.

In some embodiments, R^{B} is selected from the group consisting of phenyl, phenyl fused to a 5-7 membered heterocyclic ring, pyrrolyl, furanyl, thienyl, imidazolyl, pyrazolyl, thiazolyl, isothiazolyl, oxadiazolyl, thiadiazolyl, triazolyl, oxazolyl, isoxazolyl, tetrazolyl, pyridinyl, pyrimidinyl, pyradizinyl, pyrazinyl, furo[3,2-b]pyridinyl, pyrazolo[1,5-a]pyridinyl, pyrrolo[2,3-b]pyridinyl, pyrrolo[2,3-c]pyridinyl, imidazo[1,2-a]pyridinyl, thieno[3,2-c]pyridinyl, indolyl, indazolyl, benzofuranyl, benzimidazolyl, benzothiazolyl, benzothiophenyl, quinolinyl, isoquinolinyl, quinazolinyl, dihydrobenzofuranyl, pyrrolidinyl, tetrahydrofuranyl, tetrahydrothienyl, tetrahydropyranyl, piperidinyl, piperazinyl, morpholinyl, thiomorpholinyl, pyridone and pyridazinone; and is optionally substituted on an available atom (e.g. a carbon or nitrogen atom) by one or more R^{1B}.

In some embodiments, R^{B} is unsubstituted. In another embodiment, R^{B} is substituted by one or more (such as one, two or three e.g. one) R^{1B}. Suitably, each R^{1B} is independently selected from the group consisting of halo, C₁₋₆ alkyl, C₁₋₆ hydroxyalkyl, C₁₋₆ aminoalkyl, C₁₋₆ haloalkyl, hydroxy, OC₁₋₆ alkyl, OC₁₋₆ haloalkyl, cyano, NR^{2B}R^{3B}, C(O)OH, CONR^{2B}R^{3B}, S(O)₂NR^{2B}R^{3B}, S(O)₂C₁₋₆ alkyl, C₃₋₆ cycloalkyl and oxo; and in particular is independently selected from the group consisting of halo, C₁₋₄ alkyl, C₁₋₄ hydroxyalkyl, C₁₋₄ aminoalkyl, C₁₋₄ haloalkyl, hydroxy, OC₁₋₄ alkyl, OC₁₋₄ haloalkyl, cyano, NR^{2B}R^{3B}, C(O)OH, CONR^{2B}R^{3B}, S(O)₂NR^{2B}R^{3B}, S(O)₂C₁₋₄ alkyl, C₃₋₅ cycloalkyl and oxo; e.g. independently selected from the group consisting of F, Cl, methyl, ethyl, C₁₋₂ hydroxyalkyl, C₁₋₂ aminoalkyl, C₁₋₂ haloalkyl, hydroxy, OC₁₋₂ alkyl, OC₁₋₂ haloalkyl, cyano, NR^{2B}R^{3B}, C(O)OH, CONR^{2B}R^{3B}, S(O)₂NR^{2B}R^{3B}, S(O)₂C₁₋₂ alkyl, C₃₋₄ cycloalkyl and oxo. In one embodiment, R^{1B} is independently selected from the group consisting of F, methyl, C₁₋₂ hydroxyalkyl, C₁₋₂ aminoalkyl, hydroxy, OCH₃, cyano, NH₂, C(O)OH, CONH₂, S(O)₂NH₂, S(O)₂CH₃, cyclopropyl and oxo.

In some embodiments, R^{2B} and R^{3B} are independently selected from the group consisting of H and C₁₋₆ alkyl e.g. C₁₋₄ alkyl. Suitably, R^{2B} and R^{3B} are independently selected from the group consisting of H, methyl and ethyl. In one embodiment, R^{2B} and R^{3B} are both H. In one embodiment, R^{2B} and R^{3B} are both methyl.

In some embodiments, R^{2B} and R^{3B} together with the N atom to which they are attached combine to form a 5-7 membered heterocyclic ring which is optionally substituted on an available atom by one or more groups selected from C₁₋₂ alkyl and oxo. In one embodiment, R^{2B} and R^{3B} together with the N atom to which they are attached combine to form a 5 membered heterocyclic ring which is optionally substituted on an available atom by one or more groups selected from C₁₋₂ alkyl and oxo. In some embodiments, R^{2B} and R^{3B} together with the N atom to which they are attached combine to form a 6 membered heterocyclic ring which is optionally substituted on an available atom by one or more groups selected from C₁₋₂ alkyl and oxo. In some embodiments, R^{2B} and R^{3B} together with the N atom to which they are attached combine to form a 7 membered heterocyclic ring which is optionally substituted on an available atom by one or more groups selected from C₁₋₂ alkyl and oxo. In one embodiment, R^{2B} and R^{3B} together with the N atom to which they are attached combine to form pyrrolidin-1-yl, piperidin-1-yl, morpholin-4-yl, thiomorpholin-4-yl, 1-oxo-thiomorpholin-4-yl, 1,1-dioxo-thiomorpholin-4-yl, 1,4-piperazin-1-yl or N-methyl-1,4-piperazin-1-yl. In one embodiment, R^{2B} and R^{3B} together with the N atom to which they are attached combine to form pyrrolidin-1-yl or piperidin-1-yl. In some embodiments, R^{2B} and R^{3B} together with the N atom to which they are attached combine to form pyrrolidin-1-yl. In one embodiment, R^{2B} and R^{3B} together with the N atom to which they are attached combine to form piperidin-1-yl.

In some embodiments, R^{C} is methyl or ethyl. In some embodiments, R^{C} is H. Suitably, R^{C} is methyl.

In some embodiments, R^{D} is H, methyl, ethyl, CH₂OH or CH₂OCH₃, in particular H, CH₂OH or CH₂OCH₃. In some embodiments, R^{D} is H.

In some embodiments, X is C=O, S(=O)₂, -CH₂-S(=O)₁₋₂-, S(=O)(=NH) or -NH-S(=O)₂-. In some embodiments, X is C=O, S(=O)₁₋₂, -CH₂-S(=O)₁₋₂- or -NH-S(=O)₂-. In some embodiments, X is C=O, S(=O)₂, CH₂S(=O)₂ or -NH-S(=O)₂-, more suitably X is C=O, S=(O)₂ or -NH-S(=O)₂-, especially C=O or S(=O)₂. In some embodiments, X is C=O. In some embodiments, X is S(=O). In some embodiments, X is S(=O)₂. In some embodiments, X is -CH₂S(=O)-. In some embodiments, X is -CH₂S(=O)₂-. In some embodiments, X is S(=O)(=NH). In some embodiments, X is -NH-S(=O)₂-.

It should be noted that when X is -CH₂-S(=O)₁₋₂- or -NH-S(=O)₂- it is intended that the left hand group as drawn is directly bonded to group R^{A} and the right hand group as drawn is directly bonded to the tricyclic core structure as shown below for -CH₂-S(=O)₂-:

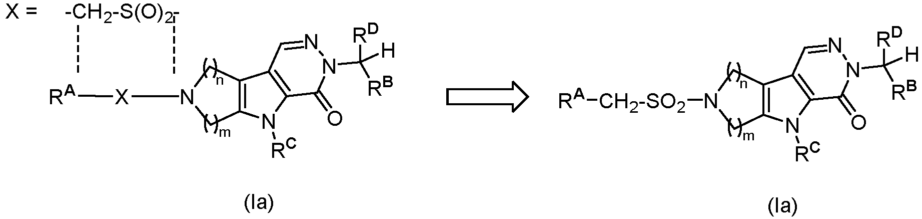

In one embodiment, n is 2 and m is 0. In another embodiment, n is 1 and m is 1. In another embodiment, n is 0 and m is 2. In all of these embodiments, n + m is 2 and the left hand ring of the tricyclic core is pyrrolidine.

In one embodiment, n is 3 and m is 0. In another embodiment, n is 2 and m is 1. In another embodiment, n is 1 and m is 2. In another embodiment, n is 0 and m is 3. In all of these embodiments, n + m is 3 and the left hand ring of the tricyclic core is piperidine.

In one embodiment, n is 4 and m is 0. In another embodiment, n is 3 and m is 1. In another embodiment, n is 2 and m is 2. In another embodiment, n is 1 and m is 3. In another embodiment, n is 0 and m is 4. In all of these embodiments, n + m is 4 and the left hand ring of the tricyclic core is homopiperidine.

In one embodiment, n is 2 and m is 1 and the compound of formula (Ia) is a compound of formula (laA) as follows:

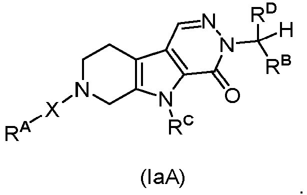

In one embodiment, n is 3 and m is 1 and the compound of formula (la) is a compound of formula (laB) as follows:

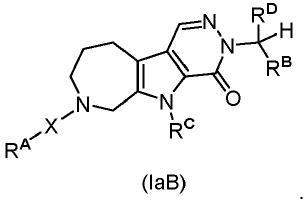

In one embodiment, n is 2 and m is 1, or n is 3 and m is 1.

In one embodiment, n is 3 and m is 0 and the compound of formula (la) is a compound of formula (laC) as follows:

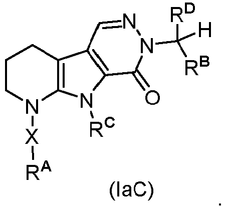

In one embodiment, n is 1 and m is 2 and the compound of formula (la) is a compound of formula (laD) as follows:

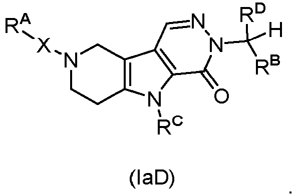

In one embodiment, n is 0 and m is 3 and the compound of formula (la) is a compound of formula (laE) as follows:

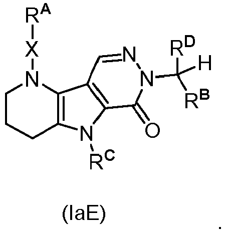

In one embodiment, n is 4 and m is 0 and the compound of formula (la) is a compound of formula (laF) as follows:

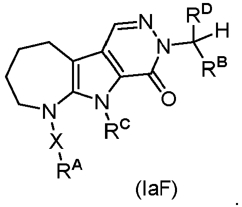

In one embodiment, n is 2 and m is 2 and the compound of formula (la) is a compound of formula (laG) as follows:

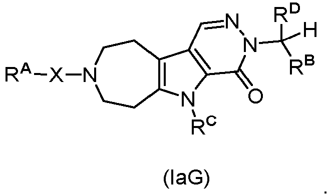

In one embodiment, n is 1 and m is 3 and the compound of formula (la) is a compound of formula (laH) as follows:

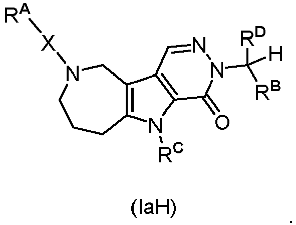

In one embodiment, n is 0 and m is 4 and the compound of formula (la) is a compound of formula (laJ) as follows:

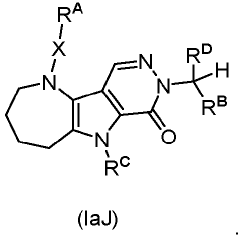

In one embodiment, n is 0 and m is 2 and the compound of formula (la) is a compound of formula (laK) as follows:

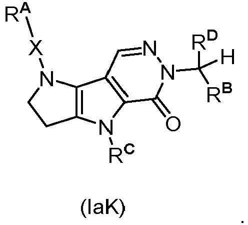

In one embodiment, n is 1 and m is 1 and the compound of formula (la) is a compound of formula (laL) as follows:

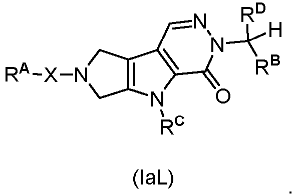

In one embodiment, n is 2 and m is 0 and the compound of formula (la) is a compound of formula (laM) as follows:

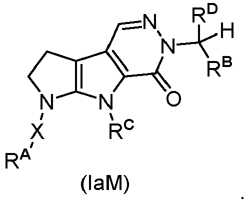

Compounds of formula (laA) are particularly suitable.

In one embodiment, the molecular weight of the compound of formula (la) is 300 Da - 550 Da, e.g. 350 Da - 480 Da.

In one embodiment there is provided a compound of formula (la), which is:
3-((2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-one;
3-((6-methoxypyridin-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(3-(1-hydroxyethyl)benzyl)-5-methyl-7-(methylsulfonyl)-3,5,6,7,8,9-hexahydro-4H-pyrido[4',3':4,5]pyrrolo[2,3-d]pyridazin-4-one;
3-((1H-pyrazol-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(2-fluoro-3-methoxybenzyl)-5-methyl-7-(methylsulfonyl)-3,5,6,7,8,9-hexahydro-4H-pyrido[4',3':4,5]pyrrolo[2,3-d]pyridazin-4-one;
3-(2,6-difluoro-4-methoxybenzyl)-5-methyl-7-(methylsulfonyl)-3,5,6,7,8,9-hexahydro-4H-pyrido[4',3':4,5]pyrrolo[2,3-d]pyridazin-4-one;
3-(3-aminobenzyl)-5-methyl-7-(methylsulfonyl)-3,5,6,7,8,9-hexahydro-4H-pyrido[4',3':4,5]pyrrolo[2,3-d]pyridazin-4-one;
7-acetyl-3-(3-aminobenzyl)-5-methyl-3,5,6,7,8,9-hexahydro-4H-pyrido[4',3':4,5]pyrrolo[2,3-d]pyridazin-4-one;
3-(3-aminobenzyl)-7-(cyclopropylsulfonyl)-5-methyl-3,5,6,7,8,9-hexahydro-4H-pyrido[4',3':4,5]pyrrolo[2,3-d]pyridazin-4-one;
3-(3-aminobenzyl)-5-methyl-7-((2-methylthiazol-5-yl)sulfonyl)-3,5,6,7,8,9-hexahydro-4H-pyrido[4',3':4,5]pyrrolo[2,3-d]pyridazin-4-one;
3-(3-aminobenzyl)-5-methyl-7-(phenylsulfonyl)-3,5,6,7,8,9-hexahydro-4H-pyrido[4',3':4,5]pyrrolo[2,3-d]pyridazin-4-one; and
3-((1H-pyrazol-3-yl)methyl)-5-methyl-7-(phenylsulfonyl)-3,5,6,7,8,9-hexahydro-4H-pyrido[4',3':4,5]pyrrolo[2,3-d]pyridazin-4-one;
3-((2-(hydroxymethyl)thiophen-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)thiophene-2-carboxamide;
3-((1H-indol-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(benzofuran-3-ylmethyl)-5-methyl-7-(methylsulfonyl)-3,5,6,7,8,9-hexahydro-4H-pyrido[4',3':4,5]pyrrolo[2,3-d]pyridazin-4-one;
3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)-1H-pyrazole-4-carboxamide;
3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)benzenesulfonamide;
5-methyl-7-(methylsulfonyl)-3-((6-(pyrrolidin-1-yl)pyridin-2-yl)methyl)-3,5,6,7,8,9-hexahydro-4H-pyrido[4',3':4,5]pyrrolo[2,3-d]pyridazin-4-one;
3-((1H-indazol-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(2-fluoro-4-methoxybenzyl)-5-methyl-7-(phenylsulfonyl)-3,5,6,7,8,9-hexahydro-4H-pyrido[4',3':4,5]pyrrolo[2,3-d]pyridazin-4-one;
3-((7-((4-methoxyphenyl)sulfonyl)-5-methyl-4-oxo-4,5,6,7,8,9-hexahydro-3H-pyrido[4',3':4,5]pyrrolo[2,3-d]pyridazin-3-yl)methyl)thiophene-2-carboxamide; 3-((2,3-dihydrobenzofuran-5-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-one;
3-((2,2-dimethyl-2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-one;
3-((1H-pyrrolo[2,3-b]pyridin-6-yl)methyl)-5-methyl-7-(methylsulfonyl)-3,5,6,7,8,9-hexahydro-4H-pyrido[4',3':4,5]pyrrolo[2,3-d]pyridazin-4-one;
(S)-3-(3-(1-aminoethyl)benzyl)-5-methyl-7-(methylsulfonyl)-3,5,6,7,8,9-hexahydro-4H-pyrido[4',3':4,5]pyrrolo[2,3-d]pyridazin-4-one;
7-((2-methoxyethyl)sulfonyl)-3-((6-methoxypyridin-3-yl)methyl)-5-methyl-3,5,6,7,8,9-hexahydro-4H-pyrido[4',3':4,5]pyrrolo[2,3-d]pyridazin-4-one;
N-(2-methoxyethyl)-3-((6-methoxypyridin-3-yl)methyl)-5-methyl-4-oxo-3,4,5,6,8,9-hexahydro-7H-pyrido[4',3':4,5]pyrrolo[2,3-d]pyridazine-7-sulfonamide;
3-((1H-pyrrolo[2,3-c]pyridin-3-yl)methyl)-7-((2-methoxyethyl)sulfonyl)-5-methyl-6,7,8,9-tetrahydro-3H-pyrido[4',3':4,5]pyrrolo[2,3-d]pyridazin-4(5H)-one;
(3-((1H-pyrrolo[2,3-c]pyridin-3-yl)methyl)-N-ethyl-5-methyl-4-oxo-5,6,8,9-tetrahydro-3H-pyrido[4',3':4,5]pyrrolo[2,3-d]pyridazine-7(4H)-sulfonamide;
5-methyl-7-(methylsulfonyl)-3-(3-(methylsulfonyl)benzyl)-3,5,6,7,8,9-hexahydro-4H-pyrido[4',3':4,5] pyrrolo[2,3-*d*]pyridazin-4-one;
3-((1*H*-indazol-4-yl)methyl)-5-methyl-7-(methylsulfonyl)-3,5,6,7,8,9-hexahydro-4*H-*pyrido[4',3':4,5]pyrrolo[2,3-d]pyridazin-4-one;
3-(3-(2-hydroxypropan-2-yl)benzyl)-5-methyl-7-(methylsulfonyl)-3,5,6,7,8,9-hexahydro-4H-pyrido[4',3':4,5]pyrrolo[2,3-d]pyridazin-4-one;
5-methyl-7-(methylsulfonyl)-3-(thieno[3,2-c]pyridin-3-ylmethyl)-3,5,6,7,8,9-hexahydro-4H-pyrido[4',3':4,5]pyrrolo[2,3-d]pyridazin-4-one;
3-((1H-pyrrolo[2,3-c]pyridin-3-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-one;
3-((1H-pyrrolo[2,3-c]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-((1H-pyrrolo[2,3-b]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-((5-fluoro-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-one;
3-((6-fluoro-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-one;
or a pharmaceutically acceptable salt and/or solvate of any one thereof.

In one embodiment is provided a compound selected from:
5-methyl-7-(methylsulfonyl)-3,5,6,7,8,9-hexahydro-4H-pyrido[4',3':4,5]pyrrolo[2,3-d]pyridazin-4-one;
tert-butyl (3-((5-methyl-4-oxo-4,5,6,7,8,9-hexahydro-3H-pyrido[4',3':4,5]pyrrolo[2,3-d]pyridazin-3-yl)methyl)phenyl)carbamate; and
5-methyl-7-(phenylsulfonyl)-3,5,6,7,8,9-hexahydro-4H-pyrido[4',3':4,5]pyrrolo[2,3-d]pyridazin-4-one;
methyl 3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)thiophene-2-carboxylate; 7-((4-methoxyphenyl)sulfonyl)-5-methyl-3,5,6,7,8,9-hexahydro-4H-pyrido[4',3':4,5]pyrrolo[2,3-d]pyridazin-4-one;
benzyl 5-methyl-4-oxo-3,4,5,6,8,9-hexahydro-7H-pyrido[4',3':4,5]pyrrolo[2,3-d]pyridazine-7-carboxylate;
3-((6-methoxypyridin-3-yl)methyl)-5-methyl-3,5,6,7,8,9-hexahydro-4H-pyrido[4',3':4,5]pyrrolo[2,3-d]pyridazin-4-one;
or a salt and/or solvate thereof.

Compounds of formula (Ia) may be synthesised as shown in the schemes below and as shown in the Example section. Where appropriate, the routes may also be used for compounds of formulae (I) and (Ib). 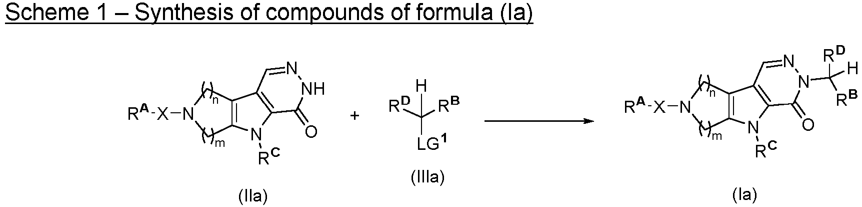 wherein R^{A}, R^{B}, R^{C}, R^{D}, X, m and n are defined elsewhere herein and LG¹ is a leaving group such as halo (e.g. chloro, bromo or iodo), OMs or OTs. A compound of formula (Ila) may be reacted with a compound of formula (Illa) in the presence of a base, such as Cs₂CO₃, K₂CO₃ or NaH, to provide a compound of formula (la). 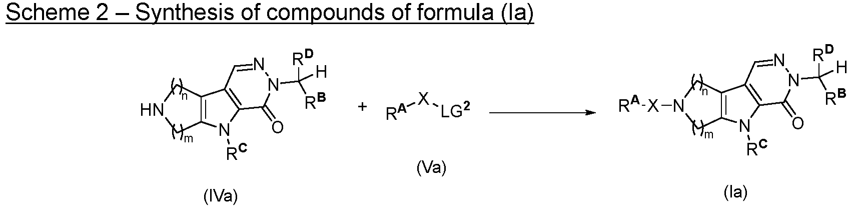 wherein R^{A}, R^{B}, R^{C}, R^{D}, X, m and n are defined elsewhere herein and LG² is a leaving group such as halo (e.g. chloro, bromo or iodo), OMs or OTs. A compound of formula (IVa) may be reacted with a compound of formula (Va) in the presence of a base, such as Cs₂CO₃, K₂CO₃ or NaH, to provide a compound of formula (Ia). 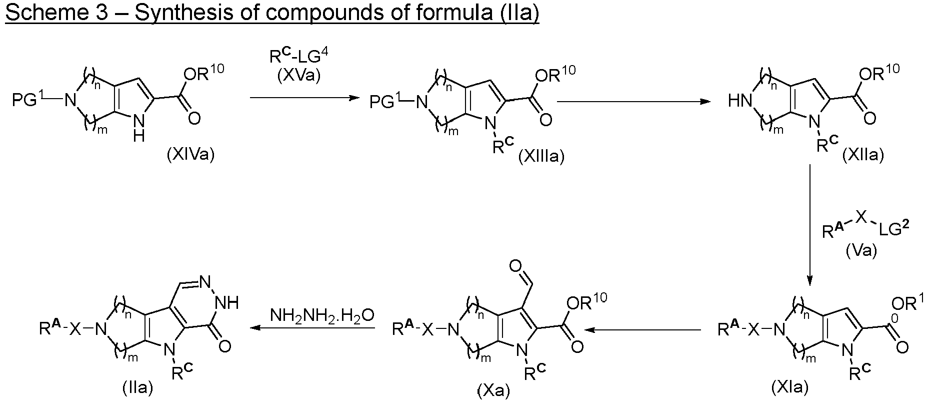 wherein R^{A}, X, R^{C}, m and n are defined elsewhere herein, R¹⁰ is C₁₋₆ alkyl, for example ethyl, LG² is as defined for Scheme 2, LG⁴ is a leaving group such as halo (e.g. chloro, bromo or iodo), mesylate or triflate, and PG¹ is a nitrogen protecting group such as those discussed below and, in particular, butyloxycarbonyl (Boc) or benzyloxycarbonyl (Cbz).

Protected compounds of formula (XIVa) may be prepared by known methods, for example as described in step 1 of the synthesis of Intermediate 1 below.

Step 1: a compound of formula (XIVa) may be reacted with a compound of formula (XVa) in the presence of a weak base such as potassium carbonate and in a solvent such as *N,N-*dimethylformamide (DMF) to give a product of formula (Xllla). The reaction may be conducted at a temperature of about 15 to 25°C, for example at room temperature.

Step 2: The compound of formula (Xllla) is deprotected using a suitable method to give a compound of formula (Xlla). The deprotection method used will depend on the protecting group. For example, when PG¹ is Boc, deprotection may be achieved by treatment with an acid such as trifluoroacetic acid (TFA) at a temperature of about 15 to 25°C, for example at room temperature. Some compounds of formula (Xlla) are also commercially available.

Step 3: the compound of formula (Xlla) is reacted with a compound of formula (Va) to give a product of formula (XIa). The reaction may be carried out under basic conditions, for example in the presence of diisopropylethylamine (DIPEA) in a polar organic solvent such as dichloromethane and a temperature of about 15 to 25°C, for example at room temperature.

Step 4: N-methyl-N-phenylformamide is treated with POCl₃ and the product is reacted with the compound of formula (Xla) to produce a compound of formula (Xa). Alternatively, POCl₃ may be added to the compound of formula (Xla) at reduced temperature, for example about -5° to 5°C, and the product reacted with N-methyl-N-phenylformamide. The initial reaction with POCl₃ is suitably conducted at reduced temperature, for example about -5° to 5°C.

Step 5: the compound of formula (Xa) is reacted with hydrazine hydrate to give the product of formula (IIa). The reaction is suitably carried out at elevated temperature, for example about 110° to 130°C. 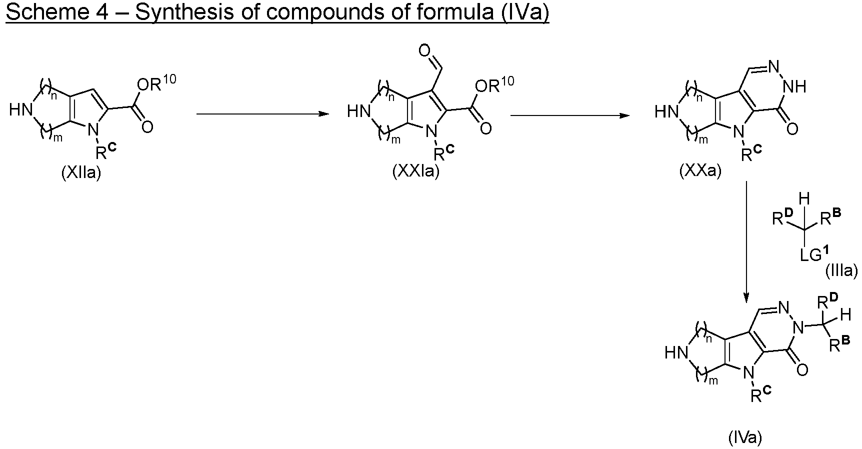 wherein R^{B}, R^{C}, R^{D}, m and n are defined elsewhere herein, R¹⁰ is as defined for Scheme 3, LG¹ is as defined for Scheme 1.

The compound of formula (Xlla) may be prepared as described above in Scheme 3, Steps 1 and 2.

Step 1: N-methyl-N-phenylformamide is treated with POCl₃ and the product is reacted with the compound of formula (Xlla) to produce a compound of formula (XXla). The reaction of N-methyl-N-phenylformamide with POCl₃ is suitably conducted at reduced temperature, for example about -5° to 5°C and the reaction with the compound of formula (Xlla) is suitably carried out at elevated temperature, for example about 70° to 90°C in an organic solvent such as dichloroethane.

Step 2: the compound of formula (XXIa) is reacted with hydrazine hydrate to give the product of formula (XXa). The reaction is carried out at elevated temperature, for example about 90° to 131°C.

Step 3: the compound of formula (XXa) is reacted with a compound of formula (Illa) in the presence of a base, such as Cs₂CO₃, K₂CO₃ or NaH, to provide a compound of formula (IVa).

The skilled person will appreciate that protecting groups may be used throughout the synthetic schemes described herein to give protected derivatives of any of the above compounds or generic formulae. For example, compounds of formulae (IVa), (Xlla), (XXa) and (XXIa) may comprise a protecting group on the nitrogen atom of the ring containing (CH₂)ₘ and (CH₂)ₙ as shown for the compound of formula (Xllla). When the group R^{B} comprises a nitrogen atom in an amine group or a nitrogen-containing heterocyclic ring, this nitrogen atom may also be protected with a suitable nitrogen protecting group. Suitably, in this case, the protecting groups will be different and will be removable by different methods to ensure that the -XR^{A} substituent is reacted with the nitrogen atom in the desired position. This is illustrated in Example 30 below.

Protective groups and the means for their removal are described in *"Protective Groups in Organic Synthesis",* by Theodora W. Greene and Peter G. M. Wuts, published by John Wiley & Sons Inc; 4th Rev Ed., 2006, ISBN-10: 0471697540. Examples of nitrogen protecting groups include trityl (Tr), tert-butyloxycarbonyl (Boc), 9-fluorenylmethyloxycarbonyl (Fmoc), benzyloxycarbonyl (Cbz), acetyl (Ac), benzyl (Bn) tetrahydropyranyl (THP) and para-methoxy benzyl (PMB). Examples of oxygen protecting groups include acetyl (Ac), methoxymethyl (MOM), para-methoxybenzyl (PMB), benzyl, tert-butyl, methyl, ethyl, tetrahydropyranyl (THP), and silyl ethers and esters (such as trimethylsilyl (TMS), tert-butyldimethylsilyl (TBDMS), tri-iso-propylsilyloxymethyl (TOM), and triisopropylsilyl (TIPS) ethers and esters). Specific examples of carboxylic acid protecting groups include alkyl esters (such as C₁₋₆ alkyl e.g. C₁₋₄ alkyl esters), benzyl esters and silyl esters.

In some cases, the process for preparing compounds of formula (la) may comprise additional steps. For example, where the group R^{B} is substituted by R^{1B}, where R^{1B} is C(O)OH, this may be obtained by hydrolysis of an analogue of a compound of formula (la) in which R^{1B} is replaced by an alkyl ester. Furthermore, a compound of formula (la) in which R^{1B} is C(O)OH or an analogue thereof in which R^{1B} is replaced by an alkyl ester may be converted to a compound of formula (la) in which R^{1B} is CH₂OH by reduction, for example using a hydride reducing agent such as lithium borohydride as described in Example 13. A compound of formula (la) in which R^{1B} is C(O)OH may also be converted to a compound of formula (la) in which R^{1B} is C(O)NH₂ by reaction with ammonium chloride in the presence of a coupling agent such as 1-[Bis(dimethylamino)methylene]-1H-1,2,3-triazolo[4,5-b]pyridinium 3-oxide hexafluorophosphate (HATU) and a base such as triethylamine in a solvent such as DMF as described in Example 22. A compound of formula (la) in which R^{B} is substituted with CH₂OH on an available nitrogen atom may be converted to a compound of formula (la) in which R^{B} is unsubstituted by reaction with aqueous ammonia as illustrated in Example 25. Similar reactions may be used to interconvert substituents R^{1A} on R^{A}.

In one embodiment, there is provided a process for preparing a compound of formula (la), or a salt, such as a pharmaceutically acceptable salt, thereof, which comprises reacting a compound of formula (IIa): 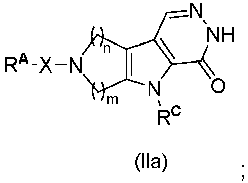
or a salt thereof;
with a compound of formula (Illa): 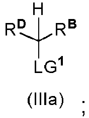
wherein R^{A}, R^{B}, R^{C}, R^{D}, X, m and n are defined elsewhere herein and LG¹ is a leaving group such as halo (e.g. chloro, bromo or iodo), OMs or OTs.

In one embodiment, there is provided a process for preparing a compound of formula (la), or a salt, such as a pharmaceutically acceptable salt, thereof, which comprises reacting a compound of formula (IVa): 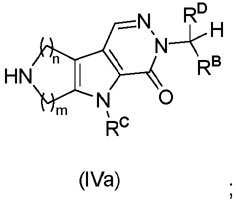
or a salt thereof;
with a compound of formula (Va): 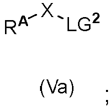
wherein R^{A}, R^{B}, R^{C}, R^{D}, X, m and n are defined elsewhere herein and LG² is a leaving group such as halo (e.g. chloro, bromo or iodo), OMs or OTs.

In one embodiment, there is provided a compound of formula (IIa): 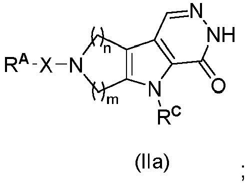
or a salt thereof;
wherein R^{A}, R^{C}, X, m and n are defined elsewhere herein.

In another embodiment, there is provided a compound of formula (IVa): 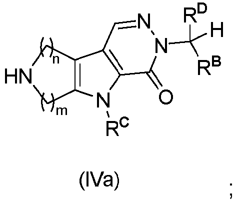
or a salt thereof;
wherein R^{B}, R^{C}, R^{D}, m and n are defined elsewhere herein.

It will be appreciated that for use in therapy the salts of the compounds of formula (Ia) should be pharmaceutically acceptable. Suitable pharmaceutically acceptable salts will be apparent to those skilled in the art. Pharmaceutically acceptable salts include acid addition salts, suitably salts of compounds of the invention comprising a basic group such as an amino group, formed with inorganic acids, e.g., hydrochloric acid, hydrobromic acid, sulfuric acid, nitric acid or phosphoric acid. Also included are salts formed with organic acids, e.g., succinic acid, maleic acid, acetic acid, fumaric acid, citric acid, tartaric acid, benzoic acid, p-toluenesulfonic acid, methanesulfonic acid, naphthalenesulfonic acid and 1,5-naphthalenedisulfonic acid. Other salts, e.g., oxalates or formates, may be used, for example in the isolation of compounds of formula (Ia) and are included within the scope of this invention, as are basic addition salts such as sodium, potassium, calcium, aluminium, zinc, magnesium and other metal salts.

Pharmaceutically acceptable salts may also be formed with organic bases such as basic amines, e.g., with ammonia, meglumine, tromethamine, piperazine, arginine, choline, diethylamine, benzathine or lysine. Thus, in one embodiment there is provided a compound of formula (Ia) in the form of a pharmaceutically acceptable salt. Alternatively, there is provided a compound of formula (Ia) in the form of a free acid. When the compound contains a basic group as well as the free acid it may be zwitterionic.

Suitably, the compound of formula (la) is not a salt, e.g., is not a pharmaceutically acceptable salt.

Suitably, where the compound of formula (la) is in the form of a salt, the pharmaceutically acceptable salt is an acid addition salt such as an ammonium salt (e.g. formed with an inorganic acid such as HCl).

The compounds of formula (la) may be prepared in crystalline or non-crystalline form and, if crystalline, may optionally be solvated, e.g., as the hydrate. This invention includes within its scope stoichiometric solvates (e.g., hydrates) as well as compounds containing variable amounts of solvent (e.g., water). Suitably, the compound of formula (la) is not a solvate.

Also disclosed is a pharmaceutically acceptable derivative thereof, such as a pharmaceutically acceptable prodrug of compounds of formula (la). Typical prodrugs of compounds of formula (Ia) which comprise a carboxylic acid include ester (e.g. C₁₋₆ alkyl e.g. C₁₋₄ alkyl ester) derivatives thereof. Thus, the compound of formula (la) is provided as a pharmaceutically acceptable prodrug (this aspect is not part of the claimed invention). In another embodiment, the compound of formula (la) is not provided as a pharmaceutically acceptable prodrug.

It is to be understood that the present invention encompasses all isomers of compounds of formula (la) including all geometric, tautomeric and optical forms, and mixtures thereof (e.g. racemic mixtures). In particular, the invention extends to all tautomeric forms of the compounds of formula (la). Where additional chiral centres are present in compounds of formula (la), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present invention also includes all isotopic forms of the compounds provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or> 99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium (²H or D), carbon-11 (¹¹C), carbon-13 (¹³C), carbon-14 (¹⁴C) , nitrogen-13 (¹³N), nitrogen-15 (¹⁵N), oxygen-15 (¹⁵O), oxygen-17 (¹⁷O), oxygen-18 (¹⁸O), phosphorus-32 (³²P), sulphur-35 (³⁵S), chlorine-36 (³⁶Cl), chlorine-37 (³⁷Cl), fluorine-18 (¹⁸F) iodine-123 (¹²³I), iodine-125 (¹²⁵I) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Unnatural variant isotopic forms which incorporate deuterium i.e. ²H or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as ¹¹C, ¹⁸F, ¹⁵O and ¹³N, and would be useful in positron emission topography (PET) studies for examining substrate receptor occupancy.

In one embodiment, the compounds of formula (la) are provided in a natural isotopic form. In one embodiment, the compounds of formula (la) are provided in an unnatural variant isotopic form. In a specific embodiment, the unnatural variant isotopic form is a form in which deuterium (i.e. ²H or D) is incorporated where hydrogen is specified in the chemical structure in one or more atoms of a compound of formula (Ia). In one embodiment, the atoms of the compounds of formula (la) are in an isotopic form which is not radioactive. In one embodiment, one or more atoms of the compounds of formula (la) are in an isotopic form which is radioactive. Suitably radioactive isotopes are stable isotopes. Suitably the unnatural variant isotopic form is a pharmaceutically acceptable form.

In one embodiment, a compound of formula (Ia) is provided whereby a single atom of the compound exists in an unnatural variant isotopic form. In another embodiment, a compound of formula (la) is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (la) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the purer forms used in the pharmaceutical compositions.

### Therapeutic indications

Compounds of formula (la) are of use in therapy, particularly for treating or preventing an inflammatory disease, a disease associated with an undesirable immune response, cancer, obesity, a diabetic disease or a blood disorder. As shown in Biological Examples 1 and 2 below, Example compounds of formula (Ia) exhibited suitable levels of *in vitro* potency for PKM2 and PKLR (using TEPP-46 as comparator), and as shown in Biological Example 3 below, the compounds of formula (la) also exhibited an *in vitro* anti-proliferative effect. As shown in Biological Example 4, all compounds exhibited significantly improved solubility compared to TEPP-46. Without wishing to be bound by theory, the present inventors' believe that the improvement in solubility (compared e.g. to compounds with similar molecular weight and/or LogD such as TEPP-46) while maintaining suitable PK (in particular PKM2 and/or PKLR) potency derives from the nature of the partially saturated, Sp3-rich tricyclic core. As such, compounds of formula (la) are expected to be suitable the treatment of diseases associated with PK, in particular PKM2 and PKLR activity.

Thus, in a first aspect, the present invention provides a compound of formula (la) or a pharmaceutically acceptable salt and/or solvate thereof as defined herein, for use as a medicament. Also provided is a pharmaceutical composition comprising a compound of formula (la) or a pharmaceutically acceptable salt and/or solvate thereof as defined herein. Such a pharmaceutical composition suitably contains the compound of formula (la) and one or more pharmaceutically acceptable diluents or carriers.

There is provided a compound of formula (Ia) or a pharmaceutically acceptable salt and/or solvate as defined herein, for use in treating or preventing, e.g. treating, a disease, disorder or condition associated with the function of PK, in particular PKM2 and/or PKLR.

There is also provided a compound of formula (la) or a pharmaceutically acceptable salt and/or solvate as defined herein, for use in treating or preventing a symptom associated with a disease, disorder or condition associated with the function of PK, in particular PKM2 and/or PKLR.

In one embodiment, a compound of formula (la) is a modulator of PKM2. In another embodiment, a compound of formula (la) is an activator of PKM2. In one embodiment, a compound of formula (la) is a modulator of PKLR. In another embodiment, a compound of formula (la) is an activator of PKLR. A compound is an "activator" of PK (e.g. PKM2 and/or PKLR) if it increases the activity of the enzyme, which can be quantified by, for example, determining the concentration of ATP generated in a suitable assay (such as Biological Example 1 for PKM2 and Biological Example 2 for PKLR).

In a further aspect, the present invention provides a compound of formula (la) or a pharmaceutically acceptable salt and/or solvate thereof as defined herein, for use in treating or preventing an inflammatory disease, a disease associated with an undesirable immune response, cancer, obesity, a diabetic disease or a blood disorder.

In a further aspect, the present invention provides a compound of formula (la) or a pharmaceutically acceptable salt and/or solvate thereof as defined herein, for use in treating or preventing a symptom associated with an inflammatory disease, a disease associated with an undesirable immune response, cancer, obesity, a diabetic disease or a blood disorder.

For all aspects of the invention, suitably the compound is administered to a subject in need thereof, wherein the subject is suitably a human subject.

In one embodiment is provided a compound of formula (Ia) or a pharmaceutically acceptable salt and/or solvate thereof as defined herein, for use in treating an inflammatory disease, a disease associated with an undesirable immune response, cancer, obesity, a diabetic disease or a blood disorder.

In one embodiment is provided a compound of formula (Ia) or a pharmaceutically acceptable salt and/or solvate thereof as defined herein, for use in treating a symptom associated with an inflammatory disease, a disease associated with an undesirable immune response, cancer, obesity, a diabetic disease or a blood disorder.

In one embodiment is provided a compound of formula (Ia) or a pharmaceutically acceptable salt and/or solvate thereof as defined herein, for use in preventing an inflammatory disease, a disease associated with an undesirable immune response, cancer, obesity, a diabetic disease or a blood disorder.

In one embodiment is provided a compound of formula (Ia) or a pharmaceutically acceptable salt and/or solvate thereof as defined herein, for use in preventing a symptom associated with an inflammatory disease, a disease associated with an undesirable immune response, cancer, obesity, a diabetic disease or a blood disorder.

In one embodiment is provided a compound of formula (Ia) or a pharmaceutically acceptable salt and/or solvate thereof as defined herein, for use in treating or preventing an inflammatory disease.

In one embodiment is provided a compound of formula (Ia) or a pharmaceutically acceptable salt and/or solvate thereof as defined herein, for use in treating or preventing a symptom associated with an inflammatory disease.

In one embodiment is provided a compound of formula (Ia) or a pharmaceutically acceptable salt and/or solvate thereof as defined herein, for use in treating or preventing inflammation associated with an inflammatory disease.

In one embodiment is provided a compound of formula (Ia) or a pharmaceutically acceptable salt and/or solvate thereof as defined herein, for use in treating or preventing a disease associated with an undesirable immune response.

In one embodiment is provided a compound of formula (Ia) or a pharmaceutically acceptable salt and/or solvate thereof as defined herein, for use in treating or preventing a symptom associated with a disease associated with an undesirable immune response.

In one embodiment is provided a compound of formula (Ia) or a pharmaceutically acceptable salt and/or solvate thereof as defined herein, for use in treating or preventing inflammation associated with a disease associated with an undesirable immune response

In one embodiment is provided a compound of formula (Ia) or a pharmaceutically acceptable salt and/or solvate thereof as defined herein, for use in treating or preventing cancer.

In one embodiment is provided a compound of formula (Ia) or a pharmaceutically acceptable salt and/or solvate thereof as defined herein, for use in treating or preventing a symptom associated with cancer.

In one embodiment is provided a compound of formula (Ia) or a pharmaceutically acceptable salt and/or solvate thereof as defined herein, for use in treating or preventing obesity.

In one embodiment is provided a compound of formula (Ia) or a pharmaceutically acceptable salt and/or solvate thereof as defined herein, for use in treating or preventing a symptom associated with obesity.

In one embodiment is provided a compound of formula (Ia) or a pharmaceutically acceptable salt and/or solvate thereof as defined herein, for use in treating or preventing a diabetic disease.

In one embodiment is provided a compound of formula (Ia) or a pharmaceutically acceptable salt and/or solvate thereof as defined herein, for use in treating or preventing a symptom associated with a diabetic disease.

In one embodiment is provided a compound of formula (Ia) or a pharmaceutically acceptable salt and/or solvate thereof as defined herein, for use in treating or preventing a blood disorder.

In one embodiment is provided a compound of formula (Ia) or a pharmaceutically acceptable salt and/or solvate thereof as defined herein, for use in treating or preventing a symptom associated with a blood disorder.

An undesirable immune response will typically be an immune response which gives rise to a pathology i.e. is a pathological immune response or reaction.

In one embodiment, the inflammatory disease or disease associated with an undesirable immune response is an auto-immune disease.

In one embodiment, the inflammatory disease or disease associated with an undesirable immune response is, or is associated with, a disease selected from the group consisting of: psoriasis (including chronic plaque, erythrodermic, pustular, guttate, inverse and nail variants), asthma, chronic obstructive pulmonary disease (COPD, including chronic bronchitis and emphysema), heart failure (including left ventricular failure), myocardial infarction, angina pectoris, other atherosclerosis and/or atherothrombosis-related disorders (including peripheral vascular disease and ischaemic stroke), a mitochondrial and neurodegenerative disease (such as Parkinson's disease, Alzheimer's disease, Huntington's disease, amyotrophic lateral sclerosis, retinitis pigmentosa or mitochondrial encephalomyopathy), autoimmune paraneoplastic retinopathy, transplantation rejection (including antibody-mediated and T cell-mediated forms), multiple sclerosis, transverse myelitis, ischaemia-reperfusion injury (e.g. during elective surgery such as cardiopulmonary bypass for coronary artery bypass grafting or other cardiac surgery, following percutaneous coronary intervention, following treatment of acute ST-elevation myocardial infarction or ischaemic stroke, organ transplantation, or acute compartment syndrome), AGE-induced genome damage, an inflammatory bowel disease (e.g. Crohn's disease or ulcerative colitis), primary sclerosing cholangitis (PSC), PSC-autoimmune hepatitis overlap syndrome, non-alcoholic fatty liver disease (non-alcoholic steatohepatitis), rheumatica, granuloma annulare, cutaneous lupus erythematosus (CLE), systemic lupus erythematosus (SLE), lupus nephritis, drug-induced lupus, autoimmune myocarditis or myopericarditis, Dressler's syndrome, giant cell myocarditis, post-pericardiotomy syndrome, drug-induced hypersensitivity syndromes (including hypersensitivity myocarditis), eczema, sarcoidosis, erythema nodosum, acute disseminated encephalomyelitis (ADEM), neuromyelitis optica spectrum disorders, MOG (myelin oligodendrocyte glycoprotein) antibody-associated disorders (including MOG-EM), optic neuritis, CLIPPERS (chronic lymphocytic inflammation with pontine perivascular enhancement responsive to steroids), diffuse myelinoclastic sclerosis, Addison's disease, alopecia areata, ankylosing spondylitis, other spondyloarthritides (including peripheral spondyloarthritis, that is associated with psoriasis, inflammatory bowel disease, reactive arthritis or juvenile onset forms), antiphospholipid antibody syndrome, autoimmune hemolytic anaemia, autoimmune hepatitis, autoimmune inner ear disease, pemphigoid (including bullous pemphigoid, mucous membrane pemphigoid, cicatricial pemphigoid, herpes gestationis or pemphigoid gestationis, ocular cicatricial pemphigoid), linear IgA disease, Behçet's disease, celiac disease, Chagas disease, dermatomyositis, diabetes mellitus type I, endometriosis, Goodpasture's syndrome, Graves' disease, Guillain-Barre syndrome and its subtypes (including acute inflammatory demyelinating polyneuropathy, AIDP, acute motor axonal neuropathy (AMAN), acute motor and sensory axonal neuropathy (AMSAN), pharyngeal-cervical-brachial variant, Miller-Fisher variant and Bickerstaff's brainstem encephalitis), progressive inflammatory neuropathy, Hashimoto's disease, hidradenitis suppurativa, inclusion body myositis, necrotising myopathy, Kawasaki disease, IgA nephropathy, Henoch-Schonlein purpura, idiopathic thrombocytopenic purpura, thrombotic thrombocytopenic purpura (TTP), Evans' syndrome, interstitial cystitis, mixed connective tissue disease, undifferentiated connective tissue disease, morphea, myasthenia gravis (including MuSK antibody positive and seronegative variants), narcolepsy, neuromyotonia, pemphigus vulgaris, pernicious anaemia, psoriatic arthritis, polymyositis, primary biliary cholangitis (also known as primary biliary cirrhosis), rheumatoid arthritis, palindromic rheumatism, schizophrenia, autoimmune (meningo-)encephalitis syndromes, scleroderma, Sjogren's syndrome, stiff person syndrome, polymylagia rheumatica, giant cell arteritis (temporal arteritis), Takayasu arteritis, polyarteritis nodosa, Kawasaki disease, granulomatosis with polyangitis (GPA; formerly known as Wegener's granulomatosis), eosinophilic granulomatosis with polyangiitis (EGPA; formerly known as Churg-Strauss syndrome), microscopic polyarteritis/polyangiitis, hypocomplementaemic urticarial vasculitis, hypersensitivity vasculitis, cryoglobulinemia, thromboangiitis obliterans (Buerger's disease), vasculitis, leukocytoclastic vasculitis, vitiligo, acute disseminated encephalomyelitis, adrenoleukodystrophy, Alexander's disease, Alper's disease, balo concentric sclerosis or Marburg disease, cryptogenic organising pneumonia (formerly known as bronchiolitis obliterans organizing pneumonia), Canavan disease, central nervous system vasculitic syndrome, Charcot-Marie-Tooth disease, childhood ataxia with central nervous system hypomyelination, chronic inflammatory demyelinating polyneuropathy (CIDP), diabetic retinopathy, globoid cell leukodystrophy (Krabbe disease), graft-versus-host disease (GVHD) (including acute and chronic forms, as well as intestinal GVHD), hepatitis C (HCV) infection or complication, herpes simplex viral infection or complication, human immunodeficiency virus (HIV) infection or complication, lichen planus, monomelic amyotrophy, fibrosis, cystic fibrosis, pulmonary arterial hypertension (PAH, including idiopathic PAH), lung sarcoidosis, idiopathic pulmonary fibrosis, kidney fibrosis, paediatric asthma, atopic dermatitis, allergic dermatitis, contact dermatitis, allergic rhinitis, rhinitis, sinusitis, conjunctivitis, allergic conjunctivitis, keratoconjunctivitis sicca, dry eye, xerophthalmia, glaucoma, macular oedema, diabetic macular oedema, central retinal vein occlusion (CRVO), macular degeneration (including dry and/or wet age related macular degeneration, AMD), post-operative cataract inflammation, uveitis (including posterior, anterior, intermediate and pan uveitis), iridocyclitis, scleritis, corneal graft and limbal cell transplant rejection, gluten sensitive enteropathy (coeliac disease), dermatitis herpetiformis, eosinophilic esophagitis, achalasia, autoimmune dysautonomia, autoimmune encephalomyelitis, autoimmune oophoritis, autoimmune orchitis, autoimmune pancreatitis, aortitis and periaortitis, autoimmune retinopathy, autoimmune urticaria, Behcet's disease, (idiopathic) Castleman's disease, Cogan's syndrome, IgG4-related disease, retroperitoneal fibrosis, juvenile idiopathic arthritis including systemic juvenile idiopathic arthritis (Still's disease), adult-onset Still's disease, ligneous conjunctivitis, Mooren's ulcer, pityriasis lichenoides et varioliformis acuta (PLEVA, also known as Mucha-Habermann disease), multifocal motor neuropathy (MMN), paediatric acute-onset neuropsychiatric syndrome (PANS) (including paediatric autoimmune neuropsychiatric disorders associated with streptococcal infections (PANDAS)), paraneoplastic syndromes (including paraneoplastic cerebellar degeneration, Lambert-Eaton myaesthenic syndrome, limbic encephalitis, brainstem encephalitis, opsoclonus myoclonus ataxia syndrome, anti-NMDA receptor encephalitis, thymoma-associated multiorgan autoimmunity), perivenous encephalomyelitis, reflex sympathetic dystrophy, relapsing polychondritis, sperm & testicular autoimmunity, Susac's syndrome, Tolosa-Hunt syndrome, Vogt-Koyanagi-Harada Disease, anti-synthetase syndrome, autoimmune enteropathy, immune dysregulation polyendocrinopathy enteropathy X-linked (IPEX), microscopic colitis, autoimmune lymphoproliferative syndrome (ALPS), autoimmune polyendocrinopathy-candidiasis-ectodermal dystrophy syndrome (APEX), gout, pseudogout, amyloid (including AA or secondary amyloidosis), eosinophilic fasciitis (Shulman syndrome) progesterone hypersensitivity (including progesterone dermatitis), familial Mediterranean fever (FMF), tumour necrosis factor (TNF) receptor-associated periodic fever syndrome (TRAPS), hyperimmunoglobulinaemia D with periodic fever syndrome (HIDS), PAPA (pyogenic arthritis, pyoderma gangrenosum, severe cystic acne) syndrome, deficiency of interleukin-1 receptor antagonist (DIRA), deficiency of the interleukin-36-receptor antagonist (DITRA), cryopyrin-associated periodic syndromes (CAPS) (including familial cold autoinflammatory syndrome [FCAS], Muckle-Wells syndrome, neonatal onset multisystem inflammatory disease [NOMID]), NLRP12-associated autoinflammatory disorders (NLRP12AD), periodic fever aphthous stomatitis (PFAPA), chronic atypical neutrophilic dermatosis with lipodystrophy and elevated temperature (CANDLE), Majeed syndrome, Blau syndrome (also known as juvenile systemic granulomatosis), macrophage activation syndrome, chronic recurrent multifocal osteomyelitis (CRMO), familial cold autoinflammatory syndrome, mutant adenosine deaminase 2 and monogenic interferonopathies (including Aicardi-Goutières syndrome, retinal vasculopathy with cerebral leukodystrophy, spondyloenchondrodysplasia, STING [stimulator of interferon genes]-associated vasculopathy with onset in infancy, proteasome associated autoinflammatory syndromes, familial chilblain lupus, dyschromatosis symmetrica hereditaria), Schnitzler syndrome; familial cylindromatosis, congenital B cell lymphocytosis, OTULIN-related autoinflammatory syndrome, type 2 diabetes mellitus, insulin resistance and the metabolic syndrome (including obesity-associated inflammation), atherosclerotic disorders (e.g. myocardial infarction, angina, ischaemic heart failure, ischaemic nephropathy, ischaemic stroke, peripheral vascular disease, aortic aneurysm), renal inflammatory disorders (e.g. diabetic nephropathy, membranous nephropathy, minimal change disease, crescentic glomerulonephritis, acute kidney injury, renal transplantation).

In one embodiment, the inflammatory disease or disease associated with an undesirable immune response is, or is associated with, a disease selected from the following autoinflammatory diseases: familial Mediterranean fever (FMF), tumour necrosis factor (TNF) receptor-associated periodic fever syndrome (TRAPS), hyperimmunoglobulinaemia D with periodic fever syndrome (HIDS), PAPA (pyogenic arthritis, pyoderma gangrenosum, and severe cystic acne) syndrome, deficiency of interleukin-1 receptor antagonist (DIRA), deficiency of the interleukin-36-receptor antagonist (DITRA), cryopyrin-associated periodic syndromes (CAPS) (including familial cold autoinflammatory syndrome [FCAS], Muckle-Wells syndrome, and neonatal onset multisystem inflammatory disease [NOMID]), NLRP12-associated autoinflammatory disorders (NLRP12AD), periodic fever aphthous stomatitis (PFAPA), chronic atypical neutrophilic dermatosis with lipodystrophy and elevated temperature (CANDLE), Majeed syndrome, Blau syndrome (also known as juvenile systemic granulomatosis), macrophage activation syndrome, chronic recurrent multifocal osteomyelitis (CRMO), familial cold autoinflammatory syndrome, mutant adenosine deaminase 2 and monogenic interferonopathies (including Aicardi-Goutières syndrome, retinal vasculopathy with cerebral leukodystrophy, spondyloenchondrodysplasia, STING [stimulator of interferon genes]-associated vasculopathy with onset in infancy, proteasome associated autoinflammatory syndromes, familial chilblain lupus, dyschromatosis symmetrica hereditaria) and Schnitzler syndrome.

In one embodiment, the inflammatory disease or disease associated with an undesirable immune response is, or is associated with, a disease selected from the following diseases mediated by excess NF-κB or gain of function in the NF-κB signalling pathway or in which there is a major contribution to the abnormal pathogenesis therefrom (including non-canonical NF-κB signalling): familial cylindromatosis, congenital B cell lymphocytosis, OTULIN-related autoinflammatory syndrome, type 2 diabetes mellitus, insulin resistance and the metabolic syndrome (including obesity-associated inflammation), atherosclerotic disorders (e.g. myocardial infarction, angina, ischaemic heart failure, ischaemic nephropathy, ischaemic stroke, peripheral vascular disease, aortic aneurysm), renal inflammatory disorders (e.g. diabetic nephropathy, membranous nephropathy, minimal change disease, crescentic glomerulonephritis, acute kidney injury, renal transplantation), asthma, COPD, type 1 diabetes mellitus, rheumatoid arthritis, multiple sclerosis, inflammatory bowel disease (including ulcerative colitis and Crohn's disease), and SLE.

In one embodiment, the disease is selected from the group consisting of rheumatoid arthritis, psoriatic arthritis, ankylosing spondylitis, systemic lupus erythematosus, multiple sclerosis, psoriasis, inflammatory bowel disease (including ulcerative colitis and Crohn's disease), atopic dermatitis, fibrosis, uveitis, cryopyrin-associated periodic syndromes, Muckle-Wells syndrome, juvenile idiopathic arthritis, chronic obstructive pulmonary disease and asthma.

In one embodiment, the disease is multiple sclerosis.

In one embodiment, the disease is psoriasis.

In one embodiment, the disease is asthma.

In one embodiment, the disease is chronic obstructive pulmonary disease.

In one embodiment, the disease is systemic lupus erythematosus.

In one embodiment, the disease is rheumatoid arthritis.

In one embodiment, the disease is inflammatory bowel disease (including ulcerative colitis and Crohn's disease).

In one embodiment, the disease is atopic dermatitis.

In one embodiment, the disease is fibrosis.

In one embodiment, cancer is selected from the group consisting of acute lymphoblastic leukaemia, adult; acute lymphoblastic leukaemia, childhood; acute myeloid leukaemia, adult; adrenocortical carcinoma; adrenocortical carcinoma, childhood; aids-related lymphoma; aids-related malignancies; anal cancer; astrocytoma, childhood cerebellar; astrocytoma, childhood cerebral; Barrett's esophagus (pre-malignant syndrome); bile duct cancer, extrahepatic; bladder cancer; bladder cancer, childhood; bone cancer, osteosarcoma/malignant fibrous histiocytoma; brain stem glioma, childhood; brain tumour, adult; brain tumour, brain stem glioma, childhood; brain tumour, cerebellar astrocytoma, childhood; brain tumour, cerebral astrocytoma/malignant glioma, childhood; brain tumour, ependymoma, childhood; brain tumour, medulloblastoma, childhood; brain tumour, supratentorial primitive neuroectodermal tumours, childhood; brain tumour, visual pathway and hypothalamic glioma, childhood; brain tumour, childhood (other); breast cancer; breast cancer and pregnancy; breast cancer, childhood; breast cancer, male; bronchial adenomas/carcinoids, childhood; carcinoid tumour, childhood; carcinoid tumour, gastrointestinal; carcinoma, adrenocortical; carcinoma, islet cell; carcinoma of unknown primary; central nervous system lymphoma, primary; cerebellar astrocytoma, childhood; cerebral astrocytoma/malignant glioma, childhood; cervical cancer; childhood cancers; chronic lymphocytic leukaemia; chronic myelogenous leukaemia; chronic myeloproliferative disorders; clear cell sarcoma of tendon sheaths; colon cancer; colorectal cancer; colorectal cancer, childhood; cutaneous t-cell lymphoma; endometrial cancer; ependymoma, childhood; epithelial cancer, ovarian; oesophageal cancer; oesophageal cancer, childhood; Ewing's family of tumours; extracranial germ cell tumour, childhood; extragonadal germ cell tumour; extrahepatic bile duct cancer; eye cancer, intraocular melanoma; eye cancer, retinoblastoma; gallbladder cancer; gastric (stomach) cancer; gastric (stomach) cancer, childhood; gastrointestinal carcinoid tumour; germ cell tumour, extracranial, childhood; germ cell tumour, extragonadal; germ cell tumour, ovarian; gestational trophoblastic tumour; glioma, childhood brain stem; glioma, childhood visual pathway and hypothalamic; hairy cell leukaemia; head and neck cancer; hepatocellular (liver) cancer; hepatocellular (liver) cancer, adult (primary); hepatocellular (liver) cancer, childhood (primary); cancer of the esophagus; Hodgkin's lymphoma; Hodgkin's lymphoma, adult; Hodgkin's lymphoma, childhood; Hodgkin's lymphoma during pregnancy; hypopharyngeal cancer; hypothalamic and visual pathway glioma, childhood; intraocular melanoma; islet cell carcinoma (endocrine pancreas); cancer of the endocrine system (e.g., cancer of the thyroid, pancreas, parathyroid or adrenal glands); Kaposi's sarcoma; kidney cancer; laryngeal cancer; laryngeal cancer, childhood; leukaemia, acute lymphoblastic, adult; leukaemia, acute lymphoblastic, childhood; leukaemia, acute myeloid, adult; leukaemia, acute myeloid, childhood; leukaemia, chronic lymphocytic; leukaemia, chronic myelogenous; leukaemia, hairy cell; lymphocytic lymphoma; lip and oral cavity cancer; liver cancer, adult (primary); liver cancer, childhood (primary); lung cancer; lung cancer, non-small cell; lung cancer, small cell; lymphoblastic leukaemia, adult acute; lymphoblastic leukaemia, childhood acute; lymphocytic leukaemia, chronic; lymphoma, aids- related; lymphoma, central nervous system (primary); lymphoma, cutaneous t-cell; lymphoma, Hodgkin's, adult; lymphoma, Hodgkin's, childhood; lymphoma, Hodgkin's during pregnancy; lymphoma, non-Hodgkin's, adult; lymphoma, non-Hodgkin's, childhood; lymphoma, non-Hodgkin's during pregnancy; lymphoma, primary central nervous system; macroglobulinemia, Waldenstrom's; male breast cancer; malignant mesothelioma, adult; malignant mesothelioma, childhood; malignant thymoma; medulloblastoma, childhood; melanoma; melanoma, intraocular; Merkel cell carcinoma; mesothelioma, malignant; metastatic squamous neck cancer with occult primary; multiple endocrine neoplasia syndrome, childhood; multiple myeloma/plasma cell neoplasm; mycosis fungoides; myelodysplastic syndromes; myelogenous leukaemia, chronic; myeloid leukaemia, childhood acute; myeloma, multiple; myeloproliferative disorders, chronic; nasal cavity and paranasal sinus cancer; nasopharyngeal cancer; nasopharyngeal cancer, childhood; neoplastic cutaneous disease; neuroblastoma; non-Hodgkin's lymphoma, adult; non-Hodgkin's lymphoma, childhood; non-Hodgkin's lymphoma during pregnancy; non-small cell lung cancer; neoplasms of the central nervous system (e.g., primary CNS lymphoma, spinal axis tumors, medulloblastoma, brain stem gliomas or pituitary adenomas); oat-cell cancer; oral cancer, childhood; oral cavity and lip cancer; oropharyngeal cancer; osteosarcoma/malignant fibrous histiocytoma of bone; ovarian cancer; ovarian cancer, childhood; ovarian epithelial cancer; ovarian germ cell tumour; ovarian low malignant potential tumour; pediatric malignancy; pancreatic cancer; pancreatic cancer, childhood; pancreatic cancer, islet cell; paranasal sinus and nasal cavity cancer; parathyroid cancer; penile cancer; pheochromocytoma; pineal and supratentorial primitive neuroectodermal tumours, childhood; pituitary tumour; plasma cell neoplasm/multiple myeloma; pleuropulmonary blastoma; pregnancy and breast cancer; pregnancy and Hodgkin's lymphoma; pregnancy and non-Hodgkin's lymphoma; primary central nervous system lymphoma; primary liver cancer, adult; primary liver cancer, childhood; prostate cancer (particularly hormone-refractory); chronic or acute leukemia; solid tumors of childhood; hypereosinophilia; rectal cancer; renal cell (kidney) cancer; renal cell cancer, childhood; renal pelvis and ureter, transitional cell cancer; retinoblastoma; rhabdomyosarcoma, childhood; salivary gland cancer; salivary gland cancer, childhood; sarcoma, Ewing's family of tumours; sarcoma, Kaposi's; sarcoma (osteosarcoma)/malignant fibrous histiocytoma of bone; sarcoma, rhabdomyosarcoma, childhood; sarcomas of soft tissues; sarcoma, soft tissue, adult; sarcoma, soft tissue, childhood; Sezary syndrome; skin cancer; skin cancer, childhood; skin cancer (melanoma); skin carcinoma, Merkel cell; small cell lung cancer; dermatofibrosarcoma protuberans; small intestine cancer; soft tissue sarcoma, adult; soft tissue sarcoma, childhood; cancer of the head and neck; squamous neck cancer with occult primary, metastatic; stomach (gastric) cancer; stomach (gastric) cancer, childhood; supratentorial primitive neuroectodermal tumours, childhood; t-cell lymphoma, cutaneous; testicular cancer; thymoma, childhood; thymoma, malignant; thyroid cancer; thyroid cancer, childhood; transitional cell cancer of the renal pelvis and ureter; trophoblastic tumour, gestational; unknown primary site, cancer of, childhood; unusual cancers of childhood; ureter and renal pelvis, transitional cell cancer; urethral cancer; cancer of the ureter (e.g., renal cell carcinoma, carcinoma of the renal pelvis); cancer of the penis; gynecologic tumors; uterine cancer; uterine sarcoma; carcinoma of the fallopian tubes; carcinoma of the endometrium; vaginal cancer; carcinoma of the vagina; carcinoma of the vulva; visual pathway and hypothalamic glioma, childhood; vulvar cancer; Waldenstrom's macro globulinemia; and Wilms' tumour.

In one embodiment, cancer is selected from the group consisting of lung cancer; NSCLC (non-small cell lung cancer); oat-cell cancer; bone cancer; pancreatic cancer; skin cancer; dermatofibrosarcoma protuberans; cancer of the head and neck; cutaneous or intraocular melanoma; uterine cancer; ovarian cancer; colo-rectal cancer; anal cancer; stomach cancer; colon cancer; breast cancer; gynecologic tumors (e.g., uterine sarcomas, carcinoma of the fallopian tubes, carcinoma of the endometrium, carcinoma of the cervix, carcinoma of the vagina or carcinoma of the vulva); Hodgkin's Disease; hepatocellular cancer; cancer of the esophagus; small intestine cancer; cancer of the endocrine system (e.g., cancer of the thyroid, pancreas, parathyroid or adrenal glands); sarcomas of soft tissues; urethral cancer; cancer of the penis; prostate cancer (particularly hormone-refractory); chronic or acute leukemia; solid tumors of childhood; hypereosinophilia; lymphocytic lymphomas; bladder cancer; kidney cancer; cancer of the ureter (e.g., renal cell carcinoma, carcinoma of the renal pelvis); pediatric malignancy; neoplasms of the central nervous system (e.g., primary CNS lymphoma, spinal axis tumors, medulloblastoma, brain stem gliomas or pituitary adenomas); Barrett's esophagus (pre-malignant syndrome) and neoplastic cutaneous disease.

"Obesity" refers to a condition in which a subject has a body mass index of greater than or equal to 30. The body mass index (BMI) is according to the "NIH Clinical Guidelines on the Identification and Evaluation, and Treatment of Overweight and Obesity in Adults" (1998).

In one embodiment, administration of a compound of formula (la) to a subject reduces the BMI of the subject to less than 30, for example less than 29, less than 28, less than 27, less than 26, or less than 25. In one embodiment, a compound of formula (la) is used to treat or prevent aberrant or inappropriate weight gain, metabolic rate, or fat deposition, for example is used to treat anorexia, bulimia, obesity, diabetes, or hyperlipidemia (e.g., elevated triglycerides and/or elevated cholesterol), as well as disorders of fat or lipid metabolism. In one embodiment, a compound of formula (la) is used to treat or prevent metabolic syndrome.

In one embodiment, a compound of formula (Ia) is used to treat obesity associated with Prader-Willi Syndrome (PWS). In one embodiment, a compound of formula (la) is used to reduce body fat, prevent increased body fat, reduce cholesterol (e.g., total cholesterol and/or ratios of total cholesterol to HDL cholesterol), and/or reduce appetite in individuals having PWS associated obesity, and/or reduce comorbidities such as diabetes, cardiovascular disease, and stroke.

A "diabetic disease" refers to diabetes mellitus ("diabetes") or a diabetic complication. The two main types of diabetes are (i) Type 1 diabetes resulting from the pancreas not producing insulin for which the usual treatment is insulin replacement therapy and (ii) Type 2 diabetes where patients either produce insufficient insulin or have insulin resistance. Diabetic complications include microvascular and macrovascular complications, and include coronary artery disease, peripheral artery disease, stroke, diabetic nephropathy, diabetic neuropathy, diabetic retinopathy, diabetic kidney disease and NASH.

In one embodiment, a "blood disorder" is selected from the group consisting of thalassemia (e.g. beta-thalassemia), hereditary spherocytosis, hereditary elliptocytosis, abetalipoproteinemia (or Bassen-Kornzweig syndrome), paroxysmal nocturnal hemoglobinuria, acquired hemolytic anaemia (e.g., congenital anaemias (e.g., enzymopathies)), and anaemia of chronic diseases.

### Administration

The compound of formula (la) is usually administered as a pharmaceutical composition. Thus, in one embodiment, there is provided a pharmaceutical composition comprising a compound of formula (la) and one or more pharmaceutically acceptable diluents or carriers.

The compound of formula (la) may be administered by any convenient method, e.g. by oral, parenteral, buccal, sublingual, nasal, rectal, intrathecal or transdermal administration, and the pharmaceutical compositions adapted accordingly.

The compound of formula (la) may be administered topically to the target organ e.g. topically to the eye, lung, nose or skin. Hence the invention provides a pharmaceutical composition comprising a compound of formula (la) optionally in combination with one or more topically acceptable diluents or carriers.

A compound of formula (Ia) which is active when given orally can be formulated as a liquid or solid, e.g. as a syrup, suspension, emulsion, tablet, capsule or lozenge.

A liquid formulation will generally consist of a suspension or solution of the compound of formula (la) in a suitable liquid carrier(s). Suitably the carrier is non-aqueous e.g. polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient can be prepared using standard carriers and then filled into a hard gelatine capsule; alternatively, a dispersion or suspension can be prepared using any suitable pharmaceutical carrier(s), e.g. aqueous gums, celluloses, silicates or oils and the dispersion or suspension then filled into a soft gelatine capsule.

Typical parenteral compositions consist of a solution or suspension of the compound of formula (la) in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the compound of formula (la) in a pharmaceutically acceptable aqueous or non-aqueous solvent and are usually presented in single or multidose quantities in sterile form in a sealed container which can take the form of a cartridge or refill for use with an atomising device. Alternatively, the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a chlorofluorocarbon (CFC) or a hydrofluorocarbon (HFC). Aerosol dosage forms can also take the form of pump-atomisers.

Topical administration to the lung may be achieved by use of an aerosol formulation. Aerosol formulations typically comprise the active ingredient suspended or dissolved in a suitable aerosol propellant, such as a chlorofluorocarbon (CFC) or a hydrofluorocarbon (HFC).

Topical administration to the lung may also be achieved by use of a non-pressurised formulation such as an aqueous solution or suspension. These may be administered by means of a nebuliser e.g. one that can be hand-held and portable or for home or hospital use (i.e. non-portable). The formulation may comprise excipients such as water, buffers, tonicity adjusting agents, pH adjusting agents, surfactants and co-solvents.

Topical administration to the lung may also be achieved by use of a dry-powder formulation. The formulation will typically contain a topically acceptable diluent such as lactose, glucose or mannitol (preferably lactose).

The compound of the invention may also be administered rectally, for example in the form of suppositories or enemas, which include aqueous or oily solutions as well as suspensions and emulsions and foams. Such compositions are prepared following standard procedures, well known by those skilled in the art. For example, suppositories can be prepared by mixing the active ingredient with a conventional suppository base such as cocoa butter or other glycerides. In this case, the drug is mixed with a suitable non-irritating excipient which is solid at ordinary temperatures but liquid at the rectal temperature and will therefore melt in the rectum to release the drug. Such materials are cocoa butter and polyethylene glycols.

Generally, for compositions intended to be administered topically to the eye in the form of eye drops or eye ointments, the total amount of the compound of the present invention will be about 0.0001 to less than 4.0% (w/w).

Preferably, for topical ocular administration, the compositions administered according to the present invention will be formulated as solutions, suspensions, emulsions and other dosage forms.

The compositions administered according to the present invention may also include various other ingredients, including, but not limited to, tonicity agents, buffers, surfactants, stabilizing polymer, preservatives, co-solvents and viscosity building agents. Suitable pharmaceutical compositions of the present invention include a compound of the invention formulated with a tonicity agent and a buffer. The pharmaceutical compositions of the present invention may further optionally include a surfactant and/or a palliative agent and/or a stabilizing polymer.

Various tonicity agents may be employed to adjust the tonicity of the composition, preferably to that of natural tears for ophthalmic compositions. For example, sodium chloride, potassium chloride, magnesium chloride, calcium chloride, simple sugars such as dextrose, fructose, galactose, and/or simply polyols such as the sugar alcohols mannitol, sorbitol, xylitol, lactitol, isomaltitol, maltitol, and hydrogenated starch hydrolysates may be added to the composition to approximate physiological tonicity. Such an amount of tonicity agent will vary, depending on the particular agent to be added. In general, however, the compositions will have a tonicity agent in an amount sufficient to cause the final composition to have an ophthalmically acceptable osmolality (generally about 150-450 mOsm, preferably 250-350 mOsm and most preferably at approximately 290 mOsm). In general, the tonicity agents of the invention will be present in the range of 2 to 4% w/w. Preferred tonicity agents of the invention include the simple sugars or the sugar alcohols, such as D-mannitol.

An appropriate buffer system (e.g. sodium phosphate, sodium acetate, sodium citrate, sodium borate or boric acid) may be added to the compositions to prevent pH drift under storage conditions. The particular concentration will vary, depending on the agent employed. Preferably however, the buffer will be chosen to maintain a target pH within the range of pH 5 to 8, and more preferably to a target pH of pH 5 to 7.

Surfactants may optionally be employed to deliver higher concentrations of compound of the present invention. The surfactants function to solubilise the compound and stabilise colloid dispersion, such as micellar solution, microemulsion, emulsion and suspension. Examples of surfactants which may optionally be used include polysorbate, poloxamer, polyosyl 40 stearate, polyoxyl castor oil, tyloxapol, Triton, and sorbitan monolaurate. Preferred surfactants to be employed in the invention have a hydrophile/lipophile/balance "HLB" in the range of 12.4 to 13.2 and are acceptable for ophthalmic use, such as TritonX114 and tyloxapol.

Additional agents that may be added to the ophthalmic compositions of compounds of the present invention are demulcents which function as a stabilising polymer. The stabilizing polymer should be an ionic/charged example with precedence for topical ocular use, more specifically, a polymer that carries negative charge on its surface that can exhibit a zeta-potential of (-)10-50 mV for physical stability and capable of making a dispersion in water (i.e. water soluble). A preferred stabilising polymer of the invention would be polyelectrolyte, or polyelectrolytes if more than one, from the family of cross-linked polyacrylates, such as carbomers and Pemulen(R), specifically Carbomer 974p (polyacrylic acid), at 0.1-0.5% w/w.

Other compounds may also be added to the ophthalmic compositions of the compound of the present invention to increase the viscosity of the carrier. Examples of viscosity enhancing agents include, but are not limited to: polysaccharides, such as hyaluronic acid and its salts, chondroitin sulfate and its salts, dextrans, various polymers of the cellulose family; vinyl polymers; and acrylic acid polymers.

Topical ophthalmic products are typically packaged in multidose form. Preservatives are thus required to prevent microbial contamination during use. Suitable preservatives include: benzalkonium chloride, chlorobutanol, benzododecinium bromide, methyl paraben, propyl paraben, phenylethyl alcohol, edentate disodium, sorbic acid, polyquaternium-1, or other agents known to those skilled in the art. Such preservatives are typically employed at a level of from 0.001 to 1.0% w/v. Unit dose compositions of the present invention will be sterile, but typically unpreserved. Such compositions, therefore, generally will not contain preservatives.

Compositions suitable for buccal or sublingual administration include tablets, lozenges and pastilles where the compound of formula (Ia) is formulated with a carrier such as sugar and acacia, tragacanth, or gelatine and glycerine.

Compositions suitable for transdermal administration include ointments, gels and patches.

The composition may contain from 0.1% to 100% by weight, for example from 10 to 60% by weight, of the compound of formula (la), depending on the method of administration. The composition may contain from 0% to 99.9% by weight, for example 40% to 90% by weight, of the carrier, depending on the method of administration. The composition may contain from 0.05mg to 1000 mg, for example from 1.0 mg to 500 mg, such as from 1.0 mg to 50 mg, e.g. about 10 mg of the compound of formula (la), depending on the method of administration. The composition may contain from 50 mg to 1000 mg, for example from 100 mg to 400 mg of the carrier, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 500 mg, such as from 1.0 mg to 50 mg, e.g. about 10 mg and such unit doses may be administered more than once a day, for example two or three times a day. Such therapy may extend for a number of weeks or months.

In one embodiment of the invention, the compound of formula (Ia) is used in combination with a further therapeutic agent or agents. When the compound of formula (la) is used in combination with other therapeutic agents, the compounds may be administered either sequentially or simultaneously by any convenient route. Alternatively, the compounds may be administered separately.

When the compound of formula (Ia) is used for treating or preventing an inflammatory disease or a disease associated with an undesirable immune response, therapeutic agents which may be used in combination with the compound of formula (Ia) include: corticosteroids (glucocorticoids), retinoids (e.g. acitretin, isotretinoin, tazarotene), anthralin, vitamin D analogues (e.g. cacitriol, calcipotriol), calcineurin inhibitors (e.g. tacrolimus, pimecrolimus), phototherapy or photochemotherapy (e.g. psoralen ultraviolet irradiation, PUVA) or other form of ultraviolet light irradiation therapy, ciclosporine, thiopurines (e.g. azathioprine, 6-mercaptopurine), methotrexate, anti-TNFα agents (e.g. infliximab, etanercept, adalimumab, certolizumab, golimumab and biosimilars), phosphodiesterase-4 (PDE4) inhibition (e.g. apremilast, crisaborole), anti-IL-17 agents (e.g. brodalumab, ixekizumab, secukinumab), anti-IL12/IL-23 agents (e.g. ustekinumab, briakinumab), anti-IL-23 agents (e.g. guselkumab, tildrakizumab), JAK (Janus Kinase) inhibitors (e.g. tofacitinib, ruxolitinib, baricitinib, filgotinib, upadacitinib), plasma exchange, intravenous immune globulin (IVIG), cyclophosphamide, anti-CD20 B cell depleting agents (e.g. rituximab, ocrelizumab, ofatumumab, obinutuzumab), anthracycline analogues (e.g. mitoxantrone), cladribine, sphingosine 1-phosphate receptor modulators or sphingosine analogues (e.g. fingolimod, siponimod, ozanimod, etrasimod), interferon beta preparations (including interferon beta 1b/1a), glatiramer, anti-CD3 therapy (e.g. OKT3), anti-CD52 targeting agents (e.g. alemtuzumab), leflunomide, teriflunomide, gold compounds, laquinimod, potassium channel blockers (e.g. dalfampridine/4-aminopyridine), mycophenolic acid, mycophenolate mofetil, purine analogues (e.g. pentostatin), mTOR (mechanistic target of rapamycin) pathway inhibitors (e.g. sirolimus, everolimus), anti-thymocyte globulin (ATG), IL-2 receptor (CD25) inhibitors (e.g. basiliximab, daclizumab), anti-IL-6 receptor or anti-IL-6 agents (e.g. tocilizumab, siltuximab), Bruton's tyrosine kinase (BTK) inhibitors (e.g. ibrutinib), tyrosine kinase inhibitors (e.g. imatinib), ursodeoxycholic acid, hydroxychloroquine, chloroquine, B cell activating factor (BAFF, also known as BlyS, B lymphocyte stimulator) inhibitors (e.g. belimumab, blisibimod), other B cell targeted therapy including fusion proteins targeting both APRIL (A Proliferation-Inducing Ligand) and BlyS (e.g. atacicept), PI3K inhibitors including pan-inhibitors or those targeting the p110δ and/or p110γ containing isoforms (e.g. idelalisib, copanlisib, duvelisib), interferon α receptor inhibitors (e.g. anifrolumab, sifalimumab), T cell co-stimulation blockers (e.g. abatacept, belatacept), thalidomide and its derivatives (e.g. lenalidomide), dapsone, clofazimine, leukotriene antagonists (e.g. montelukast), theophylline, anti-IgE therapy (e.g. omalizumab), anti-IL-5 agents (e.g. mepolizumab, reslizumab), long-acting muscarinic agents (e.g. tiotropium, aclidinium, umeclidinium), PDE4 inhibitors (e.g. roflumilast), riluzole, free radical scavengers (e.g. edaravone), proteasome inhibitors (e.g. bortezomib), complement cascade inhibitors including those directed against C5 (e.g. eculizumab), immunoadsor, antithymocyte globulin, 5-aminosalicylates and their derivatives (e.g. sulfasalazine, balsalazide, mesalamine), anti-integrin agents including those targeting α4β1 and/or α4β7 integrins (e.g. natalizumab, vedolizumab), anti-CD11-α agents (e.g. efalizumab), non-steroidal anti-inflammatory drugs (NSAIDs) including the salicylates (e.g. aspirin), propionic acids (e.g. ibuprofen, naproxen), acetic acids (e.g. indomethacin, diclofenac, etodolac), oxicams (e.g. meloxicam) and fenamates (e.g. mefenamic acid), selective or relatively selective COX-2 inhibitors (e.g. celecoxib, etroxicoxib, valdecoxib and etodolac, meloxicam, nabumetone), colchicine, IL-4 receptor inhibitors (e.g. dupilumab), topical/contact immunotherapy (e.g. diphenylcyclopropenone, squaric acid dibutyl ester), anti-IL-1 receptor therapy (e.g. anakinra), IL-1β inhibitor (e.g. canakinumab), IL-1 neutralising therapy (e.g. rilonacept), chlorambucil, specific antibiotics with immunomodulatory properties and/or ability to modulate NRF2 (e.g. tetracyclines including minocycline, clindamycin, macrolide antibiotics), anti-androgenic therapy (e.g. cyproterone, spironolactone, finasteride), pentoxifylline, ursodeoxycholic acid, obeticholic acid, fibrate, cystic fibrosis transmembrane conductance regulator (CFTR) modulators, VEGF (vascular endothelial growth factor) inhibitors (e.g. bevacizumab, ranibizumab, pegaptanib, aflibercept), pirfenidone, and mizoribine.

When the compound of formula (la) is used for treating or preventing cancer, therapeutic agents which may be used in combination with the compound of formula (la) include active agents which are used in conjunction with cancer therapy, such as agents used as palliative treatments to ameliorate unwanted side effects. Therefore, in one embodiment, the additional therapeutic agent is an agent used as a palliative treatment such as selected from the group consisting of: antiemetic agents, medication intended to alleviate pain such as opioids, medication used to decrease high blood uric acid levels such as allopurinol or rasburicase, anti-depressants, sedatives, anti-convulsant drugs, laxatives, anti-diarrhoeal drugs and/or antacids.

In another embodiment, the additional therapeutic agent is an additional cancer treatment such as chemotherapy, a targeted therapy, immunotherapy and hormonal therapy.

Examples of chemotherapy agents include antimetabolites (e.g., folic acid, purine, and pyrimidine derivatives) and alkylating agents (e.g., nitrogen mustards, nitrosoureas, platinum, alkyl sulfonates, hydrazines, triazenes, aziridines, spindle poison, cytotoxic agents, toposimerase inhibitors and others). In one embodiment, the additional therapeutic agent is a chemotherapy agent and is selected from the group consisting of Aclarubicin, Actinomycin, Alitretinon, Altretamine, Aminopterin, Aminolevulinic acid, Amrubicin, Amsacrine, Anagrelide, Arsenic trioxide, Asparaginase, Atrasentan, Belotecan, Bexarotene, endamustine, Bleomycin, Bortezomib, Busulfan, Camptothecin, Capecitabine, Carboplatin, Carboquone, Carmofur, Carmustine, Celecoxib, Chlorambucil, Chlormethine, Cisplatin, Cladribine, Clofarabine, Crisantaspase, Cyclophosphamide, Cytarabine, Dacarbazine, Dactinomycin, Daunorubicin, Decitabine, Demecolcine, Docetaxel, Doxorubicin, Efaproxiral, Elesclomol, Elsamitrucin, Enocitabine, Epirubicin, Estramustine, Etoglucid, Etoposide, Floxuridine, Fludarabine, Fluorouracil (5FU), Fotemustine, Gemcitabine, Gliadel implants, Hydroxycarbamide, Hydroxyurea, Idarubicin, Ifosfamide, Irinotecan, Irofulven, Ixabepilone, Larotaxel, Leucovorin, Liposomal doxorubicin, Liposomal daunorubicin, Lonidamine, Lomustine, Lucanthone, Mannosulfan, Masoprocol, Melphalan, Mercaptopurine, Mesna, Methotrexate, Methyl aminolevulinate, Mitobronitol, Mitoguazone, Mitotane, Mitomycin, Mitoxantrone, Nedaplatin, Nimustine, Oblimersen, Omacetaxine, Ortataxel, Oxaliplatin, Paclitaxel, Pegaspargase, Pemetrexed, Pentostatin, Pirarubicin, Pixantrone, Plicamycin, Porfimer sodium, Prednimustine, Procarbazine, Raltitrexed, Ranimustine, Rubitecan, Sapacitabine, Semustine, Sitimagene ceradenovec, Satraplatin, Streptozocin, Talaporfin, Tegafur- uracil, Temoporfin, Temozolomide, Teniposide, Tesetaxel, Testolactone, Tetranitrate, Thiotepa, Tiazofurin, Tioguanine, Tipifarnib, Topotecan, Trabectedin, Triaziquone, Triethylenemelamine, Triplatin, Tretinoin, Treosulfan, Trofosfamide, Uramustine, Valrubicin, Verteporfin, Vinblastine, Vincristine, Vindesine, Vinfhmine, Vinorelbine, Vorinostat, and Zorubicin.

Examples of targeted therapies include tyrosine kinase inhibitors, cyclin-dependent kinase inhibitors, monoclonal antibodies and fusion proteins. In one embodiment, the additional therapeutic agent is selected from the group consisting of Axitinib, Bosutinib, Cediranib, dasatinib, erlotinib, imatinib, gefitinib, lapatinib, Lestaurtinib, Nilotinib, Semaxanib, Sorafenib, Sunitinib, Vandetanib, Alvocidib, Seliciclib, Herceptin, rituximab, Tositumomab, Cetuximab, Panitumumab, Trastuzumab, Alemtuzumab, Bevacizumab, Edrecolomab, Gemtuzumab, Aflibercept, Denileukin diftitox and Bexxar.

When the compound of formula (Ia) is used for treating or preventing obesity, therapeutic agents which may be used in combination with the compound of formula (la) include a gastric or pancreatic lipase inhibitor (such as orlistat); a lipid lowering agent (such as a statin, a fibrate, niacin or a derivative thereof (such as acipimox), lecithin, a bile acid sequesterant, ezetimibe, lomitapide, a phytosterol, an omega-3 supplement, a PCSK9 inhibitor); a CB-1 antagonist; a lipoxygenase inhibitor; a somostatin analogue; an insulin compound or insulin analogue (such as human insulin, insulin lispro, insulin aspart, insulin glulisine, insulin glargine, insulin degludec); an insulin sensitising agent such as a PPAR-gamma agonist, PPAR-alpha agonist or mixed PPAR-gamma/alpha agonist (such as metformin, pioglitazone or rosiglitazone); an insulin secretagogue (such as a nateglinide or repaglinide, or a sulfonylurea such as gliclazide, glimeperide, limepiride, glyburide); an SGLT2 inhibitor (such as dapagliflozin, canagliflozin or empagliflozin); an amylin analogue (such as pramlintide); a DPPIV inhibitor (such as sitagliptin, saxagliptin, linagliptin, alogliptin or vildagliptin); a GLP-1 agonist (such as albiglutide, dulaglutide, exenatide, liraglutide, semaglutide or lixisenatide); an alpha-glucosidase inhibitor (such as acarbose, miglitol or voglibose); a phosphodiesterase inhibitor (such as pentoxifylline); a glycogen phosphorylase inhibitor; an MCH-1 antagonist; a glucokinase activator; a glucagon antagonist; an insulin signalling agonist; a PTP1B inhibitor; a gluconeogenesis inhibitor; a GSK inhibitor or a galanin receptor agonist.

When the compound of formula (la) is used for treating or preventing a diabetic disease, therapeutic agents which may be used in combination with the compound of formula (I) include a gastric or pancreatic lipase inhibitor (such as orlistat); a lipid lowering agent (such as a statin, a fibrate, niacin or a derivative thereof (such as acipimox), lecithin, a bile acid sequesterant, ezetimibe, lomitapide, a phytosterol, an omega-3 supplement, a PCSK9 inhibitor); a CB-1 antagonist; a lipoxygenase inhibitor; a somostatin analogue; an insulin compound or insulin analogue (such as human insulin, insulin lispro, insulin aspart, insulin glulisine, insulin glargine, insulin degludec); an insulin sensitising agent such as a PPAR-gamma agonist, PPAR-alpha agonist or mixed PPAR-gamma/alpha agonist (such as metformin, pioglitazone or rosiglitazone); an insulin secretagogue (such as a nateglinide or repaglinide, or a sulfonylurea such as gliclazide, glimeperide, limepiride, glyburide); an SGLT2 inhibitor (such as dapagliflozin, canagliflozin or empagliflozin); an amylin analogue (such as pramlintide); a DPPIV inhibitor (such as sitagliptin, saxagliptin, linagliptin, alogliptin or vildagliptin); a GLP-1 agonist (such as albiglutide, dulaglutide, exenatide, liraglutide, semaglutide or lixisenatide); an alpha-glucosidase inhibitor (such as acarbose, miglitol or voglibose); a phosphodiesterase inhibitor (such as pentoxifylline); a glycogen phosphorylase inhibitor; an MCH-1 antagonist; a glucokinase activator; a glucagon antagonist; an insulin signalling agonist; a PTP1B inhibitor; a gluconeogenesis inhibitor; a GSK inhibitor or a galanin receptor agonist.

Compounds of formula (Ia) may display one or more of the following desirable properties:
- low EC₅₀ and/or high Eₘₐₓ values for activating PKM2;
- low EC₅₀ and/or high Eₘₐₓ values for activating PKLR;
- low EC₅₀ and/or high Eₘₐₓ values for activating PKM2 and PKLR;
- low IC₅₀ values for reducing cellular proliferation;
- improved aqueous solubility;
- enhanced absorption through improved aqueous solubility;
- low or zero off-target activity (e.g. agonist activity against 5HT2B), for example compared with TEPP-46;
- reduced dose and dosing frequency through improved pharmacokinetics;
- improved oral systemic bioavailability;
- reduced plasma clearance following intravenous dosing;
- augmented cell permeability;
- low toxicity at the relevant therapeutic dose.

### Abbreviations

- Ac: acetyl
- ADP: adenosine diphosphate
- ADME: absorption, distribution, metabolism, and excretion
- AIBN: azobisisobutyronitrile
- aq.: Aqueous
- ATP: adenosine triphosphate
- BBFO: broadband fluorine observe
- BEH: ethylene bridged hybrid
- Bn: benzyl
- Boc: tert-butyloxycarbonyl
- Bu: butyl
- CB-1: cannabinoid-1
- Cbz: benzyloxycarbonyl
- CSH: charged surface hybrid
- DAD: diode array detector
- DCE: dichloroethane
- DCM: dichloromethane
- DHP: dihydropyran
- DIPEA: N,N-diisopropylethylamine
- DMAP: dimethylaminopyridine
- DMF: dimethylformamide
- DMSO: dimethyl sulfoxide
- dppf: diphenylphosphino)ferrocene
- DPPIV: dipeptidyl peptidase-4
- Eq: Equivalents
- ES⁺: electrospray
- Et: ethyl
- FBP: fructose-1,6-bisphosphate
- FBS: fetal bovine serum
- Fmoc: 9-fluorenylmethyloxycarbonyl
- g: gram(s)
- GLP-1: glucagon-like peptide 1
- GSK: glycogen synthase kinase
- h: hour(s)
- HATU: (1-[Bis(dimethylamino)methylene]-1H-1,2,3-triazolo[4,5-b]pyridinium 3-oxide hexafluorophosphate
- HIF: hypoxia-inducible factor
- HPLC: high-performance liquid chromatography
- 5HT2B: 5-hydroxytryptamine receptor 2B
- IPC: ion pair chromatography
- IL: interleukin
- LAH: lithium aluminium hydride
- LCMS: liquid chromatography-mass spectrometry
- M: molar concentration / molar mass
- MCH: melanin-concentrating hormone
- Me: methyl
- mm: millimetre
- (M)Hz: (megahertz
- min(s): minute(s)
- mL: millilitres
- mmol: millimole
- MOM: methoxymethyl
- MS: mass spectrometry
- Ms: methanesulfonyl
- nm: nanometre
- NASH: non-alcoholic fatty liver disease
- NBS: *N*-bromosuccinimide
- NMR: nuclear magnetic resonance
- PBS: phosphate buffered saline
- PDA: photodiode array
- PEP: phosphoenolpyruvic acid
- PK: pyruvate kinase
- PMB: *para*-methoxybenzyl
- PPAR: peroxisome proliferator-activated receptor
- PTP1B: protein tyrosine phosphatase 1B
- rpm: revolutions per minute
- RT: room temperature
- sat: saturated
- SEM: [2-(Trimethylsilyl)ethoxy]methyl
- SGLT2: sodium-glucose transport protein 2
- STAT3: signal transducer and activator of transcription 3
- TBDMS: tert-butyldimethylsilyl
- TBME: tert-butyl methyl ether
- TCA: tricarboxylic acid cycle
- TEA: triethylamine
- Tf: trifluoromethanesulfonyl, i.e., CF₃SO₂-
- TFA: trifluoroacetic acid
- THF: tetrahydrofuran
- THP: tetrahydropyranyl
- TIPS: triisopropylsilyl
- TLC: thin layer chromatography
- TMS: trimethylsilyl
- TNF: tumour necrosis factor
- TOM: tri-iso-propylsilyloxymethyl
- Tr: trityl
- Ts: toluenesulfonyl
- µL: microlitre
- µM: micromolar
- UPLC: ultra performance liquid chromatography
- wt.: weight
- °C: degrees centigrade

### EXAMPLES

### Analytical Equipment

NMR spectra were recorded using a Bruker 400 MHz Avance III spectrometer fitted with a BBFO 5 mm probe, or a Bruker 500 MHz Avance III HD spectrometer equipped with a Bruker 5 mm SmartProbeTM. Spectra were measured at 298 K, unless indicated otherwise, and were referenced relative to the solvent resonance. The chemical shifts are reported in parts per million. Data were acquired using Bruker TopSpin software.

UPLC/MS analysis was carried out on a Waters Acquity UPLC system using either a Waters Acquity CSH C18 or BEH C18 column (2.1 x 30 mm) maintained at a temperature of 40 °C and eluted with a linear acetonitrile gradient appropriate for the lipophilicity of the compound over 3 or 10 minutes at a constant flow rate of 0.77 mL/min. The aqueous portion of the mobile phase was either 0.1 % Formic Acid (CSH C18 column) or 10 mM Ammonium Bicarbonate (BEH C18 column). LC-UV chromatograms were recorded using a Waters Acquity PDA detector between 210 and 400 nm. Mass spectra were recorded using a Waters Acquity Qda detector with electrospray ionisation switching between positive and negative ion mode. Sample concentration was adjusted to give adequate UV response.

LCMS analysis was carried out on a Agilent LCMS system using either a Waters Acquity CSH C18 (4.6 x 30 mm) or BEH C18 column (4.6 x 30 mm) maintained at a temperature of 40 °C and eluted with a linear acetonitrile gradient appropriate for the lipophilicity of the compound over 4 or 15 minutes at a constant flow rate of 2.5 mL/min. The aqueous portion of the mobile phase was either 0.1 % Formic Acid (CSH C18 column) or 10 mM Ammonium Bicarbonate (BEH C18 column). LC-UV chromatograms were recorded using an Agilent VWD or DAD detector at 254 nm. Mass spectra were recorded using an Agilent MSD detector with electrospray ionisation switching between positive and negative ion mode. Sample concentration was adjusted to give adequate UV response.

### Preparative HPLC Purification Methods

### Acidic method (A):

Product was dissolved in DMSO (mL), filtered and purified by reversed phase preparative HPLC (Waters 2767 Sample Manager, Waters 2545 Binary Gradient Module, Waters Systems Fluidics Organiser, Waters 515 ACD pump, Waters 515 Makeup pump, Waters 2998 Photodiode Array Detector, Waters QDa) using a Waters X-Select CSH C18 ODB prep column, 130Å, 5 µm, 30 mm X 100 mm, flow rate 40 mL min-1 eluting with a 0.1% formic acid in water-MeCN gradient over 12.5. At-column dilution pump gives 2 mL min-1 MeCN over the entire method, which is included in the following MeCN percentages. Gradient information: 0.0-0.5 min, Ip % MeCN; 0.5-10.5 min, ramped from Ip % MeCN to fp % MeCN; 10.5-10.6 min, ramped from fp % MeCN to 100% MeCN; 10.6-12.5 min, held at 100% MeCN. The clean fractions were evaporated in a Genevac.

### Basic method (B):

Product was dissolved in DMSO (0.5mL), filtered and purified by reversed phase preparative HPLC on a Waters X-Bridge BEH C18 ODB prep column, 130Å, 5 µm, 30 mm X 100 mm, flow rate 40 mL min-1 eluting with a 0.3% ammonia in water-MeCN gradient over 12.5 mins using UV detection across all wavelengths with PDA as well as a QDA and ELS detector. At-column dilution pump gives 2 mL min-1 MeCN over the entire method, which is included in the following MeCN percentages. Gradient information: 0.0-0.5 min, 20 % MeCN; 0.5-10.5 min, ramped from 20 % MeCN to 50 % MeCN; 10.5-10.6 min, ramped from 50 % MeCN to 100% MeCN; 10.6-12.5 min, held at 100% MeCN. The clean fractions were evaporated in a Genevac.

Alternatively, the following analytical LCMS equipment and methods were also used:

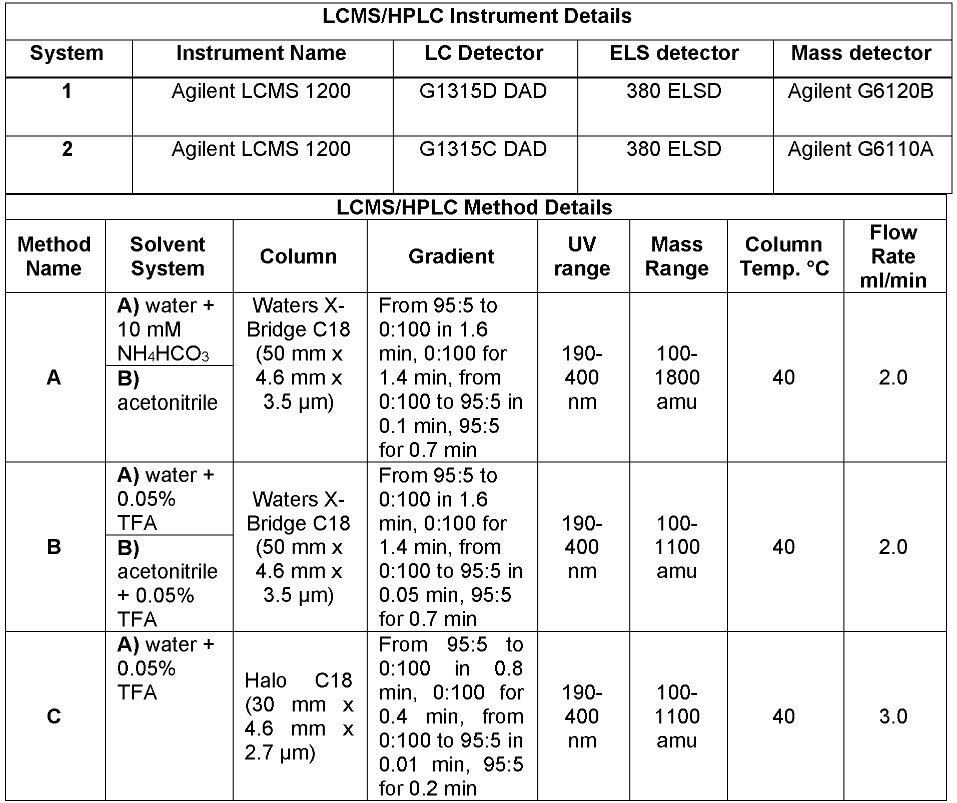

### Commercial Materials

All starting materials and solvents were obtained either from commercial sources or prepared according to the literature citation.

### General Methods

Unless otherwise stated all reactions were stirred. Organic solutions were routinely dried over anhydrous magnesium sulfate. Hydrogenations were performed on a Thales H-cube flow reactor under the conditions stated or under pressure in a gas autoclave (bomb).

### Intermediate 1 - 5-methyl-7-(methylsulfonyl)-3,5,6,7,8,9-hexahydro-4H-pyrido[4',3':4,5]pyrrolo[2,3-d]pyridazin-4-one

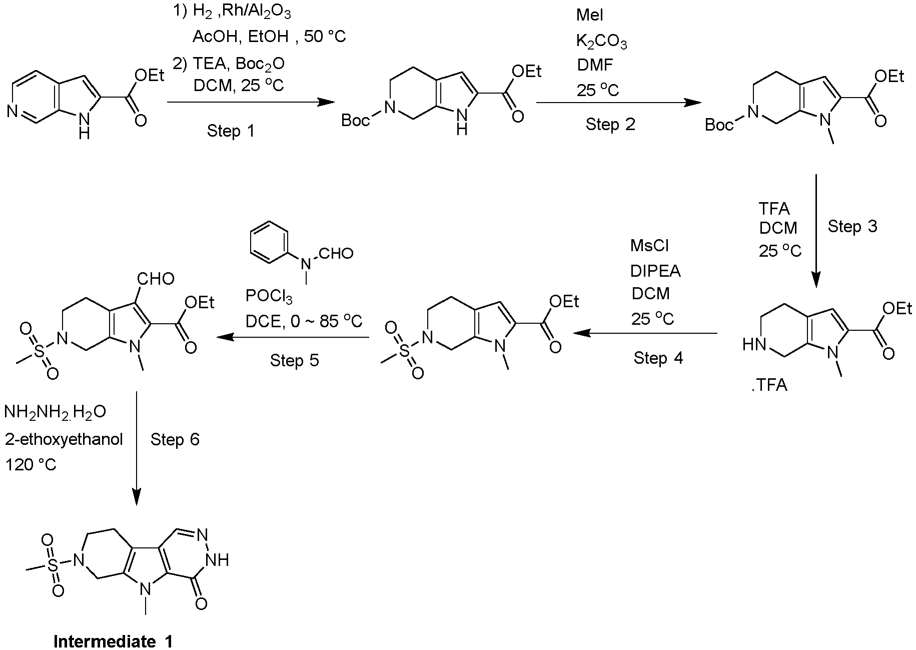

### Step 1

A mixture of ethyl 1H-pyrrolo[2,3-c]pyridine-2-carboxylate (6.00 g, 1 Eq, 31.5 mmol) and 5% Rh/Al₂O₃ (2.995 g, 5.0% Wt, 0.0461 Eq., 1.455 mmol) was suspended in acetic acid (120 mL). The mixture was stirred under hydrogen (5 bar.) at 50 °C for 21 h. The reaction mixture was allowed to cool to RT and then filtered through glass fibre filter paper washing with EtOAc. The filtrate was concentrated *in vacuo* to afford the crude product as a black oil. The crude product was azeotroped with toluene (3 times) to afford the crude product as a black oil. The crude residue was taken up in DCM (140 mL) and then treated with triethylamine (4.79 g, 6.60 mL, 1.50 Eq, 47.4 mmol) and a solution of Boc-anhydride (7.27 g, 7.65 mL, 1.06 Eq, 33.3 mmol) in DCM (20 mL) sequentially. The reaction mixture was stirred at RT for 18 h. Sat. aq. NaHCO₃ (160 mL) was added and then stirred until effervescence ceased. The organic layer was collected and the aqueous was extracted with DCM (2 x 100 mL). The combined organic extracts were washed with water (100 mL), 50% brine (100 mL), passed through a phase separator and then concentrated *in vacuo* to afford the crude product as a black oil (~10.6 g). The crude product was purified by chromatography on silica gel to afford 6-(tert-butyl) 2-ethyl 1,4,5,7-tetrahydro-6H-pyrrolo[2,3-c]pyridine-2,6-dicarboxylate (7.350 g, 90% Purity) as a sticky yellow gum. MS (ES⁺): 317 (M+Na)⁺

### Step 2

A suspension of 6-(tert-butyl) 2-ethyl 1,4,5,7-tetrahydro-6H-pyrrolo[2,3-c]pyridine-2,6-dicarboxylate (7.350 g, 90% Wt, 1 Eq, 22.47 mmol) and potassium carbonate (4.493 g, 1.447 Eq, 32.51 mmol) in DMF (100 mL) was treated with methyl iodide (3.973 g, 1.750 mL, 99% Wt, 1.233 Eq, 27.71 mmol) in one portion. The reaction mixture was stirred at RT for 18 h. Methyl iodide (708.9 mg, 312.3 µL, 99% Wt, 0.22 Eq, 4.944 mmol) and potassium carbonate (869.6 mg, 0.28 Eq, 6.293 mmol) were added and stirred for 18 h. The reaction mixture was concentrated *in vacuo* and the residue was partitioned between DCM (100 mL) and water (100 mL). The organic layer was collected and the aqueous was extracted with DCM (2 x 100 mL). The combined organic extracts were washed with 50% brine (100 mL), dried (phase separator) and concentrated *in vacuo* to afford the crude product as a yellow oil. The crude product was purified by chromatography on silica gel to afford 6-(tert-butyl) 2-ethyl 1-methyl-1,4,5,7-tetrahydro-6H-pyrrolo[2,3-c]pyridine-2,6-dicarboxylate (4.241 g, 98% Purity) as a thick yellow oil. MS (ES⁺): 331 (M+Na)⁺

### Step 3

A stirred solution of 6-(tert-butyl) 2-ethyl 1-methyl-1,4,5,7-tetrahydro-6H-pyrrolo[2,3-c]pyridine-2,6-dicarboxylate (4.240 g, 98% Wt, 1 Eq, 13.47 mmol) in DCM (100 mL) was treated with TFA (8.88 g, 6.00 mL, 5.78 Eq, 77.9 mmol) dropwise. The reaction mixture was stirred at RT for 18 h and then concentrated *in vacuo.* The residue was azeotroped with toluene (4 x 50 mL) to afford crude ethyl 1-methyl-4,5,6,7-tetrahydro-1H-pyrrolo[2,3-c]pyridine-2-carboxylate 2,2,2-trifluoroacetate (5.189 g, 83.0% Purity) as a yellow oil. ¹H NMR (400 MHz, DMSO-d6) δ 9.19 (s, 2H), 6.74 (s, 1H), 4.32 - 4.26 (m, 2H), 4.20 (q, J = 7.1 Hz, 2H), 3.74 (s, 3H), 3.31 (d, J = 6.3 Hz, 2H), 2.71 (t, J = 6.1 Hz, 2H), 1.26 (t, J = 7.1 Hz, 3H).

### Step 4

Mesyl chloride (1.834 g, 1.247 mL, 1.05 Eq, 16.01 mmol) was added dropwise to a solution of ethyl 1-methyl-4,5,6,7-tetrahydro-1H-pyrrolo[2,3-c]pyridine-2-carboxylate-2,2,2-trifluoroacetate (1/1) (5.189 g, 90% Wt, 1 Eq, 15.25 mmol) and DIPEA (5.912 g, 7.97 mL, 3 Eq, 45.74 mmol) in DCM (75 mL) under nitrogen and the mixture was stirred for 1h. 1N HCl was added and the layers separated. The organic layer was washed with water, sat. NaHCO₃, brine, then absorbed on silica. The crude product was purified by chromatography on silica gel to afford ethyl 1-methyl-6-(methylsulfonyl)-4,5,6,7-tetrahydro-1H-pyrrolo[2,3-c]pyridine-2-carboxylate (3.33 g, 76.3 %) as a white solid. ¹H NMR (400 MHz, DMSO-d6) δ 6.69 (s, 1H), 4.33 (s, 2H), 4.19 (q, J = 7.1 Hz, 2H), 3.73 (s, 3H), 3.39 (t, J = 5.7 Hz, 2H), 2.96 (s, 3H), 2.58 (t, J = 5.7 Hz, 2H), 1.25 (t, J = 7.1 Hz, 3H). MS (ES⁺): 287 (M+H)⁺

### Step 5

A stirred solution of N-methyl-N-phenylformamide (3.93 g, 3.59 mL, 2.5 Eq, 29.1 mmol) in DCE (15 mL) at 0 °C was treated with POCl₃ (4.46 g, 2.71 mL, 2.5 Eq, 29.1 mmol) dropwise. The reaction mixture was stirred at 0 °C for 0.5 h and then allowed to warm to RT for 1 h. The resultant orange solution was added to a stirred solution of ethyl 1-methyl-6-(methylsulfonyl)-4,5,6,7-tetrahydro-1H-pyrrolo[2,3-c]pyridine-2-carboxylate (3.33 g, 1 Eq, 11.6 mmol) in DCE (45 mL). The reaction mixture was heated to 80 °C overnight, then allowed to cool to RT. Sat. NaHCO₃ and DCM were added and the layers separated. The organic layer was washed with brine, then absorbed on silica. The crude product was purified by chromatography on silica gel to afford ethyl 3-formyl-1-methyl-6-(methylsulfonyl)-4,5,6,7-tetrahydro-1H-pyrrolo[2,3-c]pyridine-2-carboxylate (2.04 g, 95% Purity) as a white solid. MS (ES⁺): 315 (M+H)⁺

### Step 6

Hydrazine hydrate (677 mg, 670 µL, 35% Wt, 1.2 Eq, 7.40 mmol) was added to a mixture of ethyl 3-formyl-1-methyl-6-(methylsulfonyl)-4,5,6,7-tetrahydro-1H-pyrrolo[2,3-c]pyridine-2-carboxylate (2.04 g, 95% Wt, 1 Eq, 6.16 mmol) in 2-ethoxyethanol (40 mL) and the mixture was heated at 120°C for 2 h, then allowed to cool to RT. The mixture was poured into ice/water and the resulting precipitate was filtered, washing with water. The solid was then dried in a dessicator at 45°C overnight to afford 5-methyl-7-(methylsulfonyl)-3,5,6,7,8,9-hexahydro-4H-pyrido[4',3':4,5]pyrrolo[2,3-d]pyridazin-4-one (Intermediate 1, 1.025 g, 100% Purity) as a pale yellow solid. ¹H NMR (DMSO) δ: 12.32 (s, 1H), 8.15 (s, 1H), 4.47 (d, J = 1.5 Hz, 2H), 4.00 (s, 3H), 3.49 (t, J = 5.7, 5.7 Hz, 2H), 3.00 (s, 3H), 2.81 (t, J = 5.7, 5.7 Hz, 2H). MS (ES⁺): 283 (M+H)⁺

### Intermediate 2 - tert-butyl (3-((5-methyl-4-oxo-4,5,6,7,8,9-hexahydro-3H-pyrido[4',3':4,5]pyrrolo[2,3-d]pyridazin-3-yl)methyl)phenyl)carbamate

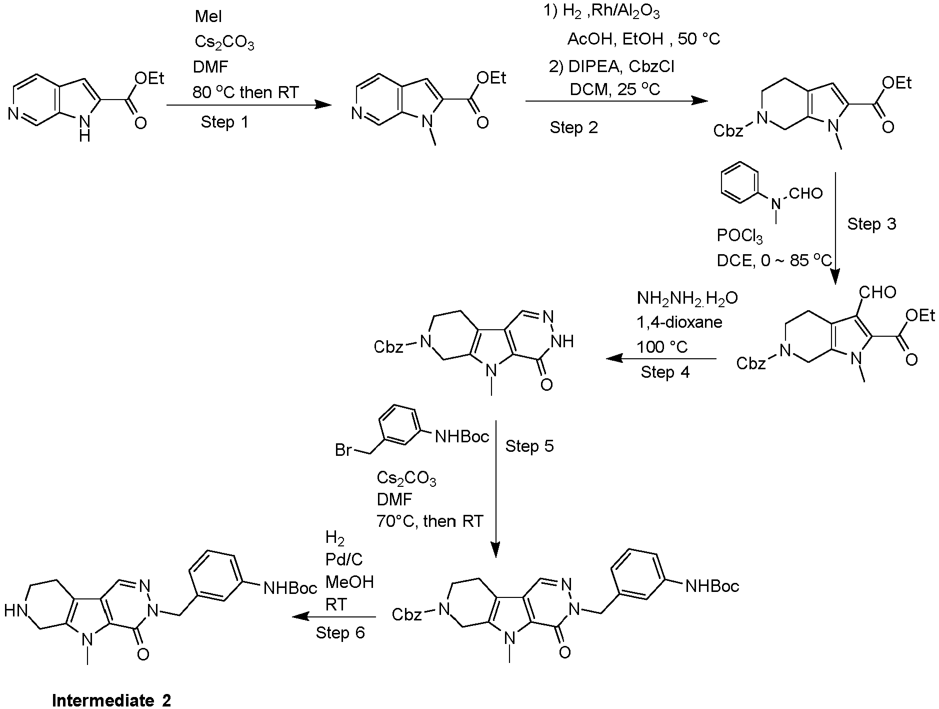

### Step 1

A suspension of ethyl 1H-pyrrolo[2,3-c]pyridine-2-carboxylate (3.00 g, 15.8 mmol, 1.0 Eq) in DMF (20.0 mL) was treated with Cs₂CO₃ (12.8 g, 39.4 mmol, 2.5 Eq). The reaction mixture was stirred at 80-85 °C for 1 h and then allowed to cool to RT. To the mixture a solution of CH₃I (1.57 g, 11.1 mmol, 687 uL, 0.7 Eq) in DMF (10.0 mL) was added drop-wise at room temperature. Then the reaction mixture was then stirred at 20-25 °C for 1 h. The reaction mixture was diluted with ethyl acetate (20.0 mL) and water (30.0 mL). The organic layer was collected and the aqueous was extracted with ethyl acetate (20.0 mL x 2). The combined organic extracts were washed with brine (20.0 mL), dried over Na₂SO₄ and concentrated *in vacuo* at 45 °C to afford ethyl 1-methyl-1H-pyrrolo[2,3-c]pyridine-2-carboxylate (3.00 g, crude) as a brown solid. ¹H NMR (400 MHz, DMSO-d6) δ 9.06 (s, 1H), 8.22 (d, J = 5.6 Hz, 1H), 7.64 (dd, J = 5.6 Hz, 1.2 Hz, 1H), 7.26 (s, 1H), 4.36 (q, J = 7.2 Hz, 2H), 4.12 (s, 3H), 1.35 (t, J = 7.2 Hz, 3H).

### Step 2

A suspension of ethyl 1-methyl-1H-pyrrolo[2,3-c]pyridine-2-carboxylate (212 mg, 1 Eq, 1.04 mmol) and 5% Rh/Al₂O₃ (107 mg, 5.0% Wt, 0.05 Eq, 51.9 µmol) in AcOH (5 mL) was stirred under H₂ (5 bar.) at 50 °C for 20 h. 5% Rh/Al₂O₃ (107 mg, 5.0% Wt, 0.05 Eq, 51.9 µmol) was added and stirred under H₂ (5 bar.) for 24 h. AcOH (10 mL) was added and stirred under H₂ (5 bar.) for 3 days. The reaction mixture was filtered through a glass fibre filter paper washing with EtOAc. The filtrate was concentrated *in vacuo* and then azeotroped with toluene (3 times) to afford the crude intermediate. A solution of the crude intermediate and DIPEA (403 mg, 542 µL, 3 Eq, 3.11 mmol) in DCM (9 mL) at 0 °C was treated with a solution of benzyl chloroformate (177 mg, 146 µL, 1 Eq, 1.04 mmol) in DCM (1 mL) dropwise. The reaction mixture was stirred at 0 °C for 15 min and then allowed to warm to RT for 20 h. The reaction mixture was diluted with DCM (10 mL) and washed with sat. aq. NaHCO₃ (10 mL). The organic layer was collected and the aqueous was extracted with DCM (2 x 10 mL). The combined organic extracts were dried (phase separator) and concentrated *in vacuo* to afford the crude product. The crude product was purified by chromatography on silica gel to afford 6-benzyl 2-ethyl 1-methyl-1,4,5,7-tetrahydro-6H-pyrrolo[2,3-c]pyridine-2,6-dicarboxylate (202 mg, 95% Purity) as a pale orange oil. MS (ES⁺): 343 (M+H)⁺

### Step 3

A stirred solution of N-methyl-N-phenylformamide (270 mg, 247 µL, 3.60 Eq, 2.00 mmol) in DCE (2 mL) at 0 °C was treated with POCI₃ (307 mg, 186 µL, 3.60 Eq, 2.00 mmol) dropwise. The solution was stirred at 0 °C for 2 h and then allowed to warm to RT for 30 min. The above solution was added to a solution of 6-benzyl 2-ethyl 1-methyl-1,4,5,7-tetrahydro-6H-pyrrolo[2,3-c]pyridine-2,6-dicarboxylate (200 mg, 95% Wt, 1 Eq, 555 µmol) in DCE (2 mL) and the reaction mixture was stirred at 85 °C for 20 h. The reaction mixture was diluted with DCM (5 mL) and sat. Na₂CO₃ (10 mL). The mixture was stirred until effervescence ceased. The organic layer was collected and the aqueous was extracted with DCM (10 mL). The combined organic extracts were dried (phase separator) and concentrated *in vacuo* to afford the crude product. The crude product was purified by chromatography on silica gel (dry load) (12 g cartridge, 0-100% EtOAc/isohexane, eluted ~50%) to afford 6-benzyl 2-ethyl 3-formyl-1-methyl-1,4,5,7-tetrahydro-6H-pyrrolo[2,3-c]pyridine-2,6-dicarboxylate (153 mg, 97% Purity) as a pale yellow oil. MS (ES⁺): 371 (M+H)⁺

### Step 4

A stirred solution of 6-benzyl 2-ethyl 3-formyl-1-methyl-1,4,5,7-tetrahydro-6H-pyrrolo[2,3-c]pyridine-2,6-dicarboxylate (150 mg, 97% Wt, 1 Eq, 393 µmol) in dioxane (4 mL) was treated hydrazine (56.3 mg, 55.0 µL, 35% Wt, 1.56 Eq, 614 µmol). The reaction mixture was stirred at 100 °C for 18 h. An aliquot (0.5 mL) of the reaction mixture was diluted into AcOH (2 mL) and stirred at 100 °C for 2 h. The remaining reaction mixture was diluted with AcOH (4 mL) and stirred at 100 °C for 2 h. The reaction mixture was concentrated *in vacuo* and the residue was azeotroped with toluene (3 times) to afford the crude product. The crude product was purified by chromatography on silica gel (12 g cartridge, 0-10% (0.7 M Ammonia/MeOH)/DCM, eluted ~4%) to afford benzyl 5-methyl-4-oxo-3,4,5,6,8,9-hexahydro-7H-pyrido[4',3':4,5]pyrrolo[2,3-d]pyridazine-7-carboxylate (82 mg, 95% Purity) as a pale yellow solid. MS (ES⁺): 339 (M+H)⁺

### Step 5

A suspension of benzyl 5-methyl-4-oxo-3,4,5,6,8,9-hexahydro-7H-pyrido[4',3':4,5]pyrrolo[2,3-d]pyridazine-7-carboxylate (74.0 mg, 95% Wt, 1 Eq, 208 µmol) and cesium carbonate (135 mg, 2.0 Eq, 416 µmol) in DMF(2 mL) was stirred at 80 °C for 2 h and then allowed to cool to RT. A solution of tert-butyl (3-(bromomethyl)phenyl)carbamate (62.4 mg, 1.05 Eq, 218 µmol) in DMF (1 mL) was added and the reaction mixture was stirred at RT for 18 h. The reaction mixture was concentrated *in vacuo* to afford the crude product. The crude product was purified by chromatography on silica gel to afford benzyl 3-(3-((tert-butoxycarbonyl)amino)benzyl)-5-methyl-4-oxo-3,4,5,6,8,9-hexahydro-7H-pyrido[4',3':4,5]pyrrolo[2,3-d]pyridazine-7-carboxylate (67 mg, 85% Purity) as a pale yellow solid. MS (ES⁺): 544 (M+H)⁺

### Step 6

A solution of benzyl 3-(3-((tert-butoxycarbonyl)amino)benzyl)-5-methyl-4-oxo-3,4,5,6,8,9-hexahydro-7H-pyrido[4',3':4,5]pyrrolo[2,3-d]pyridazine-7-carboxylate (67 mg, 85% Wt, 1 Eq, 0.10 mmol) in MeOH (5 mL) was added to palladium on carbon (10 wt% loading) (13 mg, 10% Wt, 0.12 Eq, 12 µmol). The suspension was stirred under H₂ (5 bar.) at RT for 1 h. The reaction mixture was filtered through a glass fibre filter paper, washing with MeOH, and the filtrate was concentrated *in vacuo* to afford the crude product. The crude product was purified by chromatography on silica gel to afford the tert-butyl (3-((5-methyl-4-oxo-4,5,6,7,8,9-hexahydro-3H-pyrido[4',3':4,5]pyrrolo[2,3-d]pyridazin-3-yl)methyl)phenyl)carbamate (Intermediate **2,** 35 mg, 94% Purity) as a white solid. ¹H NMR (400 MHz, DMSO-d6) δ 9.28 (s, 1H), 8.17 (s, 1H), 7.32 (d, J = 9.0 Hz, 2H), 7.16 (t, J = 7.8 Hz, 1H), 6.82 (d, J = 7.6 Hz, 1H), 5.23 (s, 2H), 3.94 (s, 3H), 3.87 (s, 2H), 2.93 (t, J = 5.6 Hz, 2H), 2.60 (t, J = 5.6 Hz, 2H), 1.44 (s, 9H). 1 proton not observed in DMSO. MS (ES⁺): 410 (M+H)⁺

### Intermediate 3 - 5-methyl-7-(phenylsulfonyl)-3,5,6,7,8,9-hexahydro-4H-pyrido[4',3':4,5]pyrrolo[2,3-d]pyridazin-4-one

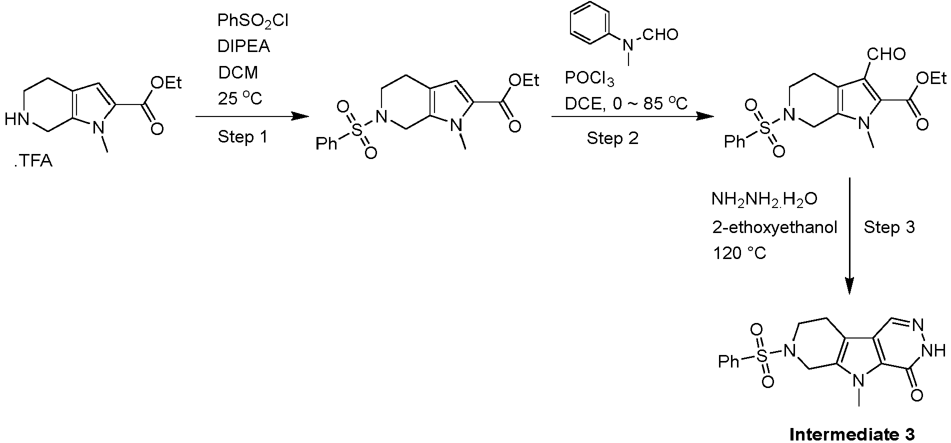

### Step 1

A stirred solution of ethyl 1-methyl-4,5,6,7-tetrahydro-1H-pyrrolo[2,3-c]pyridine-2-carboxylate 2,2,2-trifluoroacetate (prepared according to Steps 1-3 en route to Intermediate 1), 1.536 g, 78% Wt, 1 Eq, 3.718 mmol) and DIPEA (1.48 g, 2.00 mL, 3.09 Eq, 11.5 mmol) in DCM (40 mL) at 0 °C was treated with benzenesulfonyl chloride (692 mg, 500 µL, 1.05 Eq, 3.92 mmol) dropwise. The reaction mixture was allowed to warm to RT and stirred for 18 h. The reaction mixture was washed with sat. aq. NaHCO₃ (50 mL) and the organic layer was collected. The aqueous was extracted with DCM (2 x 40 mL) and the combined organic extracts were washed with 50% brine (50 mL), dried (phase separator) and concentrated *in vacuo* to afford the crude product as a yellow oil. The crude product was purified by chromatography on silica gel (dry load) (24 g cartridge, 0-100% EtOAc/isohexane) to afford ethyl 1-methyl-6-(phenylsulfonyl)-4,5,6,7-tetrahydro-1H-pyrrolo[2,3-c]pyridine-2-carboxylate (1.036 g, 76% Purity) as a clear colourless oil. MS (ES⁺): 349 (M+H)⁺

### Step 2

A stirred solution of N-methyl-N-phenylformamide (759 mg, 693 µL, 2.5 Eq, 5.62 mmol) in DCE (5 mL) at 0 °C was treated with POCl₃ (861 mg, 524 µL, 2.5 Eq, 5.62 mmol) in one portion. The reaction mixture was stirred at 0 °C for 0.5 h and then allowed to warm to RT for 1 h. The resultant orange solution was added to a stirred solution of ethyl 1-methyl-6-(phenylsulfonyl)-4,5,6,7-tetrahydro-1H-pyrrolo[2,3-c]pyridine-2-carboxylate (1.03 g, 76% Wt, 1 Eq, 2.25 mmol) in DCE (15 mL). The reaction mixture was heated to 80 °C for 44 h. The reaction mixture was washed with sat. aq. Na₂CO₃ (50 mL) and the organic layer was collected. The aqueous was extracted with DCM (2 x 40 mL) and the combined organic extracts were washed with sat. aq. Na₂CO₃ (50 mL), 50% brine (50 mL), dried (phase separator) and concentrated *in vacuo* to afford the crude products as a black oil. The crude product was purified by chromatography on silica gel to afford ethyl 3-formyl-1-methyl-6-(phenylsulfonyl)-4,5,6,7-tetrahydro-1H-pyrrolo[2,3-c]pyridine-2-carboxylate (438 mg, 91% Purity) as a yellow solid. MS (ES⁺): 377 (M+H)⁺

### Step 3

A suspension of ethyl 3-formyl-1-methyl-6-(phenylsulfonyl)-4,5,6,7-tetrahydro-1H-pyrrolo[2,3-c]pyridine-2-carboxylate (427 mg, 91% Wt, 1 Eq, 1.03 mmol) in 2-ethoxyethan-1-ol (5 mL) was treated with hydrazine (35 wt% in water) (101 mg, 100 µL, 35% Wt, 1.07 Eq, 1.10 mmol) in one portion. The reaction mixture was stirred at 130 °C for 2.5 h. Hydrazine (35 wt% in water) (10.1 mg, 10.0 µL, 35% Wt, 0.107 Eq, 110 µmol) was added and the reaction was stirred at 130 °C for 1 h. The reaction mixture was poured into water (~50 mL) and the precipitate was collected by filtration, washing with water, to afford 5-methyl-7-(phenylsulfonyl)-3,5,6,7,8,9-hexahydro-4H-pyrido[4',3':4,5]pyrrolo[2,3-d]pyridazin-4-one (Intermediate 3, 315 mg, 75% Purity) as a yellow solid. ¹H NMR (DMSO) δ: 12.29 (s, 1H), 8.07 (s, 1H), 7.92 - 7.85 (m, 2H), 7.74 - 7.65 (m, 1H), 7.68 - 7.57 (m, 2H), 4.38 (s, 2H), 3.98 (s, 3H), 3.39 (t, J = 5.7, 5.7 Hz, 2H), 2.70 (t, J = 5.7, 5.7 Hz, 2H). MS (ES⁺): 345 (M+H)⁺.

### Intermediate 4 - methyl 3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)thiophene-2-carboxylate

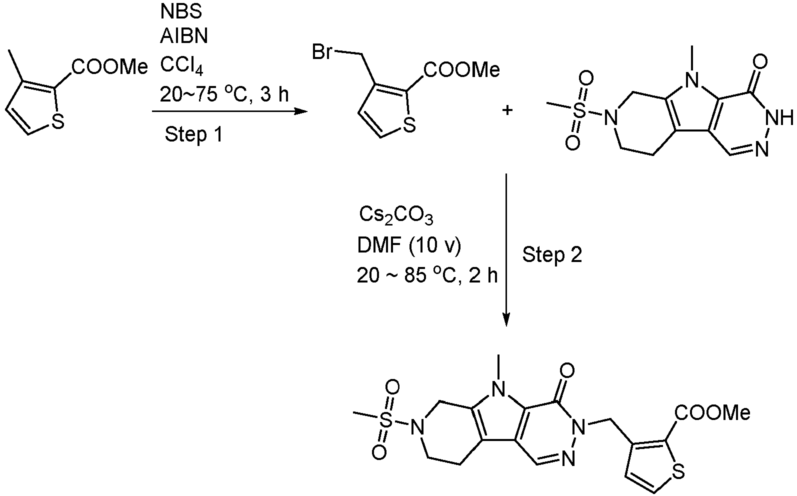

### Step 1

To a solution of methyl 3-methylthiophene-2-carboxylate (1.00 g, 6.40 mmol, 1.0 Eq) in CCl₄ (6.00 mL) was added AIBN (210 mg, 1.28 mmol, 0.2 Eq) in one portion at 20-25 °C. To the mixture was added NBS (1.48 g, 8.32 mmol, 1.3 Eq) at 70-75 °C in portions. Then the mixture was stirred at 70-75 °C for 3 h. TLC (n-heptane/ethyl acetate = 10/1, R_{f}-_{SM} = 0.4, R_{f}-_{product} = 0.45) showed the starting material was consumed completely and two new spots were detected. The mixture was cooled to 20-25 °C and filtered. The filter cake was washed with CCl₄ (2 x 5.0 mL). The filtrate was diluted with ethyl acetate (10.0 mL) and washed with water (10.0 mL). The organic layer was dried over Na₂SO₄ and concentrated at 40 °C *in vacuo* to give methyl 3-(bromomethyl)thiophene-2-carboxylate (2.00 g, crude) as a yellow oil.

The material was used in the next step without further purification

### Step 2

To a suspension of 5-methyl-7-(methylsulfonyl)-3,5,6,7,8,9-hexahydro-4H-pyrido[4',3':4,5]pyrrolo[2,3-d] pyridazin-4-one **(Intermediate 1,** 350 mg, 1.24 mmol, 1.0 Eq) in DMF (2.5 mL) was added Cs₂CO₃ (969 mg, 2.98 mmol, 2.4 Eq) in one portion at 20-25 °C. The suspension was stirred at 80-85 °C for 1 h. The suspension was then cooled to 20-25 °C. A solution of methyl 3-(bromomethyl)thiophene-2-carboxylate (874 mg, 3.72 mmol, 3.0 Eq) in DMF (1.0 mL) was added drop-wise into the suspension at 20-25 °C. And the mixture was stirred at 20-25 °C for 1 h. TLC (dichloromethane/methanol = 3/1, R_{f}-_{SM} = 0.3, R_{f}-_{product} = 0.5) showed the starting material was consumed completely and three new spots were detected. The reaction mixture was poured into ice water (25.0 mL) and stirred 30 min at 0-5 °C. The mixture was then filtered and the filter cake was washed with water (5.0 mL x 2). The filter cake was dried under vacuum at 45 °C to give the title compound (310 mg) as a white solid.

¹H NMR 400 MHz DMSO-*d₆*: δ 8.26 (s, 1H), 7.75 (d, *J* = 4.8 Hz, 1H), 6.59 (d, *J* = 5.2 Hz, 1H), 5.63 (s, 2H), 4.49 (s, 2H), 4.01 (s, 3H), 3.86 (s, 3H), 3.51 (t, *J =* 5.2 Hz, 2H), 3.01 (s, 3H), 2.83 (t, *J* = 5.2 Hz, 2H).

### Intermediate 5 - 3-(chloromethyl)benzofuran

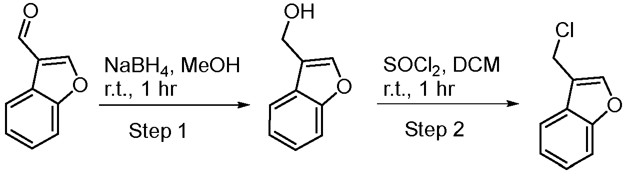

### Step 1

To a solution of benzofuran-3-carbaldehyde (150 mg, 1.03 mmol) in MeOH (5 mL) was added NaBH₄ (57 mg, 1.50 mmol) at room temperature, and the reaction mixture under nitrogen protection was stirred at room temperature for 1 hour. After LCMS indicated the reaction is completed, the reaction mixture was quenched with saturated ammonium chloride solution, concentrated to remove MeOH and extracted with DCM (10 mL×2). The combined organic layer was washed with brine, dried over Na₂SO₄, filtered, and concentrated at 40 °C under reduced pressure to give benzofuran-3-ylmethanol (160 mg, 100%) as a white solid. The crude was used in next step directly.

¹H NMR (400 MHz, CDCl₃) δ: 7.70-7.67 (m, 1H), 7.63 (s, 1H), 7.51-7.48 (m, 1H), 7.35-7.28 (m, 2H), 4.86 (s, 2H).
MS (ES⁺): 131.4 (M-OH)⁺

### Step 2

To a solution of benzofuran-3-ylmethanol (160 mg, 1.08 mmol) in DCM (5 mL) was added SOCl₂ (0.70 mL, 10 mmol) at room temperature, and the reaction mixture was stirred at room temperature for 1 hour. After LCMS indicated the reaction was complete, the reaction mixture was concentrated under vacuum to give 3-(chloromethyl)benzofuran as a yellow oil (assumed quantitative yield, 1.08mmol). The material was used crude in the next step without further purification.

### Intermediate 6 ethyl 3-(chloromethyl)-1-(tetrahydro-2H-pyran-2-yl)-1H-pyrazole-4 - carboxylate

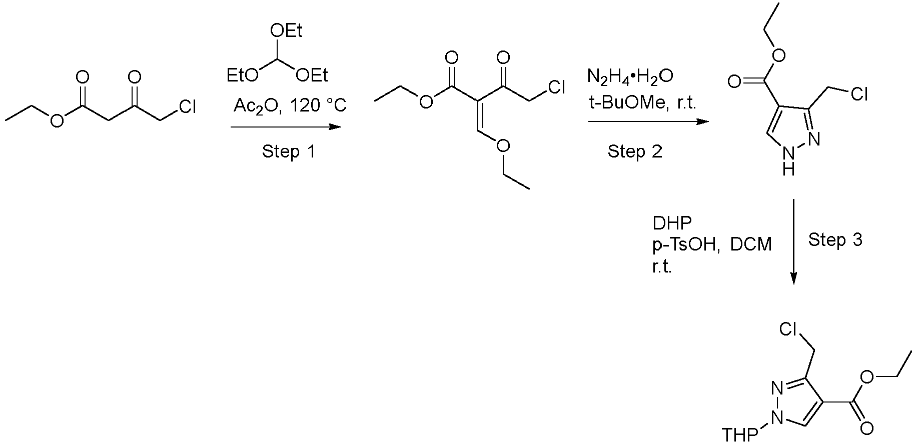

### Step 1

A solution of ethyl 4-chloro-3-oxobutanoate (4 mL, 29.5 mmol), triethoxymethane (29.5 mL) and A_{c2}O (13mL) was stirred at 120 °C for 3 h. The reaction mixture was concentrated, and n-heptane was added to the residue. A solid precipitate was obtained that upon filtration afforded ethyl-4-chloro-2-(ethoxymethylene) -3-oxobutanoate (1.5 g) as a yellow solid, which was used into next step without purification.
MS (ES⁺): 221.4 (M+H)⁺

### Step 2

To a solution of ethyl-4-chloro-2-(ethoxymethylene)-3-oxobutanoate (1.5g, 6.8mmol) in t-BuOMe (10 mL) was added N₂H₄ (3 mL). The reaction mixture was stirred at room temperature for 30 min. H2O (20mL) was added and extracted with EtOAc (20 mL x 3). The combined organic layers were washed with brine, dried over Na₂SO₄, and concentrated to give the crude product, which was purified by silica gel (20g, petroleum ether/ethyl acetate = 100:00/50:50) to obtain ethyl 3-(chloromethyl)-lH-pyrazole -4-carboxylate (300 mg, 98% Purity) as a yellow solid.
MS (ES⁺): 189.3 (M+H)⁺

### Step 3

A solution of ethyl 3-(chloromethyl)-lH-pyrazole-4-carboxylate (SCP3-6s-12, 300 mg, 1.60 mmol), DHP (269 mg, 3.20 mmol), p-TsOH (28 mg, 0.16 mmol) in DCM (10mL). Then mixture was stirred at room temperature for 4 hours. The mixture was diluted with saturated NaHCO3, extracted with DCM, and washed with brine (10mL). The organic layer was dried (MgSO4), filtered and concentrated at 40 °C under reduced pressure. The residue was purified by flash column chromatography (12g, petroleum ether/ethyl acetate = 100:00/50:50) to give the title compound (270mg, 99% Purity) as a yellow oil.
MS (ES⁺): 189.3 (M-84)⁺

### Intermediate 7 - 3-(chloromethyl)benzenesulfonamide

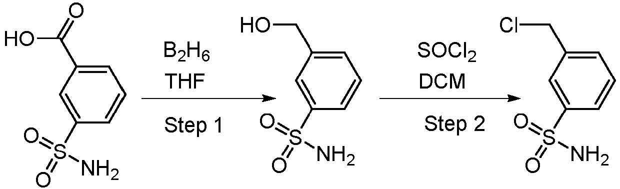

### Step 1

To the solution of 3-sulfamoylbenzoic acid (200 mg, 1.0 mmol) in Tetrahydrofuran (5mL) was add B₂H₆ (2 mL, 2.0 mmol, 1M in the Tetrahydrofuran) at 0 °C, and the reaction mixture was stirred at room temperature for 2 hours. After LCMS indicated the reaction is completed, the mixture was added MeOH (5 mL). The organic layer was concentrated at 30 °C under reduced pressure to give 3-(hydroxymethyl)benzenesulfonamide (140 mg, 95% Purity) as a yellow oil.
MS (ES⁺): 188.1 (M+H)⁺

### Step 2

To the solution of 3-(hydroxymethyl)benzenesulfonamide (140 mg, 0.75 mmol) in DCM (5 mL) was drop-wised SOCl₂ (214 mg, 1.8 mmol) at 0 °C, and the reaction mixture was stirred at room temperature for 3 hours. After LCMS indicated the reaction is completed, the mixture was concentrated at 30 °C under reduced pressure to remove DCM and most of over amounted SOCl₂ to give the title compound (120 mg, 97% Purity) as a yellow oil, which was used to the next step directly.
MS (ES⁺): 206.0 (M+H)⁺

### Intermediate 8- 2-(chloromethyl)-6-(pyrrolidin-1-yl)pyridine

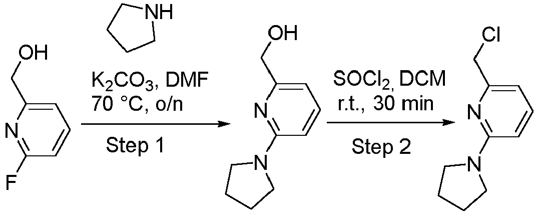

### Step 1

A mixture of (6-fluoropyridin-2-yl)methanol (400 mg, 3.15 mmol), pyrrolidine (448 mg, 6.30 mmol) and K₂CO₃ (652 mg, 4.47 mmol) in DMF (10 mL) under nitrogen protection was stirred at 70 °C overnight. After LCMS indicated the reaction is completed, the reaction mixture was diluted with H₂O (10 mL) and extracted with EtOAc (20 mL×3). The combined organic layer was washed with saturated ammonium chloride solution, concentrated at 40 °C under reduced pressure and purified by flash column chromatography (40 g, petroleum ether/methyl ether = 100:00~60:40) to give (6-(pyrrolidin-1-yl)pyridin-2-yl)methanol (250 mg, 99% Purity) as a colourless oil.
MS (ES⁺): 139.4 (M+H)⁺

### Step 2

To a solution of (6-(pyrrolidin-1-yl)pyridin-2-yl)methanol (90 mg, 0.50 mmol) in DCM (2 mL) was added SOCl₂ (0.36 mL, 5 mmol) at room temperature, and the reaction mixture was stirred at room temperature for 30 minutes. After LCMS indicated the reaction is completed, the reaction mixture was concentrated. The mixture was diluted with H₂O (2 mL), basified with K₂CO₃ until pH = 7 and then extracted with EtOAc (10 mL×3). The combined organic layer was washed with brine, dried over Na₂SO₄, filtered, and concentrated at 40 °C under reduced pressure to the title compound (95 mg, 97% Purity) as a yellow oil. The crude was used in next step directly without further purification.
MS (ES⁺): 197.3 (M+H)⁺

### Intermediate 9 - tert-butyl 3-(bromomethyl)-1H-indazole-1-carboxylate

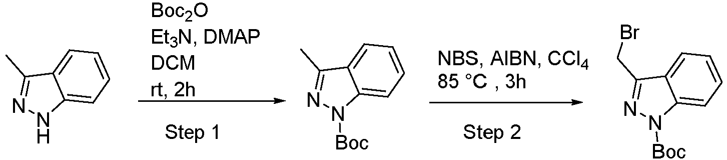

### Step 1

To a solution of 3-methyl-1H-indazole (700 mg, 5.3 mmol) and di-tert-butyl dicarbonate (1.7 g, 7.95 mmol) in DCM (10 mL) was added DMAP (112 mg, 1 mmol) at room temperature, and the reaction mixture was stirred at room temperature for 2 hours. After LCMS indicated the reaction is completed, the reaction mixture was concentrated at 40 °C under reduced pressure and extracted with EtOAc (20 mL×3). The combined organic layer was washed by HCl aq. (0.5M), dried over Na₂SO₄, filtered and concentrated at 40 °C under reduced pressure. The residue was purified by flash column chromatography (12 g, petroleum ether/ethyl acetate = 100:0-70:30) to give tert-butyl 3-methyl-1H-indazole-1-carboxylate (1.1 g, 87% Purity) as a yellow oil.
MS (ES⁺): 177.3 (M-56+H)⁺

### Step 2

To a solution of tert-butyl 3-methyl-1H-indazole-1-carboxylate (mg, 1.3 mmol), NBS (278 mg, 1.6 mmol) in CCl₄ (6 mL) was added AIBN (43 mg, 0.26 mmol) at room temperature, and the reaction mixture was stirred at 85 °C for 3 hours. After LCMS indicated the reaction was complete, the reaction mixture was concentrated at 45 °C under reduced pressure and extracted with EtOAc (10 mL×3). The combined organic layer was washed by brine, dried over Na₂SO₄, filtered, and concentrated at 40 °C under reduced pressure. The residue was purified by flash column chromatography (12 g, petroleum ether/ethyl acetate = 100:0-70:30) to give the title compound (200 mg, 99% Purity) as a yellow solid.
MS (ES⁺): 255.2 (M+H)⁺

### Intermediate 10 - tert-butyl 4-(chloromethyl)-1H-indazole-1-carboxylate

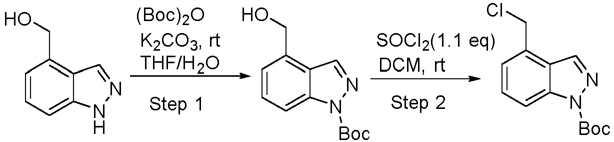

### Step 1

A mixture of (1*H*-indazol-4-yl)methanol (750 mg, 5.06 mmol), di-tert-butyl dicarbonate (2.2 g, 10.0 mmol) and K₂CO₃ (2.07 g, 15 mmol) in THF (20 mL) and H₂O (6 mL) under nitrogen protection was stirred at room temperature overnight. After LCMS indicated the reaction is completed, the reaction mixture was diluted with H₂O (10 mL) and extracted with EtOAc (30 mL×3). The combined organic layer was washed with saturated ammonium chloride solution, concentrated at 40 °C under reduced pressure and purified by flash column chromatography (40 g, petroleum ether/ethyl acetate = 100:00-60:40) to give *tert-butyl* 4-(hydroxymethyl)-1H-indazole-1-carboxylate (600 mg, 66% Purity) as a colourless oil.
MS (ES⁺): 193.3 (M+H)⁺

### Step 2

To a solution of tert-butyl 4-(hydroxymethyl)-1H-indazole-1-carboxylate (350 mg, 1.41 mmol) in DCM (7 mL) was added SOCl₂ (185 mg, 1.55 mmol) at room temperature, and the reaction mixture was stirred at room temperature for 1 hour. After LCMS indicated the reaction is completed, the reaction mixture was concentrated. The mixture was diluted with H₂O (5 mL), alkalized with K₂CO₃ until pH = 7 and then extracted with EtOAc (20 mL×3). The combined organic layer was washed with brine, dried over Na₂SO₄, filtered and concentrated at 40 °C under reduced pressure to give the crude. The crude was purified by flash column chromatography (20 g, petroleum ether/ethyl acetate = 100:00-80:20) to give the title compound (180 mg, 99% Purity) as a light-yellow oil.
MS (ES⁺): 211.4 (M+H)⁺

### Intermediate 11 - 7-((4-methoxyphenyl)sulfonyl)-5-methyl-3,5,6,7,8,9-hexahydro-4H-pyrido[4',3':4,5]pyrrolo[2,3-d]pyridazin-4-one

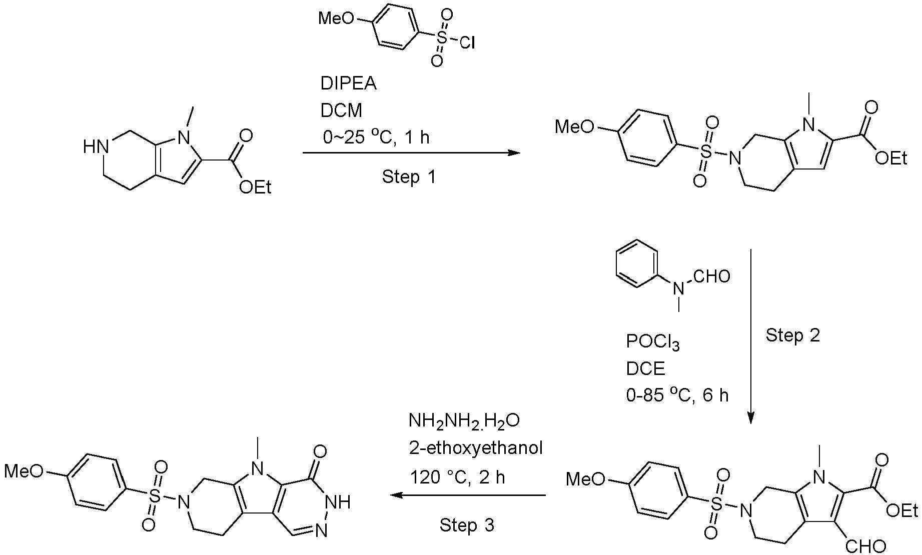

### Step 1

To a solution of compound ethyl 1-methyl-4,5,6,7-tetrahydro-1H-pyrrolo[2,3-c]pyridine-2-carboxylate **(Commercially available** 5.0 g, 24.0 mmol, 1.0 Eq) in DCM (30 mL) was added drop-wise DIPEA (12.4 g, 96.0 mmol, 4.0 Eq) at 0-5 °C. Then 4-methoxybenzenesulfonyl chloride (5.46 g, 26.4 mmol, 1.1 Eq) was added drop-wise into the reaction mixture at 0-5 °C. The reaction mixture was warmed to 20 - 25 °C and stirred for 1 hour. LCMS analysis showed the starting material was consumed completely and two major peaks with the desired mass ion were detected. The reaction mixture was poured into the ice-cold aq. NaHCO₃ (40 mL) and the layers were separated. The aqueous layer was extracted with ethyl acetate (30 mL x 3). The organic layers were combined and dried over Na₂SO₄ and concentrated at 45 °C *in vacuo* to give compound ethyl 6-((4-methoxyphenyl)sulfonyl)-1-methyl-4,5,6,7-tetrahydro-1H-pyrrolo[2,3-c]pyridine-2-carboxylate (9.1 g) which was used for next step directly.
MS (ES⁺): 379.2 (M+H)⁺

### Step 2

To a solution of ethyl 6-((4-methoxyphenyl)sulfonyl)-1-methyl-4,5,6,7-tetrahydro-1H-pyrrolo[2,3-c]pyridine-2-carboxylate (8.13 g, 60.1 mmol, 2.5 Eq) in DCE (45 mL) was added POCl₃ (29.5 g, 192 mmol, 8.0 Eq) drop-wise at 0 °C. Then the reaction mixture was warmed to 20-25 °C and stirred for 12 hours. A solution of N-methyl-N-phenylformamide (9.1 g, 24.0 mmol, 1.0 Eq) in DCE (9.0 mL) was added drop-wise to the reaction mixture. The mixture was heated to 80-85 °C and stirred for 1 hour. LCMS analysis showed the starting material was consumed completely and one major product with desired mass ion was detected. The reaction mixture was then cooled to 20-25 °C and concentrated at 45 °C to give a crude residue. The residue was diluted with DCM (50 mL) and the pH adjusted to 8 with sat. NaHCO₃. The layers were separated, and the aqueous phase was extracted with DCM (50 mL x 2). The organic layers were then combined, dried over Na₂SO₄ and concentrated at 45 °C to give a crude product. The crude product was purified by column chromatography on silica gel (n-heptane/ ethyl acetate = 10/1 to DCM/ MeOH =10/1, Rf = 0.4) to give ethyl 3-formyl-6-((4-methoxyphenyl)sulfonyl)-1-methyl-4,5,6,7-tetrahydro-1H-pyrrolo[2,3-c]pyridine-2-carboxylate (8.0 g, 96% Purity) as a yellow solid.

¹H NMR (400 MHz, DMSO-d6) δ: δ 12.24 (s, 1H), 7.78 (d, *J =* 8.8 Hz, 2H), 7.11 (d, *J =* 9.2 Hz, 2H), 4.31 (q, *J =* 7.2 Hz, 2H), 4.21 (s, 2H), 3.83 (s, 3H), 3.74 (s, 3H), 3.23 (t, *J =* 6.0 Hz, 2H), 2.71 (t, *J = 5.6* Hz, 2H), 1.30 (t, *J* = 7.2 Hz, 3H).
MS (ES⁺): 407.1 (M+H)⁺, 429.2 (M+Na)⁺

### Step 3

To a suspension of compound ethyl 3-formyl-6-((4-methoxyphenyl)sulfonyl)-1-methyl-4,5,6,7-tetrahydro-1H-pyrrolo[2,3-c]pyridine-2-carboxylate (4.0 g, 9.84 mmol, 1.0 Eq) in 2-ethoxyethanol (40 mL) was added NH₂NH₂.H₂O (831 mg, 16.6 mmol, 1.7 Eq) drop-wise at 20-25 °C. Then the mixture was stirred at 115-120 °C for 2 hours. LCMS analysis showed the starting material was consumed completely and one major peak with desired mass ion was detected. The reaction mixture was cooled to RT and then the reaction mixture was poured onto ice water (50 mL) with stirring, forming a thick slurry. The reaction mixture was filtered, and the filter cake was washed with water (20 mL x 2). The filter cake was dried under vacuum at 45 °C to give the desired product (3.0 g, 90% Purity) as a yellow solid.

¹H NMR (400 MHz, DMSO-d6) δ: δ 12.29 (s, 1H), 8.08 (s, 1H), 7.80 (d, *J =* 4.8 Hz, 2H), 7.15 (d, *J* = 4.8 Hz, 2H), 4.32 (s, 2H), 3.97 (s, 3H), 3.82 (s, 3H), 3.30 (t, *J = 5.6* Hz, 2H), 2.71 (t, *J = 5.6* Hz, 2H).
MS (ES⁺): 375.1 (M+H)⁺

### Intermediate 12 - methyl 3-(bromomethyl)thiophene-2-carboxylate

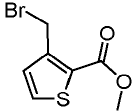

A solution of methyl 3-methylthiophene-2-carboxylate (300 mg, 3.20 mmol), NBS (576 mg, 3.20 mmol), AlBN (53 mg, 0.32 mmol) in CCl₄ (10mL). Then mixture was stirred at 75 °C for 16 hours. The mixture was diluted with saturated NaHCO₃, extracted with DCM, and washed with brine (10mL). The organic layer was dried (MgSO₄), filtered and concentrated at 40 °C under reduced pressure. The residue was purified by flash column chromatography (12g, petroleum ether/ethyl acetate = 100:00/50:50) to give the title compound (200 mg, 64% Purity) as a yellow solid.
MS (ES⁺): 235.0 (M+H)⁺

### Intermediate 13 - 5-(chloromethyl)-2,3-dihydrobenzofuran

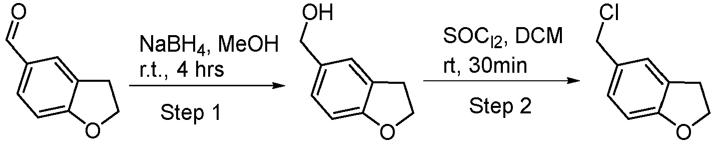

### Step 1

To a solution of 2,3-dihydrobenzofuran-5-carbaldehyde (200 mg, 1.35 mmol) in MeOH (6 mL) was added NaBH₄ (77 mg, 2.02 mmol) at room temperature, and the reaction mixture under nitrogen protection was stirred at room temperature for 4 hours. After LCMS indicated the reaction is completed, the reaction mixture was quenched with saturated ammonium chloride solution, concentrated to remove MeOH and extracted with DCM (10 mL×3). The combined organic layer was washed with brine, dried over Na₂SO₄, filtered and concentrated at 40 °C under reduced pressure to give (2,3-dihydrobenzofuran-5-yl)methanol (210 mg, 95% Purity) as a colourless oil. The crude was used in next step directly.
MS (ES⁺): 133.3 (M-OH)⁺

### Step 2

To a solution of (2,3-dihydrobenzofuran-5-yl)methanol (210 mg, 1.40 mmol) in DCM (5 mL) was added SOCl₂ (0.5 mL, 6.9 mmol) at room temperature, and the reaction mixture was stirred at room temperature for 30 minutes. After LCMS indicated the reaction was complete, the reaction mixture was concentrated in vacuum to give a colourless oil. Assumed quantitative yield obtained and used crude in the next step without purification.

### Intermediate 14 - 5-(chloromethyl)-2,2-dimethyl-2,3-dihydrobenzofuran

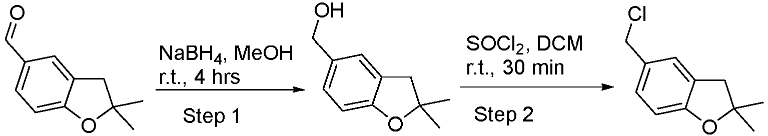

### Step 1

To a solution of 2,2-dimethyl-2,3-dihydrobenzofuran-5-carbaldehyde (100 mg, 0.57 mmol) in MeOH (3 mL) was added NaBH₄ (32 mg, 0.85 mmol) at room temperature, and the reaction mixture under nitrogen protection was stirred at room temperature for 4 hours. After LCMS indicated the mixture is no more reaction, the reaction mixture was quenched with saturated ammonium chloride solution, concentrated to remove MeOH and extracted with DCM (10 mL×2). The combined organic layer was washed with brine, dried over Na₂SO₄, filtered and concentrated at 40 °C under reduced pressure to give the mixture of (2,2-dimethyl-2,3-dihydrobenzofuran-5-yl)methanol (105 mg, 20% Purity) as a white solid. The crude was used in next step directly without further purification.
MS (ES⁺): 161.4 (M-OH)⁺

### Step 2

To a solution of crude 2,2-dimethyl-2,3-dihydrobenzofuran-5-yl)methanol (105 mg) in DCM (5 mL) was added SOCl₂ (0.4 mL, 5.5 mmol) at room temperature, and the reaction mixture was stirred at room temperature for 30 minutes. After LCMS indicated the reaction was complete, the reaction mixture was concentrated in vacuum to give a yellow oil. Assumed quantitative yield obtained and used crude in the next step without purification.

### Intermediate 15 - 6-(chloromethyl)-1-((2-(trimethylsilyl)ethoxy)methyl)-1H-pyrrolo[2,3-b]pyridine

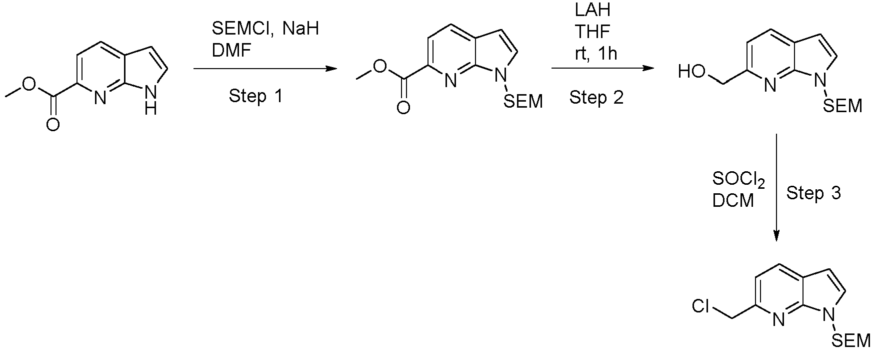

### Step 1

To a solution of methyl 1H-pyrrolo[2,3-b]pyridine-6-carboxylate (400 mg, 2.3 mmol) in DMF (10 mL) was added NaH (110 mg, 2.8 mmol) at 0 °C, and the reaction mixture was stirred at 0 °C for 0.5 hours. Then the reaction mixture was added SEM-Cl (468 mg, 2.8mmol), the reaction mixture was stirred at room temperature for 1 hours. After LCMS indicated the reaction is completed, the reaction mixture was quenched with NH₄Cl aq. and extracted with EtOAc (20 mL×3). The combined organic layer was washed by brine, dried over Na₂SO₄, filtered, and concentrated at 45 °C under reduced pressure. The residue was purified by flash column chromatography (12 g, petroleum ether/ethyl acetate = 100:0-70:30) to give methyl 1-((2-(trimethylsilyl)ethoxy)methyl)-1H-pyrrolo[2,3-b]pyridine-6-carboxylate (470 mg, 87% Purity) as a yellow solid.
MS (ES⁺): 307.3 (M+H)⁺

### Step 2

To a solution of 1-((2-(trimethylsilyl)ethoxy)methyl)-1H-pyrrolo[2,3-b]pyridine-6-carboxylate (470 mg, 1.5 mmol) in THF (10 mL) was added LAH (0.9 mL, 2.25 mmol, 2.5 M) at room temperature, and the reaction mixture was stirred at room temperature for 1 hours. After LCMS indicated the reaction is completed, the reaction mixture was quenched with Na₂SO₄.10H₂O, filtered and concentrated at 45 °C under reduced pressure. The residue was purified by flash column chromatography (12 g, petroleum ether/ethyl acetate = 100:0-30:70) to give (1-((2-(trimethylsilyl)ethoxy)methyl)-1H-pyrrolo[2,3-b]pyridin-6-yl)methanol (420 mg, 99% Purity) as a yellow solid.
MS (ES⁺): 279.3 (M+H)⁺

### Step 3

To a solution of (1-((2-(trimethylsilyl)ethoxy)methyl)-1H-pyrrolo[2,3-b]pyridin-6-yl)methanol (420 mg, 1.5 mmol) in DCM (6 mL) was added SOCl₂ (238 mg, 2 mmol) at room temperature, and the reaction mixture was stirred at room temperature for 1 hour. After LCMS indicated the reaction is completed, the mixture was concentrated at 30 °C under reduced pressure to remove DCM and most of over amounted SOCl₂. The residue was quenched with ice water (10 mL), adjusted to pH = 9 with 2N K₂CO₃ aqueous solution, and extracted with DCM (10 mL×2). The organic layer was washed with brine, dried over Na₂SO₄, filtered, and concentrated at 30 °C under reduced pressure to give the title compound (400 mg, 95% Purity) as a yellow solid, which was used to the next step directly.
MS (ES⁺): 297.2 (M+H)⁺

### Intermediate 16 - (S)-tert-butyl 1-(3-(chloromethyl)phenyl)ethylcarbamate

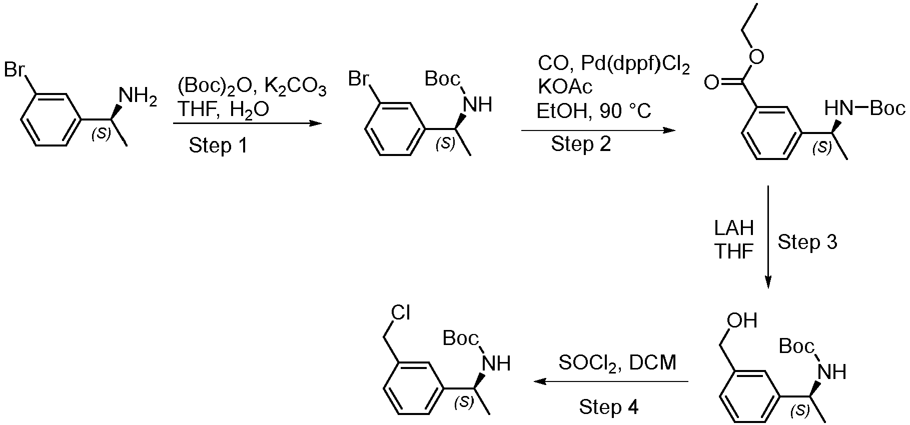

### Step 1

To a solution of (S)-1-(3-bromophenyl)ethanamine (800 mg, 4 mmol) and (Boc)₂O (1.05 mg, 4.8 mmol) in THF (10 mL) was added K₂CO₃ (1.1 g, 8 mmol) at room temperature, and the reaction mixture was stirred at room temperature for 2 hours. After LCMS indicated the reaction is completed, the reaction mixture was extracted with EtOAc (20 mL×3). The combined organic layer was washed by brine, dried over Na₂SO₄, filtered and concentrated at 45 °C under reduced pressure. The residue was purified by flash column chromatography (12 g, petroleum ether/ethyl acetate = 100:0-80:20) to give (S)-tert-butyl 1-(3-bromophenyl)ethylcarbamate (1.28 g, 99% Purity) as a yellow oil.
MS (ES⁺): 244.2 (M+H)⁺

### Step 2

To a solution of (S)-tert-butyl 1-(3-bromophenyl)ethylcarbamate (0.6 g, 2 mmol) and Pd(dppf)Cl₂ (145 mg, 0.2 mmol) in EtOH (10 mL) was added KOAc (392 mL, 4 mmol) at room temperature, and the reaction mixture was stirred at 90 °C for 4 hours under carbon monoxide atmosphere. After LCMS indicated the reaction is completed, the reaction mixture was concentrated at 45 °C under reduced pressure and extracted with EtOAc (20 mL×3). The combined organic layer was washed by brine, dried over Na₂SO₄, filtered and concentrated at 45 °C under reduced pressure. The residue was purified by flash column chromatography (12 g, petroleum ether/ethyl acetate = 100:0-70:30) to give (S)-ethyl 3-(1-(tert-butoxycarbonylamino)ethyl)benzoate (390 mg, 76% Purity) as a yellow oil.
MS (ES⁺): 316.3 (M+Na)⁺

### Step 3

To a solution of (S)-ethyl 3-(1-(tert-butoxycarbonylamino)ethyl)benzoate (390 mg, 1.3 mmol) in THF (10 mL) was added LAH (0.9 mL, 2.25 mmol, 2.5 M) at room temperature, and the reaction mixture was stirred at room temperature for 1 hours. After LCMS indicated the reaction is completed, the reaction mixture was quenched with Na₂SO₄10H₂O, filtered and concentrated at 45 °C under reduced pressure. The residue was purified by flash column chromatography (12 g, dichloromethane/methyl alcohol = 100:0-90:10) to give (S)-tert-butyl 1-(3-(hydroxymethyl)phenyl)ethylcarbamate (310 mg, 90% Purity) as a yellow solid.
MS (ES⁺): 274.4 (M+Na)⁺

### Step 4

To a solution of (S)-tert-butyl 1-(3-(hydroxymethyl)phenyl)ethylcarbamate (310 mg, 1.2 mmol) in DCM (6 mL) was added SOCl₂ (238 mg, 2 mmol) at room temperature, and the reaction mixture was stirred at room temperature for 1 hour. After LCMS indicated the reaction is completed, the mixture was concentrated at 30 °C under reduced pressure to remove DCM and most of over amounted SOCl₂. The residue was quenched with ice water (10 mL), adjusted to pH = 9 with 2N K₂CO₃ aqueous solution, and extracted with DCM (10 mL×2). The organic layer was washed with brine, dried over Na₂SO₄, filtered, and concentrated at 30 °C under reduced pressure to give (S)-tert-butyl 1-(3-(chloromethyl)phenyl)ethylcarbamate (300 mg, 58% Purity) as a yellow solid, which was used to the next step directly.
MS (ES⁺): 292.2 (M+Na)⁺

### Intermediate 17 - 2-(3-(bromomethyl)phenyl)propan-2-ol

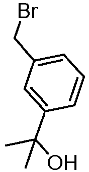

To a solution of methyl 3-(bromomethyl)benzoate (1.0 g, 4.37 mmol) in ether (20 mL), at 0 °C was added dropwise methylmagnesium chloride (3 M in ether, 4.4 mL, 13.11 mmol). The reaction mixture was warmed up to 45 °C, and stirred for 2 hours and then quenched with ammonium chloride aqueous solution, extracted with EtOAc (20 mL × 3) and concentrated to give the residue. The residue was purified by flash column chromatography (20 g, petroleum ether/ ethyl acetate = 100:0-80:20) to give a yellow oil (assumed quantitative yield). Used in the next step without further purification.

### Intermediate 18 - 3-(chloromethyl)thieno[3,2-c]pyridine

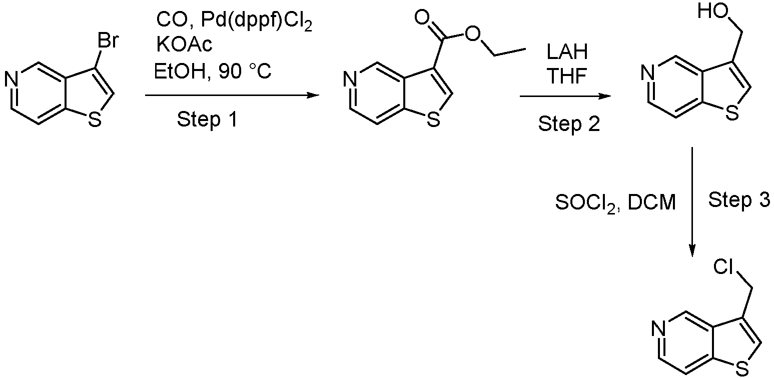

### Step 1

To a solution of 3-bromothieno[3,2-c]pyridine (1 g, 4.7 mmol) and Pd(dppf)Cl₂ (345 mg, 0.47 mmol) in EtOH (15 mL) was added KOAc (921 mg, 9.4 mmol) at room temperature, and the reaction mixture was stirred at 90 °C for 4 hours under carbon monoxide atmosphere. After LCMS indicated the reaction is completed, the reaction mixture was concentrated at 45 °C under reduced pressure and extracted with EtOAc (20 mL×3). The combined organic layer was washed by brine, dried over Na₂SO₄, filtered and concentrated at 45 °C under reduced pressure. The residue was purified by flash column chromatography (12 g, petroleum ether/ethyl acetate = 100:0-70:30) to give ethyl thieno[3,2-c]pyridine-3-carboxylate (700 mg, 75% Purity) as a yellow oil.
MS (ES⁺): 208.2 (M+H)⁺

### Step 2

To a solution of ethyl thieno[3,2-c]pyridine-3-carboxylate (700 mg, 3.4 mmol) in THF (10 mL) was added LAH (2 mL, 5.1 mmol, 2.5 M) at room temperature, and the reaction mixture was stirred at room temperature for 1 hours. After LCMS indicated the reaction is completed, the reaction mixture was quenched with Na₂SO₄10H₂O, filtered and concentrated at 45 °C under reduced pressure. The residue was purified by flash column chromatography (12 g, dichloromethane/methyl alcohol = 100:0-90:10) to give thieno[3,2-c]pyridin-3-ylmethanol (480 mg, 75% Purity) as a yellow solid.
MS (ES⁺): 166.1 (M+H)⁺.

### Step 3

To a solution of (S)-tert-butyl 1-(3-(hydroxymethyl)phenyl)ethylcarbamate (100 mg, 0.6 mmol) in DCM (6 mL) was added SOCl₂ (238 mg, 2 mmol) at room temperature, and the reaction mixture was stirred at room temperature for 1 hour. After LCMS indicated the reaction is completed, the mixture was concentrated at 30 °C under reduced pressure to remove DCM and most of over amounted SOCl₂. The residue was quenched with ice water (10 mL), adjusted to pH = 9 with 2N K₂CO₃ aqueous solution, and extracted with DCM (10 mL×2). The organic layer was washed with brine, dried over Na₂SO₄, filtered and concentrated at 30 °C under reduced pressure to give (110 mg, 83% Purity) as a yellow solid, which was used to the next step directly.
MS (ES⁺): 184.1 (M+H)⁺.

### Intermediate 19 - tert-butyl 3-(chloromethyl)-1H-pyrrolo[2,3-c]pyridine-1-carboxylate

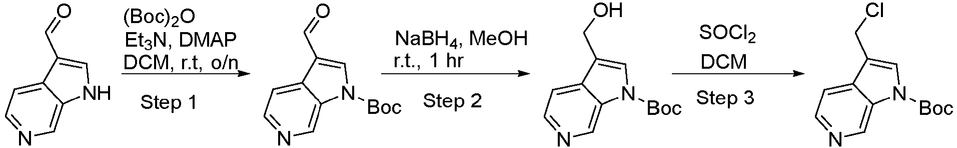

### Step 1

A mixture of 1H-pyrrolo[2,3-c]pyridine-3-carbaldehyde (510 mg, 3.49 mmol), di-tert-butyl dicarbonate (1.52 g, 7.0 mmol), Et₃N (1.5 mL, 10.8 mmol) and DMAP (43 mg, 0.35 mmol) in DCM (10 mL) under nitrogen protection was stirred at room temperature overnight. After LCMS indicated the reaction is completed, the reaction mixture was diluted with H₂O (20 mL) and extracted with DCM (30 mL × 3). The combined organic layer was washed with saturated salt water, dried over Na₂SO₄, filtered and concentrated at 40 °C under reduced pressure and purified by flash column chromatography (20 g, petroleum ether/ethyl acetate = 100:00-65:35) to give *tert* -butyl 3-formyl-1H-pyrrolo[2,3-c]pyridine-1-carboxylate (600 mg, 99% Purity) as a yellow oil. MS (ES⁺): 247.3 (M+H)⁺.

### Step 2

To a solution of *tert-butyl* 3-formyl-1H-pyrrolo[2,3-c]pyridine-1-carboxylate (300 mg, 1.22 mmol) in MeOH (5 mL) was added NaBH₄ (93 mg, 2.45 mmol) at room temperature, and the reaction mixture under nitrogen protection was stirred at room temperature for 1 hour. After LCMS indicated the reaction is completed, the reaction mixture was quenched with saturated ammonium chloride solution, concentrated to remove MeOH and extracted with DCM (10 mL×3). The combined organic layer was washed with brine, dried over Na₂SO₄, filtered and concentrated at 40 °C under reduced pressure to give *tert-butyl* 3-(hydroxymethyl)-1H-pyrrolo[2,3-c]pyridine-1-carboxylate (240 mg, 99% Purity) as a white solid. The crude was used in next step directly. MS (ES⁺): 249.4 (M+H)⁺.

### Step 3

To a solution of *tert-butyl* 3-(hydroxymethyl)-1H-pyrrolo[2,3-c]pyridine-1-carboxylate (120 mg, 0.48 mmol) in DCM (3 mL) was added SOCl₂ (85 mg, 0.72 mmol) at room temperature, and the reaction mixture was stirred at room temperature for 30 minutes. After LCMS indicated the reaction is completed, the reaction mixture was concentrated at 35 °C, diluted with H₂O (5 mL), alkalized with K₂CO₃ until pH = 7 and extracted with DCM (10 mL×3). The combined organic layer was washed with brine, dried over Na₂SO₄, filtered and concentrated at 40 °C under reduced pressure to give the title compound (130 mg, 98.7% Purity) as a light-yellow oil. The crude was used in next step directly.
MS (ES⁺): 267.2 (M+H)⁺.

### Intermediate 20 - benzyl 5-methyl-4-oxo-3,4,5,6,8,9-hexahydro-7H-pyrido[4',3':4,5]pyrrolo [2,3-d]pyridazine-7-carboxylate

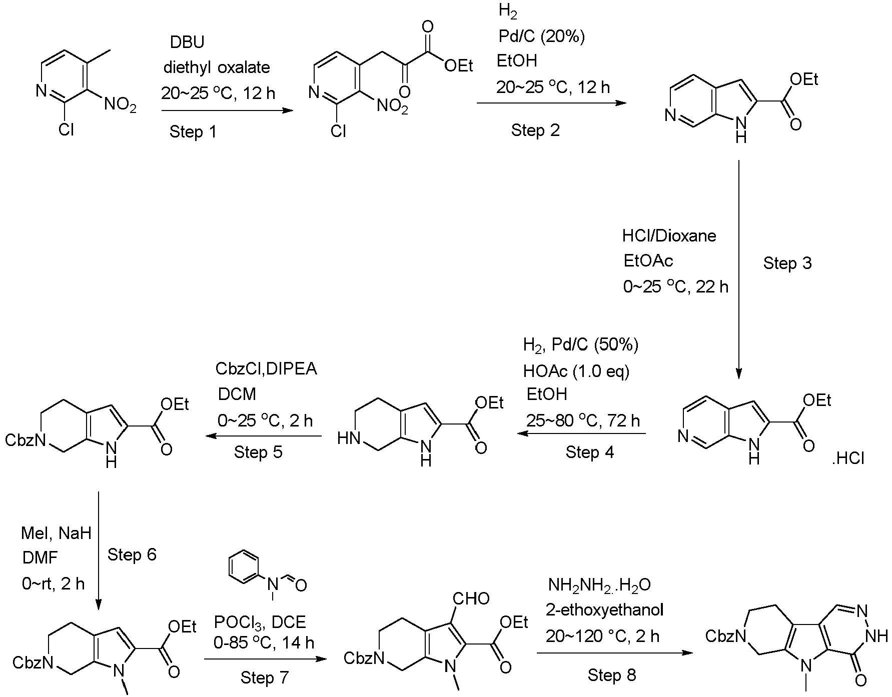

### Step 1

To a solution of 2-chloro-4-methyl-3-nitropyridine (150 g, 872 mmol) in diethyl oxalate (8.7 mol, 1.19 L) was added DBU (152.1 g, 1 mol) under N₂. The mixture was stirred at 20-25°C for 12 hours. After LCMS indicated the reaction is completed, The mixture was poured into ice water (10.0 L). The pH value of the aqueous phase was adjusted to 1-2 at 0-10 °C with HCl (1 N) to give a yellow suspension. The yellow suspension was filtered and the filter cake was dried *in vacuo.* The crude product was triturated with EtOH (2.00 L) and stirred at 25 °C for 12 hours. The mixture was filtered, the filter cake was washed with EtOH (500 mL) and dried under vacuum to give ethyl 3-(2-chloro-3-nitropyridin-4-yl)-2-oxopropanoate (165 g, 73% Purity) as a white solid. MS (ES⁺): 273.2 (M+H)⁺.

### Step 2

To a solution of ethyl 3-(2-chloro-3-nitropyridin-4-yl)-2-oxopropanoate (75 g, 276 mmol) in EtOH (0.6 L) was added 20% Pd/C (15 g, 50% purity) under H₂. The mixture was stirred at 20-25 °C for 12 hours under H₂ (1.0 Mpa). After LCMS indicated the reaction is completed, the mixture was filtered and the filter cake was washed with EtOH (1.00 L x 4). The filtrate was concentrated *in vacuo* at 45 °C to give product (55 g). The crude product (55 g) was triturated with EtOH (0.60 L) and stirred at 25 °C for 12 hours. The mixture was filtered, the filter cake was washed with EtOH (700 mL) and dried under vacuum to give ethyl 1H-pyrrolo[2,3-c]pyridine-2-carboxylate (50 g, 97% Purity) as a yellow solid.

MS (ES⁺): 191.3 (M+H)⁺.

### Step 3

To a solution of ethyl 1H-pyrrolo[2,3-c]pyridine-2-carboxylate ( 50 g, 263 mmol) in EtOAc (370 mL) was added HCl/dioxane (4M, 0.35 L) in one portion at 0-5 °C. The reaction mixture was stirred at 20-25 °C for 12 hours. After the reaction is completed, the mixture was filtered and the filter cake was washed with EtOAc (50.0 mL x 2). The filter cake was dried under vacuum at 45 °C to give ethyl 1H-pyrrolo[2,3-c]pyridine-2-carboxylate hydrochloric acid salt (60 g, 100%) as a yellow solid. Use in the next step without further characterisation.

### Step 4

To a solution of ethyl 1H-pyrrolo[2,3-c]pyridine-2-carboxylate hydrochloric acid salt (60 g, 263 mmol) in EtOH (0.5 L) was added HOAc (15.78 g, 263 mmol) and 50% Pd/C (30.0 g, 50% purity) under Ar to give a black suspension at 25 °C. The mixture was degassed under vacuum and purged with H₂ for three times. The mixture was heated to 80 °C and stirred for 15 hours under H₂ (3.0 MPa). After LCMS indicated the reaction is completed, the mixture was cooled to 20 °C and filtered through a celite pad. Two reactions were combined. The filter cake was washed with EtOH (1.00 L x 2) and the filtrate was concentrated in vacuo at 45 °C to give ethyl 4,5,6,7-tetrahydro-1H-pyrrolo[2,3-c]pyridine-2-carboxylate (47.3 g, 84% Purity) as a white solid.

MS (ES⁺): 195.3 (M+H)⁺.

### Step 5

To a solution of ethyl 4,5,6,7-tetrahydro-1H-pyrrolo[2,3-c]pyridine-2-carboxylate (47.3 g, 243 mmol) in DCM (0.5 L) was added drop-wise DIPEA (125 g, 972 mmol) at 0-5 °C. Then the mixture was added drop-wise CbzCl (44 g, 255 mmol,). The mixture was stirred at 20-25 °C for 2 hours. After LCMS indicated the reaction is completed, the reaction mixture was poured into the ice aq. NaHCO₃ (675 mL) and the layers were separated. The aqueous layer was extracted with DCM (200 mL x2). The organic layers were combined and dried over Na₂SO₄. Then the organic layer was concentrated at 45 °C in vacuo, The residue was purified by silica gel chromatography (240 g, petroleum ether: ethyl acetate= 100 : 0-50: 50)to give 6-benzyl 2-ethyl 4,5-dihydro-1H-pyrrolo[2,3-c]pyridine-2,6(7H)-dicarboxylate (64 g, 55% Purity) as a yellow solid.

MS (ES⁺): 329.2 (M+H)⁺.

### Step 6

To a solution of 6-benzyl 2-ethyl 4,5-dihydro-1H-pyrrolo[2,3-c]pyridine-2,6(7H)-dicarboxylate (64 g, 195 mmol) in DMF (0.6 L) was added NaH (9.7 g, 253 mmol) at 0 °C. The reaction mixture was stirred at 0 °C for 1 hour. A solution of Mel (27.7 g, 195 mmol, 12 mL) in DMF (186 mL) was added and the reaction mixture was stirred at 0-5 °C for 2 hour. After LCMS indicated the reaction is completed, the mixture was poured into ice water (5.0 L). The pH value of the aqueous phase was adjusted to 1-2 at 0-10 °C with HCl (1 N) to give a yellow suspension. The yellow suspension was filtered, and the filter cake was dried *in vacuo.* The residue was purified by silica gel chromatography (240 g, petroleum ether: ethyl acetate= 100 : 0-50: 50) to give 6-benzyl 2-ethyl 1-methyl-4,5-dihydro-1H-pyrrolo[2,3-c]pyridine-2,6(7H)-dicarboxylate ( 60 g, 88% Purity) as a yellow solid.

MS (ES⁺): 343.3 (M+H)⁺.

### Step 7

To a solution of N-methyl-N-phenylformamide (85 g, 632 mmol) in DCE (0.7 L) was added POCl₃ (96g, 632 mol) drop-wise at 0 °C. Then the reaction mixture was warmed to 20-25 °C and stirred at 20-25 °C for 4 hours. A solution of 6-benzyl 2-ethyl 1-methyl-4,5-dihydro-1H-pyrrolo[2,3-c]pyridine-2,6(7H)-dicarboxylate (60 g, 175 mmol,) in DCE (190 mL) was added dropwise into the mixture at 20-25 °C. The mixture was heated to 80-85 °C and stirred at 80-85 °C for 10 hours. After LCMS indicated the reaction is completed, the reaction mixture was cooled to 20-25 °C. The reaction mixture was concentrated at 45 °C to give the residue. The residue was diluted with DCM (500 mL) and the pH was adjusted to 8 with sat. NaHCO₃. The layers were separated, and the aqueous phase was extracted with DCM (500 mL x 2). The organic layers were combined and dried over Na₂SO₄. The organic layers were concentrated at 45 °C to give crude product. The residue was purified by silica gel chromatography (240 g, petroleum ether: ethyl acetate = 100 : 0-50:50) to give 6-benzyl 2-ethyl 3-formyl-1-methyl-4,5-dihydro-1H-pyrrolo[2,3-c]pyridine-2,6(7H)-dicarboxylate (54 g, 72% Purity) as a yellow solid.

MS (ES⁺): 371.3 (M+H)⁺.

### Step 8

To a solution of 6-benzyl 2-ethyl 3-formyl-1-methyl-4,5-dihydro-1 H-pyrrolo[2,3-c]pyridine-2,6(7H)-dicarboxylate (54 g, 145 mmol) in 2-ethoxyethanol (0.3 L) was added NH₂NH₂.H₂O (10.9 g, 217.5 mmol, 1.50 Eq) drop-wise at 20-25 °C. Then the mixture was stirred at 115-120 °C for 10 hours. After LCMS indicated the reaction is completed, The reaction mixture was cooled to 20-25 °C. Then the reaction mixture was poured into ice water (1.5 L). The reaction mixture was filtered and the filter cake was washed with water (100 mL x 2). The filter cake was dried under vacuum at 45 °C to give the title compound (43 g, 92% Purity) as a white solid.

MS (ES⁺): 339.3 (M+H)⁺.

### Intermediate 21 - 3-((6-methoxypyridin-3-yl)methyl)-5-methyl-3,5,6,7,8,9-hexahydro-4H-pyrido[4',3':4,5]pyrrolo[2,3-d]pyridazin-4-one

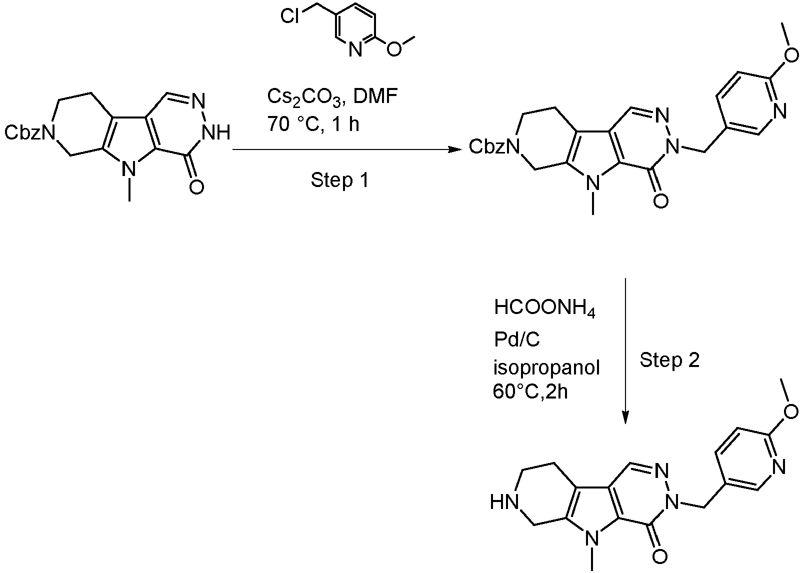

### Step 1

A mixture of benzyl 5-methyl-4-oxo-3,4,5,6,8,9-hexahydro-7H-pyrido[4',3':4,5]pyrrolo[2,3-d]pyridazine-7-carboxylate (Intermediate **20, 3.0** g, 8.87 mmol) and Cs₂CO₃(5.76 g, 17.72 mmol) in DMF (10 mL) was added 5-(chloromethyl)-2-methoxypyridine (2.10 g, 13.30 mmol) at room temperature, and the reaction mixture under nitrogen was stirred at 70 °C for 1 hour. After LCMS indicated the reaction is completed, the reaction mixture was diluted with water (20 mL) and extracted with EtOAc (30 mL × 3). The combined organic layers were washed with saturated ammonium chloride solution (30 mL × 2), dried over Na₂SO₄, filtered and concentrated at 40 °C under reduced pressure. The residual was purified by flash column chromatography (40 g, dichloromethane/methanol = 100:00-90:10) to give benzyl 3-((6-methoxypyridin-3-yl)methyl)-5-methyl-4-oxo-3,4,5,6,8,9-hexahydro-7H-pyrido[4',3':4,5]pyrrolo[2,3-d]pyridazine-7-carboxylate (3.6 g, 90% Purity) as a yellow solid.

MS (ES⁺): 460.3 (M+H)⁺.

### Step 2

A mixture of benzyl 3-((6-methoxypyridin-3-yl)methyl)-5-methyl-4-oxo-3,4,5,6,8,9-hexahydro-7H-pyrido[4',3':4,5]pyrrolo[2,3-d]pyridazine-7-carboxylate (SCP3-10-1, 3.6 g, 7.83 mmol), ammonium formate (4.93 g, 78.3 mmol) and Pd/C (10% wt, 720 mg) in isopropyl alcohol (50 mL) was stirred at 60 °C for 2 hours. After LCMS indicated the reaction is completed, the reaction mixture was basified with saturated NaHCO₃ aqueous solution until pH to 8. The suspension was filtered, and the filtrate was extracted with DCM (30 mL × 3). The organic layers were concentrated at 40 °C under reduced pressure and purified by flash column chromatography (40 g, dichloromethane/methanol = 100:00-90:10) to give to give the title compound (2.4 g, 95% Purity) as a white solid.

¹H NMR (400 MHz, DMSO-d6) δ: 8.16-8.14 (m, 2H), 7.63 (dd, J= 8.4 Hz, 2.4 Hz, 1H), 6.76 (d, *J* = 8.8 Hz, 1H), 5.24 (s, 2H), 3.94 (s, 3H), 3.83-3.81 (m, 5H), 2.89 (t, *J = 5.6* Hz, 2H), 2.57 (t, *J* = 5.6 Hz, 2H).

MS (ES⁺): 326.3 (M+H)⁺.

### Intermediate 22 - N-(2-methoxyethyl)-2-oxooxazolidine-3-sulfonamide

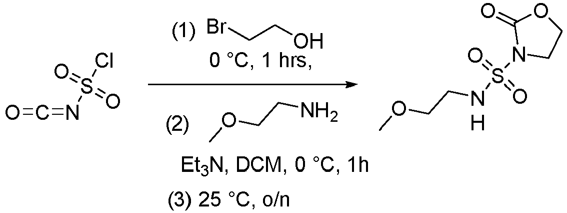

To a solution of sulfurisocyanatidic chloride (2 g, 14 mmol) in DCM (150 mL) was added 2-bromoethanol (1.74 g, 14 mmol) at 0 °C, and the reaction mixture was stirred at 0 °C for 1 hour. Then the reaction was added 2-methoxyethanamine (1.26 g, 16.8 mmol) and Et₃N (4.2 mg, 42 mmol) at 0 °C, the reaction mixture was stirred at 25 °C for overnight. TLC indicated the reaction to be complete, the reaction mixture was extracted with DCM (20 mL×3). The combined organic layer was washed by brine, dried over Na₂SO₄, filtered and concentrated at 45 °C under reduced pressure. The residue was purified by flash column chromatography (12 g, petroleum ether/ethyl acetate = 100:0-70:30) to give N-(2-methoxyethyl)-2-oxooxazolidine-3-sulfonamide (2.2 g, 95% purity) as a yellow oil.

1H NMR (400 MHz, DMSO-d6) δ: 8.44 (t, *J =* 5.6 Hz, 1H), 4.38-4.34 (m, 2H), 3.94-3.91 (m, 2H), 3.36 (t, *J =* 5.6 Hz, 2H), 3.24 (s, 3H), 3.20-3.16 (m, 2H).

### Intermediate 23 - tert-butyl 3-(chloromethyl)-1H-pyrrolo[2,3-b]pyridine-1-carboxylate

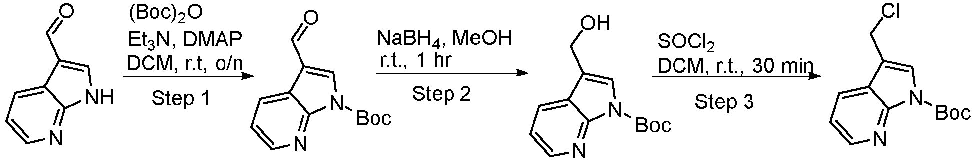

### Step 1

A mixture of 1H-pyrrolo[2,3-b]pyridine-3-carbaldehyde (1.7 g, 11.63 mmol), di-tert-butyl dicarbonate (5.08 g, 23.26 mmol), Et₃N (4.85 mL, 34.89 mmol) and DMAP (141 mg, 1.16 mmol) in DCM (50 mL) under nitrogen protection was stirred at room temperature overnight. After LCMS indicated the reaction is completed, the reaction mixture was diluted with H₂O (100 mL) and extracted with DCM (100 mL × 3). The combined organic layer was washed with saturated salt water, dried over Na₂SO₄, filtered and concentrated at 40 °C under reduced pressure and purified by flash column chromatography (40 g, petroleum ether/ethyl acetate = 100:00-66:34) to give *tert* -butyl 3-formyl-1H-pyrrolo[2,3-b]pyridine-1-carboxylate (2.4 g, 90% Purity) as a yellow oil.

MS (ES⁺): 191.2 (M-56+H)⁺.

### Step 2

To a solution of *tert-butyl* 3-formyl-1H-pyrrolo[2,3-b]pyridine-1-carboxylate (2.4 g, 9.76 mmol) in MeOH (50 mL) was added NaBH₄ (556 mg, 14.63 mmol) at room temperature, and the reaction mixture under nitrogen protection was stirred at room temperature for 1 hour. After LCMS indicated the reaction is completed, the reaction mixture was quenched with saturated ammonium chloride solution, concentrated to remove MeOH and extracted with DCM (100 mL×3). The combined organic layer was washed with brine, dried over Na₂SO₄, filtered, and concentrated at 40 °C under reduced pressure to give *tert-butyl* 3-(hydroxymethyl)-1H-pyrrolo[2,3-b]pyridine-1-carboxylate (2.3 g, 87% Purity) as a white solid. The crude was used in next step directly. MS (ES⁺): 249.3 (M+H)⁺.

### Step 3

To a solution of *tert-butyl* 3-(hydroxymethyl)-1H-pyrrolo[2,3-b]pyridine-1-carboxylate (250 mg, 1.01 mmol) in DCM (5 mL) was added SOCl₂ (178 mg, 1.50 mmol) at room temperature, and the reaction mixture was stirred at room temperature for 30 minutes. After LCMS indicated the reaction is completed, the reaction mixture was concentrated at 35 °C under reduced pressure to give t the title compound as a light-yellow solid. The material was used crude in the next step without further purification.

### Intermediate 24 - tert-butyl 3-(chloromethyl)-5-fluoro-1 H-indole-1-carboxylate

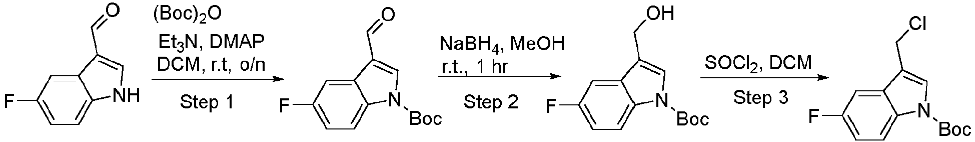

### Step 1

A mixture of 5-fluoro-1H-indole-3-carbaldehyde (400 mg, 2.45 mmol), di-tert-butyl dicarbonate (1.07 g, 4.90 mmol), Et₃N (1.00 mL, 7.35 mmol) and DMAP (61 mg, 0.50 mmol) in DCM (12 mL) under nitrogen protection was stirred at room temperature overnight. After LCMS indicated the reaction is completed, the reaction mixture was diluted with H₂O (40 mL), separated, and extracted with DCM (30 mL × 3). The combined organic layers were washed with saturated salt water (40 mL), dried over Na₂SO₄, filtered, and concentrated at 40 °C under reduced pressure. The mixture was purified by flash column chromatography (20 g, petroleum ether/ethyl acetate = 100:00-88:12) to give *tert-butyl* 5-fluoro-3-formyl-1H-indole-1-carboxylate (620 mg, 86% Purity) as a light-yellow oil.

MS (ES⁺): 208.1 (M-56+H)⁺.

### Step 2

To a solution of *tert-butyl* 5-fluoro-3-formyl-1H-indole-1-carboxylate (620 mg, 2.36 mmol) in MeOH (10 mL) was added NaBH₄ (134 mg, 5.34 mmol) at room temperature, and the reaction mixture under nitrogen protection was stirred at room temperature for 1 hour. After LCMS indicated the reaction is completed, the reaction mixture was quenched with saturated ammonium chloride aqueous solution, concentrated to remove MeOH and extracted with DCM (20 mL × 3). The combined organic layers were washed with brine, dried over Na₂SO₄, filtered and concentrated at 40 °C under reduced pressure to give *tert-butyl* 5-fluoro-3-(hydroxymethyl)-1H-indole-1-carboxylate (600 mg, 99% Purity) as a light-yellow solid. The crude was used in next step directly.

MS (ES⁺): 192.3 (M-56+H)⁺.

### Step 3

To a solution of *tert-butyl* 5-fluoro-3-(hydroxymethyl)-1H-indole-1-carboxylate (280 mg, 1.06 mmol) in DCM (6 mL) was added SOCl₂ (190 mg, 1.60 mmol) at room temperature, and the reaction mixture was stirred at room temperature for 30 minutes. After LCMS indicated the raw material is disappeared, the reaction mixture was concentrated at 35 °C under reduced pressure to give the title compound as a yellow oil. The material was used crude in the next step without further purification.

### Intermediate 25 - tert-butyl 3-(chloromethyl)-6-fluoro-1 H-indole-1-carboxylate

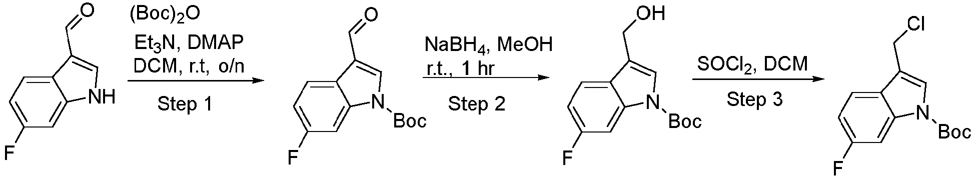

### Step 1

A mixture of 6-fluoro-1H-indole-3-carbaldehyde (1 g, 6.13 mmol), di-tert-butyl dicarbonate (2.68 g, 12.26 mmol), Et₃N (2.56 mL, 18.4 mmol) and DMAP (154 mg, 1.26 mmol) in DCM (30 mL) under nitrogen protection was stirred at room temperature overnight. After LCMS indicated the reaction is completed, the reaction mixture was diluted with H₂O (40 mL), separated and extracted with DCM (30 mL × 3). The combined organic layers were washed with saturated salt water (40 mL), dried over Na₂SO₄, filtered and concentrated at 40 °C under reduced pressure and purified by flash column chromatography (20 g, petroleum ether/ethyl acetate = 100:00-90:10) to give *tert-*butyl 6-fluoro-3-formyl-1H-indole-1-carboxylate (1.6 g, 99% Purity) as a light-yellow oil. MS (ES⁺): 264.3 (M+H)⁺.

### Step 2

To a solution of tert-butyl 6-fluoro-3-formyl-1H-indole-1-carboxylate (1.6 g, 6.08 mmol) in MeOH (25 mL) was added NaBH₄ (346 mg, 9.12 mmol) at room temperature, and the reaction mixture under nitrogen protection was stirred at room temperature for 1 hour. After LCMS indicated the reaction is completed, the reaction mixture was quenched with saturated ammonium chloride aqueous solution, concentrated to remove MeOH and extracted with DCM (40 mL × 3). The combined organic layers were washed with brine, dried over Na₂SO₄, filtered and concentrated at 40 °C under reduced pressure to give *tert-butyl* 6-fluoro-3-(hydroxymethyl)-1H-indole-1-carboxylate (1.66 g, 99% Purity) as a light-yellow solid. The crude was used in next step directly. MS (ES⁺): 192.2 (M-56-18+H)⁺.

### Step 3

To a solution of *tert-butyl* 6-fluoro-3-(hydroxymethyl)-1H-indole-1-carboxylate (300 mg, 1.13 mmol) in DCM (8 mL) was added SOCl₂ (202 mg, 1.70 mmol) at room temperature, and the reaction mixture was stirred at room temperature for 30 minutes. After LCMS indicated the raw material is disappeared, the reaction mixture was concentrated at 35 °C under reduced pressure to give the title compound as a yellow oil. The material was used crude in the next step without further purification.

### Example 1 - 3-((2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-one

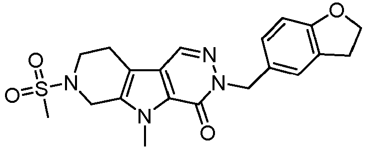

A suspension of Intermediate 1 (40 mg, 100% Wt, 1 Eq, 0.14 mmol) and cesium carbonate (92 mg, 2 Eq, 0.28 mmol) in DMF (1 mL) was stirred at 85 °C for 2 h and then allowed to cool to RT. A solution of 5-(chloromethyl)-2,3-dihydrobenzofuran (45 mg, 80% Wt, 1.5 Eq, 0.21 mmol) in DMF (0.5 mL) was added and the reaction mixture was stirred at RT for 18 h. The reaction mixture was concentrated *in vacuo* to afford the crude product. The crude product was purified by chromatography on silica gel (4 g cartridge, 0-4% MeOH/DCM) to afford the title compound (39.8 mg, 97% Purity) as a pale yellow solid. ¹H NMR (400 MHz, DMSO-d6) δ 8.20 (s, 1H), 7.15 (d, J = 1.7 Hz, 1H), 7.03 (dd, J = 8.1, 1.8 Hz, 1H), 6.67 (d, J = 8.1 Hz, 1H), 5.20 (s, 2H), 4.50 - 4.43 (m, 4H), 4.01 (s, 3H), 3.49 (t, J = 5.7 Hz, 2H), 3.11 (t, J = 8.7 Hz, 2H), 3.00 (s, 3H), 2.80 (t, J = 5.8 Hz, 2H). MS (ES⁺): 415 (M+H)⁺.

### Example 2 - 3-((6-methoxypyridin-3-yl)methyl)-5-methyl-7-(methylsulfonyl)-3,5,6,7,8,9-hexahydro-4H-pyrido[4',3':4,5]pyrrolo[2,3-d]pyridazin-4-one

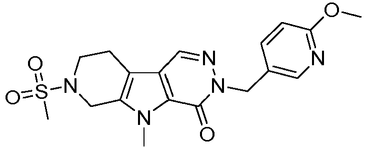

A suspension of Intermediate 1 (30 mg, 100% Wt, 1 Eq, 0.11 mmol) and cesium carbonate (69 mg, 2 Eq, 0.21 mmol) in DMF (1 mL) was stirred at 85 °C for 2 h and then allowed to cool to RT. A solution of 5-(chloromethyl)-2-methoxypyridine (19 mg, 16 µL, 95% Wt, 1.05 Eq, 0.11 mmol) in DMF (0.5 mL) was added and the reaction mixture was stirred at RT for 18 h. The reaction mixture was partitioned between DCM (5 mL) and water (5 mL). The organic layer was collected and the aqueous was extracted with DCM (5 mL). The combined organic extracts were dried (phase separator) and concentrated *in vacuo* to afford the crude product. The crude product was purified by preparative HPLC (Basic method (B)) to afford the title compound (12.8 mg, 100% Purity) as a flocculent white solid. ¹H NMR (400 MHz, DMSO-d6) δ 8.22 (s, 1H), 8.17 - 8.12 (m, 1H), 7.63 (dd, J = 8.6, 2.5 Hz, 1H), 6.76 (dd, J = 8.5, 0.7 Hz, 1H), 5.24 (s, 2H), 4.47 (s, 2H), 4.01 (s, 3H), 3.81 (s, 3H), 3.48 (t, J = 5.7 Hz, 2H), 2.99 (s, 3H), 2.80 (t, J = 5.8 Hz, 2H). MS (ES⁺): 404 (M+H)⁺.

### Example 3 - 3-(3-(1-hydroxyethyl)benzyl)-5-methyl-7-(methylsulfonyl)-3,5,6,7,8,9-hexahydro-4H-pyrido[4',3':4,5]pyrrolo[2,3-d]pyridazin-4-one

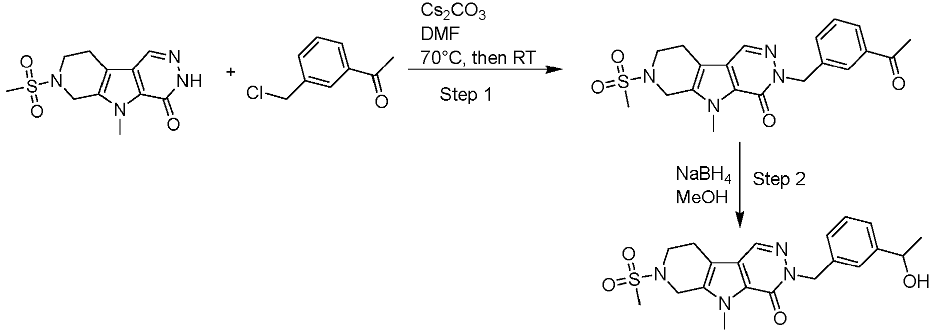

### Step 1

A mixture of Intermediate 1 (60 mg, 1 Eq, 0.21 mmol) and cesium carbonate (0.21 g, 3 Eq, 0.64 mmol) in DMF (1 mL) was stirred at 70 °C for 30 minutes. 1-(3-(chloromethyl)phenyl)ethan-1-one (39 mg, 1.1 Eq, 0.23 mmol) was added and the mixture was allowed to cool to RT and stirred overnight. Water and DCM were added and the layers separated through a phase separating cartridge. The organic layer was washed with brine, then absorbed on silica. The crude product was purified by chromatography on silica gel to afford 3-(2,6-difluoro-4-methoxybenzyl)-5-methyl-7-(methylsulfonyl)-3,5,6,7,8,9-hexahydro-4H-pyrido[4',3':4,5]pyrrolo[2,3-d]pyridazin-4-one (34 mg, 100% Purity) as a white solid. MS (ES⁺): 415 (M+H)⁺.

### Step 2

Sodium borohydride (2.7 mg, 2 Eq, 72 µmol) was added to a suspension of 3-(3-acetylbenzyl)-5-methyl-7-(methylsulfonyl)-3,5,6,7,8,9-hexahydro-4H-pyrido[4',3':4,5]pyrrolo[2,3-d]pyridazin-4-one (15 mg, 1 Eq, 36 µmol) in MeOH (0.2 mL). THF (0.2 mL) was added and further small portions of sodium borohydride were added until completion of reaction (3.5 h). 1N HCl and DCM were added and the phases were separated through a phase separator. The organic layer was washed with water, brine, dried (MgSO₄) and concentrated *in vacuo* to afford the title compound (4.9 mg, 99% Purity) as a white solid. ¹H NMR (DMSO-d6) δ: 8.23 (s, 1H), 7.28-7.17 (m, 3H), 7.11-7.04 (m, 1H), 5.29 (s, 2H), 5.11 (d, 1H), 4.71 - 4.60 (m, 1H), 4.48 (s, 2H), 4.01 (s, 3H), 3.50 (t, 2H), 3.00 (s, 3H), 2.81 (t, 2H), 1.27 (d, 3H). MS(ES⁺): 417 (M+H)⁺.

### Example 4 - 3-((1H-pyrazol-3-yl)methyl)-5-methyl-7-(methylsulfonyl)-3,5,6,7,8,9-hexahydro-4H-pyrido[4',3':4,5]pyrrolo[2,3-d]pyridazin-4-one

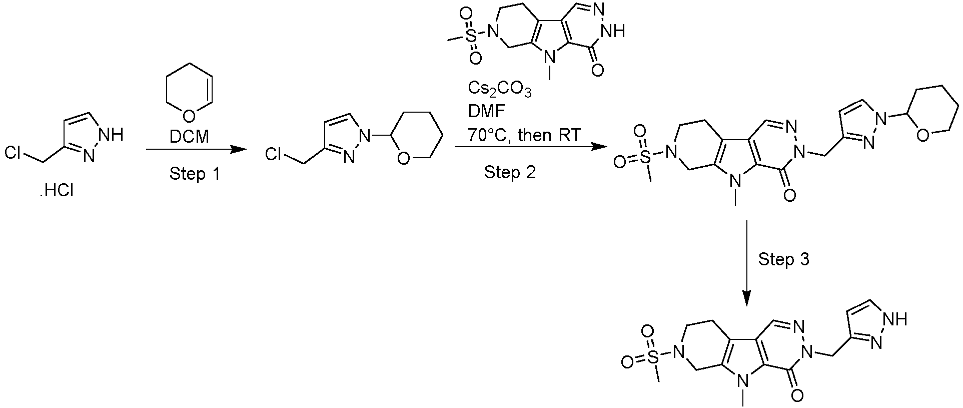

### Step 1

A suspension of 3-(chloromethyl)-1H-pyrazole, HCl (500 mg, 96% Wt, 1 Eq, 3.14 mmol) in DCM was treated with 3,4-dihydro-2H-pyran (286 mg, 310 µL, 97% Wt, 1.05 Eq, 3.29 mmol) dropwise. The reaction mixture was stirred at RT for 24 h. DCM (10 mL) and sat. aq. NaHCO₃ (10 mL) were added and the phases were mixed. The mixture was passed through a phase separator and the aqueous was extracted with DCM (2 x 10 mL). The combined organic extracts were dried (phase separator) and concentrated *in vacuo* to afford the crude 3-(chloromethyl)-1-(tetrahydro-2H-pyran-2-yl)-1H-pyrazole (501 mg, 80% Purity) as a pale yellow oil. MS (ES⁺): 223/225 (M+Na)⁺.

### Step 2

A mixture of Intermediate 1 (30 mg, 1 Eq, 0.11 mmol) and cesium carbonate (0.10 g, 3 Eq, 0.32 mmol) in DMF (0.5 mL) was stirred at 70°C for 30 minutes. 3-(chloromethyl)-1-(tetrahydro-2H-pyran-2-yl)-1H-pyrazole (23 mg, 1.1 Eq, 0.12 mmol) was added and the mixture was allowed to cool to RT and stirred overnight. Water and DCM were added and the layers separated through a phase separating cartridge. The organic layer was washed with brine, then absorbed on silica. The crude product was purified by chromatography on silica gel to afford 5-methyl-7-(methylsulfonyl)-3-((1-(tetrahydro-2H-pyran-2-yl)-1H-pyrazol-3-yl)methyl)-3,5,6,7,8,9-hexahydro-4H-pyrido[4',3':4,5]pyrrolo[2,3-d]pyridazin-4-one (16 mg, 100% Purity) as a white solid. MS(ES⁺): 447 (M+H)⁺.

### Step 3

A mixture of 5-methyl-7-(methylsulfonyl)-3-((1-(tetrahydro-2H-pyran-2-yl)-1H-pyrazol-3-yl)methyl)-3,5,6,7,8,9-hexahydro-4H-pyrido[4',3':4,5]pyrrolo[2,3-d]pyridazin-4-one (16 mg, 1 Eq, 36 µmol) and hydrogen chloride (4N in dioxane) (13 mg, 90 µL, 4 molar, 10 Eq, 0.36 mmol) in MeOH (0.3 mL) was stirred for 1.5h. Further hydrogen chloride (4N in dioxane) (45 µL, 4 molar, 5 Eq, 0.18 mmol) was added and the mixture stirred for 15 minutes, then concentrated *in vacuo.* Sat. NaHCO₃ and DCM were added to the residue and layers separated through a phase separator. The organic layer was washed with brine, then absorbed on silica. The crude product was purified by chromatography on silica gel to afford the title compound (4.2 mg, 99% Purity) as a white solid. ¹H NMR (DMSO-d6) δ: 12.59 (s, 1H), 8.18 (s, 1H), 7.58 (s, 1H), 6.04 (s, 1H), 5.28 (s, 2H), 4.47 (s, 2H), 4.01 (d, 3H), 3.53 - 3.45 (m, 2H), 3.00 (d, 3H), 2.84 - 2.78 (m, 2H) (trace of tautomers observed). MS (ES⁺): 363 (M+H)⁺.

### Example 5 - 3-(2-fluoro-3-methoxybenzyl)-5-methyl-7-(methylsulfonyl)-3,5,6,7,8,9-hexahydro-4H-pyrido[4',3':4,5]pyrrolo[2,3-d]pyridazin-4-one

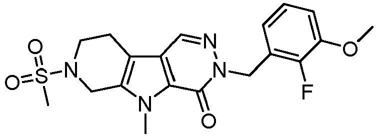

A mixture of Intermediate 1 (30 mg, 1 Eq, 0.11 mmol) and cesium carbonate (0.10 g, 3 Eq, 0.32 mmol) in DMF (0.5 mL) was stirred at 70 °C for 30 minutes. 1-(bromomethyl)-2-fluoro-3-methoxybenzene (26 mg, 1.1 Eq, 0.12 mmol) was added and the mixture was allowed to cool to RT and stirred overnight. Water and DCM were added and the layers separated through a phase separating cartridge. The organic layer was washed with brine, then absorbed on silica. The crude product was purified by chromatography on silica gel to afford 3-(2-fluoro-3-methoxybenzyl)-5-methyl-7-(methylsulfonyl)-3,5,6,7,8,9-hexahydro-4H-pyrido[4',3':4,5]pyrrolo[2,3-d]pyridazin-4-one. The product was dissolved in DCM, then washed with water, brine, dried (MgSO₄) and concentrated *in vacuo* to afford the title compound (8.6 mg, 97% Purity) as a white solid. ¹H NMR (DMSO-d6) δ: 8.23 (s, 1H), 7.11 - 6.98 (m, 2H), 6.65 - 6.57 (m, 1H), 5.35 (s, 2H), 4.48 (s, 2H), 4.00 (s, 3H), 3.82 (s, 3H), 3.50 (t, 2H), 3.00 (s, 3H), 2.85 - 2.78 (m, 2H). MS (ES⁺): 421 (M+H)⁺.

### Example 6 - 3-(2,6-difluoro-4-methoxybenzyl)-5-methyl-7-(methylsulfonyl)-3,5,6,7,8,9-hexahydro-4H-pyrido[4',3':4,5]pyrrolo[2,3-d]pyridazin-4-one

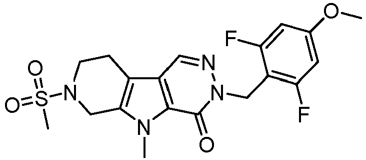

A mixture of Intermediate 1 (30 mg, 1 Eq, 0.11 mmol) and cesium carbonate (0.10 g, 3 Eq, 0.32 mmol) in DMF (0.5 mL) was stirred at 70 °C for 30 minutes. 2-(bromomethyl)-1,3-difluoro-5-methoxybenzene (28 mg, 1.1 Eq, 0.12 mmol) was added and the mixture was allowed to cool to RT and stirred overnight. Water and DCM were added and the layers separated through a phase separating cartridge. The organic layer was washed with brine, then absorbed on silica. The crude product was purified by chromatography on silica gel to afford the title compound (21.3 mg, 99% Purity) as a white solid. ¹H NMR (DMSO-d6) δ: 8.13 (s, 1H), 6.75 - 6.65 (m, 2H), 5.28 (s, 2H), 4.47 (s, 2H), 4.01 (s, 3H), 3.77 (s, 3H), 3.48 (t, 2H), 2.99 (s, 3H), 2.78 (t, 2H). MS (ES)⁺: 439 (M+H)⁺

### Example 7 - 3-(3-aminobenzyl)-5-methyl-7-(methylsulfonyl)-3,5,6,7,8,9-hexahydro-4H-pyrido[4',3':4,5]pyrrolo[2,3-d]pyridazin-4-one

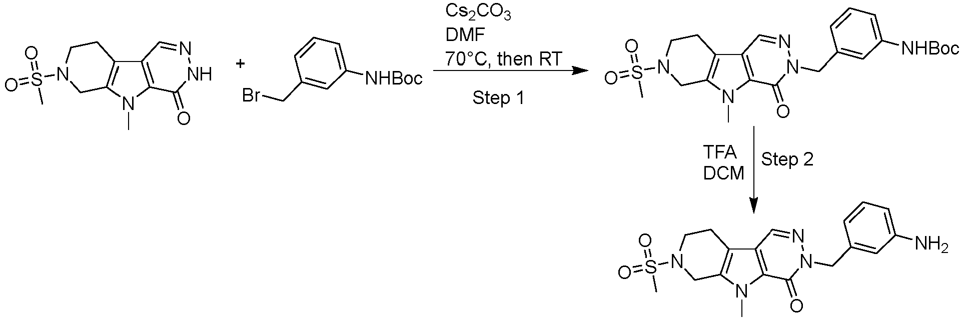

### Step 1

A suspension of Intermediate 1 (69.0 mg, 100% Wt, 1 Eq, 244 µmol) and cesium carbonate (166 mg, 2.08 Eq, 509 µmol) in DMF (3 mL) was stirred at 80 °C for 30 min. A solution of tert-butyl (3-(bromomethyl)phenyl)carbamate (78 mg, 1.1 Eq, 0.27 mmol) in DMF (1 mL) was added dropwise and the reaction mixture was stirred at 80 °C for 1 h. The reaction mixture was concentrated *in vacuo* to afford the crude product. The crude product was purified by chromatography on silica gel to afford tert-butyl (3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)phenyl)carbamate (126 mg, 68% Purity) as a pale yellow solid. MS (ES⁺): 510 (M+Na)⁺.

### Step 2

A stirred solution of tert-butyl (3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)phenyl)carbamate (126 mg, 68% Wt, 1 Eq, 176 µmol) in DCM (1.5 mL) was treated with TFA (0.74 g, 0.50 mL, 37 Eq, 6.5 mmol) dropwise. The reaction mixture was stirred at RT for 1.5 h, diluted with DCM (4 mL) and then quenched with aq. 2 M NaOH (5 mL). The organic layer was collected and the aqueous was extracted with DCM (2 x 5 mL). The combined organic extracts were dried (phase separator) and concentrated *in vacuo* to afford the crude product. The crude product was triturated with diethyl ether (3 x 5 mL), the solid was collected by filtration and then dried *in vacuo* (45 °C) to afford the title compound (60.7 mg, 95% Purity) as a pale yellow solid. ¹H NMR (400 MHz, DMSO-d6) δ 8.21 (s, 1H), 6.92 (dd, J = 8.6, 7.4 Hz, 1H), 6.40 (td, J = 7.7, 7.0, 2.6 Hz, 3H), 5.15 (s, 2H), 5.01 (s, 2H), 4.48 (s, 2H), 4.01 (s, 3H), 3.50 (t, J = 5.7 Hz, 2H), 3.00 (s, 3H), 2.81 (t, J = 5.8 Hz, 2H). MS (ES⁺): 388 (M+H)⁺.

### Example 8 - 7-acetyl-3-(3-aminobenzyl)-5-methyl-3,5,6,7,8,9-hexahydro-4H-pyrido [4',3':4,5]pyrrolo[2,3-d]pyridazin-4-one

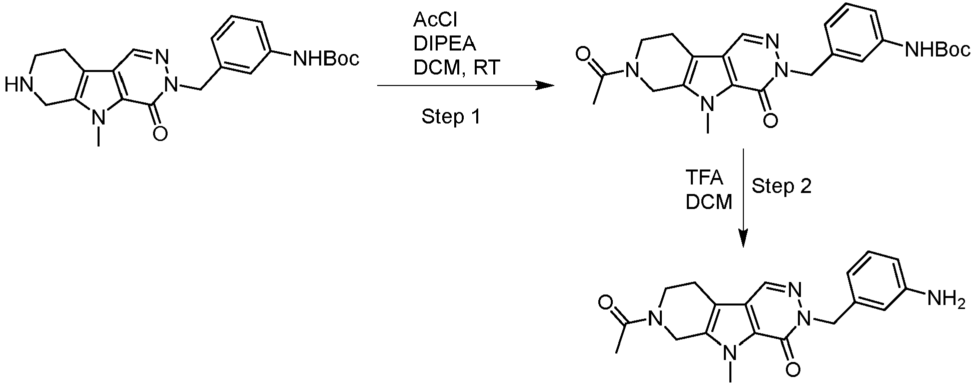

### Step 1

A solution of Intermediate 2 (19 mg, 94% Wt, 1 Eq, 44 µmol) and DIPEA (8.5 mg, 11 µL, 1.5 Eq, 65 µmol) in DCM (1.5 mL) at 0 °C was treated with a solution of acetyl chloride (3.5 mg, 3.2 µL, 98% Wt, 1 Eq, 44 µmol) in DCM (0.5 mL). The reaction mixture was stirred at 0 °C for 1 h and then allowed to warm to RT for 1 h. The reaction mixture was diluted with DCM (3 mL) and washed with sat. aq. NaHCO₃ (5 mL). The organic layer was collected and the aqueous was extracted with DCM (5 mL). The combined organic extracts were dried (phase separator) and concentrated *in vacuo* to afford the crude product tert-butyl (3-((7-acetyl-5-methyl-4-oxo-4,5,6,7,8,9-hexahydro-3H-pyrido[4',3':4,5]pyrrolo[2,3-d]pyridazin-3-yl)methyl)phenyl)carbamate (22 mg, 88% Purity) as a pale yellow solid. The crude product was used without further purification in the next step. MS (ES⁺): 452 M+H)⁺.

### Step 2

A solution of tert-butyl (3-((7-acetyl-5-methyl-4-oxo-4,5,6,7,8,9-hexahydro-3H-pyrido[4',3':4,5]pyrrolo[2,3-d]pyridazin-3-yl)methyl)phenyl)carbamate (22 mg, 88% Wt, 1 Eq, 43 µmol) in DCM (1 mL) was treated with TFA (148 mg, 100 µL, 30 Eq, 1.30 mmol). The reaction mixture was stirred at RT for 1 h, diluted with DCM (4 mL), quenched with 2 M NaOH (aq.) (2 mL) and then stirred for 1 h. The organic layer was collected and the aqueous was extracted with DCM (5 mL). The combined organic extracts were dried (phase separator) and concentrated *in vacuo* to afford the crude product. The crude product was dissolved in DMSO (0.5 mL), filtered and purified by reversed phase preparative HPLC (Acidic method (A)). The clean fractions were evaporated in a Genevac to afford the title compound (4.1 mg, 98% Purity) (2 steps) as a flocculent white solid. ¹H NMR (400 MHz, DMSO-d6) δ 8.20 - 8.19 (m, 1H), 6.95 - 6.87 (m, 1H), 6.43 - 6.35 (m, 3H), 5.14 (s, 2H), 5.00 (s, 2H), 4.72 - 4.64 (m, 2H), 4.05 - 4.00 (m, 3H), 3.76 - 3.67 (m, 2H), 2.80 - 2.62 (m, 2H), 2.16 - 2.12 (m, 3H). MS (ES⁺): 352 (M+H)⁺.

### Example 9 - 3-(3-aminobenzyl)-7-(cyclopropylsulfonyl)-5-methyl-3,5,6,7,8,9-hexahydro-4H-pyrido[4',3':4,5]pyrrolo[2,3-d]pyridazin-4-one

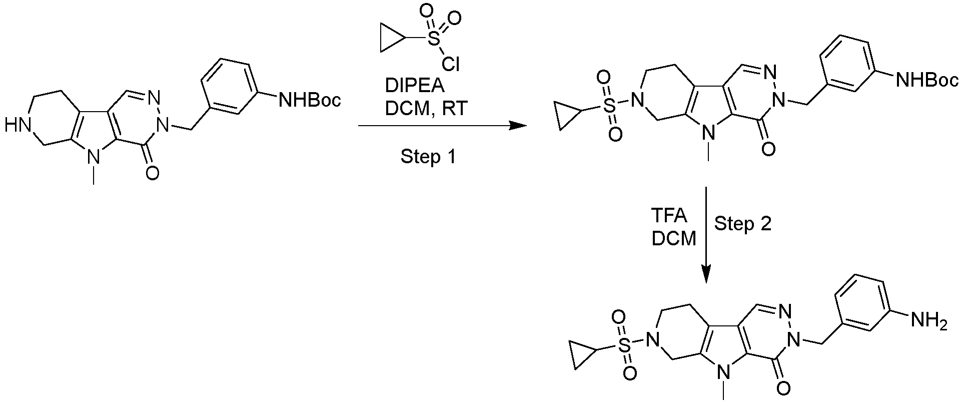

### Step 1

A solution of Intermediate 2 (14 mg, 94% Wt, 1 Eq, 32 µmol) and DIPEA (6.7 mg, 9.0 µL, 1.6 Eq, 52 µmol) in DCM (1.5 mL) at 0 °C was treated with a solution of cyclopropanesulfonyl chloride (4.5 mg, 3.3 µL, 1 Eq, 32 µmol) in DCM (0.5 mL). The reaction mixture was stirred at 0 °C for 1 h and then allowed to warm to RT for 18 h. Further cyclopropanesulfonyl chloride (4.5 mg, 3.3 µL, 1 Eq, 32 µmol) in DCM (0.5 mL) was added and stirred for 18 h. A third portion of cyclopropanesulfonyl chloride (4.5 mg, 3.3 µL, 1 Eq, 32 µmol) in DCM (0.5 mL) was added and stirred for 18 h. The reaction mixture was diluted with DCM (5 mL) and washed with sat. aq. NaHCO₃ (5 mL). The organic layer was collected and the aqueous was extracted with DCM (5 mL). The combined organic extracts were dried (phase separator) and concentrated *in vacuo* to afford crude tert-butyl (3-((7-(cyclopropylsulfonyl)-5-methyl-4-oxo-4,5,6,7,8,9-hexahydro-3H-pyrido[4',3':4,5]pyrrolo[2,3-d]pyridazin-3-yl)methyl)phenyl)carbamate (23 mg, 83% Purity). The crude product was used without further purification in the next step. MS (ES⁺): 514 M+H)⁺.

### Step 2

A solution of tert-butyl (3-((7-(cyclopropylsulfonyl)-5-methyl-4-oxo-4,5,6,7,8,9-hexahydro-3H-pyrido[4',3':4,5]pyrrolo[2,3-d]pyridazin-3-yl)methyl)phenyl)carbamate (16 mg, 1 Eq, 32 µmol) in DCM (1 mL) was treated with TFA (0.15 g, 0.10 mL, 41 Eq, 1.3 mmol). The reaction mixture was stirred at RT for 1 h, diluted with DCM (4 mL), quenched with 2 M NaOH (aq.) (5 mL) and then stirred for 0.5 h. The organic layer was collected and the aqueous was extracted with DCM (5 mL). The combined organic extracts were dried (phase separator) and concentrated *in vacuo* to afford the crude product. The crude product was purified by chromatography on silica gel to afford the title compound (10.6 mg, 98% Purity) (over 2 steps) as a flocculent white solid. ¹H NMR (400 MHz, DMSO-d6) δ 8.21 (s, 1H), 6.95 - 6.88 (m, 1H), 6.44 - 6.36 (m, 3H), 5.14 (s, 2H), 5.01 (s, 2H), 4.54 (s, 2H), 4.02 (s, 3H), 3.56 (t, J = 5.7 Hz, 2H), 2.81 (t, J = 5.7 Hz, 2H), 2.73 - 2.64 (m, 1H), 1.01 (dt, J = 5.3, 2.9 Hz, 2H), 0.98 - 0.90 (m, 2H). MS (ES⁺): 414 (M+H)⁺.

### Example 10 - 3-(3-aminobenzyl)-5-methyl-7-((2-methylthiazol-5-yl)sulfonyl)-3,5,6,7,8,9-hexahydro-4H-pyrido[4',3':4,5]pyrrolo[2,3-d]pyridazin-4-one

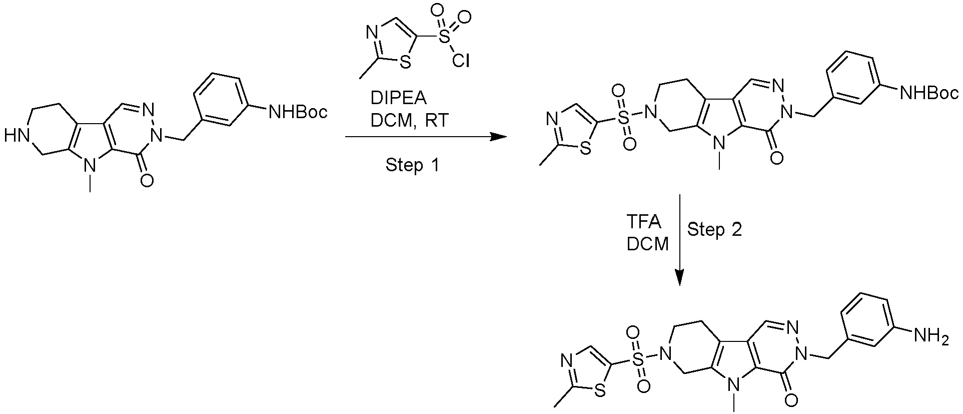

### Step 1

A stirred solution of Intermediate 2 (28 mg, 97% Wt, 1 Eq, 66 µmol) and DIPEA (14.8 mg, 20.0 µL, 1.7 Eq, 115 µmol) in DCM (1 mL) at 0 °C was treated with a solution of 2-methylthiazole-5-sulfonyl chloride (14.2 mg, 9.00 µL, 95% Wt, 1.0 Eq, 68.3 µmol) in DCM (0.5 mL) dropwise. The reaction mixture was stirred at 0 °C for 2 h. The reaction mixture was diluted with DCM (4 mL) and washed with sat. aq. NaHCO₃ (5 mL). The organic layer was collected and the aqueous was extracted with DCM (5 mL). The combined organic extracts were dried (phase separator) and concentrated *in vacuo* to afford the crude product. The crude product was purified by chromatography on silica gel to afford tert-butyl (3-((5-methyl-7-((2-methylthiazol-5-yl)sulfonyl)-4-oxo-4,5,6,7,8,9-hexahydro-3H-pyrido[4',3':4,5]pyrrolo[2,3-d]pyridazin-3-yl)methyl)phenyl)carbamate (35 mg, 98% Purity) as a pale yellow gum. The crude product was used without further purification in the next step. MS (ES⁺): 571 M+H)⁺.

### Step 2

A solution of tert-butyl (3-((5-methyl-7-((2-methylthiazol-5-yl)sulfonyl)-4-oxo-4,5,6,7,8,9-hexahydro-3H-pyrido[4',3':4,5]pyrrolo[2,3-d]pyridazin-3-yl)methyl)phenyl)carbamate (35 mg, 60 µmol, 91 %, 98% Purity) in DCM was treated with TFA (148 mg, 100 µL, 20 Eq, 1.30 mmol) in one portion and the reaction mixture was allowed to stand for 1 h. The reaction mixture was diluted with DCM (3 mL) and washed with 2 M NaOH (aq.) (4 mL). The organic layer was collected and the aqueous was extracted with DCM (2 x 4 mL). The combined organic extracts were dried (phase separator) and concentrated *in vacuo* to afford the crude product. The crude product was purified by chromatography on silica gel to afford the title compound (24.7 mg, 97% Purity) (yield over 2 steps) as a white solid after drying *in vacuo* (45 °C, 3 h). ¹H NMR (400 MHz, DMSO-d6) δ 8.26 (s, 1H), 8.18 (s, 1H), 6.94 - 6.88 (m, 1H), 6.43 - 6.35 (m, 3H), 5.13 (s, 2H), 5.00 (d, J = 8.4 Hz, 2H), 4.43 (s, 2H), 4.01 (s, 3H), 3.45 (t, J = 5.6 Hz, 2H), 2.79 (t, J = 5.8 Hz, 2H), 2.71 (s, 3H). MS (ES⁺): 471 (M+H)⁺.

### Example 11 - 3-(3-aminobenzyl)-5-methyl-7-(phenylsulfonyl)-3,5,6,7,8,9-hexahydro-4H-pyrido[4',3':4,5]pyrrolo[2,3-d]pyridazin-4-one

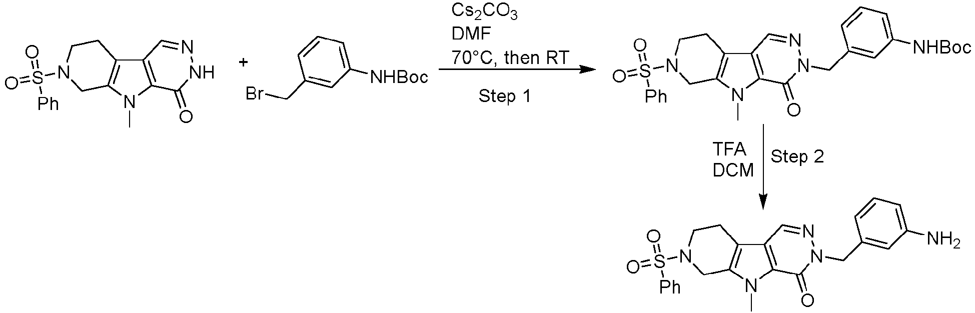

### Step 1

A suspension of Intermediate 3 (100 mg, 75% Wt, 1 Eq, 218 µmol) and cesium carbonate (165 mg, 2.33 Eq, 506 µmol) in DMF (2 mL) was stirred at 80 °C for 1 h. The reaction mixture was cooled to RT and treated with tert-butyl (3-(bromomethyl)phenyl)carbamate (62.3 mg, 1 Eq, 218 µmol) in one portion. The reaction mixture was stirred at RT for 18 h. The reaction mixture was concentrated *in vacuo* to afford the crude product. The crude product was purified by chromatography on silica gel to afford tert-butyl (3-((5-methyl-4-oxo-7-(phenylsulfonyl)-4,5,6,7,8,9-hexahydro-3H-pyrido[4',3':4,5]pyrrolo[2,3-d]pyridazin-3-yl)methyl)phenyl)carbamate (33 mg, 77% Purity) as a yellow solid. MS (ES⁺): 572 M+H)⁺.

### Step 2

A solution of tert-butyl (3-((5-methyl-4-oxo-7-(phenylsulfonyl)-4,5,6,7,8,9-hexahydro-3H-pyrido[4',3':4,5]pyrrolo[2,3-d]pyridazin-3-yl)methyl)phenyl)carbamate (33 mg, 77% Wt, 1 Eq, 46 µmol) in DCM (1 mL) was treated with TFA (296 mg, 200 µL, 56 Eq, 2.60 mmol) in one portion. The reaction mixture was allowed to stand at RT for 2 h and then concentrated *in vacuo* to afford the crude product. The crude product was purified by chromatography on silica gel to afford the title compound(13.0 mg, 99% Purity) as a pale yellow solid. ¹H NMR (400 MHz, DMSO-d6) δ 8.13 (s, 1H), 7.91 = 7.84 (m, 2H), 7.73 - 7.65 (m, 1H), 7.65 - 7.58 (m, 2H), 6.90 (dd, J = 8.2, 7.1 Hz, 1H), 6.44 - 6.33 (m, 3H), 5.12 (s, 2H), 5.00 (s, 2H), 4.38 (s, 2H), 4.00 (s, 3H), 3.39 (t, J = 5.7 Hz, 2H), 2.71 (t, J = 5.7 Hz, 2H). MS (ES⁺): 450 (M+H)⁺.

### Example 12 - 3-((1H-pyrazol-3-yl)methyl)-5-methyl-7-(phenylsulfonyl)-3,5,6,7,8,9-hexahydro-4H-pyrido[4',3':4,5]pyrrolo[2,3-d]pyridazin-4-one

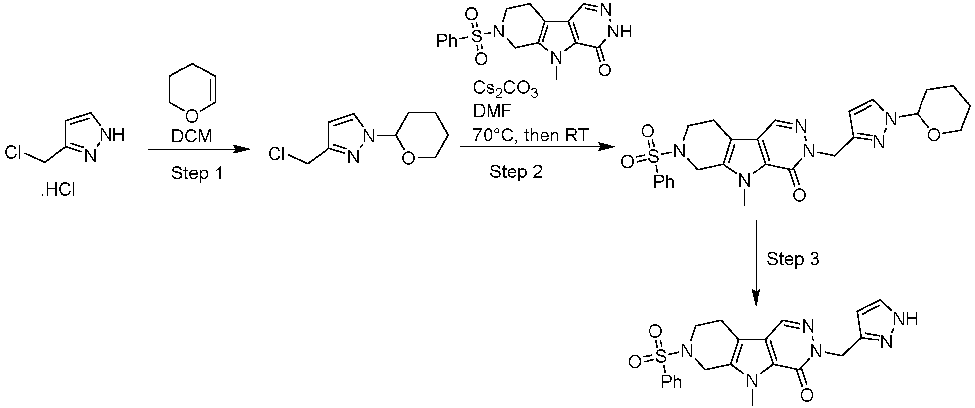

### Step 1

A suspension of 3-(chloromethyl)-1H-pyrazole, HCl (500 mg, 96% Wt, 1 Eq, 3.14 mmol) in DCM was treated with 3,4-dihydro-2H-pyran (286 mg, 310 µL, 97% Wt, 1.05 Eq, 3.29 mmol) dropwise. The reaction mixture was stirred at RT for 24 h. DCM (10 mL) and sat. aq. NaHCO₃ (10 mL) were added and the phases were mixed. The mixture was passed through a phase separator and the aqueous was extracted with DCM (2 x 10 mL). The combined organic extracts were dried (phase separator) and concentrated *in vacuo* to afford the crude 3-(chloromethyl)-1-(tetrahydro-2H-pyran-2-yl)-1H-pyrazole (501 mg, 80% Purity) as a pale yellow oil. MS (ES⁺): 223/225 (M+Na)⁺.

### Step 2

A suspension of Intermediate 3 (25.0 mg, 95% Wt, 1 Eq, 69.0 µmol) and cesium carbonate (27.0 mg, 1.2 Eq, 82.8 µmol) was stirred at 60 °C for 1 h and then allowed to cool to RT. 3-(chloromethyl)-1-(tetrahydro-2H-pyran-2-yl)-1H-pyrazole (23 mg, 70% Wt, 1.2 Eq, 80 µmol) was added at rt and the reaction mixture was stirred for 18 h. The reaction mixture was heated to 50 °C for 1 h and then cesium carbonate (36 mg, 0.11 mmol) was added and stirred for 30 min. The reaction mixture was allowed to cool to RT and 3-(chloromethyl)-1-(tetrahydro-2H-pyran-2-yl)-1H-pyrazole (46 mg, 70% Wt, 2.4 Eq, 0.16 mmol) was added and stirred at 50 °C for 3 h. The reaction mixture was concentrated onto silica gel and the crude product was purified by chromatography on silica gel to afford 5-methyl-7-(phenylsulfonyl)-3-((1-(tetrahydro-2H-pyran-2-yl)-1H-pyrazol-3-yl)methyl)-3,5,6,7,8,9-hexahydro-4H-pyrido[4',3':4,5]pyrrolo[2,3-d]pyridazin-4-one (19.0 mg, 90% Purity) as a pale yellow oil. MS (ES⁺): 509 (M+H)⁺.

### Step 3

HCl (4 M in dioxane) (0.53 g, 0.50 mL, 4.00 molar, 59 Eq, 2.0 mmol) was added to 5-methyl-7-(phenylsulfonyl)-3-((1-(tetrahydro-2H-pyran-2-yl)-1H-pyrazol-3-yl)methyl)-3,5,6,7,8,9-hexahydro-4H-pyrido[4',3':4,5]pyrrolo[2,3-d]pyridazin-4-one (19.0 mg, 90% Wt, 1 Eq, 33.6 µmol) in one portion. The reaction mixture was allowed to stand at RT for 18 h. The reaction mixture was diluted with DCM (3 mL) and washed with 2 M NaOH (aq.) (3 mL). The organic layer was collected and the aqueous was extracted with DCM (2 x 3 mL). The combined organic extracts were dried (phase separator) and concentrated *in vacuo* to afford the crude product. The crude product was purified by chromatography on silica gel to afford the title compound (8.00 mg, 95% Purity) as a white solid after drying *in vacuo* (45 °C). ¹H NMR (400 MHz, DMSO-d6) δ 12.58 (s, 1H), 8.10 (s, 1H), 7.91 - 7.85 (m, 2H), 7.71 - 7.66 (m, 1H), 7.65 - 7.59 (m, 2H), 7.58 (s, 1H), 6.01 (s, 1H), 5.24 (s, 2H), 4.38 (s, 2H), 4.00 (s, 3H), 3.38 (t, J = 5.7 Hz, 2H), 2.70 (t, J = 6.0 Hz, 2H). MS (ES⁺): 425 (M+H)⁺.

### Example 13 - 3-((2-(hydroxymethyl)thiophen-3-yl)methyl)-5-methyl-7-(methylsulfonyl)-3,5,6,7,8,9-hexahydro-4H-pyrido[4',3':4,5]pyrrolo[2,3-d]pyridazin-4-one

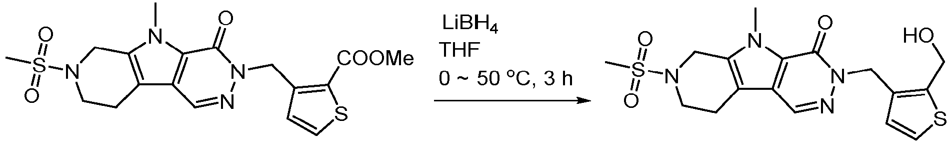

To solution of methyl 3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)thiophene-2-carboxylate **(Intermediate 4** 100 mg, 0.229 mmol, 1.0 Eq) in THF (1.0 mL) was added LiBH₄ (7.5 mg, 0.34 mmol, 1.5 Eq) in portions at 0-10 °C under N₂. Then the mixture was stirred at 45-50 °C for 3 h. LCMS_IPC (R_{t-SM} = 0.899 min, R_{f-product} = 0.847 min) showed the starting material was consumed completely and the desired mass was detected by LCMS. The reaction mixture was cooled to 20-25 °C and quenched with ice aq. NH₄Cl (10.0 mL) and extracted with ethyl acetate (10.0 mL x 2). The organic layer was washed with brine (10.0 mL), dried with Na₂SO₄, filtered, and concentrated *in vacuo* at 45 °C to give the crude product. The crude product was purification by pre-HPLC (column: phenomenex C18 75 * 30 mm * 3 um; mobile phase: [water (NH₄HCO₃)-ACN]; B%: 30%-65%, 8 min) to give the title compound (34 mg, 99.7% purity) as a white solid.

¹H NMR (400 MHz DMSO-d6): δ 8.21 (s, 1H), 7.27 (d, *J* = 5.2 Hz, 1H), 6.88 (d, *J* = 5.2 Hz, 1H), 5.44 (t, *J =* 5.6 Hz, 1H), 5.23 (s, 2H), 4.78 (d, *J* = 5.2 Hz, 2H), 4.46 (s, 2H), 4.00 (s, 3H), 3.48 (t, *J = 5.6* Hz, 2H), 2.99 (s, 3H), 2.80 (t, *J =* 5.6 Hz, 2H).

MS (ES⁺): 431.3 (M+Na)⁺.

### Example 14 - 3-((5-methyl-7-(methylsulfonyl)-4-oxo-4,5,6,7,8,9-hexahydro-3H-pyrido [4',3':4,5]pyrrolo[2,3-d]pyridazin-3-yl)methyl)thiophene-2-carboxamide

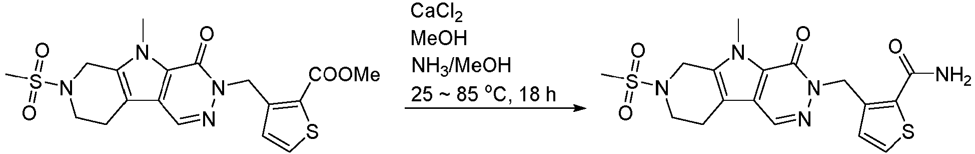

To a solution of methyl 3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)thiophene-2-carboxylate **(Intermediate 4,** 200 mg, 0.458 mmol, 1.0 Eq) in MeOH (2.0 mL) was added NH₃/MeOH (7 M, 654 uL, 10.0 Eq) and CaCl₂ (50.8 mg, 0.458 mmol, 1.0 Eq) in one portion at 20-25 °C. Then the mixture was stirred at 80-85 °C for 18 h. LCMS_IPC (R_{t-SM} = 0.899 min, R_{f-product} = 0.817 min) showed the starting material was consumed completely and the desired mass was detected. The reaction mixture was cooled to 20-25 °C. Then the reaction mixture was filtered, and the filter cake was washed with MeOH (5.0 mL). The filtrate was concentrated at 45 °C *in vacuo* to give the crude product. The crude product was purified by prep-HPLC (column: waters xbridge BEH C18 100 * 25 mm * 5 µm; mobile phase: [water (NH₄HCO₃)-ACN]; B%: 15%-45%, 10 min) to give the title compound (22 mg, 98.6% purity) as a white solid.

¹H NMR (400 MHz DMSO-d6): δ 8.29 (s, 1H), 7.58 (d, *J* = 6.0 Hz, 1H), 6.74 (d, *J* = 5.2 Hz, 1H), 5.52 (s, 2H), 4.48 (s, 2H), 4.00 (s, 3H), 3.50 (t, *J* = 4.4 Hz, 2H), 3.00 (s, 3H), 2.82 (t, *J =* 4.4 Hz, 2H).
MS (ES⁺): 422.1 (M+H)⁺

### Example 15 - 3-((1H-indol-3-yl)methyl)-5-methyl-7-(methylsulfonyl)-3,5,6,7,8,9-hexahydro-4H-pyrido[4',3':4,5]pyrrolo[2,3-d]pyridazin-4-one

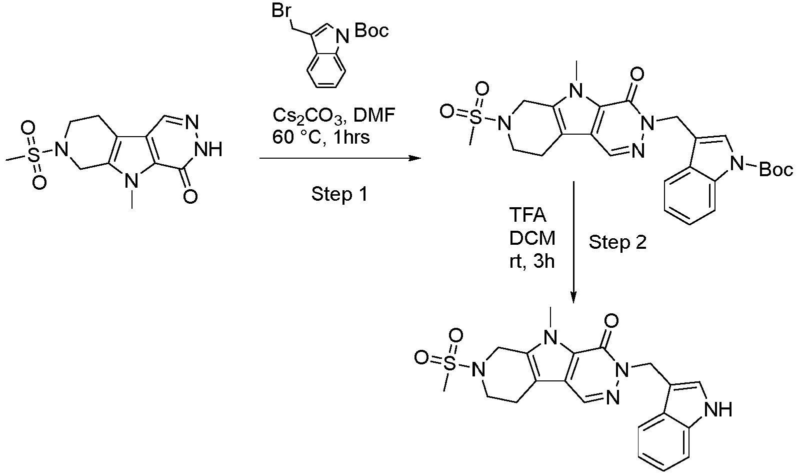

### Step 1

To the solution of 5-methyl-7-(methylsulfonyl)-3,5,6,7,8,9-hexahydro-4H-pyrido[4',3':4,5]pyrrolo[2,3-d] pyridazin-4-one (Intermediate 1, 73 mg, 0.23 mmol), Cs₂CO₃ (254 mg, 0.78 mmol) in DMF (5 mL) was added tert-butyl 3-(bromomethyl)-1H-indole-1-carboxylate (80 mg, 0.26 mmol) at 60 °C for 1 hours. After LCMS indicated the reaction completed, the reaction mixture was filtered and extracted with DCM (5 mL×3). The organic layer was washed with brine, dried over Na₂SO₄, filtered and concentrated at 30 °C under reduced pressure the residue was purified by column chromatography (12g, petroleum ether/ethyl acetate = 100:00~50:50) to give tert-butyl 3-((5-methyl-7-(methylsulfonyl)-4-oxo-4,5,6,7,8,9-hexahydro-3H-pyrido [4',3':4,5]pyrrolo[2,3-d]pyridazin-3-yl)methyl)-1H-indole-1-carboxylate (90 mg, 91% purity) as a yellow solid.

MS (ES⁺): 512.2 (M+H)⁺.

### Step 2

To the solution of tert-butyl 3-((5-methyl-7-(methylsulfonyl)-4-oxo-4,5,6,7,8,9-hexahydro-3H-pyrido[4',3':4,5] pyrrolo[2,3-d]pyridazin-3-yl)methyl)-1H-indole-1-carboxylate (90 mg, 0.18 mmol) in TFA (2 MI) and DCM (5 MI) at room temperature for 3 hours. After LCMS indicated the reaction completed, the mixture was adjusted to PH=8 with 1 N aq. NaOH. And extracted with DCM (10 MI×3), the organic layer was concentrated at 30 °C under reduced pressure. The residue was purified by prep-HPLC (Column: Waters X-SELECT C18 OBD 10µm 19*250mm; Flow Rate: 20 MI/min; solvent system: MeCN/(10 mmol/L NH₄HCO₃/water) gradient: MeCN: 40%~95%; collection wavelength: 214 nm). The fractions were concentrated at 42 °C under reduced pressure to remove MeCN, and the residue was lyophilized to give the title compound (5.11 mg, 95% Purity) as a white solid.

¹H NMR (400 MHz, DMSO-d₆) δ: 8.17 (s, 1H), 7.71(d, J= 8.0Hz, 1H), 7.35-7.31 (m, 2H), 7.04(t, J= 7.6Hz, 1H), 6.96(t, J= 7.6Hz, 1H), 5.42 (s, 2H), 4.45 (s, 2H), 4.03 (s, 3H), 3.48-3.44 (m, 2H), 2.98 (s, 3H), 2.79-2.75 (m, 2H).

MS (ES⁺): 412.3 (M+H)⁺.

### Example 16 - 3-(benzofuran-3-ylmethyl)-5-methyl-7-(methylsulfonyl)-3,5,6,7,8,9-hexahydro-4H-pyrido[4',3':4,5]pyrrolo[2,3-d]pyridazin-4-one

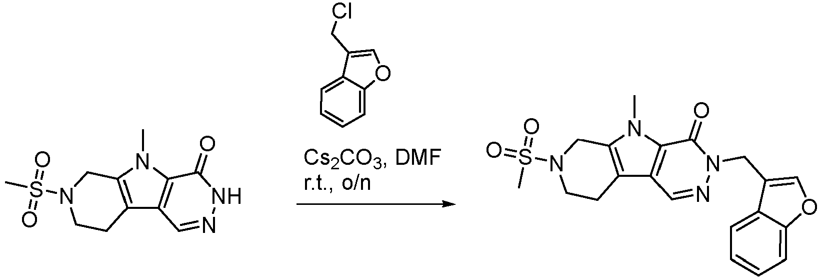

A solution of 3-(chloromethyl)benzofuran (Intermediate **5,** 1.08mmol) and Cs₂CO₃ (1.3 g, 4 mmol) in DMF (4 mL) was added 5-methyl-7-(methylsulfonyl)-3,5,6,7,8,9-hexahydro-4H-pyrido[4',3':4,5]pyrrolo[2,3-d]pyridazin-4-one (Intermediate 1, 90 mg, 0.32 mmol) at room temperature, and the reaction mixture under nitrogen protection was stirred at room temperature overnight. After LCMS indicated the reaction is completed, the reaction mixture was diluted with water (10 mL) and extracted with EtOAc (10 mL×3). The organic layer was concentrated at 40 °C under reduced pressure. The residue was purified by prep-HPLC (Column: Waters X-Bridge C18 OBD 10µm 19*250mm; Flow Rate: 20 mL/min; solvent system: MeCN/(10mmol/L NH₄HCO₃/water) gradient: MeCN: 40%~95%;collection wavelength: 214 nm). The fractions were concentrated at 42 °C under reduced pressure to remove MeCN, and the residue was lyophilized to give the title compound (84.59 mg, 99% Purity) as a white solid.

1H NMR (400 MHz, DMSO-d6) δ: 8.23 (s, 1H), 8.01 (s, 1H), 7.74-7.72 (m, 1H), 7.55 (d, J = 8.0 Hz, 1H), 7.31-7.27 (m, 1H), 7.25-7.21 (m, 1H), 5.42 (s, 2H), 4.47 (s, 2H), 4.03 (s, 3H), 3.47 (t, J = 6.0 Hz, 2H), 2.99 (s, 3H), 2.78 (t, J = 5.2 Hz, 2H).

MS (ES⁺): 413.3 (M+H)⁺.

### Example 17 - 3-((5-methyl-7-(methylsulfonyl)-4-oxo-4,5,6,7,8,9-hexahydro-3H-pyrido [4',3":4,5]pyrrolo[2,3-d]pyridazin-3-yl)methyl)-1H-pyrazole-4-carboxamide

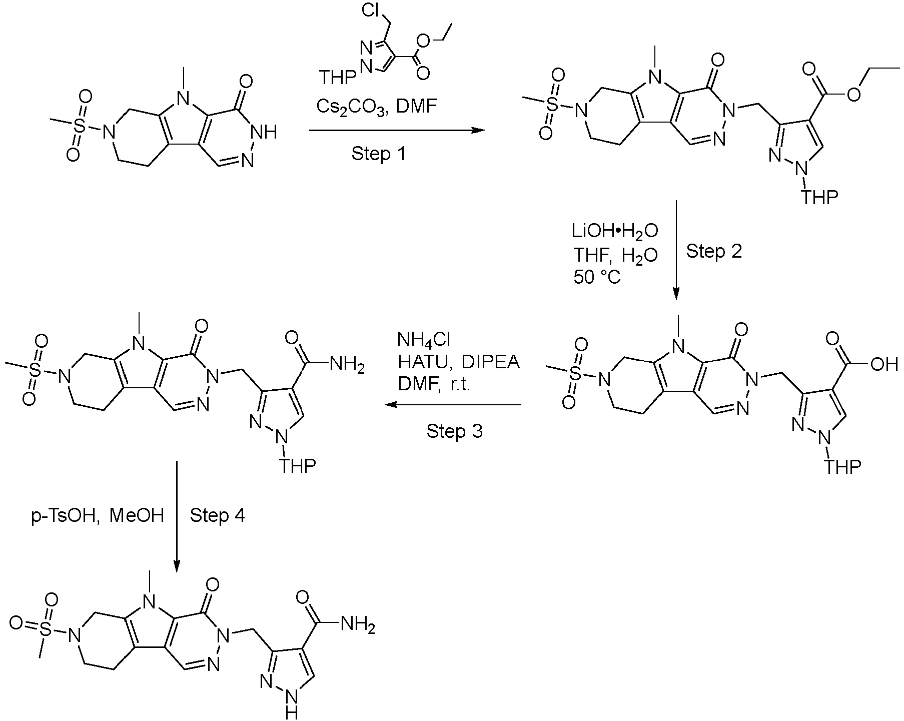

### Step 1

To the solution of 5-methyl-7-(methylsulfonyl)-3,5,6,7,8,9-hexahydro-4H-pyrido[4',3':4,5]pyrrolo[2,3-d] pyridazin-4-one (Intermediate 1, 282 mg, 1.0 mmol), Cs₂CO₃ (650 mg, 2.0 mmol) in DMF (10 mL) was added ethyl 3-(chloromethyl)-1-(tetrahydro-2H-pyran-2-yl)-1H-pyrazole-4-carboxylate (Intermediate **6,** 272 mg, 1.0 mmol) at 60 °C for 1 hours. After LCMS indicated the reaction was complete, the mixture was quenched with ice water (10 mL), and extracted with DCM (10 mL×2). The organic layer was washed with brine, dried over Na₂SO₄, filtered and concentrated at 30 °C under reduced pressure; the residue was purified by flash column chromatography (12g, dichloromethane/methyl alcohol = 100:00-90:10) to give ethyl 3-((5-methyl -7-(methylsulfonyl)-4-oxo-4,5,6,7,8,9-hexahydro-3H-pyrido[4',3':4,5]pyrrolo[2,3-d]pyridazin-3-yl)methyl)-1-(tetrahydro-2H-pyran-2-yl)-1H-pyrazole-4-carboxylate (180 mg, 89% Purity) as a yellow solid.

MS (ES⁺): 519.2 (M+H)⁺.

### Step 2

A mixture of ethyl 3-((5-methyl-7-(methylsulfonyl)-4-oxo-4,5,6,7,8,9-hexahydro-3H-pyrido[4',3':4,5]pyrrolo [2,3-d]pyridazin-3-yl)methyl)-1-(tetrahydro-2H-pyran-2-yl)-1H-pyrazole-4-carboxylate (180 mg, 0.35 mmol) and LiOH (26 mg, 1.05 mmol) in H₂O(5mL) and MeOH (5mL) was stirred at 50 °C overnight. After LCMS indicated the reaction completed, the mixture was quenched with 2 N HCl. The mixture was concentrated at 40 °C to remove organic solvent; the residue was separated and extracted with dichloromethane (10mL×3). The separated organics were washed with brine, dried over Na₂SO₄ and filtered. The filtrate was concentrated 40 °C under reduced pressure to give 3-((5-methyl-7-(methylsulfonyl)-4 -oxo-4,5,6,7,8,9-hexahydro-3H-pyrido[4',3':4,5]pyrrolo[2,3-d]pyridazin-3-yl)methyl)-1-(tetrahydro-2H-pyran-2-yl)-1H-pyrazole-4-carboxylic acid (140 mg, 93% Purity) as a yellow oil.

MS (ES⁺): 491.2 (M+H)⁺.

### Step 3

A mixture of 3-((5-methyl-7-(methylsulfonyl)-4-oxo-4,5,6,7,8,9-hexahydro-3H-pyrido[4',3':4,5]pyrrolo[2,3-d] pyridazin-3-yl)methyl)-1-(tetrahydro-2H-pyran-2-yl)-1H-pyrazole-4-carboxylic acid (140 mg, 0.29 mmol), NH₄Cl (62 mg, 1.16 mmol), HATU (220 mg, 0.58mmol), and DIPEA (112mg, 0.87mmol) in DMF (5mL) was stirred at room temperature for 16 hours. After LCMS indicated the reaction is completed, the mixture was quenched with ice water (10 mL), and extracted with DCM (10 mL×2). The organic layer was washed with brine, dried over Na₂SO₄, filtered and concentrated at 30 °C under reduced pressure the residue was purified by flash column chromatography (12g, DCM/MeOH= 100:00~90:10) to give 3-((5-methyl-7-(methylsulfonyl)-4-oxo-4,5,6,7,8,9-hexahydro-3H-pyrido[4',3':4,5]pyrrolo[2,3-d] pyridazin-3-yl)methyl)-1-(tetrahydro-2H-pyran-2-yl)-1H-pyrazole-4-carboxamide (90 mg, 95% Purity) as a yellow soild.

MS (ES⁺): 490.2 (M+H)⁺.

### Step 4

A mixture of 3-((5-methyl-7-(methylsulfonyl)-4-oxo-4,5,6,7,8,9-hexahydro-3H-pyrido[4',3':4,5]pyrrolo[2,3-d] pyridazin-3-yl)methyl)-1-(tetrahydro-2H-pyran-2-yl)-1H-pyrazole-4-carboxamide (90 mg, 0.18 mmol), p-TsOH (31 mg, 0.18 mmol) in MeOH (5 mL) was stirred at room temperature for 16 hours. After LCMS indicated the reaction completed. The mixture was concentrated at 30 °C under reduced pressure the residue was purified by prep-HPLC (Column: Waters X-SELECT C18 OBD 10µm 19*250mm; Flow Rate: 20 mL/min; solvent system: MeCN/(10mmol/L NH₄HCO₃/water) gradient: MeCN: 40%~95%; collection wavelength: 214 nm). The fractions were concentrated at 42 °C under reduced pressure to remove MeCN, and the residue was lyophilized to give the title compound (23.87mg, 98.7% Purity) as a white solid. ¹H NMR (400 MHz, DMSO-d₆) δ: 12.79-12.69 (m, 1H), 8.16 (s, 1H), 7.54 (s, 1H), 7.05 (s, 1H), 4.49(s, 2H), 4.01 (s, 3H), 3.50 (t, J=5.6 Hz, 2H), 3.01 (s, 3H), 2.83 (t, J=5.2 Hz, 2H).

MS (ES⁺): 406.1 (M+H)⁺.

### Example 18 - 3-((5-methyl-7-(methylsulfonyl)-4-oxo-4,5,6,7,8,9-hexahydro-3H-pyrido [4',3':4,5]pyrrolo[2,3-d]pyridazin-3-yl)methyl)benzenesulfonamide

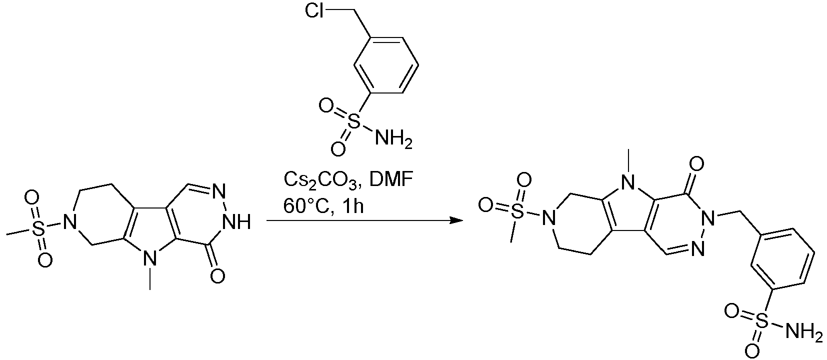

To the solution of 5-methyl-7-(methylsulfonyl)-3,5,6,7,8,9-hexahydro-4H-pyrido[4',3':4,5]pyrrolo[2,3-d] pyridazin-4-one **(Intermediate 1,** 160 mg, 0.57 mmol), Cs₂CO₃(557 mg, 1.7 mmol) in DMF (5 mL) was added 3-(chloromethyl)benzenesulfonamide (**Intermediate 7,** 117 mg, 0.57 mmol) at 60 °C for 1 hours. After LCMS indicated the reaction completed, the reaction mixture was filtered and extracted with DCM (10 mL×3), the organic layer was concentrated at 30 °C under reduced pressure. The residue was purified by prep-HPLC (Column: Waters X-SELECT C18 OBD 10µm 19*250mm; Flow Rate: 20 mL/min; solvent system: MeCN/(10 mmol/L NH₄HCO₃/water) gradient: MeCN: 40%~95%; collection wavelength: 214 nm). The fractions were concentrated at 42 °C under reduced pressure to remove MeCN, and the residue was lyophilized to give the title compound (5.07 mg, 95% Purity) as a white solid.

¹H NMR (400 MHz, DMSO-d₆) δ: 8.26 (s, 1H), 7.74-7.69(m, 2H), 7.55-7.50 (m, 2H), 7.36(s, 2H), 5.38 (s, 2H), 4.49 (s, 2H), 4.01 (s, 3H), 3.50 (t, *J=* 5.6 Hz, 2H), 3.01 (s, 3H), 2.82 (t, *J=* 5.2 Hz, 2H).

MS (ES⁺): 452.2 (M+H)⁺.

### Example 19 - 5-methyl-7-(methylsulfonyl)-3-((6-(pyrrolidin-1-yl)pyridin-2-yl)methyl)-3,5,6,7,8,9-hexahydro-4H-pyrido[4',3':4,5]pyrrolo[2,3-d]pyridazin-4-one

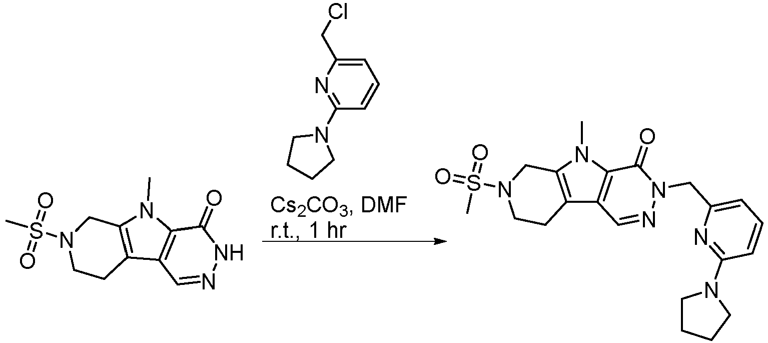

A solution of 2-(chloromethyl)-6-(pyrrolidin-1-yl)pyridine **(Intermediate** 8 95 mg, 0.48 mmol) and Cs₂CO₃(312 mg, 0.96 mmol) in DMF (3 mL) was added 5-methyl-7-(methylsulfonyl)-3,5,6,7,8,9-hexahydro-4H-pyrido[4',3':4,5]pyrrolo[2,3-d]pyridazin-4-one **(Intermediate** 1, 90 mg, 0.32 mmol) at room temperature, and the reaction mixture under nitrogen protection was stirred at room temperature for 1 hour. After LCMS indicated the reaction is completed, the reaction mixture was diluted with water (3 mL) and extracted with EtOAc (5 mL×4). The organic layer was concentrated at 40 °C under reduced pressure. The residue was purified by prep-HPLC (Column: SunFire Prep C18 OBD 10µm 19*250mm; Flow Rate: 20 mL/min; solvent system: MeCN/(0.2% formic/water) gradient: MeCN: 10%~95%; collection wavelength: 214 nm). The fractions were concentrated at 42 °C under reduced pressure to remove MeCN, and the residue was lyophilized to give the title compound (27.50 mg, 98% Purity) as a white solid.

¹H NMR (400 MHz, DMSO-d6) δ: 8.24 (s, 1H), 7.34 (d, *J =* 8.0 Hz, 1H), 6.27 (d, *J =* 8.4 Hz, 1H), 5.99 (d, *J* = 7.2 Hz, 1H), 5.21 (s, 2H), 4.49 (s, 2H), 4.01 (s, 3H), 3.51 (t, *J* = 5.6 Hz, 2H), 3.33-3.27 (m, 4H), 3.01 (s, 3H), 2.83 (t, *J =* 5.2 Hz, 2H), 1.93-1.89 (m, 4H).

¹H NMR (400 MHz, CDCl₃) δ: 8.06 (s, 1H), 7.37-7.29 (m, 1H), 6.22 (t, *J =* 7.2 Hz, 2H), 5.49-5.42 (m, 2H), 4.48 (s, 2H), 4.08 (s, 3H), 3.65 (t, *J =* 6.0 Hz, 2H), 3.41 (s, 4H), 2.92-2.89 (m, 5H), 1.97-1.71 (m, 4H).

MS (ES⁺): 443.2 (M+H)⁺.

### Example 20 - 3-((1H-indazol-3-yl)methyl)-5-methyl-7-(methylsulfonyl)-3,5,6,7,8,9-hexahydro-4H-pyrido[4',3':4,5]pyrrolo[2,3-d]pyridazin-4-one

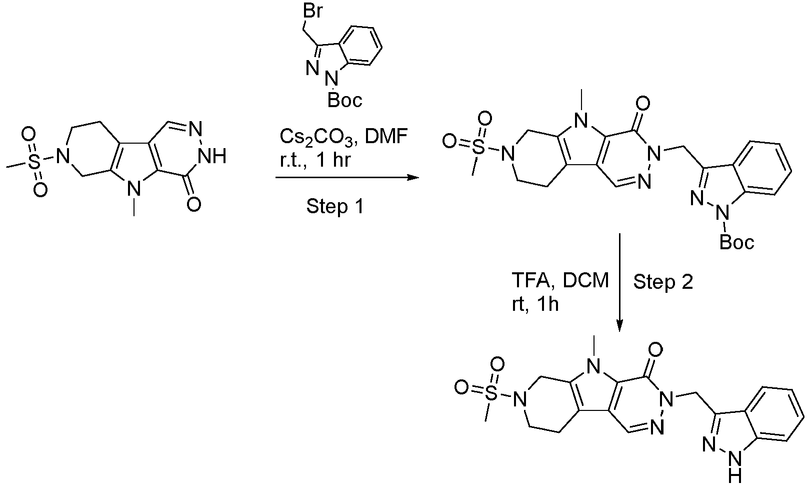

To a solution of 5-methyl-7-(methylsulfonyl)-3,5,6,7,8,9-hexahydro-4H-pyrido[4',3':4,5]pyrrolo[2,3-d]pyridazin-4-one **(Intermediate 1,** 100 mg, 0.35 mmol), tert-butyl 3-(bromomethyl)-1H-indazole-1-carboxylate **(Intermediate 9,** 130 mg, 0.42 mmol) in DMF (3 mL) was added Cs₂CO₃ (228 mg, 0.7 mmol) at room temperature, and the reaction mixture was stirred at 60 °C for 1 hour. After LCMS indicated the reaction is completed, the reaction mixture was filtered through Celite and washed with MeOH and DCM (1:3). The combined organic layer was concentrated at 45 °C under reduced pressure and extracted with EtOAc (20 mL×3). The combined organic layer was washed by brine, dried over Na₂SO₄, filtered and concentrated at 45 °C under reduced pressure. The residue was purified by flash column chromatography (12 g, dichloromethane/methyl alcohol = 100:0~90:10) to give tert-butyl3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)-1H-indazole-1-carboxylate (110 mg, 85% Purity) as a yellow solid.

MS (ES⁺): 513.2 (M+H)⁺.

### Step 2

To the solution of tert-butyl3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)-1H-indazole-1-carboxylate (110 mg, 0.2 mmol) in DCM (3 mL) was added TFA (1 mL) at room temperature, and the reaction mixture was stirred at room temperature for 1 hours. After LCMS indicated the reaction is completed, the reaction mixture was adjusted to pH to 8 with aq. K₂CO₃aq. and extracted with EtOAc (20 mL×3). The combined organic layer was washed by brine, dried over Na₂SO₄, filtered and concentrated at 40 °C under reduced pressure, and the residue was purified by prep-HPLC (Column: Waters X-Bridge C18 OBD 10µm 19*250mm; Flow Rate: 20 mL/min; solvent system: MeCN/(10mmol/L NH4HCO3/water) gradient: MeCN: 39%~95%; collection wavelength: 214 nm). The fractions were concentrated at 42 °C under reduced pressure to remove MeCN, and the residue was lyophilized to give the title compound (69.29 mg, 99% Purity) as a white solid.

1H NMR (400 MHz, DMSO-d6) δ: 12.89 (s, 1H), 8.20 (s, 1H), 7.67 (d, *J* = 8.0 Hz, 1H), 7.47 (d, *J* = 8.4 Hz 1H), 7.30 (t, *J* = 7.2 Hz, 1H), 7.03 (t, *J =* 7.6 Hz, 1H), 5.64 (s, 2H), 4.47 (s, 2H), 4.03 (s, 3H), 3.48 (t, *J* = 5.2 Hz, 2H), 3.00 (s, 3H), 2.79 (t, *J =* 5.2 Hz, 2H).

MS (ES⁺): 413.3 (M+H)⁺.

### Example 21 - 3-(2-fluoro-4-methoxybenzyl)-5-methyl-7-(phenylsulfonyl)-3,5,6,7,8,9-hexahydro-4H-pyrido[4',3':4,5]pyrrolo[2,3-d]pyridazin-4-one

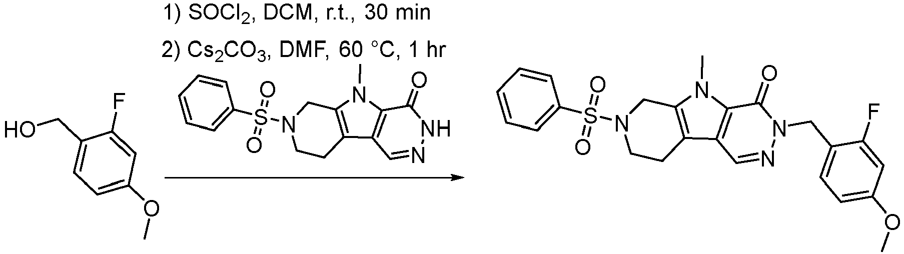

To a solution of (2-fluoro-4-methoxyphenyl)methanol (100 mg, 0.64 mmol) in DCM (4 mL) was added SOCl₂ (0.5 mL, 6.7 mmol) at room temperature, and the reaction mixture was stirred at room temperature for 30 minutes. After LCMS indicated the starting material had been consumed, the reaction was concentrated at 40 °C under reduced pressure to give the crude mixture as a yellow oil. To a mixture of the yellow oil and Cs₂CO₃ (624 mg, 1.92 mmol) in DMF (4 mL) was then added 5-methyl-7-(phenylsulfonyl)-3,5,6,7,8,9-hexahydro-4*H*-pyrido[4',3':4,5]pyrrolo[2,3-*d*]pyridazin-4-one **(Intermediate 3,** 110 mg, 0.32 mmol) at room temperature, and the reaction mixture was stirred at 60 °C for 1 hour. After LCMS indicated the reaction to be complete, the reaction mixture was diluted with water (10 mL) and extracted with EtOAc (10 mL × 4). The combined organic layer was concentrated at 40 °C under reduced pressure to give the residue. The residue was purified by prep-HPLC (Column: Waters X-Bridge shield Prep C18 OBD 10µm 19*250mm; Flow Rate: 20 mL/min; solvent system: (CH₃CN/CH₃OH=1/1)/(10mmol/L NH₄HCO₃/water) gradient: MeCN: 63%-95%; collection wavelength: 214 nm). The fractions were concentrated at 42 °C under reduced pressure to remove MeCN, and the residue was lyophilized to give the title compound (64.83 mg, 99% Purity) as a white solid.

¹H NMR (400 MHz, DMSO-d6) δ: 8.14 (s, 1H), 7.89-7.87 (m, 2H), 7.69-7.67 (m, 1H), 7.64-7.60 (m, 2H), 7.08 (t, *J =* 8.4 Hz, 1H), 6.80 (dd, *J* = 12.4 Hz, 2.8 Hz, 1H), 6.70 (dd, *J* = 8.8 Hz, 2.4 Hz, 1H), 5.25 (s, 2H), 4.38 (s, 2H), 3.99 (s, 3H), 3.73 (s, 3H), 3.38 (t, *J* = 5.6 Hz, 2H), 2.71-2.67 (m, 2H).

MS (ES⁺): 483.3 (M+H)⁺.

### Example 22 - 3-((7-((4-methoxyphenyl)sulfonyl)-5-methyl-4-oxo-4,5,6,7,8,9-hexahydro-3H-pyrido[4',3':4,5]pyrrolo[2,3-d]pyridazin-3-yl)methyl)thiophene-2-carboxamide

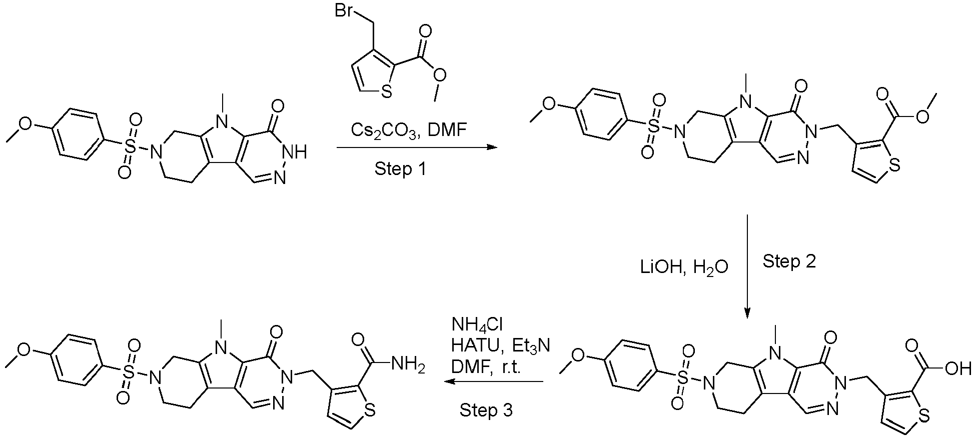

### Step 1

To the solution of 7-((4-methoxyphenyl)sulfonyl)-5-methyl-3,5,6,7,8,9-hexahydro-4H-pyrido[4',3':4,5]pyrrolo[2,3-d]pyridazin-4-one (**Intermediate 11, 141** mg, 0.5 mmol), Cs₂CO₃ (325 mg, 1.0 mmol) in DMF (5 mL) was added ethyl 3-(chloromethyl)-1-(tetrahydro-2H-pyran-2-yl)-1H-pyrazole-4-carboxylate (**Intermediate 12,** 217 mg, 0.5 mmol) at 60 °C for 1 hours. After LCMS indicated the reaction is completed, the mixture was quenched with ice water (10 mL), and extracted with DCM (10 mL×2). The organic layer was washed with brine, dried over Na₂SO₄, filtered and concentrated at 30 °C under reduced pressure the residue was purified by flash column chromatography (12g, dichloromethane/methyl alcohol = 100:00~90:10) to give methyl 3-((7-((4-methoxyphenyl)sulfonyl)-5-methyl-4-oxo-4,5,6,7,8,9-hexahydro-3H-pyrido[4',3':4,5]pyrrolo[2,3-d]pyridazin-3-yl)methyl)thiophene-2-carboxylate (120 mg, 77.5% Purity) as a yellow solid.

MS (ES⁺): 529.2 (M+H)⁺.

### Step 2

A mixture of methyl 3-((7-((4-methoxyphenyl)sulfonyl)-5-methyl-4-oxo-4,5,6,7,8,9-hexahydro-3H-pyrido [4',3':4,5]pyrrolo[2,3-d]pyridazin-3-yl)methyl)thiophene-2-carboxylate (120 mg, 0.23 mmol) and LiOH (26 mg, 1.05 mmol) in H₂O(5mL) and MeOH (5mL) was stirred at 40 °C overnight. After LCMS indicated the reaction completed, the mixture was quenched with 2 N HCl. The mixture was concentrated at 40 °C to remove organic solvent; the residue was separated and extracted with ethyl acetate (10mL×3). The separated organics were washed with brine, dried over Na₂SO₄ and filtered. The filtrate was concentrated 40 °C under reduced pressure to give 3-((7-((4-methoxyphenyl)sulfonyl)-5-methyl-4-oxo-4,5,6,7,8,9 -hexahydro-3H-pyrido[4',3':4,5]pyrrolo[2,3-d]pyridazin-3-yl)methyl)thiophene-2-carboxylic acid (100 mg, 89.5% Purity) as a yellow solid. MS (ES⁺): 515.2 (M+H)⁺.

### Step 3

A mixture of 3-((7-((4-methoxyphenyl)sulfonyl)-5-methyl-4-oxo-4,5,6,7,8,9-hexahydro-3H-pyrido[4',3':4,5] pyrrolo[2,3-d]pyridazin-3-yl)methyl)thiophene-2-carboxylic acid (100 mg, 0.19 mmol), NH₄Cl (62 mg, 1.16 mmol), HATU (220 mg, 0.58mmol), and DIPEA (112mg, 0.87mmol) in DMF (5mL) was stirred at room temperature for 16 hours. After LCMS indicated the reaction is completed, the mixture was quenched with ice water (10 mL) and extracted with DCM (10 mL×2). The organic layer was washed with brine, dried over Na₂SO₄, filtered, and concentrated at 30 °C under reduced pressure the residue was purified by prep-HPLC (Column: Waters X-SELECT C18 OBD 10µm 19*250mm; Flow Rate: 20 mL/min; solvent system: MeCN/(10mmol/L NH₄HCO₃/water) gradient: MeCN: 40%~95%; collection wavelength: 214 nm). The fractions were concentrated at 42 °C under reduced pressure to remove MeCN, and the residue was lyophilized to afford the title compound (39.34 mg, 99% Purity) as a white solid.

¹H NMR (400 MHz, DMSO-d₆) δ: 18.23 (s, 1H), 8.21-8.18(m, 1H), 7.81 (d, *J*= 8.8 Hz, 2H), 7.63-7.60(m, 1H), 7.55(d, *J*= 8.8 Hz, 1H), 7.12 (d, *J*= 9.2 Hz, 2H), 6.71 (d, *J*= 5.2 Hz, 1H), 5.50 (s, 2H), 4.33 (s, 2H) , 3.98 (s, 3H), 3.82 (s, 3H), 3.34-3.29 (m, 2H), 2.73-7.67 (m, 2H).

MS (ES⁺): 414.2 (M+H)⁺.

### Example 23 - 3-((2,3-dihydrobenzofuran-5-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-one

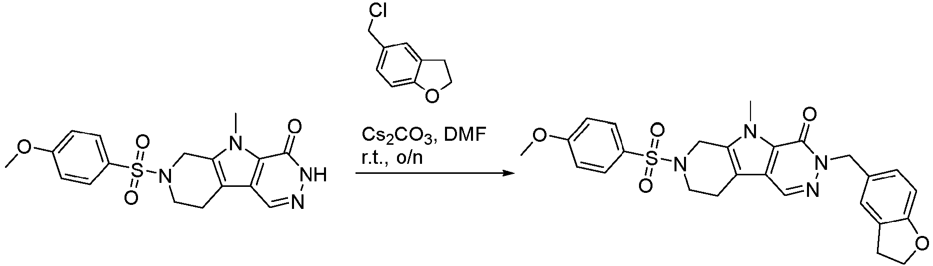

5-(chloromethyl)-2,3-dihydrobenzofuran **(Intermediate 13,** 1.4mmol) and Cs₂CO₃ (1.82 g, 5.6 mmol) in DMF (4 mL) was added 7-((4-methoxyphenyl)sulfonyl)-5-methyl-3,5,6,7,8,9-hexahydro-4H-pyrido[4',3':4,5]pyrrolo[2,3-d]pyridazin-4-one (**Intermediate 11,** 112 mg, 0.30 mmol) at room temperature, and the reaction mixture under nitrogen protection was stirred at room temperature overnight. After LCMS indicated the reaction is completed, the reaction mixture was diluted with water (15 mL) and extracted with EtOAc (20 mL×3). The organic layer was concentrated at 40 °C under reduced pressure. The residue was purified by prep-HPLC (Column: Waters X-Bridge C18 OBD 10µm 19*250mm; Flow Rate: 20 mL/min; solvent system: MeCN/(10mmol/L NH₄HCO₃/water) gradient: MeCN: 50%-95%; collection wavelength: 214 nm). The fractions were concentrated at 42 °C under reduced pressure to remove MeCN, and the residue was lyophilized to give the title compound (65.31 mg, 99% Purity) as a white solid.

¹H NMR (400 MHz, DMSO-d6) δ: 8.14 (s, 1H), 7.83-7.79 (m, 2H), 7.13-7.09 (m, 3H), 7.02 (d, *J* = 8.4 Hz, 1H), 6.66 (d, *J* = 8.4 Hz, 1H), 5.17 (s, 2H), 4.46 (s, *J =* 8.8 Hz, 2H), 4.31 (s, 2H), 3.99 (s, 3H), 3.81 (s, 3H), 3.33-3.32 (m, 2H), 3.10 (t, *J =* 8.8 Hz, 2H), 2.72-2.67 (m, 2H).
MS (ES⁺): 507.2 (M+H)⁺

### Example 24 - 3-((2,2-dimethyl-2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-one

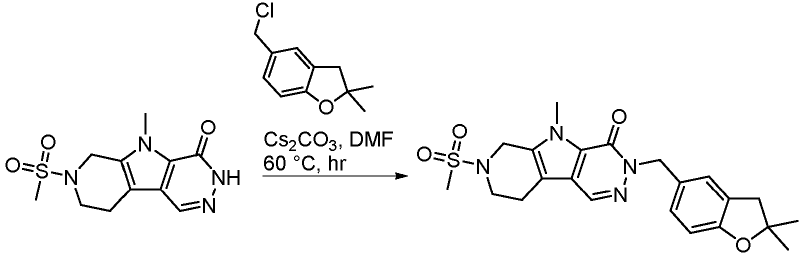

To a solution of the crude 5-(chloromethyl)-2,2-dimethyl-2,3-dihydrobenzofuran **(Intermediate 14,** 0.59 mmol) and Cs₂CO₃ (780 mg, 2.4 mmol) in DMF (3 mL) was added 5-methyl-7-(methylsulfonyl)-3,5,6,7,8,9-hexahydro-4H-pyrido[4',3':4,5]pyrrolo[2,3-d]pyridazin-4-one **(Intermediate 1, 90** mg, 0.32 mmol) at room temperature, and the reaction mixture under nitrogen protection was stirred at room temperature overnight. After LCMS indicated the reaction is completed, the reaction mixture was diluted with water (10 mL) and extracted with EtOAc (10 mL×3). The organic layer was concentrated at 40 °C under reduced pressure. The residue was purified by prep-HPLC (Column: Waters X-Bridge shield Prep C18 OBD 10µm 19*250mm; Flow Rate: 20 mL/min; solvent system: (CH₃CN/CH₃OH=1/1)/(10mmol/L NH₄HCO₃/water) gradient: MeCN: 60%~95%; collection wavelength: 214 nm). The fractions were concentrated at 42 °C under reduced pressure to remove MeCN, and the residue was lyophilized to give the title compound (30.95 mg, 99% Purity) as a white solid.

¹H NMR (400 MHz, DMSO-d6) δ: 8.21 (s, 1H), 7.11 (s, 1H), 7.03 (d, *J* = 8.0 Hz, 1H), 6.60 (d, *J* = 8.0 Hz, 1H), 5.19 (s, 2H), 4.47 (s, 2H), 4.01 (s, 3H), 3.49 (t, *J* = 5.6 Hz, 2H), 3.00 (s, 3H), 2.94 (s, 2H), 2.80 (t, *J* = 5.6 Hz, 2H), 1.36 (s, 6H).

MS (ES⁺): 443.3 (M+H)⁺.

### Example 25 - 3-((1H-pyrrolo[2,3-b]pyridin-6-yl)methyl)-5-methyl-7-(methylsulfonyl)-3,5,6,7,8,9-hexahydro-4H-pyrido[4',3':4,5]pyrrolo[2,3-d]pyridazin-4-one

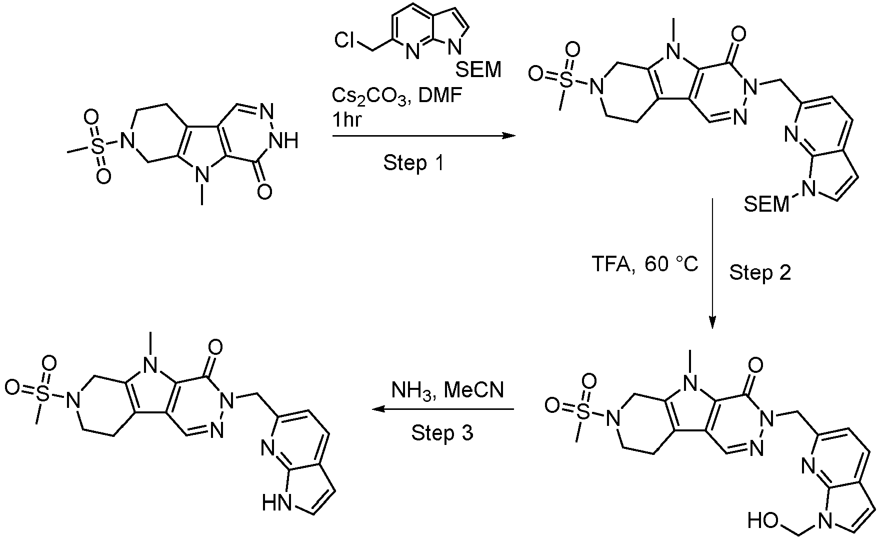

### Step 1

To a solution of 5-methyl-7-(methylsulfonyl)-3,5,6,7,8,9-hexahydro-4H-pyrido[4',3':4,5]pyrrolo[2,3-d]pyridazin-4-one **(Intermediate 1,** 150mg, 0.53mmol), 6-(chloromethyl)-1-((2-(trimethylsilyl)ethoxy)methyl)-1H-pyrrolo[2,3-b]pyridine (165 mg, 0.6 mmol) in DMF (3 mL) was added Cs₂CO₃ (326 mg, 1 mmol) at room temperature, and the reaction mixture was stirred at 60 °C for 1 hour. After LCMS indicated the reaction is completed, the reaction mixture was filtered through Celite and washed with MeOH and DCM (1:3). The combined organic layer was concentrated at 45 °C under reduced pressure and extracted with EtOAc (20 mL×3). The combined organic layer was washed by brine, dried over Na₂SO₄, filtered and concentrated at 45 °C under reduced pressure. The residue was purified by flash column chromatography (12 g, dichloromethane/methyl alcohol = 100:0~90:10) to give 5-methyl-7-(methylsulfonyl)-3-((1-((2-(trimethylsilyl)ethoxy)methyl)-1H-pyrrolo[2,3-b]pyridin-6-yl)methyl)-3,5,6,7,8,9-hexahydro-4H-pyrido[4',3':4,5]pyrrolo[2,3-d]pyridazin-4-one (160 mg, 89% Purity) as a yellow solid.

MS (ES⁺): 543.2 (M+H)⁺.

### Step 2

To a solution of 5-methyl-7-(methylsulfonyl)-3-((1-((2-(trimethylsilyl)ethoxy)methyl)-1H-pyrrolo[2,3-b]pyridin-6-yl)methyl)-3,5,6,7,8,9-hexahydro-4H-pyrido[4',3':4,5]pyrrolo[2,3-d]pyridazin-4-one (160 mg, 0.3 mmol) in TFA (5 mL) at room temperature, and the reaction mixture was stirred at 60 °C for 2 hours. After LCMS indicated the reaction is completed, the reaction mixture was concentrated at 45 °C under reduced pressure to give 3-((1-(hydroxymethyl)-1H-pyrrolo[2,3-b]pyridin-6-yl)methyl)-5-methyl-7-(methylsulfonyl)-3,5,6,7,8,9-hexahydro-4H-pyrido[4',3':4,5]pyrrolo[2,3-d]pyridazin-4-one as a yellow solid, which was used to the next step directly without further purification.

MS (ES⁺): 443.3 (M+H)⁺.

### Step 3

To a solution of 3-((1-(hydroxymethyl)-1H-pyrrolo[2,3-b]pyridin-6-yl)methyl)-5-methyl-7-(methylsulfonyl)-3,5,6,7,8,9-hexahydro-4H-pyrido[4',3':4,5]pyrrolo[2,3-d]pyridazin-4-one (in MeCN (5 mL) was added NH₃H₂O (5 mL, 25-28 wt%) at room temperature, and the reaction mixture was stirred at room temperature for 2 hours. After LCMS indicated the reaction is completed, the reaction was concentrated at 40 °C under reduced pressure, and the residue was purified by prep-HPLC (Column: Waters X-Bridge C18 OBD 10µm 19*250mm; Flow Rate: 20 mL/min; solvent system: MeCN/(10mmol/L NH4HCO3/water) gradient: MeCN: 38%-95%; collection wavelength: 214 nm). The fractions were concentrated at 42 °C under reduced pressure to remove MeCN, and the residue was lyophilized to the title compound (66.28 mg, 99% Purity) as a white solid.

1H NMR (400 MHz, DMSO-d6) δ: 11.60 (br, 1H), 8.23 (s, 1H), 7.87 (d, *J* = 8.0 Hz, 1H), 7.40 (t, *J* = 3.2 Hz, 1H), 6.84 (d, *J =* 8.0 Hz, 1H), 6.39 (t, *J* = 2.8 Hz, 1H), 5.47 (s, 2H), 4.50 (s, 2H), 4.01 (s, 3H), 3.51 (t, *J* = 5.2 Hz, 2H), 3.02 (s, 3H), 2.83 (t, *J =* 5.6 Hz, 2H).

MS (ES⁺): 413.3 (M+H)⁺.

### Example 26 - (S)-3-(3-(1-aminoethyl)benzyl)-5-methyl-7-(methylsulfonyl)-3,5,6,7,8,9-hexahydro-4H-pyrido[4',3':4,5]pyrrolo[2,3-d]pyridazin-4-one

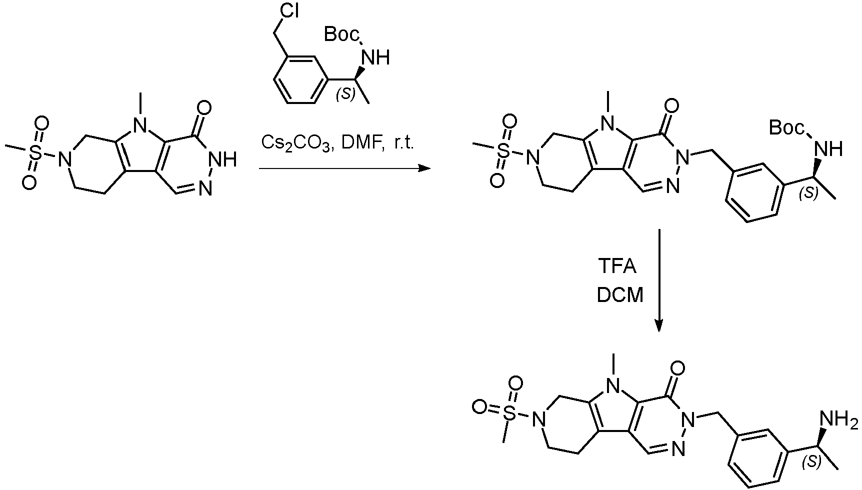

### Step 1

To a solution of 5-methyl-7-(methylsulfonyl)-3,5,6,7,8,9-hexahydro-4H-pyrido[4',3':4,5]pyrrolo[2,3-d]pyridazin-4-one **(Intermediate 1,** 150mg, 0.53mmol), (S)-tert-butyl 1-(3-(chloromethyl)phenyl)ethylcarbamate (**Intermediate 16,** 240 mg, 0.93 mmol) in DMF (3 mL) was added Cs₂CO₃ (326 mg, 1 mmol) at room temperature, and the reaction mixture was stirred at 60 °C for 1 hour. After LCMS indicated the reaction is completed, the reaction mixture was filtered through Celite and washed with MeOH and DCM (1:3). The combined organic layer was concentrated at 45 °C under reduced pressure and extracted with EtOAc (10 mL×3). The combined organic layer was washed by brine, dried over Na₂SO₄, filtered and concentrated at 45 °C under reduced pressure. The residue was purified by flash column chromatography (12 g, dichloromethane/methyl alcohol = 100:0~90:10) to give tert-butyl(S)-(1-(3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)phenyl)ethyl)carbamate (130 mg, 78% Purity) as a yellow solid.

MS (ES⁺): 460.4 (M+H)⁺.

### Step 2

To a solution of tert-butyl(S)-(1-(3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)phenyl)ethyl)carbamate (130 mg, 0.25 mmol) in DCM (5 mL) at room temperature, and the reaction mixture was stirred at room temperature for 1 hour1. After LCMS indicated the reaction is completed, the reaction was concentrated at 40 °C under reduced pressure. The residue was quenched with ice water (10 mL), adjusted to pH = 9 with 2N K₂CO₃ aqueous solution, and the mixture was extracted with EtOAc (10 mL×3). The combined organic layer was washed by brine, dried over Na₂SO₄, filtered and concentrated at 45 °C under reduced pressure. The residue was purified by prep-HPLC (Column: Waters X-Bridge C18 OBD 10µm 19*250mm; Flow Rate: 20 mL/min; solvent system: MeCN/(10mmol/L NH4HCO3/water) gradient: MeCN: 38%-95%; collection wavelength: 214 nm). The fractions were concentrated at 42 °C under reduced pressure to remove MeCN, and the residue was lyophilized to give the title compound (49.88 mg, 99% Purity) as a white solid.

1H NMR (400 MHz, DMSO-d6) δ: 8.23 (s, 1H), 7.29-7.19 (m, 3H), 7.02 (d, *J =* 7.6 Hz, 1H), 5.29 (s, 2H), 4.48 (s, 2H), 4.01 (s, 3H), 3.93 (q, *J* = 6.4 Hz, 1H), 3.49 (t, *J* = 6.0 Hz, 2H), 3.01 (s, 3H), 2.81 (t, *J* = 5.2 Hz, 2H), 1.92 (br, 2H), 1.20 (d, *J* = 6.8 Hz, 3H).

MS (ES⁺): 416.3 (M+H)⁺.

### Example 27 - 7-((2-methoxyethyl)sulfonyl)-3-((6-methoxypyridin-3-yl)methyl)-5-methyl-3,5,6,7,8,9-hexahydro-4H-pyrido[4',3':4,5]pyrrolo[2,3-d]pyridazin-4-one

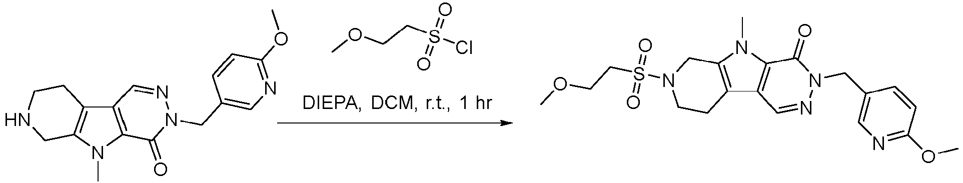

A suspension of 3-((6-methoxypyridin-3-yl)methyl)-5-methyl-3,5,6,7,8,9-hexahydro-4H-pyrido[4',3':4,5]pyrrolo[2,3-d]pyridazin-4-one (**Intermediate 21,** 160 mg, 0.49 mmol) and DIPEA (322 mg, 2.5 mmol) in DCM (5 mL) was added 2-methoxyethanesulfonyl chloride (93 mg, 0.59 mmol) and the reaction mixture was stirred at room temperature for 1 hour. After LCMS indicated the reaction completed, the mixture was diluted with H₂O (5 mL) and extracted with DCM (10 mL × 3). The combined organic layers were concentrated at 40 °C under reduced pressure to give the residue. The residue was purified by flash column chromatography (40 g, dichloromethane/methanol = 100:00-90:10) to give the crude product. The crude product was purified by prep-HPLC (Column: Waters Xbridge shield Prep C18 OBD 10µm 19*250mm; Flow Rate: 20 mL/min; solvent system: CH₃CN/(10mmol/L NH₄HCO₃/water) gradient: MeCN: 40%-95%; collection wavelength: 214 nm). The fractions were concentrated at 42 °C under reduced pressure to remove MeCN, and the residue was lyophilized to give the title compound (87.69 mg, 99% Purity) as a white solid.

¹H NMR (400 MHz, DMSO-d6) δ: 8.22 (s, 1H), 8.15 (d, *J* = 2.0 Hz, 1H), 7.63 (dd, J= 8.4 Hz, 2.4 Hz, 1H), 6.76 (d, *J* = 8.4 Hz, 1H), 5.25 (s, 2H), 4.48 (s, 2H), 4.00 (s, 3H), 3.81 (s, 3H), 3.67 (t, *J* = 6.0 Hz, 2H), 3.51 (t, *J =* 5.6 Hz, 2H) , 3.46 (t, *J* = 6.0 Hz, 2H), 3.23 (s, 3H), 2.78 (d, *J* = 5.2 Hz, 2H).

MS (ES⁺): 448.3 (M+H)⁺.

### Example 28 - N-(2-methoxyethyl)-3-((6-methoxypyridin-3-yl)methyl)-5-methyl-4-oxo-3,4,5,6,8,9-hexahydro-7H-pyrido[4',3':4,5]pyrrolo[2,3-d]pyridazine-7-sulfonamide

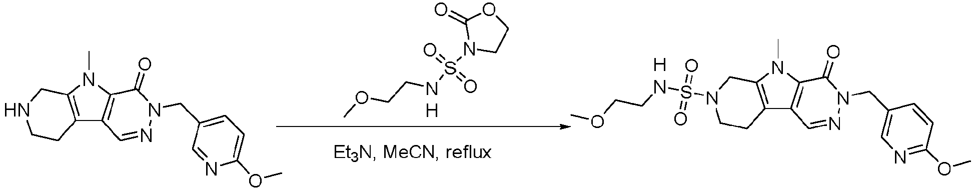

To a solution of 3-((6-methoxypyridin-3-yl)methyl)-5-methyl-3,5,6,7,8,9-hexahydro-4H-pyrido[4',3':4,5]pyrrolo[2,3-d]pyridazin-4-one (**Intermediate 21,** 200 mg, 0.6 mmol), N-(2-methoxyethyl)-2-oxooxazolidine-3-sulfonamide **(Intermediate 22,** 179.2 mg, 0.8 mmol) in MeCN (10 mL) was added Et₃N (182 mg, 1.8 mmol) at room temperature, and the reaction mixture was stirred at 90 °C for overnight. After LCMS indicated the reaction is completed, the reaction mixture was concentrated at 45 °C under reduced pressure and extracted with EtOAc (10 mL×3). The combined organic layer was washed by brine, dried over Na₂SO₄, filtered, and concentrated at 45 °C under reduced pressure. The residue was purified by prep-HPLC (Column: Waters X-Bridge C18 OBD 10µm 19*250mm; Flow Rate: 20 mL/min; solvent system: MeCN/(10mmol/L NH4HCO3/water) gradient: MeCN: 38%-95%; collection wavelength: 214 nm). The fractions were concentrated at 42 °C under reduced pressure to remove MeCN, and the residue was lyophilized to give the title compound (52.03 mg, 99% Purity) as a white solid.

1H NMR (400 MHz, DMSO-d6) δ: 8.22 (s, 1H), 8.15 (d, *J* = 2.0 Hz, 1H), 7.63 (dd, *J* = 8.8, 2.4 Hz, 1H), 6.76 (d, *J* = 8.8 Hz, 1H), 5.25 (s, 2H), 4.36 (s, 2H), 4.00 (s, 3H), 3.81 (s, 3H), 3.42 (t, *J* = 5.6 Hz, 2H), 3.36 (t, *J* = 5.6 Hz, 2H), 3.21 (s, 3H), 3.04 (t, *J =* 5.6 Hz, 2H), 2.76 (t, *J* = 5.2 Hz, 2H). MS (ES⁺): 463.3 (M+H)⁺.

### Example 29 - 3-((1H-pyrrolo[2,3-c]pyridin-3-yl)methyl)-7-((2-methoxyethyl)sulfonyl)-5-methyl-6,7,8,9-tetrahydro-3H-pyrido[4',3':4,5]pyrrolo[2,3-d]pyridazin-4(5H)-one

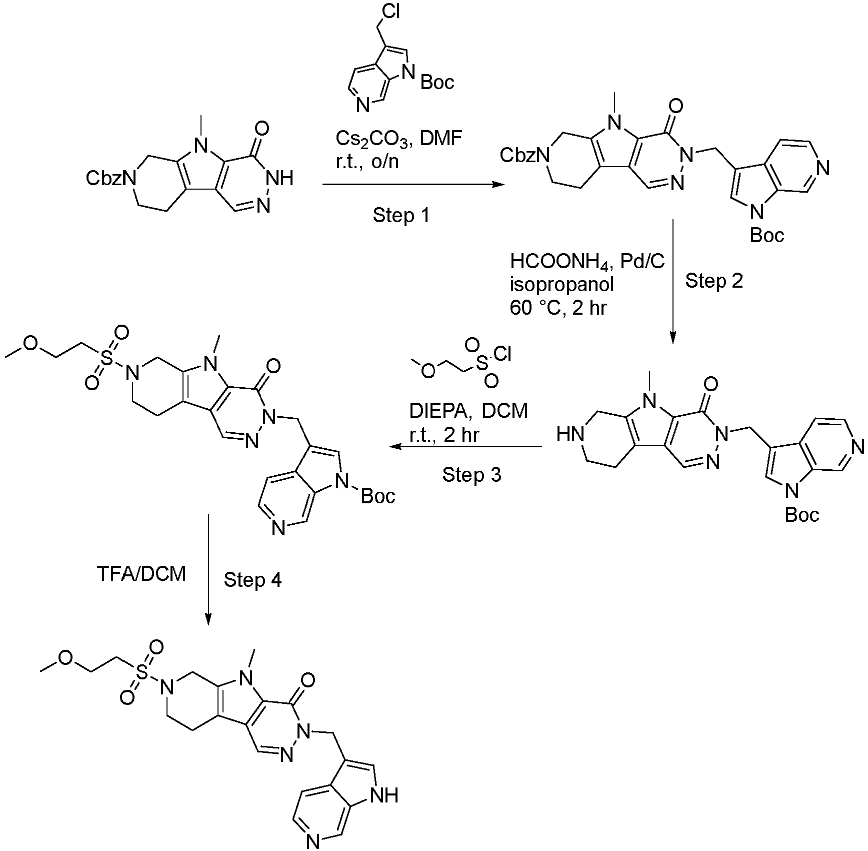

### Step 1

A mixture tert-butyl 3-(chloromethyl)-1H-pyrrolo[2,3-c]pyridine-1-carboxylate **(Intermediate 19,** 1.5mmol), Cs₂CO₃ (6.14 g, 18.9 mmol) and 5-methyl-7-(methylsulfonyl)-3,5,6,7,8,9-hexahydro-4*H*-pyrido[4',3':4,5]pyrrolo[2,3-d]pyridazin-4-one (**Intermediate 20,** 500 mg, 1.48 mmol) in DMF (10 mL) under nitrogen protection was stirred at room temperature overnight. After LCMS indicated the reaction is completed, the reaction mixture was diluted with water (20 mL) and extracted with EtOAc (30 mL × 4). The organic layers were washed with saturated NH₄Cl aqueous solution (40 mL × 2), concentrated at 40 °C under reduced pressure and purified by flash column chromatography (12 g, petroleum ether/ethyl acetate = 100:00~20:80) to give benzyl 3-((1-(*tert-*butoxycarbonyl)-1*H*-pyrrolo[2,3-*c*]pyridin-3-yl)methyl)-5-methyl-4-oxo-5,6,8,9-tetrahydro-3*H-*pyrido[4',3':4,5]pyrrolo[2,3-*d*]pyridazine-7(4*H*)-carboxylate (600 mg, 80% Purity) as a yellow solid. MS (ES⁺): 569.3 (M+H)⁺.

### Step 2

A mixture of benzyl 3-((1-(*tert*-butoxycarbonyl)-1*H*-pyrrolo[2,3-*c*]pyridin-3-yl)methyl)-5-methyl-4-oxo-5,6,8,9-tetrahydro-3*H*-pyrido[4',3':4,5]pyrrolo[2,3-*d*]pyridazine-7(4*H*)-carboxylate (600 mg, 1.06 mmol), ammonium formate (668 mg, 10.6 mmol) and Pd/C (10% wt, 120 mg) in isopropyl alcohol (5 mL) under nitrogen protection was stirred at 60 °C for 2 hours. After LCMS indicated the reaction is completed, the reaction mixture was filtered, washed Pd/C with DCM (80 mL) and the filtrate was concentrated at 40 °C under reduced pressure to remove isopropanol. The residue was diluted with water (10 mL), extracted with EtOAc (20 mL × 4) and concentrated at 40 °C under reduced pressure to give *tert*-butyl 3-((5-methyl-4-oxo-4,5,6,7,8,9-hexahydro-3*H-*pyrido[4',3':4,5]pyrrolo[2,3-*d*]pyridazin-3-yl)methyl)-1*H*-pyrrolo[2,3-c]pyridine-1-carboxylate (420 mg, 88% Purity) as a white solid. The crude was used in next step directly.

MS (ES⁺): 435.3 (M+H)⁺.

### Step 3

A suspension of *tert*-butyl 3-((5-methyl-4-oxo-4,5,6,7,8,9-hexahydro-3*H-*pyrido[4',3':4,5]pyrrolo[2,3-*d*]pyridazin-3-yl)methyl)-1*H*-pyrrolo[2,3-c]pyridine-1-carboxylate (420 mg, 0.97 mmol) and DIPEA (774 mg, 6.0 mmol) in DCM (5 mL) was added 2-methoxyethanesulfonyl chloride (184 mg, 1.16 mmol) and the reaction mixture was stirred at room temperature for 2 hours. After LCMS indicated the reaction completed, the mixture was diluted with H₂O (10 mL) and extracted with DCM (20 mL × 3). The combined organic layers were concentrated at 40 °C under reduced pressure and purified by flash column chromatography (12 g, dichloromethane/methanol = 100:00~96:4) to give *tert*-butyl 3-((7-((2-methoxyethyl)sulfonyl)-5-methyl-4-oxo-4,5,6,7,8,9-hexahydro-3*H*-pyrido[4',3':4,5]pyrrolo[2,3-*d*]pyridazin-3-yl)methyl)-1*H-*pyrrolo[2,3-*c*]pyridine-1-carboxylate (270 mg, 78% Purity) as a white solid.

MS (ES⁺): 557.3 (M+H)⁺.

### Step 4

To a solution of *tert*-butyl 3-((7-((2-methoxyethyl)sulfonyl)-5-methyl-4-oxo-4,5,6,7,8,9-hexahydro-3*H*-pyrido[4',3':4,5]pyrrolo[2,3-*d*]pyridazin-3-yl)methyl)-1*H*-pyrrolo[2,3-*c*]pyridine-1-carboxylate (270 mg, 0.48 mol) in DCM (3 mL) was added TFA (3 mL) and the mixture was stirred at room temperature for 1 hour. After LCMS indicated the reaction completed, the mixture was concentrated under reduced pressure. The residue was diluted with H₂O (15 mL), adjusted pH to 8 with K₂CO₃ and filtered. The filtered cake was purified by prep-HPLC (Column: Waters Xbridge Prep C18 OBD 10µm 19*250mm; Flow Rate: 20 mL/min; solvent system: MeCN/(10 mmol/L NH₄HCO₃/water) gradient: MeCN: 18%~95%; collection wavelength: 214 nm). The fractions were concentrated at 40 °C under reduced pressure to remove MeCN, and the residue was lyophilized to give the title compound (82.17 mg, 99% Purity) as a white solid.

¹H NMR (400 MHz, DMSO-d6) δ: 11.55 (br, 1H), 8.70 (s, 1H), 8.19 (s, 1H), 8.06 (d, *J* = 5.6 Hz, 1H), 7.66 (d, *J* = 5.6 Hz, 1H), 7.64 (s, 1H), 5.43 (s, 2H), 4.46 (s, 2H), 4.02 (s, 3H), 3.66 (t, *J* = 6.4 Hz, 2H), 3.49 (t, *J* = 5.6 Hz, 2H), 3.44 (t, *J* = 6.0 Hz, 2H), 3.22 (s, 3H), 2.74 (t, *J* = 5.6 Hz, 2H).

¹H NMR (400 MHz, CDCl₃) δ: 8.88 (s, 1H), 8.20 (d, *J* = 5.6 Hz, 1H), 8.02 (s, 1H), 7.92 (d, *J* = 5.6 Hz, 1H), 7.68 (s, 1H), 5.56 (s, 2H), 4.45 (s, 2H), 4.05 (s, 3H), 3.76 (t, *J* = 5.6 Hz, 2H), 3.59 (t, *J =* 5.6 Hz, 2H), 3.32 (s, 3H), 3.30 (t, *J* = 5.6 Hz, 2H), 2.81 (t, *J* = 5.6 Hz, 2H).

MS (ES⁺): 457.3 (M+H)⁺.

### Example 30 - (3-((1H-pyrrolo[2,3-c]pyridin-3-yl)methyl)-N-ethyl-5-methyl-4-oxo-5,6,8,9-tetrahydro-3H-pyrido[4',3':4,5]pyrrolo[2,3-d]pyridazine-7(4H)-sulfonamide

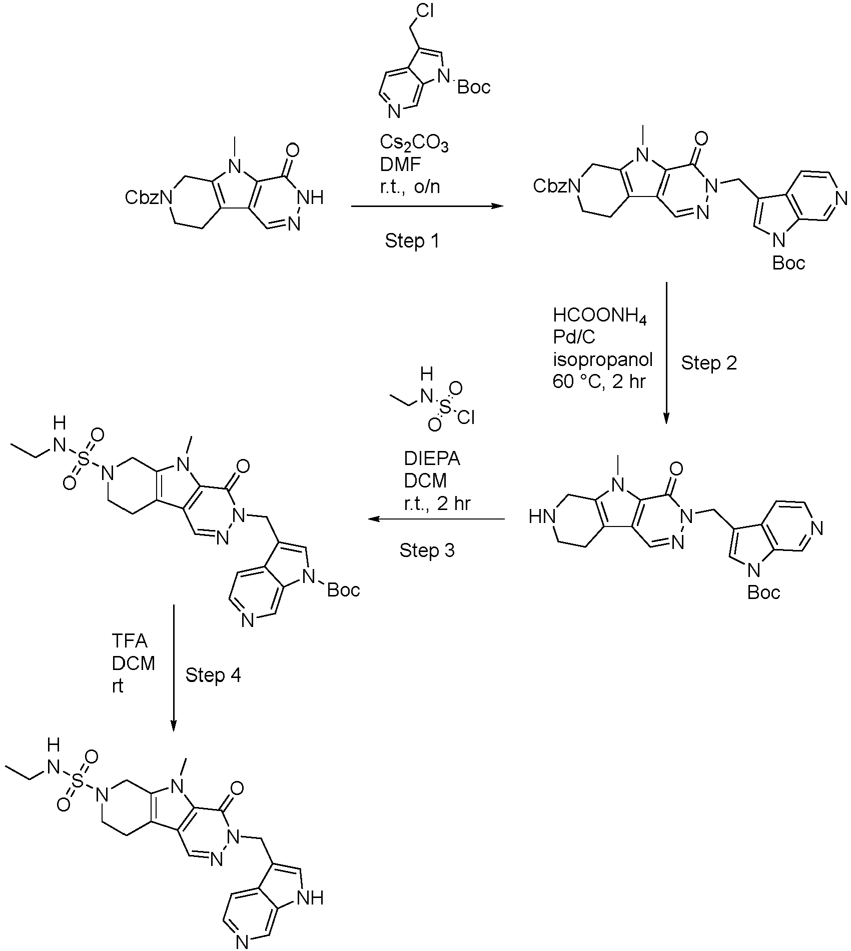

### Step 1

A mixture tert-butyl 3-(chloromethyl)-1H-pyrrolo[2,3-c]pyridine-1-carboxylate **(Intermediate 19,** 1.5mmol), Cs₂CO₃ (5.49g, 16.9 mmol) and 5-methyl-7-(methylsulfonyl)-3,5,6,7,8,9-hexahydro-4*H*-pyrido[4',3':4,5]pyrrolo[2,3-d]pyridazin-4-one (**Intermediate 20,** 420 mg, 1.24 mmol) in DMF (10 mL) under nitrogen protection was stirred at room temperature overnight. After LCMS indicated the reaction is completed, the reaction mixture was diluted with water (20 mL) and extracted with EtOAc (30 mL × 4).

The organic layers were washed with saturated NH₄Cl aqueous solution (40 mL × 2), concentrated at 40 °C under reduced pressure and purified by flash column chromatography (12 g, petroleum ether/ethyl acetate = 100:00~40:60) to give benzyl 3-((1-(*tert*-butoxycarbonyl)-1*H-*pyrrolo[2,3-*c*]pyridin-3-yl)methyl)-5-methyl-4-oxo-5,6,8,9-tetrahydro-3*H-*pyrido[4',3':4,5]pyrrolo[2,3-*d*]pyridazine-7(4*H*)-carboxylate (540 mg, 89% Purity) as a yellow solid. MS (ES⁺): 569.3 (M+H)⁺.

### Step 2

A mixture of benzyl 3-((1-(*tert*-butoxycarbonyl)-1*H*-pyrrolo[2,3-*c*]pyridin-3-yl)methyl)-5-methyl-4-oxo-5,6,8,9-tetrahydro-3*H*-pyrido[4',3':4,5]pyrrolo[2,3-*d*]pyridazine-7(4*H*)-carboxylate (540 mg, 0.95 mmol), ammonium formate (598 mg, 9.5 mmol) and Pd/C (10 wt%, 104 mg) in isopropyl alcohol (5 mL) under nitrogen protection was stirred at 60 °C for 2 hours. After LCMS indicated the reaction is completed, the reaction mixture was filtered, washed Pd/C with DCM (80 mL) and the filtrate was concentrated at 40 °C under reduced pressure to remove isopropanol. The residue was diluted with water (10 mL), extracted with EtOAc (20 mL × 4) and concentrated at 40 °C under reduced pressure to give *tert-butyl* 3-((5-methyl-4-oxo-4,5,6,7,8,9-hexahydro-3H-pyrido[4',3':4,5]pyrrolo[2,3-d]pyridazin-3-yl)methyl)-1H-pyrrolo[2,3-c]pyridine-1-carboxylate (300 mg, 91% Purity) as white solid. The crude was used in next step directly.

MS (ES⁺): 435.3 (M+H)⁺.

### Step 3

To a suspension of *tert*-butyl 3-((5-methyl-4-oxo-4,5,6,7,8,9-hexahydro-3*H-*pyrido[4',3':4,5]pyrrolo [2,3-*d*]pyridazin-3-yl)methyl)-1*H*-pyrrolo[2,3-*c*]pyridine-1-carboxylate (300 mg, 0.69 mmol) and DIPEA (451 mg, 3.5 mmol) in DCM (5 mL) was added ethylsulfamoyl chloride ( 119 mg, 0.83 mmol) and the reaction mixture was stirred at room temperature for 2 hours. After LCMS indicated the reaction completed, the mixture was diluted with H₂O (10 mL) and extracted with DCM (20 mL × 3). The combined organic layers were concentrated at 40 °C under reduced pressure and purified by flash column chromatography (12 g, dichloromethane/methanol = 100:00~94:6) to give *tert*-butyl 3-((7-(N-ethylsulfamoyl)-5-methyl-4-oxo-4,5,6,7,8,9-hexahydro-3*H*-pyrido[4',3':4,5]pyrrolo[2,3-*d*]pyridazin-3-yl)methyl)-1*H*-pyrrolo[2,3-*c*]pyridine-1-carboxylate (220 mg, 83% Purity) as white solid.

MS (ES⁺): 542.2 (M+H)⁺.

### Step 4

To a solution of *tert-butyl* 3-((7-(N-ethylsulfamoyl)-5-methyl-4-oxo-4,5,6,7,8,9-hexahydro-3H-pyrido [4',3':4,5]pyrrolo[2,3-*d*]pyridazin-3-yl)methyl)-1*H*-pyrrolo[2,3-c]pyridine-1-carboxylate (220 mg, 0.40 mol) in DCM (3 mL) was added TFA (3 mL) and the mixture was stirred at room temperature for 1 hour. After LCMS indicated the reaction completed, the mixture was concentrated under reduced pressure to give the residue. The residue was diluted with H₂O (15 mL), adjusted pH to 8 with K₂CO₃ and filtered. The filtered cake was purified by prep-HPLC (Column: Waters Xbridge Prep C18 OBD 10µm 19*250mm; Flow Rate: 20 mL/min; solvent system: MeCN/(10 mmol/L NH₄HCO₃/water) gradient: MeCN: 23%~95%; collection wavelength: 214 nm). The fractions were concentrated at 40 °C under reduced pressure to remove MeCN, and the residue was lyophilized to give the title compound (85.18 mg, 1000% Purity) as a white solid.

¹H NMR (400 MHz, DMSO-d6) δ: 11.51 (br, 1H), 8.68 (s, 1H), 8.18 (s, 1H), 8.06 (d, *J* = 5.6 Hz, 1H), 7.65 (d, *J =* 5.6 Hz, 1H), 7.61 (d, *J =* 2.0 Hz, 1H), 7.40 (d, *J =* 5.6 Hz, 1H), 5.43 (s, 2H), 4.34 (s, 2H), 4.02 (s, 3H), 3.39 (t, *J =* 5.6 Hz, 2H), 2.94-2.87 (m, 2H), 2.74 (t, *J =* 5.2 Hz, 2H), 1.04 (t, *J =* 7.6 Hz, 3H).

MS (ES⁺): 442.3 (M+H)⁺.

Further examples were prepared as set out in the following table.

| **Example No.** | **Example Structure / Name** | **LCMS / ¹H NMR data** | **Method and starting materials** |
|---|---|---|---|
| 31 | 5-methyl-7-(methylsulfonyl)-3-(3-(methylsulfonyl)benzyl)-3,5,6,7,8,9-hexahydro-4H-pyrido[4',3':4,5] pyrrolo[2,3-*d*]pyridazin-4-one 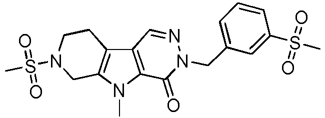 | MS (ES+): 451.2 (M+H)+ | Method of Example 1 |
| | | ¹H NMR (400 MHz, DMSO-d₆) δ: 8.28 (s, 1H), 7.86-7.82 (m, 2H), 7.62-7.59 (m, 2H), 5.42 (s, 2H), 4.48 (s, 2H), 4.45 (s, 2H), 4.01 (s, 3H), 3.50 (d, *J*= 5.6Hz, 2H), 3.20 (s, 3H), 3.01 (s, 3H), 2.82 (d, *J*= 5.2Hz, 2H). | Intermediate 1 and 3-chloromethyl-1-(methylsulfonyl)benzene |
| 32 | 3-((1H-indazol-4-yl)methyl)-5-methyl-7-(methylsulfonyl)-3,5,6,7,8,9-hexahydro-4H-pyrido[4',3':4,5]pyrrolo[2,3-*d*]pyridazin-4-one 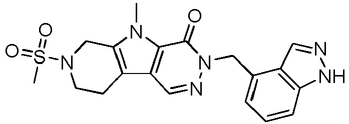 | MS (ES+): 413.3 (M+H)+ | Method of Example 20 |
| | | 1H NMR (400 MHz, DMSO-d6) δ: 13.10 (br, 1H), 8.24 (s, 1H), 8.09 (s, 1H), 7.45 (d, J = 8.4 Hz, 1H), 7.26 (dd, J = 8.0 Hz, 7.2 H, 1H), 6.89 (d, J = 7.2 Hz, 1H), 5.61 (s, 2H), 4.48 (s, 2H), 4.02 (s, 3H), 3.49 (t, J = 5.6 Hz, 2H), 3.00 (s, 3H), 2.81 (t, J = 5.2 Hz, 2H). | Intermediate 1 and tert-butyl 4-(chloromethyl)-1H-indazole-1-carboxylate |
| 33 | 3-(3-(2-hydroxypropan-2-yl)benzyl)-5-methyl-7-(methylsulfonyl)-3,5,6,7,8,9-hexahydro-4H-pyrido[4',3':4,5]pyrrolo[2,3-d]pyridazin-4-one 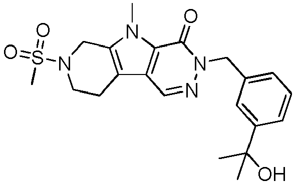 | MS (ES+): 416.3 (M+H)+ | Method of Example 1 |
| | | 1H NMR (400 MHz, DMSO-d6) δ: 8.24 (s, 1H), 7.45 (s, 1H), 7.31 (d, J = 8.0 Hz 1H), 7.21 (t, J = 8.0 Hz 1H), 7.02 (d, J = 7.2 Hz 1H), 5.30 (s, 2H), 4.99 (s, 1H), 4.48 (s, 2H), 4.02 (s, 3H), 3.49 (t, J = 5.6 Hz, 2H), 3.00 (s, 3H), 2.83-2.80 (m, 2H), 1.41-1.38 (m, 6H). | Intermediate 1 and Intermediate 17 |
| 34 | 5-methyl-7-(methylsulfonyl)-3-(thieno[3,2-c]pyridin-3-ylmethyl)-3,5,6,7,8,9-hexahydro-4H-pyrido[4',3':4,5]pyrrolo[2,3-d]pyridazin-4-one 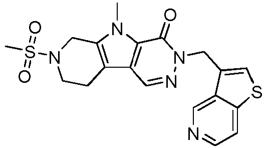 | MS (ES+): 430.3 (M+H)+ | Method of Example 1 |
| | | 1H NMR (400 MHz, DMSO-d6) δ: 9.30 (s, 1H), 8.42 (d, J = 5.2 Hz, 1H), 8.26 (s, 1H), 8.05 (dd, J = 5.6, 0.8 Hz, 1H), 7.70 (s, 1H), 5.63 (s, 2H), 4.48 (s, 2H), 4.03 (s, 3H), 3.48 (t, J = 5.6 Hz, 2H), 2.99 (s, 3H), 2.80 (t, J = 5.2 Hz, 2H). | Intermediate 1 and Intermediate 18 |
| 35 | 3-((1H-pyrrolo[2,3-c]pyridin-3-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-one 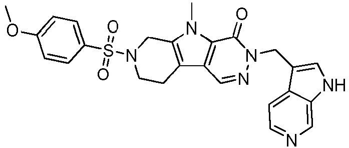 | MS (ES+): 505.3 (M+H)+ | Method of Example 22 |
| | | 1H NMR (400 MHz, DMSO-d6) δ: 11.50 (br, 1H), 8.67 (d, J = 1.2 Hz, 1H), 8.12 (s, 1H), 8.04 (d, J =5.6 Hz, 1H), 7.81-7.77 (m, 2H), 7.63 (d, J = 5.6 Hz, 1H), 7.59 (d, J = 2.8 Hz, 1H), 7.12-7.09 (m, 2H), 5.40 (s, 2H), 4.30 (s, 2H), 4.00 (s, 3H), 3.78 (s, 3H), 3.30 (t, J = 6.0 Hz, 2H), 2.67 (t, J = 5.2 Hz, 2H). | Intermediate 11 and Intermediate 19 |
| 36 | 3-((1H-pyrrolo[2,3-c]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-one 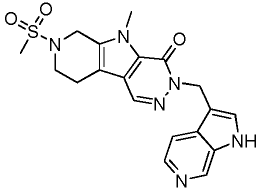 | MS (ES+): 413.3 (M+H)+ | Method of Example 1 |
| | | 1H NMR (400 MHz, DMSO-d6) δ: 11.54 (br, 1H), 8.69 (s, 1H), 8.19 (s, 1H), 8.06 (d, J = 5.6 Hz, 1H), 7.77 (d, J = 5.6 Hz, 1H), 7.63 (d, J = 2.0 Hz, 1H), 5.42 (s, J = 7.2 Hz, 2H), 4.46 (s, 2H), 4.01 (s, J = 9.2 Hz, 3H), 3.46 (t, J = 6.0 Hz, 2H), 2.98 (s, 3H), 2.77 (t, J = 5.2 Hz, 2H). | Intermediate 1 and Intermediate 19 |
| 37 | 3-((1H-pyrrolo[2,3-b]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-one 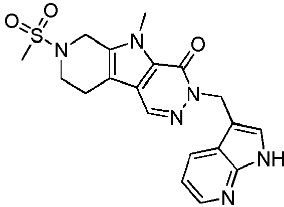 | MS (ES+): 413.4 (M+H)+ | Method of Example 1 |
| | | 1H NMR (400 MHz, DMSO-d6) δ: 11.58 (br, 1H), 8.20-8.18 (m, 2H), 8.09 (d, J = 7.6 Hz, 1H), 7.49 (s, 1H), 7.04 (s, 1H), 5.42 (s, 2H), 4.46-4.42 (m, 2H), 4.03 (s, 3H), 3.46 (s, 2H), 2.99-2.94 (m, 3H) 2.78 (s, 2H). | Intermediate 1 and Intermediate 23 |
| 38 | 3-((5-fluoro-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-one 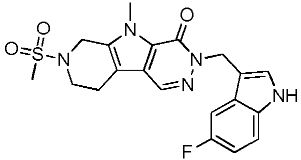 | MS (ES+): 430.3 (M+H)+ | Method of Example 1 |
| | | 1H NMR (400 MHz, DMSO-d₆) δ: 11.11 (br, 1H), 8.19 (s, 1H), 7.48-7.44 (m, 2H), 7.32 (q, J = 4.4 Hz, 1H), 6.90 (td, J = 9.2 Hz, 2.4 Hz, 1H), 5.39 (s, 2H), 4.46 (s, 2H), 4.03 (s, 3H), 3.46 (t, J = 5.6 Hz, 2H), 2.98 (s, 3H), 2.77 (t, J = 5.6 Hz, 2H). | Intermediate 1 and Intermediate 24 |
| 39 | 3-((6-fluoro-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-one 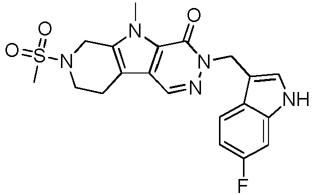 | MS (ES+): 430.2 (M+H)+ | Method of Example 1 |
| | | 1H NMR (400 MHz, DMSO-d6) δ: 11.06 (br, 1H), 8.18 (s, 1H), 7.70 (dd, J = 8.8 Hz, 5.6 Hz, 1H), 7.36 (d, J = 5.6 Hz, 1H), 7.11 (dd, J = 10.4 Hz, 2.4 Hz, 1H), 6.86-6.81 (m, 1H), 5.40 (s, 2H), 4.46 (s, 2H), 4.02 (s, 3H), 3.46 (t, J = 6.0 Hz, 2H), 2.98 (s, 3H), 2.77 (t, J = 5.6 Hz, 2H). | Intermediate 1 and Intermediate 25 |

### Biological Example 1 - human PKM2 activation assay

### Measuring in vitro activation of recombinant human PKM2

Compound activation of recombinant human PKM2 pyruvate kinase activity was determined by biochemical assay. N-terminal His-tagged hPKM2 was sourced from R&D Systems and its substrates phosphoenolpyruvate (PEP) and ADP from Sigma-Aldrich and 2BScientific Ltd, respectively. The Kinase-Glo^{®} Plus luminescence assay was from Promega. All other reagents were from Sigma-Aldrich. Test Compounds were prepared as 10 mM DMSO stocks and dilution series prepared in DMSO for direct dilution into Assay Buffer comprising 50 mM imidazole, 50 mM KCI, 7 mM MgCl₂, 0.01% Tween20, 0.05% BSA (pH 7.2).

### Assay Procedure

Human PKM2 was diluted into Assay Buffer comprising 50 mM imidazole, 50 mM KCI, 7 mM MgCl₂, 0.01% Tween20, 0.05% BSA (pH 7.2) to a final concentration of 5 pM. Enzyme-Assay Buffer mix was dispensed into a 384-well shallow-well white-walled plate (PerkinElmer) and Test Compounds added by acoustic dispense (Echo^{®}, Labcyte Inc.). Following 10 minutes' incubation at room temperature, the enzyme reaction was initiated by acoustic dispensing of ADP+PEP substrate to final concentrations of 254 µM ADP and 53 µM ADP.

After 60 minutes' incubation on an orbital shaker (300 rpm, 26 °C), enzyme activity was quantified by the luminescent detection of generated ATP. Kinase-Glo^{®} Plus reagent was added to each well and the plates incubated for a further 15 minutes on an orbital shaker in the dark (300 rpm, 26 °C) before luminescence measurement on a plate reader (PHERAstar^{®} FSX, BMG Labtech).

Percentage activation was calculated by normalising fluorescence signals to plate LOW (DMSO vehicle) and HIGH (5 µM TEPP-46) controls. EC₅₀ and Eₘₐₓ values were determined from 4-parameter logistic fits of compound concentration-response curves.

All Example compounds of formula (Ia) were tested and the results are shown in Table 1 below.

**Table 1 - PKM2 EC₅₀ values (µM) and Eₘₐₓ values (%)**

| **Compound** | **hPKM2 EC₅₀ (µM)** | **hPKM2 Eₘₐₓ (%)** |
|---|---|---|
| TEPP-46 | 0.185 | 113 |
| Example 1 | 0.1797 | 86.88 |
| Example 2 | 0.3055 | 93.65 |
| Example 3 | 0.3031 | 81.43 |
| Example 4 | 1.139 | 89.82 |
| Example 5 | 0.5907 | 96.72 |
| TEPP-46 | 0.185 | 113 |
| Example 6 | 0.3172 | 85.31 |
| Example 7 | 0.3464 | 107.4 |
| Example 8 | 1.736 | 83.14 |
| Example 9 | 1.768 | 47.27 |
| Example 10 | 4.678 | 72.05 |
| Example 11 | 0.8459 | 88.41 |
| Example 12 | 1.489 | 79.66 |
| Example 13 | 0.702 | 88.5 |
| Example 14 | 0.512 | 102 |
| Example 15 | 0.099 | 99.9 |
| Example 16 | 0.129 | 76.6 |
| Example 17 | 0.901 | 98.8 |
| Example 18 | 0.394 | 58.7 |
| Example 19 | 0.869 | 63.8 |
| Example 20 | 0.272 | 111 |
| Example 21 | 0.986 | 71.4 |
| Example 22 | 0.385 | 76.8 |
| Example 23 | 0.313 | 58.3 |
| Example 24 | 0.188 | 90.9 |
| Example 25 | 0.342 | 99.5 |
| Example 26 | 0.372 | 81.1 |
| Example 27 | 0.919 | 78 |
| Example 28 | 0.385 | 56.4 |
| Example 29 | 0.28 | 89 |
| Example 30 | 0.083 | 62.2 |
| Example 31 | 0.423 | 75.3 |
| Example 32 | 0.365 | 94.6 |
| Example 33 | 0.354 | 96.5 |
| Example 34 | 0.44 | 82.6 |
| Example 35 | 0.569 | 78.1 |
| Example 36 | 0.071 | 100 |
| Example 37 | 0.207 | 106 |
| Example 38 | 0.759 | 93.2 |
| Example 39 | 0.217 | 92.7 |

Compounds of formula (la) that were tested in this assay exhibited acceptable or good PKM2-modulatory activity, as demonstrated by their EC₅₀ and/or Eₘₐₓ values for PKM2 activation. Certain compounds of formula (la) exhibited improved PKM2-activation potency compared with TEPP-46, as demonstrated by their lower EC₅₀ values.

### Biological Example 2 - human PKLR activation assay

### Measuring in vitro activation of recombinant human PKLR

Compound activation of recombinant human PKLR pyruvate kinase activity was determined by biochemical assay. N-terminal His-tagged enzyme was sourced from R&D Systems and its substrates phosphoenolpyruvate (PEP) and ADP from Sigma-Aldrich and 2BScientific Ltd, respectively. The Kinase-Glo^{®} Plus luminescence assay was from Promega. All other reagents were from Sigma-Aldrich. Test Compounds were prepared as 10 mM DMSO stocks and dilution series prepared in DMSO for direct dilution into Assay Buffer comprising 50 mM imidazole, 50 mM KCI, 7 mM MgCl₂, 0.01% Tween20, 0.05% BSA (pH 7.2).

### Assay Procedure

Human PKLR was diluted into Assay Buffer to a final concentration of 5 pM. Enzyme-Assay Buffer mix was dispensed into 384-well shallow-well white-walled plates and Test Compounds added by acoustic dispense (Echo^{®}, Labcyte Inc). Following 10 minutes' incubation at room temperature, the enzyme reaction was initiated by acoustic dispensing of ADP+PEP substrate to final concentrations of 254 µM ADP and 53 µM ADP.

After 60 minutes' incubation on an orbital shaker (300 rpm, 26 °C), enzyme activity was quantified by the luminescent detection of generated ATP. Kinase-Glo^{®} Plus reagent was added to each well and the plates incubated for a further 15 minutes on an orbital shaker in the dark (300 rpm, 26 °C) before luminescence measurement on a plate reader (PHERAstar^{®} FSX, BMG Labtech).

Percentage activation was calculated by normalising fluorescence signals to plate LOW (DMSO vehicle) and HIGH (5 µM TEPP46) controls. EC₅₀ and Eₘₐₓ values were determined from 4-parameter logistic fits of compound concentration-response curves.

The compounds of formula (Ia) of Examples 1 to 12 and 36 were tested and the results are shown in Table 2 below.

**Table 2 - PKLR EC₅₀ values (µM) and Eₘₐₓ values (%)**

| **Compound** | **hPKLR EC₅₀ (µM)** | **hPKLR Eₘₐₓ (%)** |
|---|---|---|
| TEPP46 | 0.0435 | 116.6 |
| Example 1 | ++ | §§ |
| Example 2 | ++ | §§§ |
| Example 3 | ++ | §§§ |
| TEPP46 | 0.0435 | 116.6 |
| Example 4 | ++ | §§§ |
| Example 5 | ++ | § |
| Example 6 | +++ | § |
| Example 7 | ++ | §§§ |
| Example 8 | + | § |
| Example 9 | + | § |
| Example 10 | + | §§ |
| Example 11 | ++ | § |
| Example 12 | ++ | §§§ |
| Example 36 | +++ | §§ |

| | | |
|---|---|---|
| "+++" refers to an EC₅₀ of greater than 0.0435 µM but less than 0.0600 µM; "++" refers to an EC₅₀ of between 0.0600 µM and 0.3200 µM; and "+" refers to an EC₅₀ of greater than 0.3200 µM. "§§§" refers to an Eₘₐₓ of greater than 100 % but less than 116.6 %; "§§" refers to an Eₘₐₓ of between 90 % and 100 %; "§" refers to an Eₘₐₓ of less than 90 %. | | |

Compounds of formula (la) that were tested in this assay exhibited acceptable or good PKLR-modulatory activity, as demonstrated by their EC₅₀ and/or Eₘₐₓ values for PKLR activation.

### Biological Example 3 - A549 Proliferation and Cytotoxicity Assay

### Induction of serine auxotrophy as a cell-based readout of PKM2 activation

In highly proliferative, glycolytically active cells, PKM2 activation accelerates flux through glycolysis and induces a powerful dependence of cell proliferation on the non-essential amino acid serine (Kung *et al.,* 2012). The effect of compounds of Formula (I) on A549 cell proliferation in low-serine medium was assessed using a luminescence assay of viable cell number (CellTiter-Glo^{®} 2.0, Promega) duplexed with a fluorescent cytotoxicity assessment (CellTox^{™} Green, Promega). Cell culture reagents were from ThermoFisher unless specified otherwise. Assay reagents were from Sigma-Aldrich unless specified otherwise. Test Compounds were prepared as 10 mM DMSO stocks and dilution series prepared in DMSO for direct dilution into culture medium.

### Assay Procedure

A549 cells were maintained in T225 flasks in a humidified incubator (37 °C, 5% CO₂), in complete growth medium comprising RPMI 1640 supplemented with 10% heat-inactivated FBS plus 2 mM L-glutamine + 1% penicillin/streptomycin (Sigma-Aldrich). Cells were passaged every 3-4 days at 80-90% confluence. The cell monolayer was washed twice with room-temperature Ca²⁺/Mg-free PBS and 4 ml room-temperature trypsin-EDTA added to the flask. After 5 minutes' incubation (37 °C, 5% CO₂) to detach cells, trypsin was quenched with 13 ml warmed complete growth medium and the cells harvested and diluted to 2.4×10⁴ cells/ml for dispensing into 384-well clear-bottom black-walled plates (600 cells/well).

Following overnight incubation, growth medium was aspirated from each well and the cells washed twice with Assay Medium comprising Basal Medium Eagle supplemented with 3 % heat-inactivated dialysed FBS plus 2 mM L-glutamine plus 20 µM L-serine (Sigma-Aldrich). After aspiration of the final wash medium, Assay Medium containing Test Compound was added to each well. DMSO vehicle controls were included on each plate.

Cells were incubated for 3 days in a humidified incubator (37 °C, 5% CO₂) at which point CellTox^{™} Green and CellTiter-Glo^{®} reagents were prepared according to the manufacturer's instructions. Triton-X was added to HIGH wells for cytotoxicity determination and the plate incubated for 30 minutes (20 °C, ambient humidity and pCO₂). CellTox^{™} Green reagent was added to each well and the plate covered, placed on an orbital shaker @450 rpm for 2 minutes, and incubated in the dark for 15 minutes (20 °C, ambient humidity and pCO₂). Endpoint fluorescence was measured using a plate reader (PHERAstar^{®} FSX, BMG Labtech).

Following cytotoxicity determination, CellTiter-Glo^{®} reagent was added to each well and the plate placed on an orbital shaker @450 rpm for 2 minutes to lyse cells. Plates were incubated in the dark for 15-30 minutes (20 °C, ambient humidity and pCO₂) prior to endpoint luminescence measurement (PHERAstar^{®} FSX, BMG Labtech).

Cytotoxicity was calculated by normalising fluorescence signals to plate LOW (DMSO vehicle) and HIGH (Triton X-treated) controls. CC₅₀ values were determined from a 4-parameter logistic fits of the compound concentration-response curve.

Proliferation was calculated by normalizing luminescence signals to LOW controls obtained from a parallel plate processed at the time of Test Compound challenge and HIGH (DMSO vehicle) wells from the compound plate. IC₅₀ values were determined from a 4-parameter logistic fit of compound concentration-response curves.

The compounds of formula (la) of Examples 1 to 12 were tested and the results are shown in Table 3 below.

**Table 3 - A549 Cell Proliferation and Cytotoxicity**

| **Compound** | **Proliferation IC₅₀ (µM)** |
|---|---|
| TEPP-46 | 0.0016 |
| Example 1 | 0.0016 |
| Example 2 | 0.0045 |
| Example 3 | 0.0105 |
| Example 4 | NT* |
| Example 5 | 0.0133 |
| Example 6 | 0.0168 |
| Example 7 | 0.0009 |
| Example 8 | 0.0254 |
| Example 9 | 0.0258 |
| Example 10 | 0.1174 |
| Example 11 | 0.014 |
| Example 12 | 0.0135 |

Compounds of formula (la) that were tested in this assay exhibited acceptable or good anti-proliferative activity, as demonstrated by their IC₅₀ values. Certain compounds of formula (la) exhibited improved anti-proliferative activity compared with TEPP-46, as demonstrated by their lower IC₅₀ values.

### Stability Example 1 - Kinetic Aqueous Solubility

Solubility of a given compound is the amount of the compound that passes into solution to achieve a saturated solution at constant temperature and pressure. It is expressed in terms of maximum volume or mass of the solute that dissolves in a given volume of a solvent.

General considerations in measuring solubility by the described method herein:
- Filtration of the saturated solution for separation
- Quantification of the compound in the solution by UV spectroscopy
- Both filtration and analysis were performed at room temperature
- This assay has been validated for Millipore Multiscreen Filter plates (ref: MSS LB PC 10) as well as for Porvair plates (ref: 360043)

### Standard Assay Method

### Assay Preparation

Phosphate buffered saline (PBS) pH 7.4 was prepared using PBS tablets as per the manufacturer's instructions. Compounds and marker stocks are prepared at 10 mM in DMSO.

### Assay Procedure

Compounds were incubated at 200 µM (2% DMSO) in Millipore - MSS LB PC 10 or Porvair - ref: 36004 plates in the presence of the prepared PBS pH 7.4 solution for 1.5h, at RT, with gentle shaking. After the incubation time, the samples were filtrated by centrifugation (for Millipore plates) or by Vacuum (Porvair plates) and the filtrates were diluted 1.25x in Acetonitrile and compared to a matrix matched calibration curve. The samples were then analysed in a 96 well UV Star analysis plate on a plate reader from 220 nm to 500 nm at 10 nm increments. When analysis was not possible by UV plate reader, filtrates were diluted 150x in methanol: water 50:50 and then analysed by LC-MS/MS.

### Data Processing

The concentration of the samples was calculated against the standard curves. Compounds with a concentration greater than 200 µM are reported as solubility >200 µM and compounds with a concentration lower than 5.12 µM (Lower concentration point in the standard curve) are reported as solubility <5.12 µM.

Kinetic solubility was determined for all Example compounds of formula (la) and the results are shown in Table 4 below.

**Table 4 - Kinetic solubility**

| **Compound** | **Kinetic solubility (Ksol; (µg/ml)** |
|---|---|
| TEPP-46 | 0.15 |
| Example 1 | 13.89 |
| | 12.85* |
| Example 2 | 36.19 |
| | 36.10* |
| Example 3 | 18.98 |
| | 17.91* |
| Example 4 | 42.01 |
| Example 5 | 5.13 |
| Example 6 | 0.64 |
| | 0.87* |
| Example 7 | 72.24 |
| Example 8 | >70.30 |
| Example 9 | 43.14 |
| Example 10 | 17.14 |
| Example 11 | 9.90 |
| Example 12 | 61.62 |
| Example 13 | 37.97 |
| Example 14 | 41.33 |
| TEPP-46 | 0.15 |
| Example 15 | 2.49 |
| Example 16 | 7.58 |
| Example 17 | 73.09 |
| Example 18 | 18.27 |
| Example 19 | 5.42 |
| Example 20 | 8.43 |
| Example 21 | 1.04 |
| Example 22 | 2.53 |
| Example 25 | 3.94 |
| Example 26 | >83.01 |
| Example 28 | >92.5 |
| Example 29 | 37.45 |
| Example 30 | 30.29 |
| Example 31 | 18.84 |
| Example 32 | 16.16 |
| Example 33 | 19.80 |
| Example 34 | 1.80 |
| Example 36 | >82.49 |
| Example 37 | 59.28 |
| Example 39 | 1.09 |

| | |
|---|---|
| * * repeated value | |

Compounds of formula (la) that were tested in this assay exhibited significantly improved solubility compared with TEPP-46.

### References

Abulizi et al. Cell Metab. 2020, 32(5):751-766.e11.
Alves-Filho et al. Front Immunol. 2016, 7(145), 1-7.
Barazzoni et al. Eating and Weight Disorders - Studies on Anorexia, Bulimia and Obesity 2018, 23, 149-157.
Bettaieb et al. The Journal of Biological Chemistry 2013, 288(24), 17360-17371.
Bianchi et al. Haematologica 2020, 105(9), 2218-2228.
Cangado et al., Hematology, Transfusion and Cell Therapy, 2018, 40 (1), 1-2.
Chhipa et al. Life Sciences 2018, 280, DOI: 10.1016/j.lfs.2021.119694.
Dong et al. Oncol Lett. 2016, 11(3), 1980-1986.
Grace et al. N. Engl. J. Med. 2019, 381(10), 933-944
Kung et al. Chemistry & Biology 2012, 19, 1187-1198
Kung et al. Blood 2017, 14;130(11), 1347-1356.
Lewandowski et al. Cell Metab. 2020, 32(5):736-750.e5.
Liu et al. J. Diabetes Investig. 2020, 12(5):697-709.
Pålsson-McDermott et al. Cell Research 2020, 30:300-314.
Puckett et al. International Journal of Molecular Sciences 2021, 22, 1171.
Qi et al. Nat Med. 2017, 23(6), 753-762
Yi et al. Front. Immunol. 2021, DOI: 10.3389/fimmu.2020.595316.
Jiang et al. Biorganic & Medicinal Chemistry Letters, 2010, 20, 11, 3387-3393.

## Claims

1. A compound of formula (la): 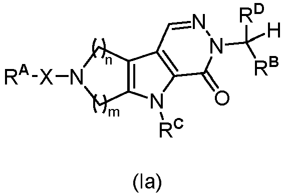 wherein,
R^{A} is C₁₋₁₀ alkyl, C₂₋₁₀ alkenyl, C₂₋₁₀ alkynyl, C₃₋₁₀ cycloalkyl, phenyl or 5-10 membered heteroaryl; wherein R^{A} is optionally substituted on an available atom by one or more R^{1A}, wherein each R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano, NR^{2A}R^{3A}, CO₂H, CONR^{2A}R^{3A} and C₃₋₆ cycloalkyl;
R^{2A} and R^{3A} are independently selected from the group consisting of H and C₁₋₆ alkyl; or R^{2A} and R^{3A} together with the N atom to which they are attached combine to form a 5-7 membered heterocyclic ring which is optionally substituted on an available atom by one or more groups selected from C₁₋₂ alkyl and oxo;
R^{B} is phenyl, phenyl fused to a 5-7 membered heterocyclic ring, 5-10 membered heteroaryl, or 4-7 membered heterocyclyl; wherein R^{B} is optionally substituted on an available atom by one or more R^{1B}, wherein each R^{1B} is independently selected from the group consisting of halo, C₁₋₆ alkyl, C₁₋₆ hydroxyalkyl, C₁₋₆ aminoalkyl, C₁₋₆ haloalkyl, hydroxy, OC₁₋₆ alkyl, OC₁₋₆ haloalkyl, cyano, NR^{2B}R^{3B} , CO₂H, CONR^{2B}R^{3B}, S(O)₂NR^{2B}R^{3B}, S(O)₂C₁₋₆ alkyl, C₃₋₆ cycloalkyl and oxo;
R^{2B} and R^{3B} are independently selected from the group consisting of H and C₁₋₆ alkyl; or R^{2B} and R^{3B} together with the N atom to which they are attached combine to form a 5-7 membered heterocyclic ring which is optionally substituted on an available atom by one or more groups selected from C₁₋₂ alkyl and oxo;
R^{C} is H or C₁₋₂ alkyl;
R^{D} is H, C₁₋₂ alkyl, C₁₋₂ hydroxyalkyl or C₁₋₂ methoxyalkyl;
X is C=O, S(=O)₁₋₂, -CH₂-S(=O)₁₋₂-, -S(=O)(=NH) or -NH-S(=O)₂-;
n is 0 to 4 and m is 0 to 4, wherein n + m is 2, 3 or 4;
or a pharmaceutically acceptable salt and/or solvate thereof.

2. The compound, pharmaceutically acceptable salt and/or solvate thereof according to claim 1, wherein R^{A} is C₁₋₁₀ alkyl, C₃₋₁₀ cycloalkyl, phenyl or 5-10 membered heteroaryl, and is optionally substituted on an available atom by one or more R^{1A}, wherein each R^{1A} is independently selected from the group consisting of halo, C₁₋₆ alkyl, C₁₋₆ hydroxyalkyl, C₁₋₆ haloalkyl, hydroxy, OC₁₋₆ alkyl, OC₁₋₆ haloalkyl, cyano and NR^{2A}R^{3A}.

3. The compound, pharmaceutically acceptable salt and/or solvate thereof according to claim 2, wherein R^{A} is methyl, ethyl, or n-propyl and is optionally substituted by OC₁₋₄ alkyl, or wherein R^{A} is phenyl optionally substituted on an available carbon atom by OC₁₋₆ alkyl.

4. The compound, pharmaceutically acceptable salt and/or solvate thereof according to any one of claims 1 to 3, wherein R^{B} is selected from the group consisting of phenyl, phenyl fused to a 5-7 membered heterocyclic ring, pyrrolyl, furanyl, thienyl, imidazolyl, pyrazolyl, thiazolyl, isothiazolyl, oxadiazolyl, thiadiazolyl, triazolyl, oxazolyl, isoxazolyl, tetrazolyl, pyridinyl, pyrimidinyl, pyradizinyl, pyrazinyl, furo[3,2-b]pyridinyl, pyrazolo[1,5-a]pyridinyl, pyrrolo[2,3-b]pyridinyl, pyrrolo[2,3-c]pyridinyl, imidazo[1,2-a]pyridinyl, thieno[3,2-c]pyridinyl, indolyl, indazolyl, benzofuranyl, benzimidazolyl, benzothiazolyl, benzothiophenyl, quinolinyl, isoquinolinyl, quinazolinyl, pyrrolidinyl, tetrahydrofuranyl, tetrahydrothienyl, tetrahydropyranyl, piperidinyl, piperazinyl, morpholinyl, thiomorpholinyl, pyridone and pyridazinone; and is optionally substituted on an available atom by one or more R^{1B}, wherein each R^{1B} is independently selected from the group consisting of halo, C₁₋₄ alkyl, C₁₋₄ hydroxyalkyl, C₁₋₆ aminoalkyl, C₁₋₄ haloalkyl, hydroxy, OC₁₋₄ alkyl, OC₁₋₄ haloalkyl, cyano, NR^{2B}R^{3B}, CONR^{2B}R^{3B}, S(O)₂NR^{2B}R^{3B}, S(O)₂C₁₋₆ alkyl, C₃₋₅ cycloalkyl and oxo.

5. The compound, pharmaceutically acceptable salt and/or solvate thereof according to any one of claims 1 to 4, wherein R^{C} is methyl, R^{D} is H, and X is S(=O)₂.

6. The compound, pharmaceutically acceptable salt and/or solvate thereof according to any one of claims 1 to 5, wherein n is 2 and m is 1 and the compound of formula (la) is a compound of formula (laA): 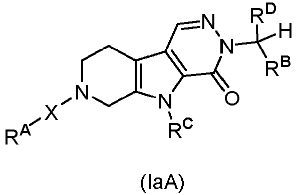
wherein R^{A}, R^{B}, R^{C}, R^{D} and X are as defined in any one of claims 1 to 5;
or a pharmaceutically acceptable salt and/or solvate thereof.

7. The compound, pharmaceutically acceptable salt and/or solvate thereof according to claim 1, selected from the group consisting of:
3-((2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-one;
3-((6-methoxypyridin-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(3-(1-hydroxyethyl)benzyl)-5-methyl-7-(methylsulfonyl)-3,5,6,7,8,9-hexahydro-4H-pyrido[4',3':4,5]pyrrolo[2,3-d]pyridazin-4-one;
3-((1H-pyrazol-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(2-fluoro-3-methoxybenzyl)-5-methyl-7-(methylsulfonyl)-3,5,6,7,8,9-hexahydro-4H-pyrido[4',3':4,5]pyrrolo[2,3-d]pyridazin-4-one;
3-(2,6-difluoro-4-methoxybenzyl)-5-methyl-7-(methylsulfonyl)-3,5,6,7,8,9-hexahydro-4H-pyrido[4',3':4,5]pyrrolo[2,3-d]pyridazin-4-one;
3-(3-aminobenzyl)-5-methyl-7-(methylsulfonyl)-3,5,6,7,8,9-hexahydro-4H-pyrido[4',3':4,5]pyrrolo[2,3-d]pyridazin-4-one;
7-acetyl-3-(3-aminobenzyl)-5-methyl-3,5,6,7,8,9-hexahydro-4H-pyrido[4',3':4,5]pyrrolo[2,3-d]pyridazin-4-one;
3-(3-aminobenzyl)-7-(cyclopropylsulfonyl)-5-methyl-3,5,6,7,8,9-hexahydro-4H-pyrido[4',3':4,5]pyrrolo[2,3-d]pyridazin-4-one;
3-(3-aminobenzyl)-5-methyl-7-((2-methylthiazol-5-yl)sulfonyl)-3,5,6,7,8,9-hexahydro-4H-pyrido[4',3':4,5]pyrrolo[2,3-d]pyridazin-4-one;
3-(3-aminobenzyl)-5-methyl-7-(phenylsulfonyl)-3,5,6,7,8,9-hexahydro-4H-pyrido[4',3':4,5]pyrrolo[2,3-d]pyridazin-4-one; and
3-((1H-pyrazol-3-yl)methyl)-5-methyl-7-(phenylsulfonyl)-3,5,6,7,8,9-hexahydro-4H-pyrido[4',3':4,5]pyrrolo[2,3-d]pyridazin-4-one;
3-((2-(hydroxymethyl)thiophen-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)thiophene-2-carboxamide;
3-((1H-indol-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(benzofuran-3-ylmethyl)-5-methyl-7-(methylsulfonyl)-3,5,6,7,8,9-hexahydro-4H-pyrido[4',3':4,5]pyrrolo[2,3-d]pyridazin-4-one;
3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)-1H-pyrazole-4-carboxamide;
3-((5-methyl-7-(methylsulfonyl)-4-oxo-4,5,6,7,8,9-hexahydro-3H-pyrido[4',3':4,5]pyrrolo[2,3-d]pyridazin-3-yl)methyl)benzenesulfonamide;
5-methyl-7-(methylsulfonyl)-3-((6-(pyrrolidin-1-yl)pyridin-2-yl)methyl)-3,5,6,7,8,9-hexahydro-4H-pyrido[4',3':4,5]pyrrolo[2,3-d]pyridazin-4-one;
3-((1H-indazol-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-(2-fluoro-4-methoxybenzyl)-5-methyl-7-(phenylsulfonyl)-3,5,6,7,8,9-hexahydro-4H-pyrido[4',3':4,5]pyrrolo[2,3-d]pyridazin-4-one;
3-((7-((4-methoxyphenyl)sulfonyl)-5-methyl-4-oxo-4,5,6,7,8,9-hexahydro-3H-pyrido[4',3':4,5]pyrrolo[2,3-d]pyridazin-3-yl)methyl)thiophene-2-carboxamide;
3-((2,3-dihydrobenzofuran-5-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-one;
3-((2,2-dimethyl-2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-one;
3-((1H-pyrrolo[2,3-b]pyridin-6-yl)methyl)-5-methyl-7-(methylsulfonyl)-3,5,6,7,8,9-hexahydro-4H-pyrido[4',3':4,5]pyrrolo[2,3-d]pyridazin-4-one;
(S)-3-(3-(1-aminoethyl)benzyl)-5-methyl-7-(methylsulfonyl)-3,5,6,7,8,9-hexahydro-4H-pyrido[4',3':4,5]pyrrolo[2,3-d]pyridazin-4-one;
7-((2-methoxyethyl)sulfonyl)-3-((6-methoxypyridin-3-yl)methyl)-5-methyl-3,5,6,7,8,9-hexahydro-4H-pyrido[4',3':4,5]pyrrolo[2,3-d]pyridazin-4-one;
N-(2-methoxyethyl)-3-((6-methoxypyridin-3-yl)methyl)-5-methyl-4-oxo-3,4,5,6,8,9-hexahydro-7H-pyrido[4',3':4,5]pyrrolo[2,3-d]pyridazine-7-sulfonamide;
3-((1H-pyrrolo[2,3-c]pyridin-3-yl)methyl)-7-((2-methoxyethyl)sulfonyl)-5-methyl-6,7,8,9-tetrahydro-3H-pyrido[4',3':4,5]pyrrolo[2,3-d]pyridazin-4(5H)-one;
(3-((1H-pyrrolo[2,3-c]pyridin-3-yl)methyl)-N-ethyl-5-methyl-4-oxo-5,6,8,9-tetrahydro-3H-pyrido[4',3':4,5]pyrrolo[2,3-d]pyridazine-7(4H)-sulfonamide;
5-methyl-7-(methylsulfonyl)-3-(3-(methylsulfonyl)benzyl)-3,5,6,7,8,9-hexahydro-4H-pyrido[4',3':4,5] pyrrolo[2,3-*d*]pyridazin-4-one;
3-((1*H*-indazol-4-yl)methyl)-5-methyl-7-(methylsulfonyl)-3,5,6,7,8,9-hexahydro-4*H-*pyrido[4',3':4,5]pyrrolo[2,3-d]pyridazin-4-one;
3-(3-(2-hydroxypropan-2-yl)benzyl)-5-methyl-7-(methylsulfonyl)-3,5,6,7,8,9-hexahydro-4H-pyrido[4',3':4,5]pyrrolo[2,3-d]pyridazin-4-one;
5-methyl-7-(methylsulfonyl)-3-(thieno[3,2-c]pyridin-3-ylmethyl)-3,5,6,7,8,9-hexahydro-4H-pyrido[4',3':4,5]pyrrolo[2,3-d]pyridazin-4-one;
3-((1H-pyrrolo[2,3-c]pyridin-3-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-one;
3-((1H-pyrrolo[2,3-c]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-((1H-pyrrolo[2,3-b]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-one;
3-((5-fluoro-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-one; and
3-((6-fluoro-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-one;
or a pharmaceutically acceptable salt and/or solvate of any one thereof.

8. A pharmaceutical composition comprising a compound, pharmaceutically acceptable salt and/or solvate thereof according to any one of claims 1 to 7, and one or more pharmaceutically acceptable diluents or carriers.

9. A compound, pharmaceutically acceptable salt and/or solvate thereof according to any one of claims 1 to 7 or a pharmaceutical composition according to claim 8, for use as a medicament.

10. The compound, or pharmaceutically acceptable salt and/or solvate thereof for use, or pharmaceutical composition for use according to claim 9, for treating or preventing an inflammatory disease, a disease associated with an undesirable immune response, cancer, obesity, a diabetic disease or a blood disorder.

11. The compound, or pharmaceutically acceptable salt and/or solvate thereof for use, or pharmaceutical composition for use according to claim 10, wherein:
- the inflammatory disease or disease associated with an undesirable immune response is, or is associated with, a disease selected from the group consisting of: psoriasis, asthma, chronic obstructive pulmonary disease (COPD), heart failure, myocardial infarction, angina pectoris, other atherosclerosis and/or atherothrombosis-related disorders, a mitochondrial and neurodegenerative disease, autoimmune paraneoplastic retinopathy, transplantation rejection, multiple sclerosis, transverse myelitis, ischaemia-reperfusion injury, AGE-induced genome damage, an inflammatory bowel disease, primary sclerosing cholangitis (PSC), PSC-autoimmune hepatitis overlap syndrome, non-alcoholic fatty liver disease (non-alcoholic steatohepatitis), rheumatica, granuloma annulare, cutaneous lupus erythematosus (CLE), systemic lupus erythematosus (SLE), lupus nephritis, drug-induced lupus, autoimmune myocarditis or myopericarditis, Dressler's syndrome, giant cell myocarditis, post-pericardiotomy syndrome, drug-induced hypersensitivity syndromes, eczema, sarcoidosis, erythema nodosum, acute disseminated encephalomyelitis (ADEM), neuromyelitis optica spectrum disorders, MOG (myelin oligodendrocyte glycoprotein) antibody-associated disorders, optic neuritis, CLIPPERS (chronic lymphocytic inflammation with pontine perivascular enhancement responsive to steroids), diffuse myelinoclastic sclerosis, Addison's disease, alopecia areata, ankylosing spondylitis, other spondyloarthritides, antiphospholipid antibody syndrome, autoimmune hemolytic anaemia, autoimmune hepatitis, autoimmune inner ear disease, pemphigoid, linear IgA disease, Behçet's disease, celiac disease, Chagas disease, dermatomyositis, diabetes mellitus type I, endometriosis, Goodpasture's syndrome, Graves' disease, Guillain-Barre syndrome and its subtypes, progressive inflammatory neuropathy, Hashimoto's disease, hidradenitis suppurativa, inclusion body myositis, necrotising myopathy, Kawasaki disease, IgA nephropathy, Henoch-Schonlein purpura, idiopathic thrombocytopenic purpura, thrombotic thrombocytopenic purpura (TTP), Evans' syndrome, interstitial cystitis, mixed connective tissue disease, undifferentiated connective tissue disease, morphea, myasthenia gravis, narcolepsy, neuromyotonia, pemphigus vulgaris, pernicious anaemia, psoriatic arthritis, polymyositis, primary biliary cholangitis (also known as primary biliary cirrhosis), rheumatoid arthritis, palindromic rheumatism, schizophrenia, autoimmune encephalitis syndromes, autoimmune meningoencephalitis syndromes, scleroderma, Sjogren's syndrome, stiff person syndrome, polymylagia rheumatica, giant cell arteritis (temporal arteritis), Takayasu arteritis, polyarteritis nodosa, Kawasaki disease, granulomatosis with polyangitis (GPA; formerly known as Wegener's granulomatosis), eosinophilic granulomatosis with polyangiitis (EGPA; formerly known as Churg-Strauss syndrome), microscopic polyarteritis/polyangiitis, hypocomplementaemic urticarial vasculitis, hypersensitivity vasculitis, cryoglobulinemia, thromboangiitis obliterans (Buerger's disease), vasculitis, leukocytoclastic vasculitis, vitiligo, acute disseminated encephalomyelitis, adrenoleukodystrophy, Alexander's disease, Alper's disease, balo concentric sclerosis or Marburg disease, cryptogenic organising pneumonia (formerly known as bronchiolitis obliterans organizing pneumonia), Canavan disease, central nervous system vasculitic syndrome, Charcot-Marie-Tooth disease, childhood ataxia with central nervous system hypomyelination, chronic inflammatory demyelinating polyneuropathy (CIDP), diabetic retinopathy, globoid cell leukodystrophy (Krabbe disease), graft-versus-host disease (GVHD), hepatitis C (HCV) infection or complication, herpes simplex viral infection or complication, human immunodeficiency virus (HIV) infection or complication, lichen planus, monomelic amyotrophy, fibrosis, cystic fibrosis, pulmonary arterial hypertension (PAH), lung sarcoidosis, idiopathic pulmonary fibrosis, kidney fibrosis, paediatric asthma, atopic dermatitis, allergic dermatitis, contact dermatitis, allergic rhinitis, rhinitis, sinusitis, conjunctivitis, allergic conjunctivitis, keratoconjunctivitis sicca, dry eye, xerophthalmia, glaucoma, macular oedema, diabetic macular oedema, central retinal vein occlusion (CRVO), macular degeneration, post-operative cataract inflammation, uveitis, iridocyclitis, scleritis, corneal graft and limbal cell transplant rejection, gluten sensitive enteropathy (coeliac disease), dermatitis herpetiformis, eosinophilic esophagitis, achalasia, autoimmune dysautonomia, autoimmune encephalomyelitis, autoimmune oophoritis, autoimmune orchitis, autoimmune pancreatitis, aortitis and periaortitis, autoimmune retinopathy, autoimmune urticaria, Behcet's disease, Castleman's disease, Cogan's syndrome, IgG4-related disease, retroperitoneal fibrosis, juvenile idiopathic arthritis, adult-onset Still's disease, ligneous conjunctivitis, Mooren's ulcer, pityriasis lichenoides et varioliformis acuta (PLEVA, also known as Mucha-Habermann disease), multifocal motor neuropathy (MMN), paediatric acute-onset neuropsychiatric syndrome (PANS), paraneoplastic syndromes, perivenous encephalomyelitis, reflex sympathetic dystrophy, relapsing polychondritis, sperm & testicular autoimmunity, Susac's syndrome, Tolosa-Hunt syndrome, Vogt-Koyanagi-Harada Disease, anti-synthetase syndrome, autoimmune enteropathy, immune dysregulation polyendocrinopathy enteropathy X-linked (IPEX), microscopic colitis, autoimmune lymphoproliferative syndrome (ALPS), autoimmune polyendocrinopathycandidiasis-ectodermal dystrophy syndrome (APEX), gout, pseudogout, amyloid, eosinophilic fasciitis (Shulman syndrome) progesterone hypersensitivity, amilial Mediterranean fever (FMF), tumour necrosis factor (TNF) receptor-associated periodic fever syndrome (TRAPS), hyperimmunoglobulinaemia D with periodic fever syndrome (HIDS), PAPA (pyogenic arthritis, pyoderma gangrenosum, severe cystic acne) syndrome, deficiency of interleukin-1 receptor antagonist (DIRA), deficiency of the interleukin-36-receptor antagonist (DITRA), cryopyrin-associated periodic syndromes (CAPS), NLRP12-associated autoinflammatory disorders (NLRP12AD), periodic fever aphthous stomatitis (PFAPA), chronic atypical neutrophilic dermatosis with lipodystrophy and elevated temperature (CANDLE), Majeed syndrome, Blau syndrome (also known as juvenile systemic granulomatosis), macrophage activation syndrome, chronic recurrent multifocal osteomyelitis (CRMO), familial cold autoinflammatory syndrome, mutant adenosine deaminase 2 and monogenic interferonopathies, Schnitzler syndrome; familial cylindromatosis, congenital B cell lymphocytosis, OTULIN-related autoinflammatory syndrome, type 2 diabetes mellitus, insulin resistance and the metabolic syndrome, atherosclerotic disorders, and renal inflammatory disorders; and
- the cancer is selected from the group consisting of: acute lymphoblastic leukaemia, adult; acute lymphoblastic leukaemia, childhood; acute myeloid leukaemia, adult; adrenocortical carcinoma; adrenocortical carcinoma, childhood; aids-related lymphoma; aids-related malignancies; anal cancer; astrocytoma, childhood cerebellar; astrocytoma, childhood cerebral; Barrett's esophagus (pre-malignant syndrome); bile duct cancer, extrahepatic; bladder cancer; bladder cancer, childhood; bone cancer, osteosarcoma/malignant fibrous histiocytoma; brain stem glioma, childhood; brain tumour, adult; brain tumour, brain stem glioma, childhood; brain tumour, cerebellar astrocytoma, childhood; brain tumour, cerebral astrocytoma/malignant glioma, childhood; brain tumour, ependymoma, childhood; brain tumour, medulloblastoma, childhood; brain tumour, supratentorial primitive neuroectodermal tumours, childhood; brain tumour, visual pathway and hypothalamic glioma, childhood; brain tumour, childhood (other); breast cancer; breast cancer and pregnancy; breast cancer, childhood; breast cancer, male; bronchial adenomas/carcinoids, childhood; carcinoid tumour, childhood; carcinoid tumour, gastrointestinal; carcinoma, adrenocortical; carcinoma, islet cell; carcinoma of unknown primary; central nervous system lymphoma, primary; cerebellar astrocytoma, childhood; cerebral astrocytoma/malignant glioma, childhood; cervical cancer; childhood cancers; chronic lymphocytic leukaemia; chronic myelogenous leukaemia; chronic myeloproliferative disorders; clear cell sarcoma of tendon sheaths; colon cancer; colorectal cancer; colorectal cancer, childhood; cutaneous t-cell lymphoma; endometrial cancer; ependymoma, childhood; epithelial cancer, ovarian; oesophageal cancer; oesophageal cancer, childhood; Ewing's family of tumours; extracranial germ cell tumour, childhood; extragonadal germ cell tumour; extrahepatic bile duct cancer; eye cancer, intraocular melanoma; eye cancer, retinoblastoma; gallbladder cancer; gastric (stomach) cancer; gastric (stomach) cancer, childhood; gastrointestinal carcinoid tumour; germ cell tumour, extracranial, childhood; germ cell tumour, extragonadal; germ cell tumour, ovarian; gestational trophoblastic tumour; glioma, childhood brain stem; glioma, childhood visual pathway and hypothalamic; hairy cell leukaemia; head and neck cancer; hepatocellular (liver) cancer; hepatocellular (liver) cancer, adult (primary); hepatocellular (liver) cancer, childhood (primary); cancer of the esophagus; Hodgkin's lymphoma; Hodgkin's lymphoma, adult; Hodgkin's lymphoma, childhood; Hodgkin's lymphoma during pregnancy; hypopharyngeal cancer; hypothalamic and visual pathway glioma, childhood; intraocular melanoma; islet cell carcinoma (endocrine pancreas); cancer of the endocrine system; Kaposi's sarcoma; kidney cancer; laryngeal cancer; laryngeal cancer, childhood; leukaemia, acute lymphoblastic, adult; leukaemia, acute lymphoblastic, childhood; leukaemia, acute myeloid, adult; leukaemia, acute myeloid, childhood; leukaemia, chronic lymphocytic; leukaemia, chronic myelogenous; leukaemia, hairy cell; lymphocytic lymphoma; lip and oral cavity cancer; liver cancer, adult (primary); liver cancer, childhood (primary); lung cancer; lung cancer, non-small cell; lung cancer, small cell; lymphoblastic leukaemia, adult acute; lymphoblastic leukaemia, childhood acute; lymphocytic leukaemia, chronic; lymphoma, aids-related; lymphoma, central nervous system (primary); lymphoma, cutaneous t-cell; lymphoma, Hodgkin's, adult; lymphoma, Hodgkin's, childhood; lymphoma, Hodgkin's during pregnancy; lymphoma, non-Hodgkin's, adult; lymphoma, non-Hodgkin's, childhood; lymphoma, non-Hodgkin's during pregnancy; lymphoma, primary central nervous system; macroglobulinemia, Waldenstrom's; male breast cancer; malignant mesothelioma, adult; malignant mesothelioma, childhood; malignant thymoma; medulloblastoma, childhood; melanoma; melanoma, intraocular; Merkel cell carcinoma; mesothelioma, malignant; metastatic squamous neck cancer with occult primary; multiple endocrine neoplasia syndrome, childhood; multiple myeloma/plasma cell neoplasm; mycosis fungoides; myelodysplastic syndromes; myelogenous leukaemia, chronic; myeloid leukaemia, childhood acute; myeloma, multiple; myeloproliferative disorders, chronic; nasal cavity and paranasal sinus cancer; nasopharyngeal cancer; nasopharyngeal cancer, childhood; neoplastic cutaneous disease; neuroblastoma; non-Hodgkin's lymphoma, adult; non-Hodgkin's lymphoma, childhood; non-Hodgkin's lymphoma during pregnancy; non-small cell lung cancer; neoplasms of the central nervous system; oat-cell cancer; oral cancer, childhood; oral cavity and lip cancer; oropharyngeal cancer; osteosarcoma/malignant fibrous histiocytoma of bone; ovarian cancer; ovarian cancer, childhood; ovarian epithelial cancer; ovarian germ cell tumour; ovarian low malignant potential tumour; pediatric malignancy; pancreatic cancer; pancreatic cancer, childhood; pancreatic cancer, islet cell; paranasal sinus and nasal cavity cancer; parathyroid cancer; penile cancer; pheochromocytoma; pineal and supratentorial primitive neuroectodermal tumours, childhood; pituitary tumour; plasma cell neoplasm/multiple myeloma; pleuropulmonary blastoma; pregnancy and breast cancer; pregnancy and Hodgkin's lymphoma; pregnancy and non-Hodgkin's lymphoma; primary central nervous system lymphoma; primary liver cancer, adult; primary liver cancer, childhood; prostate cancer; chronic or acute leukemia; solid tumors of childhood; hypereosinophilia; rectal cancer; renal cell (kidney) cancer; renal cell cancer, childhood; renal pelvis and ureter, transitional cell cancer; retinoblastoma; rhabdomyosarcoma, childhood; salivary gland cancer; salivary gland cancer, childhood; sarcoma, Ewing's family of tumours; sarcoma, Kaposi's; sarcoma (osteosarcoma)/malignant fibrous histiocytoma of bone; sarcoma, rhabdomyosarcoma, childhood; sarcomas of soft tissues; sarcoma, soft tissue, adult; sarcoma, soft tissue, childhood; Sezary syndrome; skin cancer; skin cancer, childhood; skin cancer (melanoma); skin carcinoma, Merkel cell; small cell lung cancer; dermatofibrosarcoma protuberans; small intestine cancer; soft tissue sarcoma, adult; soft tissue sarcoma, childhood; cancer of the head and neck; squamous neck cancer with occult primary, metastatic; stomach (gastric) cancer; stomach (gastric) cancer, childhood; supratentorial primitive neuroectodermal tumours, childhood; t-cell lymphoma, cutaneous; testicular cancer; thymoma, childhood; thymoma, malignant; thyroid cancer; thyroid cancer, childhood; transitional cell cancer of the renal pelvis and ureter; trophoblastic tumour, gestational; unknown primary site, cancer of, childhood; unusual cancers of childhood; ureter and renal pelvis, transitional cell cancer; urethral cancer; cancer of the ureter; cancer of the penis; gynecologic tumors; uterine cancer; uterine sarcoma; carcinoma of the fallopian tubes; carcinoma of the endometrium; vaginal cancer; carcinoma of the vagina; carcinoma of the vulva; visual pathway and hypothalamic glioma, childhood; vulva cancer; Waldenstrom's macro globulinemia; and Wilms' tumour.

12. The compound, or pharmaceutically acceptable salt and/or solvate thereof for use, or pharmaceutical composition for use according to claim 11, wherein the inflammatory disease or disease associated with an undesirable immune response is selected from the group consisting of rheumatoid arthritis, psoriatic arthritis, ankylosing spondylitis, systemic lupus erythematosus, multiple sclerosis, psoriasis, inflammatory bowel disease, atopic dermatitis, fibrosis, uveitis, cryopyrin-associated periodic syndromes, Muckle-Wells syndrome, juvenile idiopathic arthritis, chronic obstructive pulmonary disease and asthma.

13. The compound or pharmaceutically acceptable salt and/or solvate thereof for use, or pharmaceutical composition for use, according to any one of claims 9 to 12, for use in combination with a further therapeutic agent wherein the further therapeutic agent is selected from the group consisting of:
- a corticosteroid (glucocorticoid), retinoid, anthralin, vitamin D analogue, calcineurin inhibitors, phototherapy or photochemotherapy or other form of ultraviolet light irradiation therapy, ciclosporine, a thiopurine, methotrexate, an anti-TNFα agent, phosphodiesterase-4 (PDE4) inhibition, anti-IL-17 agent, anti-IL12/IL-23 agent, anti-IL-23 agent, JAK (Janus Kinase) inhibitor, plasma exchange, intravenous immune globulin (IVIG), cyclophosphamide, anti-CD20 B cell depleting agent, anthracycline analogue, cladribine, sphingosine 1-phosphate receptor modulator or sphingosine analogue, interferon beta preparation, glatiramer, anti-CD3 therapy, anti-CD52 targeting agent, leflunomide, teriflunomide, gold compound, laquinimod, potassium channel blocker, mycophenolic acid, mycophenolate mofetil, purine analogue, mTOR (mechanistic target of rapamycin) pathway inhibitor, anti-thymocyte globulin (ATG), IL-2 receptor (CD25) inhibitor, anti-IL-6 receptor or anti-IL-6 agent, Bruton's tyrosine kinase (BTK) inhibitor, tyrosine kinase inhibitor, ursodeoxycholic acid, hydroxychloroquine, chloroquine, B cell activating factor (BAFF, also known as BlyS, B lymphocyte stimulator) inhibitor, other B cell targeted therapy including a fusion protein targeting both APRIL (A Proliferation-Inducing Ligand) and BlyS, PI3K inhibitor including pan-inhibitor or one targeting the p110δ and/or p110γ containing isoforms, an interferon α receptor inhibitor, T cell co-stimulation blocker, thalidomide and its derivatives, dapsone, clofazimine, a leukotriene antagonist, theophylline, anti-IgE therapy, an anti-IL-5 agent, a longacting muscarinic agent, a PDE4 inhibitor, riluzole, a free radical scavenger, a proteasome inhibitor, a complement cascade inhibitor including one directed against C5, immunoadsor, antithymocyte globulin, 5-aminosalicylates and their derivatives, an anti-integrin agent including one targeting α4β1 and/or α4β7 integrins, an anti-CD11-α agent, a non-steroidal antiinflammatory drug (NSAID) including a salicylate, a propionic acid, an acetic acid, an oxicam a fenamate, a selective or relatively selective COX-2 inhibitor, colchicine, an IL-4 receptor inhibitor, topical/contact immunotherapy, anti-IL-1 receptor therapy, IL-1β inhibitor, IL-1 neutralising therapy, chlorambucil, a specific antibiotic with immunomodulatory properties and/or ability to modulate NRF2, anti-androgenic therapy, pentoxifylline, ursodeoxycholic acid, obeticholic acid, fibrate, a cystic fibrosis transmembrane conductance regulator (CFTR) modulator, a VEGF (vascular endothelial growth factor) inhibitor, pirfenidone, and mizoribine;
- a palliative treatment selected from the group consisting of: antiemetic agents, medication intended to alleviate pain, medication used to decrease high blood uric acid levels, anti-depressants, sedatives, anti-convulsant drugs, laxatives, anti-diarrhoeal drugs and antacids;
- an additional cancer treatment selected from the group consisting of chemotherapy, a targeted therapy, immunotherapy and a hormonal therapy; and
- a gastric or pancreatic lipase inhibitor; a lipid lowering agent; a CB-1 antagonist; a lipoxygenase inhibitor; a somostatin analogue; an insulin compound or insulin analogue; an insulin sensitising agent such as a PPAR-gamma agonist, PPAR-alpha agonist or mixed PPAR-gamma/alpha agonist; an insulin secretagogue; an SGLT2 inhibitor; an amylin analogue; a DPPIV inhibitor; a GLP-1 agonist; an alpha-glucosidase inhibitor; a phosphodiesterase inhibitor; a glycogen phosphorylase inhibitor; an MCH-1 antagonist; a glucokinase activator; a glucagon antagonist; an insulin signalling agonist; a PTP1B inhibitor; a gluconeogenesis inhibitor; a GSK inhibitor or a galanin receptor agonist.

14. A process for preparing a compound of formula (la) as described in any one of claims 1 to 7, or a salt thereof, which comprises:
(a) reacting a compound of formula (IIa): 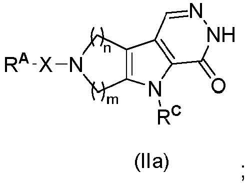
or a salt thereof;
with a compound of formula (Illa): 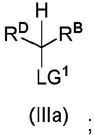
wherein R^{A}, R^{B}, R^{C}, R^{D}, X, m and n are defined according to any one of claims 1 to 6, and LG¹ is a leaving group; or
(b) reacting a compound of formula (IVa): 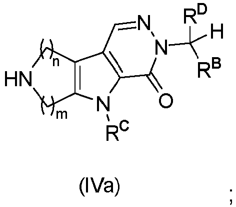
or a salt thereof;
with a compound of formula (Va): 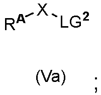
wherein R^{A}, R^{B}, R^{C}, R^{D}, X, m and n are defined according to any one of claims 1 to 6, and LG² is a leaving group.

15. A compound selected from the group consisting of:
(a) a compound of formula (IIa): 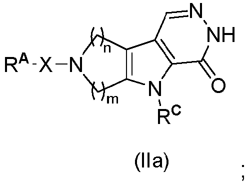
or a salt thereof;
wherein R^{A}, R^{C}, X, m and n are defined according to any one of claims 1 to 3, 5 or 6; and
(b) a compound of formula (IVa): 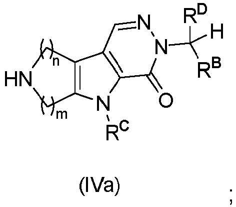
or a salt thereof;
wherein R^{B}, R^{C}, R^{D}, m and n are defined according to any one of claims 1 or 4 to 6.

## Patentansprüche

1. Verbindung der Formel (Ia): 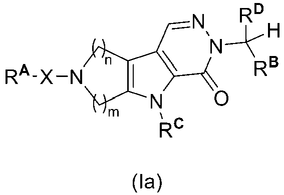 wobei
R^{A} ein C₁₋₁₀-Alkyl, C₂₋₁₀-Alkenyl, C₂₋₁₀-Alkinyl, C₃₋₁₀-Cycloalkyl, Phenyl oder 5-10-gliedriges Heteroaryl ist; wobei R^{A} gegebenenfalls an einem verfügbaren Atom durch ein oder mehrere R^{1A} substituiert ist, wobei jedes R^{1A} unabhängig ausgewählt ist aus der Gruppe, bestehend aus Halogen, C₁₋₆-Alkyl, C₁₋₆-Hydroxyalkyl, C₁₋₆-Halogenalkyl, Hydroxy, OC₁₋₆-Alkyl, OC₁₋₆-Halogenalkyl, Cyano, NR^{2A}R^{3A}, CO₂H, CONR^{2A}R^{3A} und C₃₋₆-Cycloalkyl; R^{2A} und R^{3A} unabhängig voneinander ausgewählt sind aus der Gruppe, bestehend aus H und C₁₋₆-Alkyl; oder R^{2A} und R^{3A} sich zusammen mit dem N-Atom, an das sie gebunden sind, unter Bilden eines 5-7-gliedrigen heterocyclischen Ring vereinigen, der gegebenenfalls an einem verfügbaren Atom durch eine oder mehrere Gruppen substituiert ist, ausgewählt aus C₁₋₂-Alkyl und Oxo;
R^{B} Phenyl, an einen 5-7-gliedrigen heterocyclischen Ring kondensiertes Phenyl, ein 5-10-gliedriges Heteroaryl oder ein 4-7-gliedriges Heterocyclyl ist; wobei R^{B} gegebenenfalls an einem verfügbaren Atom durch ein oder mehrere R^{1B} substituiert ist, wobei jedes R^{1B} unabhängig ausgewählt ist aus der Gruppe, bestehend aus Halogen, C₁₋₆-Alkyl, C₁₋₆-Hydroxyalkyl, C₁₋₆-Aminoalkyl, C₁₋₆-Halogenalkyl, Hydroxy, OC₁₋₆-Alkyl, OC₁₋₆-Halogenalkyl, Cyano, NR^{2B}R^{3B}, CO₂H, CONR^{2B}R^{3B}, S(O)₂NR^{2B}R^{3B}, S(O)₂C₁₋₆-Alkyl, C₃₋₆-Cycloalkyl und Oxo;
R^{2B} und R^{3B} unabhängig voneinander ausgewählt sind aus der Gruppe, bestehend aus H und C₁₋₆-Alkyl; oder R^{2B} und R^{3B} sich zusammen mit dem N-Atom, an das sie gebunden sind, unter Bilden eines 5-7-gliedrigen heterocyclischen Ring vereinigen, der gegebenenfalls an einem verfügbaren Atom durch eine oder mehrere Gruppen substituiert ist, ausgewählt aus C₁₋₂-Alkyl und Oxo;
R^{C} H oder C₁₋₂-Alkyl ist;
R^{D} H, C₁₋₂-Alkyl, C₁₋₂-Hydroxyalkyl oder C₁₋₂-Methoxyalkyl ist; X C=O, S(=O)₁₋₂, -CH₂-S(=O)₁₋₂-, -S(=O)(=NH) oder -NH-S(=O)₂- ist; n 0 bis 4 ist und m 0 bis 4 ist, wobei n + m gleich 2, 3 oder 4 ist;
oder ein pharmazeutisch unbedenkliches Salz und/oder Solvat davon.

2. Verbindung, pharmazeutisch unbedenkliches Salz und/oder Solvat davon nach Anspruch 1, wobei R^{A} C₁₋₁₀-Alkyl, C₃₋₁₀-Cycloalkyl, Phenyl oder 5-10-gliedriges Heteroaryl ist und gegebenenfalls an einem verfügbaren Atom durch ein oder mehrere R^{1A} substituiert ist, wobei jedes R^{1A} unabhängig ausgewählt ist aus der Gruppe, bestehend aus Halogen, C₁₋₆-Alkyl, C₁₋₆-Hydroxyalkyl, C₁₋₆-Halogenalkyl, Hydroxy, OC₁₋₆-Alkyl, OC₁₋₆-Halogenalkyl, Cyano und NR^{2A}R^{3A}.

3. Verbindung, pharmazeutisch unbedenkliches Salz und/oder Solvat davon nach Anspruch 2, wobei R^{A} Methyl, Ethyl oder n-Propyl ist und gegebenenfalls durch OC₁₋₄-Alkyl substituiert ist oder wobei R^{A} Phenyl ist, gegebenenfalls an einem verfügbaren Kohlenstoffatom substituiert durch OC₁₋₆-Alkyl.

4. Verbindung, pharmazeutisch unbedenkliches Salz und/oder Solvat davon nach einem der Ansprüche 1 bis 3, wobei R^{B} ausgewählt ist aus der Gruppe bestehend aus Phenyl, an einen 5-7-gliedrigen heterocyclischen Ring kondensiertes Phenyl, Pyrrolyl, Furanyl, Thienyl, Imidazolyl, Pyrazolyl, Thiazolyl, Isothiazolyl, Oxadiazolyl, Thiadiazolyl, Triazolyl, Oxazolyl, Isoxazolyl, Tetrazolyl, Pyridinyl, Pyrimidinyl, Pyradizinyl, Pyrazinyl, Furo[3,2-b]pyridinyl, Pyrazolo[1,5-a]pyridinyl, Pyrrolo[2,3-b]pyridinyl, Pyrrolo[2,3-c]pyridinyl, Imidazo[1,2-a]pyridinyl, Thieno[3,2-c]pyridinyl, Indolyl, Indazolyl, Benzofuranyl, Benzimidazolyl, Benzothiazolyl, Benzothiophenyl, Chinolinyl, Isochinolinyl, Chinazolinyl, Pyrrolidinyl, Tetrahydrofuranyl, Tetrahydrothienyl, Tetrahydropyranyl, Piperidinyl, Piperazinyl, Morpholinyl, Thiomorpholinyl, Pyridon und Pyridazinon; und gegebenenfalls an einem verfügbaren Atom durch einen oder mehrere R^{1B} substituiert ist, wobei jedes R^{1B} unabhängig ausgewählt ist aus der Gruppe, bestehend aus Halogen, C₁₋₄-Alkyl, C₁₋₄-Hydroxyalkyl, C₁₋₆-Aminoalkyl, C₁₋₄-Halogenalkyl, Hydroxy, OC₁₋₄-Alkyl, OC₁₋₄-Halogenalkyl, Cyano, NR^{2B}R^{3B}, CONR^{2B}R^{3B}, S(O)₂NR^{2B}R^{3B}, S(O)₂C₁₋₆-Alkyl, C₃₋₅-Cycloalkyl und Oxo.

5. Verbindung oder ein pharmazeutisch unbedenkliches Salz und/oder Solvat davon nach einem der Ansprüche 1 bis 4, wobei R^{C} Methyl ist, R^{D} H ist und X S(=O)₂ ist.

6. Verbindung, pharmazeutisch unbedenkliches Salz und/oder Solvat davon nach einem der Ansprüche 1 bis 5, wobei n 2 ist und m 1 ist und die Verbindung der Formel (Ia) eine Verbindung der Formel (IaA) ist: 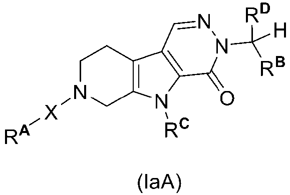
wobei R^{A}, R^{B}, R^{C}, R^{D} und X wie in einem der Ansprüche 1 bis 5 definiert sind;
oder ein pharmazeutisch unbedenkliches Salz und/oder Solvat davon.

7. Verbindung, pharmazeutisch unbedenkliches Salz und/oder Solvat davon nach Anspruch 1, ausgewählt aus der Gruppe, bestehend aus:
3-((2,3-Dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-on;
3-((6-Methoxypyridin-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-(3-(1-Hydroxyethyl)benzyl)-5-methyl-7-(methylsulfonyl)-3,5,6,7,8,9-hexahydro-4H-pyrido[4',3':4,5]pyrrolo[2,3-d]pyridazin-4-on;
3-((1H-Pyrazol-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-(2-Fluor-3-methoxybenzyl)-5-methyl-7-(methylsulfonyl)-3,5,6,7,8,9-hexahydro-4H-pyrido[4',3':4,5]pyrrolo[2,3-d]pyridazin-4-on;
3-(2,6-Difluor-4-methoxybenzyl)-5-methyl-7-(methylsulfonyl)-3,5,6,7,8,9-hexahydro-4H-pyrido[4',3':4,5]pyrrolo[2,3-d]pyridazin-4-on;
3-(3-Aminobenzyl)-5-methyl-7-(methylsulfonyl)-3,5,6,7,8,9-hexahydro-4H-pyrido[4',3':4,5]pyrrolo[2,3-d]pyridazin-4-on;
7-Acetyl-3-(3-aminobenzyl)-5-methyl-3,5,6,7,8,9-hexahydro-4H-pyrido[4',3':4,5]pyrrolo[2,3-d]pyridazin-4-on;
3-(3-Aminobenzyl)-7-(cyclopropylsulfonyl)-5-methyl-3,5,6,7,8,9-hexahydro-4H-pyrido[4',3':4,5]pyrrolo[2,3-d]pyridazin-4-on;
3-(3-Aminobenzyl)-5-methyl-7-((2-methylthiazol-5-yl)sulfonyl)-3,5,6,7,8,9-hexahydro-4H-pyrido[4',3':4,5]pyrrolo[2,3-d]pyridazin-4-on;
3-(3-Aminobenzyl)-5-methyl-7-(phenylsulfonyl)-3,5,6,7,8,9-hexahydro-4H-pyrido[4',3':4,5]pyrrolo[2,3-d]pyridazin-4-on; und
3-((1H-Pyrazol-3-yl)methyl)-5-methyl-7-(phenylsulfonyl)-3,5,6,7,8,9-hexahydro-4H-pyrido[4',3':4,5]pyrrolo[2,3-d]pyridazin-4-on;
3-((2-(Hydroxymethyl)thiophen-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-((5-Methyl-7-(methylsulfonyl)-4-oxo-4,5,6,7,8,9-hexahydro-3H-pyrido[4',3':4,5]pyrrolo[2,3-d]pyridazin-3-yl)methyl)thiophen-2-carboxamid;
3-((1H-Indol-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-(Benzofuran-3-ylmethyl)-5-methyl-7-(methylsulfonyl)-3,5,6,7,8,9-hexahydro-4H-pyrido[4',3':4,5]pyrrolo[2,3-d]pyridazin-4-on;
3-((5-Methyl-7-(methylsulfonyl)-4-oxo-4,5,6,7,8,9-hexahydro-3H-pyrido[4',3':4,5]pyrrolo[2,3-d]pyridazin-3-yl)methyl)-1H-pyrazol-4-carboxamid;
3-((5-Methyl-7-(methylsulfonyl)-4-oxo-4,5,6,7,8,9-hexahydro-3H-pyrido[4',3':4,5]pyrrolo[2,3-d]pyridazin-3-yl)methyl)benzolsulfonamid;
5-Methyl-7-(methylsulfonyl)-3-((6-(pyrrolidin-1-yl)pyridin-2-yl)methyl)-3,5,6,7,8,9-hexahydro-4H-pyrido[4',3':4,5]pyrrolo[2,3-d]pyridazin-4-on;
3-((1H-Indazol-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-(2-Fluor-4-methoxybenzyl)-5-methyl-7-(phenylsulfonyl)-3,5,6,7,8,9-hexahydro-4H-pyrido[4',3':4,5]pyrrolo[2,3-d]pyridazin-4-on;
3-((7-((4-Methoxyphenyl)sulfonyl)-5-methyl-4-oxo-4,5,6,7,8,9-hexahydro-3H-pyrido[4',3':4,5]pyrrolo[2,3-d]pyridazin-3-yl)methyl)thiophen-2-carboxamid;
3-((2,3-Dihydrobenzofuran-5-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-on;
3-((2,2-Dimethyl-2,3-dihydrobenzofuran-5-yl)methyl)-5-methyl-7-(methylsulfonyl)-3,5,6,7,8,9-hexahydro-4H-pyrido[4',3':4,5]pyrrolo[2,3-d]pyridazin-4-on;
3-((1H-Pyrrolo[2,3-b]pyridin-6-yl)methyl)-5-methyl-7-(methylsulfonyl)-3,5,6,7,8,9-hexahydro-4H-pyrido[4',3':4,5]pyrrolo[2,3-d]pyridazin-4-on;
(S)-3-(3-(1-Aminoethyl)benzyl)-5-methyl-7-(methylsulfonyl)-3,5,6,7,8,9-hexahydro-4H-pyrido[4',3':4,5]pyrrolo[2,3-d]pyridazin-4-on;
7-((2-Methoxyethyl)sulfonyl)-3-((6-methoxypyridin-3-yl)methyl)-5-methyl-3,5,6,7,8,9-hexahydro-4H-pyrido[4',3':4,5]pyrrolo[2,3-d]pyridazin-4-on;
N-(2-Methoxyethyl)-3-((6-methoxypyridin-3-yl)methyl)-5-methyl-4-oxo-3,4,5,6,8,9-hexahydro-7H-pyrido[4',3':4,5]pyrrolo[2,3-d]pyridazin-7-sulfonamid;
3-((1H-Pyrrolo[2,3-c]pyridin-3-yl)methyl)-7-((2-methoxyethyl)sulfonyl)-5-methyl-6,7,8,9-tetrahydro-3H-pyrido[4',3':4,5]pyrrolo[2,3-d]pyridazin-4(5H)-on;
(3-((1H-Pyrrolo[2,3-c]pyridin-3-yl)methyl)-N-ethyl-5-methyl-4-oxo-5,6,8,9-tetrahydro-3H-pyrido[4',3':4,5]pyrrolo[2,3-d]pyridazin-7(4H)-sulfonamid;
5-Methyl-7-(methylsulfonyl)-3-(3-(methylsulfonyl)benzyl)-3,5,6,7,8,9-hexahydro-4H-pyrido[4',3':4,5]pyrrolo[2,3-*d*]pyridazin-4-on;
3-((1*H*-Indazol-4-yl)methyl)-5-methyl-7-(methylsulfonyl)-3,5,6,7,8,9-hexahydro-4*H*-pyrido[4',3':4,5]pyrrolo[2,3-*d*]pyridazin-4-on;
3-(3-(2-Hydroxypropan-2-yl)benzyl)-5-methyl-7-(methylsulfonyl)-3,5,6,7,8,9-hexahydro-4H-pyrido[4',3':4,5]pyrrolo[2,3-d]pyridazin-4-on;
5-Methyl-7-(methylsulfonyl)-3-(thieno[3,2-c]pyridin-3-ylmethyl)-3,5,6,7,8,9-hexahydro-4H-pyrido[4',3':4,5]pyrrolo[2,3-d]pyridazin-4-on;
3-((1H-Pyrrolo[2,3-c]pyridin-3-yl)methyl)-7-((4-methoxyphenyl)sulfonyl)-5-methyl-3,5,6,7,8,9-hexahydro-4H-pyrido[4',3':4,5]pyrrolo[2,3-d]pyridazin-4-on;
3-((1H-Pyrrolo[2,3-c]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-((1H-Pyrrolo[2,3-b]pyridin-3-yl)methyl)-5-methyl-7-(methylsulfonyl)-3,5,6,7,8,9-hexahydro-4H-pyrido[4',3':4,5]pyrrolo[2,3-d]pyridazin-4-on;
3-((5-Fluor-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-on; und
3-((6-Fluor-1H-indol-3-yl)methyl)-5-methyl-7-(methylsulfonyl)-6,7,8,9-tetrahydro-3H-pyrido[4',3':4,5]pyrrolo[2,3-d]pyridazin-4(5H)-on;
oder ein pharmazeutisch unbedenkliches Salz und/oder Solvat einer beliebigen davon.

8. Pharmazeutische Zusammensetzung, umfassend eine Verbindung, ein pharmazeutisch unbedenkliches Salz und/oder ein Solvat davon nach einem der Ansprüche 1 bis 7 und einen oder mehrere pharmazeutisch unbedenkliche Verdünnungsmittel oder Träger.

9. Verbindung, pharmazeutisch unbedenkliches Salz und/oder Solvat davon nach einem der Ansprüche 1 bis 7 oder pharmazeutische Zusammensetzung nach Anspruch 8 zur Verwendung als Medikament.

10. Verbindung oder pharmazeutisch unbedenkliches Salz und/oder Solvat davon zur Verwendung oder pharmazeutische Zusammensetzung zur Verwendung nach Anspruch 9 zum Behandeln oder Verhindern einer entzündlichen Krankheit, einer mit einer unerwünschten Immunantwort verknüpften Krankheit, Krebs, Adipositas, einer diabetischen Krankheit oder einer Blutstörung.

11. Verbindung oder pharmazeutisch unbedenkliches Salz und/oder Solvat davon zur Verwendung oder pharmazeutische Zusammensetzung zur Verwendung nach Anspruch 10, wobei:
- die entzündliche Krankheit oder die mit einer unerwünschten Immunantwort verknüpfte Krankheit eine Krankheit ist oder damit verknüpft ist, ausgewählt aus der Gruppe, bestehend aus: Psoriasis, Asthma, chronisch obstruktiver Lungenkrankheit (COPD), Herzinsuffizienz, Myokardinfarkt, Angina pectoris, anderen mit Atherosklerose und/oder Atherothrombose zusammenhängenden Störungen, einer mitochondrialen und neurodegenerativen Krankheit, autoimmuner paraneoplastischer Retinopathie, Transplantationsabstoßung , multipler Sklerose, transversaler Myelitis, Ischämie-Reperfusionsverletzung, AGEinduzierten Genomschäden, einer entzündlichen Darmkrankheit, primär sklerosierender Cholangitis (PSC), PSC-Autoimmunhepatitis-Überlappungssyndrom, nichtalkoholischer Fettleberkrankheit (nichtalkoholischer Steatohepatitis), Rheumatica, Granuloma annulare, kutanem Lupus erythematodes (CLE), systemischem Lupus erythematodes (SLE), Lupusnephritis, medikamenteninduziertem Lupus, autoimmuner Myokarditis oder Myoperikarditis, Dressler-Syndrom, Riesenzellmyokarditis, Postperikardiotomie-Syndrom, arzneimittelinduzierten Überempfindlichkeitssyndromen, Ekzem, Sarkoidose, Erythema nodosum, akuter disseminierter Enzephalomyelitis (ADEM), Neuromyelitis-optica-Spektrum-Störungen, MOG(Myelin-Oligodendrozyten-Glykoprotein)-Antikörper-assoziierten Störungen, Sehnervenentzündung, CLIPPERS (chronisch lymphozytärer Entzündung mit perivaskulärer Anreicherung im Gehirn, die auf Steroide anspricht), diffuser myelinoklastischer Sklerose, Morbus Addison, Alopecia areata, ankylosierender Spondylitis, anderen Spondyloarthritiden, Antiphospholipid-Antikörper-Syndrom, autoimmuner hämolytischer Anämie, autoimmuner Hepatitis, autoimmunen Innenohrkrankheiten, Pemphigoid, linearer IgA-Krankheit, Behçet-Krankheit, Zöliakie, Chagas-Krankheit, Dermatomyositis, Diabetes mellitus Typ I, Endometriose, Goodpasture-Syndrom, Morbus Basedow, Guillain-Barre-Syndrom und seinen Subtypen , progressiver entzündlicher Neuropathie, Hashimoto-Krankheit, Hidradenitis suppurativa, Einschlusskörpermyositis, nekrotisierender Myopathie, Kawasaki-Krankheit, IgA-Nephropathie, Henoch-Schonlein-Purpura, idiopathischer thrombozytopenischer Purpura, thrombotischer thrombozytopenischer Purpura (TTP), Evans-Syndrom, interstitieller Zystitis, gemischter Bindegewebskrankheit, undifferenzierter Bindegewebskrankheit, Morphea, Myasthenia gravis, Narkolepsie, Neuromyotonie, Pemphigus vulgaris, perniziöser Anämie, Psoriasis-Arthritis, Polymyositis, primär biliärer Cholangitis (auch bekannt als primär biliäre Zirrhose), rheumatoider Arthritis, palindromischem Rheumatismus, Schizophrenie, Autoimmun-Enzephalitis-Syndromen, Autoimmun-Meningoenzephalitis-Syndromen, Sklerodermie, Sjögren-Syndrom, Stiff-Person-Syndrom, Polymylagia rheumatica, Riesenzellarteriitis (Arteriitis temporalis), Takayasu-Arteriitis, Polyarteriitis nodosa, Kawasaki-Krankheit, Granulomatose mit Polyangiitis (GPA; früher bekannt als Wegenersche Granulomatose), eosinophiler Granulomatose mit Polyangiitis (EGPA; früher bekannt als Churg-Strauss-Syndrom), mikroskopischer Polyarteriitis/Polyangiitis, hypokomplementämischer urtikarieller Vaskulitis, Überempfindlichkeitsvaskulitis, Kryoglobulinämie, Thromboangiitis obliterans (Morbus Buerger), Vaskulitis, leukozytoklastischer Vaskulitis, Vitiligo, akuter disseminierter Enzephalomyelitis, Adrenoleukodystrophie, Alexander-Krankheit, Alper-Krankheit, balo-konzentrischer Sklerose oder Marburg-Krankheit, kryptogener organisierender Lungenentzündung (früher bekannt als Bronchiolitis obliterans organisierende Lungenentzündung), Canavan-Krankheit, vaskulitischem Syndrom des Zentralnervensystems, Charcot-Marie-Tooth-Krankheit, kindlicher Ataxie mit Hypomyelinisierung des Zentralnervensystems, chronisch entzündlicher demyelinisierender Polyneuropathie (CIDP), diabetischer Retinopathie, Globoidzell-Leukodystrophie (Krabbe-Krankheit), Transplantat-gegen-Wirt-Krankheit (GVHD) , Hepatitis-C(HCV)-Infektion oder Komplikation, Herpes-simplex-Virusinfektion oder
- Komplikation, Infektion oder Komplikation mit dem Humanen Immundefizienz-Virus (HIV), Lichen planus, monomelischer Amyotrophie, Fibrose, zystischer Fibrose, pulmonaler arterieller Hypertonie (PAH), Lungensarkoidose, idiopathischer Lungenfibrose, Nierenfibrose, pädiatrischem Asthma, atopischer Dermatitis, allergischer Dermatitis, Kontaktdermatitis, allergischer Rhinitis, Rhinitis, Sinusitis, Bindehautentzündung, allergischer Bindehautentzündung, Keratokonjunktivitis sicca, trockenem Auge, Xerophthalmie, Glaukom, Makulaödem, diabetischem Makulaödem, retinalem Zentralvenenverschluss (ZVV), Makuladegeneration, postoperativer Kataraktentzündung, Uveitis, Iridozyklitis, Skleritis, Abstoßung von Hornhauttransplantaten und Limbustransplantaten, glutensensitiver Enteropathie (Zöliakie), Dermatitis herpetiformis, eosinophiler Ösophagitis, Achalasie, Autoimmundysautonomie, Autoimmun-Enzephalomyelitis, Autoimmun-Oophoritis, Autoimmun-Orchitis, Autoimmun-Pankreatitis, Aortitis und Periaortitis, Autoimmun-Retinopathie, Autoimmun-Urtikaria, Morbus Behcet, Castleman-Krankheit, Cogan-Syndrom, IgG4-bedingter Krankheit, retroperitonealer Fibrose, juveniler idiopathischer Arthritis, Morbus Still mit Beginn im Erwachsenenalter, holziger Bindehautentzündung, Ulcus Mooren, Pityriasis lichenoides et varioliformis acuta (PLEVA, auch bekannt als Mucha-Habermann-Krankheit), multifokaler motorischer Neuropathie (MMN), pädiatrischem akutem neuropsychiatrischem Syndrom (PANS), paraneoplastischen Syndromen, perivenöser Enzephalomyelitis, Reflex-Sympathikus-Dystrophie, rezidivierender Polychondritis, Sperma- & Hoden-Autoimmunität, Susac-Syndrom, Tolosa-Hunt-Syndrom, Vogt-Koyanagi-Harada-Krankheit, Anti-Synthetase-Syndrom, Autoimmun-Enteropathie, X-chromosomaler Immundysregulation-Polyendokrinopathie-Enteropathie (IPEX), mikroskopischer Kolitis, lymphoproliferativem Autoimmun-Syndrom (ALPS), ektodermalem Autoimmun-Polyendokrinopathie-Candidiasis-Dystrophie-Syndrom (APEX), Gicht, Pseudogicht, Amyloid, eosinophiler Fasziitis (Shulman-Syndrom), Progesteron-Überempfindlichkeit, amilialem Mittelmeerfieber (FMF), Tumornekrosefaktor(TNF)-Rezeptor-assoziiertem periodischem Fiebersyndrom (TRAPS), Hyperimmunoglobulinämie D mit periodischem Fiebersyndrom (HIDS), PAPA-Syndrom (pyogene Arthritis, Pyoderma gangrenosum, schwere zystische Akne), Mangel an Interleukin-1-Rezeptor-Antagonist (DIRA), Mangel an Interleukin-36-Rezeptor-Antagonist (DITRA), Cryopyrin-assoziierten periodischen Syndromen (CAPS), NLRP12-assoziierten autoinflammatorischen Störungen (NLRP12AD), periodischer aphthöser Fieberstomatitis (PFAPA), chronischer atypischer neutrophiler Dermatose mit Lipodystrophie und erhöhter Temperatur (CANDLE), Majeed-Syndrom, Blau-Syndrom (auch bekannt als juvenile systemische Granulomatose), Makrophagenaktivierungssyndrom, chronisch rezidivierender multifokaler Osteomyelitis (CRMO), familiärem autoinflammatorischem Kälte-Syndrom, mutierten Adenosin-Deaminase-2- und monogenen Interferonopathien, Schnitzler-Syndrom; familiärer Zylindromatose, kongenitaler B-Zell-Lymphozytose, OTULIN-assoziiertem autoinflammatorischem Syndrom, Diabetes mellitus, Typ 2, Insulinresistenz und metabolischem Syndrom, atherosklerotischen Krankheiten, und entzündlichen Nierenkrankheiten; und
- der Krebs ausgewählt ist aus der Gruppe, bestehend aus: akuter lymphoblastischer Leukämie, Erwachsene; akuter lymphoblastischer Leukämie, im Kindesalter; akuter myeloischer Leukämie, Erwachsene; Nebennierenrindenkarzinom; Nebennierenrindenkarzinom, im Kindesalter; AIDS-assoziiertem Lymphom; AIDS-assoziierten Malignomen; Analkrebs; Astrozytom, Kleinhirn, im Kindesalter; Astrozytom, Großhirn, im Kindesalter; Barrett-Ösophagus (prämalignes Syndrom); Gallengangskrebs, extrahepatisch; Blasenkrebs; Blasenkrebs im Kindesalter; Knochenkrebs, Osteosarkom/malignem fibrösem Histiozytom; Hirnstammgliom im Kindesalter; Hirntumor, Erwachsene; Hirntumor, Hirnstammgliom, im Kindesalter; Hirntumor, Kleinhirnastrozytom, im Kindesalter; Hirntumor, zerebralem Astrozytom/malignem Gliom, im Kindesalter; Hirntumor, Ependymom, im Kindesalter; Hirntumor, Medulloblastom, im Kindesalter; Hirntumor, supratentorialen primitiven neuroektodermalen Tumoren, im Kindesalter; Hirntumor, Gliom der Sehbahnen und des Hypothalamus, im Kindesalter; Hirntumor, im Kindesalter (sonstigen); Brustkrebs; Brustkrebs und Schwangerschaft; Brustkrebs, im Kindesalter; Brustkrebs, Männer; Bronchialadenomen/Karzinoiden, im Kindesalter; Karzinoidtumor, im Kindesalter; Karzinoidtumor des Magen-Darm-Trakts; Karzinom, Nebennierenrinde; Karzinom, Inselzellen; Karzinom unbekannter Primärherkunft; Lymphom des Zentralnervensystems, primär; Kleinhirnastrozytom, im Kindesalter; Großhirnastrozytom/malignem Gliom, im Kindesalter; Gebärmutterhalskrebs; Krebskrankheiten im Kindesalter; chronischer lymphatischer Leukämie; chronischer myeloischer Leukämie; chronischen myeloproliferativen Störungen; klarzelligem Sarkom der Sehnenscheiden; Dickdarmkrebs; kolorektalem Krebs; kolorektalem Krebs, im Kindesalter; kutanem T-Zell-Lymphom; Gebärmutterkörperkrebs; Ependymom, im Kindesalter; epithelialem Eierstockkrebs; Speiseröhrenkrebs; Speiseröhrenkrebs, im Kindesalter; Ewing-Tumoren; extrakraniellem Keimzelltumor, im Kindesalter; extragonadalem Keimzelltumor; extrahepatischem Gallengangskrebs; Augenkrebs, intraokularem Melanom; Augenkrebs, Retinoblastom; Gallenblasenkrebs; Magenkrebs; Magenkrebs, im Kindesalter; gastrointestinalem Karzinoidtumor; Keimzelltumor, extrakraniell, im Kindesalter; Keimzelltumor, extragonadal; Keimzelltumor, Eierstöcke; gestationellem Trophoblastentumor; Gliom, im Kindesalter, Hirnstamm; Gliom, im Kindesalter, Sehbahnbereich und Hypothalamus; Haarzell-Leukämie; Kopf- und Halskrebs; hepatozellulärem (Leber) Krebs; hepatozellulärem (Leber) Krebs, Erwachsene (primär); hepatozellulärem (Leber) Krebs, im Kindesalter (primär); Krebs der Speiseröhre; Hodgkin-Lymphom; Hodgkin-Lymphom, Erwachsene; Hodgkin-Lymphom, im Kindesalter; Hodgkin-Lymphom während der Schwangerschaft; Hypopharynxkrebs; Hypothalamus- und Sehbahnengliom, im Kindesalter; intraokularem Melanom; Inselzellkarzinom (endokrine Bauchspeicheldrüse); Krebs des endokrinen Systems; Kaposi-Sarkom; Nierenkrebs; Kehlkopfkrebs; Kehlkopfkrebs, im Kindesalter; Leukämie, akut lymphoblastisch, Erwachsene; Leukämie, akut lymphoblastisch, im Kindesalter; Leukämie, akut myeloisch, Erwachsene; Leukämie, akut myeloisch, im Kindesalter; Leukämie, chronisch lymphatisch; Leukämie, chronisch myeloisch; Leukämie, Haarzellen; lymphozytischem Lymphom; Lippen- und Mundhöhlenkrebs; Leberkrebs, Erwachsene (primär); Leberkrebs, im Kindesalter (primär); Lungenkrebs; Lungenkrebs, nichtkleinzellig; Lungenkrebs, kleinzellig; lymphoblastischer Leukämie, akut, Erwachsene; lymphoblastischer Leukämie, akut, im Kindesalter; lymphozytärer Leukämie, chronisch; Lymphom, AIDS-assoziiert; Lymphom, Zentralnervensystem (primär); Lymphom, kutan, T-Zellen; Lymphom, Hodgkin, Erwachsene; Lymphom, Hodgkin, im Kindesalter; Lymphom, Hodgkin, während der Schwangerschaft; Lymphom, Non-Hodgkin, Erwachsene; Lymphom, Non-Hodgkin, im Kindesalter; Lymphom, Non-Hodgkin, während der Schwangerschaft; Lymphom, primär, Zentralnervensystem; Makroglobulinämie, Waldenström; männlichem Brustkrebs Mann; malignem Mesotheliom, Erwachsene; malignem Mesotheliom, im Kindesalter; malignem Thymom; Medulloblastom, im Kindesalter; Melanom; Melanom, intraokular; Merkelzellkarzinom; Mesotheliom, maligne; metastasierendem Plattenepithelkrebs des Halses mit okkultem Primärtumor; Syndrom multipler endokriner Neoplasien, im Kindesalter; Multiplem Myelom/Plasmazellneoplasma; Mycosis fungoides; myelodysplastischen Syndromen; myeloischer Leukämie, chronisch; myeloischer Leukämie, akut, im Kindesalter; Myelom, multipel; myeloproliferativen Störungen, chronisch; Krebs der Nasenhöhle und der Nasennebenhöhlen; Nasopharynxkrebs; Nasopharynxkrebs, im Kindesalter; neoplastischer Hautkrankheit; Neuroblastom; Non-Hodgkin-Lymphom, Erwachsene; Non-Hodgkin-Lymphom, im Kindesalter; Non-Hodgkin-Lymphom während der Schwangerschaft; nichtkleinzelligem Lungenkrebs; Neoplasien des Zentralnervensystems; Haferzellkrebs; Mundhöhlenkrebs, im Kindesalter; Mundhöhlen- und Lippenkrebs; Oropharynxkrebs; Osteosarkom/malignem fibrösem Histiozytom des Knochens; Eierstockkrebs; Eierstockkrebs, im Kindesalter; epithelialem Eierstockkrebs; Eierstock-Keimzelltumor; Eierstocktumor mit geringem Malignitätspotenzial; pädiatrischer Malignität; Bauchspeicheldrüsenkrebs; Bauchspeicheldrüsenkrebs, im Kindesalter; Bauchspeicheldrüsekrebs, Inselzellen; Krebs der Nasennebenhöhlen und der Nasenhöhle; Nebenschilddrüsenkrebs; Peniskrebs; Phäochromozytom; primitiven neuroektodermalen Tumoren der Zirbeldrüse und des supratentoriellen Raums, im Kindesalter; Hypophysentumor; Plasmazellneoplasma/multiplem Myelom; pleuropulmonalem Blastom; Schwangerschaft und Brustkrebs; Schwangerschaft und Hodgkin-Lymphom; Schwangerschaft und Non-Hodgkin-Lymphom; primärem Lymphom des Zentralnervensystems; primärem Leberkrebs, Erwachsene; primärem Leberkrebs, im Kindesalter; Prostatakrebs; chronischer oder akuter Leukämie; soliden Tumoren, im Kindesalter; Hypereosinophilie; Rektumkrebs; Nierenzell(Nieren)-Krebs; Nierenzellkrebs, im Kindesalter; Nierenbecken und Harnleiter, Übergangszellkrebs; Retinoblastom; Rhabdomyosarkom, im Kindesalter; Speicheldrüsenkrebs; Speicheldrüsenkrebs, im Kindesalter; Sarkom, Ewing-Tumoren; Sarkom, Kaposi; Sarkom (Osteosarkom)/malignem fibröse Histiozytom des Knochens; Sarkom, Rhabdomyosarkom, im Kindesalter; Sarkomen, Weichteile; Sarkomen, Weichteile, Erwachsene; Sarkomen, Weichteile, im Kindesalter; Sezary-Syndrom; Hautkrebs; Hautkrebs, im Kindesalter; Hautkrebs (Melanom); Hautkarzinom, Merkelzellen; kleinzelligem Lungenkrebs; Dermatofibrosarkom protuberans; Dünndarmkrebs; Weichteilsarkom, Erwachsene; Weichteilsarkom, im Kindesalter; Kopf-Hals-Krebs; Plattenepithelkrebs des Halses mit okkultem Primärtumor, metastasiert; Magenkrebs; Magenkrebs, im Kindesalter; supratentorialen primitiven neuroektodermalen Tumoren, im Kindesalter; T-Zell-Lymphom, kutan; Hodenkrebs; Thymom, im Kindesalter; Thymom, maligne; Schilddrüsenkrebs; Schilddrüsenkrebs, im Kindesalter; Übergangszellkrebs des Nierenbeckens und des Harnleiters; Gestations-Trophoblastentumor; Krebs mit unbekanntem Primärtumor, im Kindesalter; seltenen Krebskrankheiten, im Kindesalter; Harnleiter und Nierenbecken, Übergangszellkrebs; Harnröhrenkrebs; Krebs des Harnleiters; Krebs des Penis; gynäkologischen Tumoren; Gebärmutterkrebs; Gebärmuttersarkom; Karzinom der Eileiter; Karzinom des Endometriums; Vaginalkrebs; Karzinom der Vagina; Karzinom der Vulva; Sehbahn- und Hypothalamusgliom, im Kindesalter; Vulvakrebs; Waldenström-Makroglobulinämie; und Wilms-Tumor.

12. Verbindung oder pharmazeutisch unbedenkliches Salz und/oder Solvat davon zur Verwendung oder pharmazeutische Zusammensetzung zur Verwendung nach Anspruch 11, wobei die entzündliche Krankheit oder die mit einer unerwünschten Immunantwort verknüpfte Krankheit ausgewählt ist aus der Gruppe, bestehend aus rheumatoider Arthritis, Psoriasis-Arthritis, ankylosierender Spondylitis, systemischem Lupus erythematodes, Multipler Sklerose, Psoriasis, entzündlicher Darmkrankheit, atopischer Dermatitis, Fibrose, Uveitis, Cryopyrin-assoziierten periodischen Syndromen, Muckle-Wells-Syndrom, juveniler idiopathischer Arthritis, chronisch obstruktiver Lungenkrankheit und Asthma.

13. Verbindung oder pharmazeutisch unbedenkliches Salz und/oder Solvat davon zur Verwendung oder pharmazeutische Zusammensetzung zur Verwendung nach einem der Ansprüche 9 bis 12 zur Verwendung in Kombination mit einem weiteren therapeutischen Wirkstoff, wobei der weitere therapeutische Wirkstoff ausgewählt ist aus der Gruppe, bestehend aus:
- einem Kortikosteroid (Glukokortikoid), Retinoid, Anthralin, Vitamin-D-Analogon, Calcineurin-Hemmern, Phototherapie oder Photochemotherapie oder anderen Formen der UV-Licht-Bestrahlungstherapie, Ciclosporin, einem Thiopurin, Methotrexat, einem Anti-TNFα-Wirkstoff, Phosphodiesterase-4(PDE4)-Hemmer, Anti-IL-17-Wirkstoff, Anti-IL12/IL-23-Wirkstoff, Anti-IL-23-Wirkstoff, JAK(Januskinase)-Hemmer, Plasmaaustausch, intravenösem Immunglobulin (IVIG), Cyclophosphamid, Anti-CD20-B-Zell-depletierendem Wirkstoff, Anthracyclin-Analogon, Cladribin, Sphingosin-1-Phosphat-Rezeptor-Modulator oder Sphingosin-Analogon, Interferon-beta-Präparat, Glatiramer, Anti-CD3-Therapie, Anti-CD52-Targeting-Wirkstoff, Leflunomid, Teriflunomid, Gold-Verbindung, Laquinimod, Kaliumkanalblocker, Mycophenolsäure, Mycophenolatmofetil, Purinanalogon, mTOR-Hemmer (mechanistisches Ziel von Rapamycin), Anti-Thymozytenglobulin (ATG), IL-2-Rezeptor(CD25)-Hemmer, Anti-IL-6-Rezeptor- oder Anti-IL-6-Wirkstoff, Bruton-Tyrosinkinase-Hemmer (BTK), Tyrosinkinase-Hemmer, Ursodeoxycholsäure, Hydroxychloroquin, Chloroquin, Hemmer des B-Zell-aktivierenden Faktors (BAFF, auch bekannt als BlyS, B-Lymphozyten-Stimulator), anderen B-Zellgerichtete Therapien, einschließlich eines Fusionsproteins, das sowohl gegen APRIL (A-Proliferation-Inducing Ligand) als auch den BlyS-PI3K-Hemmer, einschließlich Pan-Hemmer, gerichtet ist, oder eines, das gegen die p110δ- und/oder p110γ-haltige Isoform eines Interferon-α-Rezeptor-Hemmers gerichtet ist, T-Zell-Kostimulationsblocker, Thalidomid und seinen Derivaten, Dapson, Clofazimin, einem Leukotrienantagonist, Theophyllin, Anti-IgE-Therapie, einem Anti-IL-5-Wirkstoff, einem lang wirksamen Muskarinrezeptor, einem PDE4-Hemmer, Riluzol, einem Radikalfänger, einem Proteasom-Hemmer, einem Komplementkaskaden-Hemmer, einschließlich eines gegen C5 gerichteten Immunadsorbens, Antithymozytenglobulin, 5-Aminosalicylaten und deren Derivaten, einem Anti-Integrin-Wirkstoff, einschließlich eines gegen α4β1- und/oder α4β7-Integrine gerichteten, einem Anti-CD11-α-Wirkstoff, einem nichtsteroidalen Antirheumatikum (NSAR), einschließlich eines Salicylats, einer Propionsäure, einer Essigsäure, eines Oxicams, eines Fenamats, einem selektiven oder relativ selektiven COX-2-Hemmer, Colchicin, einem IL-4-Rezeptor-Hemmer, topischer/Kontaktimmuntherapie, Anti-IL-1-Rezeptortherapie, IL-1β-Hemmer, IL-1-neutralisierender Therapie, Chlorambucil, einem spezifisches Antibiotikum mit immunmodulatorischen Eigenschaften und/oder der Fähigkeit, NRF2 zu modulieren, antiandrogener Therapie, Pentoxifyllin, Ursodeoxycholsäure, Obeticholsäure, Fibrat, einem Modulator des Cystic Fibrosis Transmembrane Conductance Regulators (CFTR), einem VEGF-Hemmer (vaskulärer endothelialer Wachstumsfaktor), Pirfenidon und Mizoribin;
- einer palliativen Behandlung, ausgewählt aus der Gruppe, bestehend aus: Antiemetika, Schmerzmitteln, Medikamenten zur Senkung eines hohen Harnsäurespiegels im Blut, Antidepressiva, Sedativa, Antikonvulsiva, Laxanzien, Antidiarrhoika und Antazida;
- einer zusätzlichen Krebsbehandlung, ausgewählt aus der Gruppe, bestehend aus Chemotherapie, einer gerichteten Therapie, Immuntherapie und einer Hormontherapie; und
- einem Magen- oder Pankreaslipasehemmer; einem Lipidsenker; einem CB-1-Antagonisten; einem Lipoxygenasehemmer; einem Somostatinanalogon; einer Insulinverbindung oder einem Insulinanalogon; einem Insulinsensitizer, wie einem PPAR-gamma-Agonisten, PPAR-alpha-Agonisten oder gemischten PPAR-gamma/alpha-Agonisten; einem Insulinsekretagogum; einem SGLT2-Hemmer; einem Amylinanalogon; einem DPPIV-Hemmer; einem GLP-1-Agonisten; einem Alpha-Glucosidasehemmer; einem Phosphodiesterasehemmer; einem Glykogenphosphorylasehemmer; einem MCH-1-Antagonisten; einem Glucokinaseaktivator; einem Glucagonantagonisten; einem Insulinsignalagonisten; einem PTP1B-Hemmer; einem Glukoneogenesehemmer; einem GSK-Hemmer oder einem Galaninrezeptoragonisten.

14. Verfahren zum Herstellen einer Verbindung der Formel (Ia), wie in einem der Ansprüche 1 bis 7 beschrieben, oder ein Salz davon, das Folgendes umfasst:
(a) Umsetzen einer Verbindung der Formel (IIa): 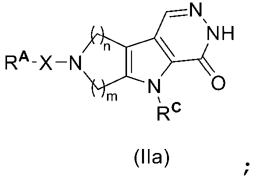
oder ein Salz davon;
mit einer Verbindung der Formel (IIIa): 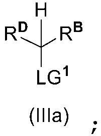
wobei R**^{A}**, R**^{B}**, R**^{C}**, R**^{D}**, X, m und n nach einem der Ansprüche 1 bis 6 definiert sind und LG**¹** eine Abgangsgruppe ist; oder
(b) Umsetzen einer Verbindung der Formel (IVa): 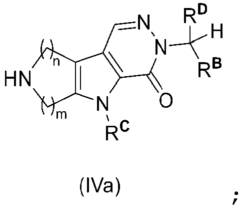
oder ein Salz davon;
mit einer Verbindung der Formel (Va): 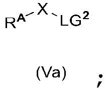
wobei R**^{A}**, R**^{B}**, R**^{C}**, R**^{D}**, X, m und n nach einem der Ansprüche 1 bis 6 definiert sind und LG**²** eine Abgangsgruppe ist.

15. Verbindung, ausgewählt aus der Gruppe, bestehend aus:
(a) einer Verbindung der Formel (IIa): 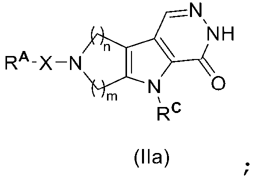
oder einem Salz davon;
wobei R**^{A}**, R**^{C}**, X, m und n nach einem der Ansprüche 1 bis 3, 5 oder 6 definiert sind; und
(b) einer Verbindung der Formel (IVa): 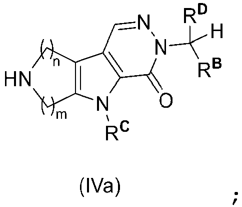
oder einem Salz davon;
wobei R**^{B}**, R**^{C}**, R**^{D}**, m und n nach einem der Ansprüche 1 oder 4 oder 6 definiert sind.

## Revendications

1. Composé de formule (Ia) : 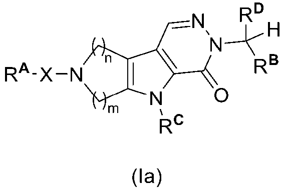 dans lequel,
R**^{A}** est alkyle en C₁₋₁₀, alcényle en C₂₋₁₀, alcynyle en C₂₋₁₀, cycloalkyle en C₃₋₁₀, phényle ou hétéroaryle à 5 à 10 chaînons ; dans lequel R^{A} est éventuellement substitué sur un atome disponible par un ou plusieurs R**^{1A}**, dans lequel chaque R**^{1A}** est choisi indépendamment parmi le groupe constitué de halo, alkyle en C₁₋₆, hydroxyalkyle en C₁₋₆, haloalkyle en C₁₋₆, hydroxy, O-alkyle en C₁₋₆, Ohaloalkyle en C₁₋₆, cyano, NR**^{2A}**R**^{3A}**, CO₂H, CONR**^{2A}**R**^{3A}** et cycloalkyle en C₃₋₆ ;
R**^{2A}** et R**^{3A}** sont choisis indépendamment parmi le groupe constitué de H et d'alkyle en C₁₋₆ ; ou R**^{2A}** et R**^{3A}**, conjointement avec l'atome N auquel ils sont attachés, se combinent pour former un cycle hétérocyclique à 5 à 7 chaînons qui est éventuellement substitué sur un atome disponible par un ou plusieurs groupes choisis parmi alkyle en C₁₋₂ et oxo ;
R**^{B}** est phényle, phényle fusionné à un cycle hétérocyclique à 5 à 7 chaînons, hétéroaryle à 5 à 10 chaînons ou hétérocyclyle à 4 à 7 chaînons ; dans lequel R**^{B}** est éventuellement substitué sur un atome disponible par un ou plusieurs R**^{1B}**, dans lequel chaque R**^{1B}** est choisi indépendamment parmi le groupe constitué de halo, alkyle en C₁₋₆, hydroxyalkyle en C₁₋₆, aminoalkyle en C₁₋₆, haloalkyle en C₁₋₆, hydroxy, O-alkyle en C₁₋₆, Ohaloalkyle en C₁₋₆, cyano, NR**^{2B}**R**^{3B}**, CO₂H, CONR**^{2B}**R**^{3B}**, S(O)₂NR**^{2B}**R**^{3B}**, S(O)₂alkyle en C₁₋₆, cycloalkyle en C₃₋₆ et oxo ;
R**^{2B}** et R**^{3B}** sont choisis indépendamment parmi le groupe constitué de H et d'alkyle en C₁₋₆ ; ou R**^{2B}** et R**^{3B}**, ainsi que l'atome N auquel ils sont attachés, se combinent pour former un cycle hétérocyclique à 5 à 7 chaînons qui est éventuellement substitué sur un atome disponible par un ou plusieurs groupes choisis parmi alkyle en C₁₋₂ et oxo ;
R**^{C}** est H ou alkyle en C₁₋₂ ;
R**^{D}** est H, alkyle en C₁₋₂, hydroxyalkyle en C₁₋₂ ou méthoxyalkyle en C₁₋₂ ;
X est C=O, S(=O)₁₋₂, -CH₂-S(=O)₁₋₂-, -S(=O)(=NH) ou -NH-S(=O)₂- ; n est compris entre 0 et 4 et m est compris entre 0 et 4, dans lequel n + m vaut 2, 3 ou 4 ;
ou un sel et/ou solvate pharmaceutiquement acceptables de celui-ci.

2. Composé, sel pharmaceutiquement acceptables et/ou solvate de celui-ci selon la revendication 1, dans lequel R**^{A}** est alkyle en C₁₋₁₀, cycloalkyle en C₃₋₁₀, phényle ou hétéroaryle à 5 à 10 chaînons, et est éventuellement substitué sur un atome disponible par un ou plusieurs R**^{1A},** dans lequel chaque R**^{1A}** est choisi indépendamment parmi le groupe constitué de halo, alkyle en C₁₋₆, hydroxyalkyle en C₁₋₆, haloalkyle en C₁₋₆, hydroxy, O-alkyle en C₁₋₆, Ohaloalkyle en C₁₋₆, cyano et NR**^{2A}**R**^{3A}**.

3. Composé, sel pharmaceutiquement acceptables et/ou solvate de celui-ci selon la revendication 2, dans lequel R**^{A}** est méthyle, éthyle ou n-propyle et est éventuellement substitué par O-alkyle en C₁₋₄, ou dans lequel R**^{A}** est phényle éventuellement substitué sur un atome de carbone disponible par O-alkyle en C₁₋₆.

4. Composé, sel pharmaceutiquement acceptables et/ou solvate de celui-ci selon l'une quelconque des revendications 1 à 3, dans lequel R**^{B}** est choisi parmi le groupe constitué de phényle, phényle fusionné à un cycle hétérocyclique à 5 à 7 chaînons, pyrrolyle, furanyle, thiényle, imidazolyle, pyrazolyle, thiazolyle, isothiazolyle, oxadiazolyle, thiadiazolyle, triazolyle, oxazolyle, isoxazolyle, tétrazolyle, pyridinyle, pyrimidinyle, pyridazinyle, pyrazinyle, furo[3,2-b]pyridinyle, pyrazolo[1,5-a]pyridinyle, pyrrolo[2,3-b]pyridinyle, pyrrolo[2,3-c]pyridinyle, imidazo[1,2-a]pyridinyle, thiéno[3,2-c]pyridinyle, indolyle, indazolyle, benzofuranyle, benzimidazolyle, benzothiazolyle, benzothiophényle, quinolinyle, isoquinolinyle, quinazolinyle, pyrrolidinyle, tétrahydrofuranyle, tétrahydrothiényle, tétrahydropyranyle, pipéridinyle, pipérazinyle, morpholinyle, thiomorpholinyle, pyridone et pyridazinone ; et est éventuellement substitué sur un atome disponible par un ou plusieurs R**^{1B}**, dans lequel chaque R**^{1B}** est choisi indépendamment parmi le groupe constitué de halo, alkyle en C₁₋₄, hydroxyalkyle en C₁₋₄, aminoalkyle en C₁₋₆, haloalkyle en C₁₋₄, hydroxy, O-alkyle en C₁₋₄, Ohaloalkyle en C₁₋₄, cyano, NR**^{2B}**R**^{3B}**, CONR**^{2B}**R**^{3B}**, S(O)₂NR**^{2B}**R**^{3B}**, S(O)₂alkyle en C₁₋₆, cycloalkyle en C₃₋₅ et oxo.

5. Composé, sel pharmaceutiquement acceptables et/ou solvate de celui-ci selon l'une quelconque des revendications 1 à 4, dans lequel R**^{C}** est méthyle, R**^{D}** est H et X est S(=O)₂.

6. Composé, sel pharmaceutiquement acceptables et/ou solvate de celui-ci selon l'une quelconque des revendications 1 à 5, dans lequel n vaut 2 et m vaut 1 et le composé de formule (Ia) est un composé de formule (IaA) : 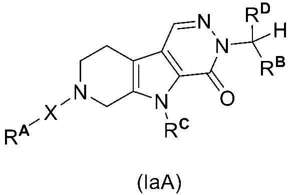
dans lequel R**^{A}**, R**^{B}**, R**^{C}**, R**^{D}** et X sont tels que définis selon l'une quelconque des revendications 1 à 5 ;
ou un sel et/ou solvate pharmaceutiquement acceptables de celui-ci.

7. Composé, sel et/ou solvate pharmaceutiquement acceptables de celui-ci selon la revendication 1, choisi parmi le groupe constitué des éléments suivants :
3-((2,3-dihydrobenzofuran-5-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-((6-méthoxypyridin-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-(3-(1-hydroxyéthyl)benzyl)-5-méthyl-7-(méthylsulfonyl)-3,5,6,7,8,9-hexahydro-4H-pyrido[4',3':4,5]pyrrolo[2,3-d]pyridazin-4-one ;
3-((1H-pyrazol-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-(2-fluoro-3-méthoxybenzyl)-5-méthyl-7-(méthylsulfonyl)-3,5,6,7,8,9-hexahydro-4H-pyrido[4',3':4,5]pyrrolo[2,3-d]pyridazin-4-one ;
3-(2,6-difluoro-4-méthoxybenzyl)-5-méthyl-7-(méthylsulfonyl)-3,5,6,7,8,9-hexahydro-4H-pyrido[4',3':4,5]pyrrolo[2,3-d]pyridazin-4-one ;
3-(3-aminobenzyl)-5-méthyl-7-(méthylsulfonyl)-3,5,6,7,8,9-hexahydro-4H-pyrido[4',3':4,5]pyrrolo[2,3-d]pyridazin-4-one ; 7-acétyl-3-(3-aminobenzyl)-5-méthyl-3,5,6,7,8,9-hexahydro-4H-pyrido[4',3':4,5]pyrrolo[2,3-d]pyridazin-4-one ;
3-(3-aminobenzyl)-7-(cyclopropylsulfonyl)-5-méthyl-3,5,6,7,8,9-hexahydro-4H-pyrido[4',3':4,5]pyrrolo[2,3-d]pyridazin-4-one ;
3-(3-aminobenzyl)-5-méthyl-7-((2-méthylthiazol-5-yl)sulfonyl)-3,5,6,7,8,9-hexahydro-4H-pyrido[4',3':4,5]pyrrolo[2,3-d]pyridazin-4-one ;
3-(3-aminobenzyl)-5-méthyl-7-(phénylsulfonyl)-3,5,6,7,8,9-hexahydro-4H-pyrido[4',3':4,5]pyrrolo[2,3-d]pyridazin-4-one ; et
3-((1H-pyrazol-3-yl)méthyl)-5-méthyl-7-(phénylsulfonyl)-3,5,6,7,8,9-hexahydro-4H-pyrido[4',3':4,5]pyrrolo[2,3-d]pyridazin-4-one ;
3-((2-(hydroxyméthyl)thiophén-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-((5-méthyl-7-(méthylsulfonyl)-4-oxo-4,5,6,7,8,9-hexahydro-3H-pyrido[4',3':4,5]pyrrolo[2,3-d]pyridazin-3-yl)méthyl)thiophène-2-carboxamide ;
3-((1H-indol-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-(benzofuran-3-ylméthyl)-5-méthyl-7-(méthylsulfonyl)-3,5,6,7,8,9-hexahydro-4H-pyrido[4',3':4,5]pyrrolo[2,3-d]pyridazin-4-one ;
3-((5-méthyl-7-(méthylsulfonyl)-4-oxo-4,5,6,7,8,9-hexahydro-3H-pyrido[4',3':4,5]pyrrolo[2,3-d]pyridazin-3-yl)méthyl)-1H-pyrazole-4-carboxamide ;
3-((5-méthyl-7-(méthylsulfonyl)-4-oxo-4,5,6,7,8,9-hexahydro-3H-pyrido[4',3':4,5]pyrrolo[2,3-d]pyridazin-3-yl)méthyl)benzènesulfonamide ;
5-méthyl-7-(méthylsulfonyl)-3-((6-(pyrrolidin-1-yl)pyridin-2-yl)méthyl)-3,5,6,7,8,9-hexahydro-4H-pyrido[4',3':4,5]pyrrolo[2,3-d]pyridazin-4-one ;
3-((1H-indazol-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-(2-fluoro-4-méthoxybenzyl)-5-méthyl-7-(phénylsulfonyl)-3,5,6,7,8,9-hexahydro-4H-pyrido[4',3':4,5]pyrrolo[2,3-d]pyridazin-4-one ;
3-((7-((4-méthoxyphényl)sulfonyl)-5-méthyl-4-oxo-4,5,6,7,8,9-hexahydro-3H-pyrido[4',3':4,5]pyrrolo[2,3-d]pyridazin-3-yl)méthyl)thiophène-2-carboxamide ;
3-((2,3-dihydrobenzofuran-5-yl)méthyl)-7-((4-méthoxyphényl)sulfonyl)-5-méthyl-3,5,6,7,8,9-hexahydro-4H-pyrido[4',3':4,5]pyrrolo[2,3-d]pyridazin-4-one ;
3-((2,2-diméthyl-2,3-dihydrobenzofuran-5-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-((1H-pyrrolo[2,3-b]pyridin-6-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
(S)-3-(3-(1-aminoéthyl)benzyl)-5-méthyl-7-(méthylsulfonyl)-3,5,6,7,8,9-hexahydro-4H-pyrido[4',3':4,5]pyrrolo[2,3-d]pyridazin-4-one ;
7-((2-méthoxyéthyl)sulfonyl)-3-((6-méthoxypyridin-3-yl)méthyl)-5-méthyl-3,5,6,7,8,9-hexahydro-4H-pyrido[4',3':4,5]pyrrolo[2,3-d]pyridazin-4-one ;
N-(2-méthoxyéthyl)-3-((6-méthoxypyridin-3-yl)méthyl)-5-méthyl-4-oxo-3,4,5,6,8,9-hexahydro-7H-pyrido[4',3':4,5]pyrrolo[2,3-d]pyridazine-7-sulfonamide ;
3-((1H-pyrrolo[2,3-c]pyridin-3-yl)méthyl)-7-((2-méthoxyéthyl)sulfonyl)-5-méthyl-6,7,8,9-tétrahydro-3H-pyrido[4',3':4,5]pyrrolo[2,3-d]pyridazin-4(5H)-one ;
(3-((1H-pyrrolo[2,3-c]pyridin-3-yl)méthyl)-N-éthyl-5-méthyl-4-oxo-5,6,8,9-tétrahydro-3H-pyrido[4',3':4,5]pyrrolo[2,3-d]pyridazine-7(4H)-sulfonamide ;
5-méthyl-7-(méthylsulfonyl)-3-(3-(méthylsulfonyl)benzyl)-3,5,6,7,8,9-hexahydro-4H-pyrido[4',3':4,5]pyrrolo[2,3-*d*]pyridazin-4-one ;
3-((1*H*-indazol-4-yl)méthyl)-5-méthyl-7-(méthylsulfonyl)-3,5,6,7,8,9-hexahydro-4*H*-pyrido[4',3':4,5]pyrrolo[2,3-*d*]pyridazin-4-one ;
3-(3-(2-hydroxypropan-2-yl)benzyl)-5-méthyl-7-(méthylsulfonyl)-3,5,6,7,8,9-hexahydro-4H-pyrido[4',3':4,5]pyrrolo[2,3-d]pyridazin-4-one ;
5-méthyl-7-(méthylsulfonyl)-3-(thiéno[3,2-c]pyridin-3-ylméthyl)-3,5,6,7,8,9-hexahydro-4H-pyrido[4',3':4,5]pyrrolo[2,3-d]pyridazin-4-one ;
3-((1H-pyrrolo[2,3-c]pyridin-3-yl)méthyl)-7-((4-méthoxyphényl)sulfonyl)-5-méthyl-3,5,6,7,8,9-hexahydro-4H-pyrido[4',3':4,5]pyrrolo[2,3-d]pyridazin-4-one ;
3-((1H-pyrrolo[2,3-c]pyridin-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-((1H-pyrrolo[2,3-b]pyridin-3-yl)méthyl)-5-méthyl-7-(méthylsulfonyl)-3,5,6,7,8,9-hexahydro-4H-pyrido[4',3':4,5]pyrrolo[2,3-d]pyridazin-4-one ;
3-((5-fluoro-1H-indol-3-yl)méthyl)-5-méthyl-7-(méthylsulfonyl)-6,7,8,9-tétrahydro-3H-pyrido[4',3':4,5]pyrrolo[2,3-d]pyridazin-4(5H)-one ; et
3-((6-fluoro-1H-indol-3-yl)méthyl)-5-méthyl-7-(méthylsulfonyl)-6,7,8,9-tétrahydro-3H-pyrido[4',3':4,5]pyrrolo[2,3-d]pyridazin-4(5H)-one ;
ou un sel et/ou solvate pharmaceutiquement acceptables de ceux-ci.

8. Composition pharmaceutique comprenant un composé, un sel et/ou solvate pharmaceutiquement acceptables de celui-ci selon l'une quelconque des revendications 1 à 7, et un ou plusieurs diluants ou supports pharmaceutiquement acceptabless.

9. Composé, sel et/ou solvate pharmaceutiquement acceptables de celui-ci selon l'une quelconque des revendications 1 à 7 ou composition pharmaceutique selon la revendication 8 pour une utilisation en tant que médicament.

10. Composé, ou sel et/ou solvate pharmaceutiquement acceptables de celui-ci pour une utilisation, ou composition pharmaceutique pour une utilisation selon la revendication 9, pour traiter ou prévenir une maladie inflammatoire, une maladie associée à une réponse immunitaire indésirable, un cancer, l'obésité, une maladie diabétique ou un trouble sanguin.

11. Composé, ou sel et/ou solvate pharmaceutiquement acceptables de celui-ci pour une utilisation, ou composition pharmaceutique pour une utilisation selon la revendication 10, dans lequel :
- la maladie inflammatoire ou la maladie associée à une réponse immunitaire indésirable est, ou est associée à, une maladie choisie parmi le groupe constitué des éléments suivants : psoriasis, asthme, bronchopneumopathie chronique obstructive (BPCO), insuffisance cardiaque, infarctus du myocarde, angine de poitrine, autres troubles liés à l'athérosclérose et/ou à l'athérothrombose, maladie mitochondriale et neurodégénérative, rétinopathie paranéoplasique auto-immune, rejet de greffe, sclérose en plaques, myélite transverse, lésion d'ischémie-reperfusion, lésion du génome induite par les AGE, maladie inflammatoire de l'intestin, cholangite sclérosante primitive (CSP), syndrome de chevauchement CSP-hépatite auto-immune, stéatose hépatique non alcoolique (stéatohépatite non alcoolique), rhumatisme granulome annulaire, lupus érythémateux cutané (LEC), lupus érythémateux disséminé (LED), néphrite lupique, lupus médicamenteux, myocardite ou myopéricardite auto-immune, syndrome de Dressler, myocardite à cellules géantes, syndrome post-péricardiotomie, syndromes d'hypersensibilité médicamenteuse, eczéma, sarcoïdose, érythème noueux, encéphalomyélite aiguë disséminée (ADEM), troubles du spectre de la neuromyélite optique, troubles associés aux anticorps anti-MOG (glycoprotéine de la myéline oligodendrocytaire), névrite optique, CLIPPERS (inflammation lymphocytaire chronique avec rehaussement périvasculaire pontique sensible aux stéroïdes), sclérose myélinoclastique diffuse, maladie d'Addison, alopécie areata, spondylarthrite ankylosante, autres spondylarthrites, syndrome des anticorps antiphospholipides, anémie hémolytique auto-immune, hépatite auto-immune, maladie auto-immune de l'oreille interne, pemphigoïde, maladie à IgA linéaire, maladie de Behçet, maladie cœliaque, maladie de Chagas, dermatomyosite, diabète sucré de type I, endométriose, syndrome de Goodpasture, maladie de Graves, syndrome de Guillain-Barré et ses sous-types, neuropathie inflammatoire progressive, maladie de Hashimoto, hidradénite suppurée, myosite à corps d'inclusion, myopathie nécrosante, maladie de Kawasaki, néphropathie à IgA, purpura de Henoch-Schönlein, purpura thrombocytopénique idiopathique, purpura thrombocytopénique thrombotique (PTT), syndrome d'Evans, cystite interstitielle, connectivite mixte, connectivite indifférenciée, morphée, myasthénie grave, narcolepsie, neuromyotonie, pemphigus vulgaire, anémie pernicieuse, rhumatisme psoriasique, polymyosite, cholangite biliaire primitive (également connue sous le nom de cirrhose biliaire primitive), polyarthrite rhumatoïde, rhumatisme palindromique, schizophrénie, syndromes d'encéphalite auto-immune, syndromes de méningoencéphalite auto-immune, sclérodermie, syndrome de Sjögren, syndrome de la personne raide, polymyalgie rhumatismale, artérite à cellules géantes (artérite temporale), artérite de Takayasu, périartérite noueuse, maladie de Kawasaki, granulomatose avec polyangéite (GPA ; anciennement connue sous le nom de granulomatose de Wegener), granulomatose éosinophile avec polyangéite (EGPA ; anciennement connue sous le nom de syndrome de Churg-Strauss), polyartérite/polyangéite microscopique, vascularite urticarienne hypocomplémentémique, vascularite d'hypersensibilité, cryoglobulinémie, thromboangéite oblitérante (maladie de Buerger), vascularite, vascularite leucocytoclasique, vitiligo, encéphalomyélite aiguë disséminée, adrénoleucodystrophie, maladie d'Alexander, maladie d'Alper, sclérose concentrique de Balo ou maladie de Marburg, pneumonie cryptogénique organisée (anciennement connue sous le nom de bronchiolite oblitérante organisée), maladie de Canavan, syndrome de vascularite du système nerveux central, maladie de Charcot-Marie-Tooth, ataxie infantile avec hypomyélinisation du système nerveux central, polyneuropathie inflammatoire démyélinisante chronique (PIDC), rétinopathie diabétique, leucodystrophie à cellules globoïdes (maladie de Krabbe), maladie du greffon contre l'hôte (GVHD), infection ou complication liée à l'hépatite C (VHC), infection ou complication liée au virus de l'herpès simplex, infection ou complication liée au virus de l'immunodéficience humaine (VIH), lichen plan, amyotrophie monomélique, fibrose, mucoviscidose, hypertension artérielle pulmonaire (HTAP), sarcoïdose pulmonaire, fibrose pulmonaire idiopathique, fibrose rénale, asthme pédiatrique, dermatite atopique, dermatite allergique, dermatite de contact, rhinite allergique, rhinite, sinusite, conjonctivite, conjonctivite allergique, kératoconjonctivite sèche, sécheresse oculaire, xérophtalmie, glaucome, œdème maculaire, œdème maculaire diabétique, occlusion de la veine centrale de la rétine (OVCR), dégénérescence maculaire, inflammation postopératoire de la cataracte, uvéite, iridocyclite, sclérite, rejet de greffe de cornée et de cellules limbiques, entéropathie sensible au gluten (maladie cœliaque), dermatite herpétiforme, œsophagite à éosinophiles, achalasie, dysautonomie auto-immune, encéphalomyélite auto-immune, oophorite auto-immune, orchite auto-immune, pancréatite auto-immune, aortite et péri-aortite, rétinopathie auto-immune, urticaire auto-immune, maladie de Behçet, maladie de Castleman, syndrome de Cogan, maladie liée aux IgG4, fibrose rétropéritonéale, arthrite juvénile idiopathique, maladie de Still de l'adulte, conjonctivite ligneuse, ulcère de Mooren, pityriasis lichénoïde et varioliforme aigu (PLEVA, également connu sous le nom de maladie de Mucha-Habermann), neuropathie motrice multifocale (NMM), syndrome neuropsychiatrique pédiatrique aigu (PANS), syndromes paranéoplasiques, encéphalomyélite périveineuse, dystrophie sympathique réflexe, polychondrite récidivante, auto-immunité spermatique et testiculaire, syndrome de Susac, syndrome de Tolosa-Hunt, maladie de Vogt-Koyanagi-Harada, syndrome des antisynthétases, entéropathie auto-immune, entéropathie polyendocrinienne avec dysrégulation immunitaire liée à l'X (IPEX), colite microscopique, syndrome lymphoprolifératif auto-immun (ALPS), syndrome de polyendocrinopathie auto-immune-candidose-dystrophie ectodermique (APEX), goutte, pseudogoutte, amyloïde, fasciite éosinophile (syndrome de Shulman), hypersensibilité à la progestérone, fièvre méditerranéenne familiale (FMF), syndrome de fièvre périodique associé au récepteur du facteur de nécrose tumorale (TNF) (TRAPS), syndrome d'hyperimmunoglobulinémie D avec fièvre périodique (HIDS), syndrome PAPA (arthrite pyogène, pyoderma gangrenosum, acné kystique sévère), déficit en antagoniste du récepteur de l'interleukine-1 (DIRA), déficit en antagoniste du récepteur de l'interleukine-36 (DITRA), syndromes périodiques associés à la cryopyrine (CAPS), troubles auto-inflammatoires associés à NLRP12 (NLRP12AD), stomatite aphteuse à fièvre périodique (PFAPA), dermatose neutrophilique atypique chronique avec lipodystrophie et température élevée (CANDLE), syndrome de Majeed, syndrome de Blau (également connu sous le nom de granulomatose systémique juvénile), syndrome d'activation des macrophages, ostéomyélite multifocale chronique récurrente (CRMO), syndrome auto-inflammatoire familial au froid, adénosine désaminase 2 mutante et interféronopathies monogéniques, syndrome de Schnitzler ; cylindromatose familiale, lymphocytose congénitale à cellules B, syndrome auto-inflammatoire lié à OTULIN, diabète sucré de type 2, résistance à l'insuline et syndrome métabolique, troubles athérosclérotiques et troubles inflammatoires rénaux ; et
- le cancer est choisi parmi le groupe constitué des éléments suivants : leucémie lymphoblastique aiguë de l'adulte ; leucémie lymphoblastique aiguë de l'enfant ; leucémie myéloïde aiguë de l'adulte ; carcinome corticosurrénalien ; carcinome corticosurrénalien de l'enfant ; lymphome associé au sida ; tumeurs malignes associées au sida ; cancer anal ; astrocytome cérébelleux de l'enfant ; astrocytome cérébral de l'enfant ; œsophage de Barrett (syndrome prémalin) ; cancer des voies biliaires extrahépatiques ; cancer de la vessie ; cancer de la vessie de l'enfant ; cancer des os, ostéosarcome/histiocytome fibreux malin ; gliome du tronc cérébral de l'enfant ; tumeur cérébrale de l'adulte ; tumeur cérébrale, gliome du tronc cérébral de l'enfant ; tumeur cérébrale, astrocytome cérébelleux de l'enfant ; tumeur cérébrale, astrocytome cérébral/gliome malin de l'enfant ; tumeur cérébrale, épendymome de l'enfant ; tumeur cérébrale, médulloblastome de l'enfant ; tumeur cérébrale, tumeurs neuroectodermiques primitives supratentorielles de l'enfant ; tumeur cérébrale, gliome des voies visuelles et hypothalamique de l'enfant ; tumeur cérébrale de l'enfant (autre) ; cancer du sein ; cancer du sein et grossesse ; cancer du sein de l'enfant ; cancer du sein chez l'homme ; adénomes/tumeurs carcinoïdes bronchiques de l'enfant ; tumeur carcinoïde de l'enfant ; tumeur carcinoïde gastro-intestinale ; carcinome corticosurrénalien ; carcinome à cellules insulaires ; carcinome de primitif inconnu ; lymphome primitif du système nerveux central ; astrocytome cérébelleux de l'enfant ; astrocytome cérébral/gliome malin de l'enfant ; cancer du col de l'utérus ; cancers de l'enfant ; leucémie lymphoïde chronique ; leucémie myéloïde chronique ; syndromes myéloprolifératifs chroniques ; sarcome à cellules claires des gaines tendineuses ; cancer du côlon ; cancer colorectal ; cancer colorectal de l'enfant ; lymphome cutané à cellules T ; cancer de l'endomètre ; épendymome de l'enfant ; cancer épithélial de l'ovaire ; cancer de l'œsophage ; cancer de l'œsophage de l'enfant ; tumeurs de la famille du sarcome d'Ewing ; tumeur germinale extracrânienne de l'enfant ; tumeur germinale extragonadique ; cancer des voies biliaires extrahépatiques ; cancer de l'œil, mélanome intraoculaire ; cancer de l'œil, rétinoblastome ; cancer de la vésicule biliaire ; cancer de l'estomac ; cancer de l'estomac de l'enfant ; tumeur carcinoïde gastro-intestinale ; tumeur germinale extracrânienne de l'enfant ; tumeur germinale extragonadique ; tumeur germinale ovarienne ; tumeur trophoblastique gestationnelle ; gliome du tronc cérébral de l'enfant ; gliome des voies visuelles et hypothalamique de l'enfant ; leucémie à tricholeucocytes ; cancer de la tête et du cou ; carcinome hépatocellulaire (foie) ; carcinome hépatocellulaire (foie) primitif de l'adulte ; carcinome hépatocellulaire (foie) primitif de l'enfant ; cancer de l'œsophage ; lymphome de Hodgkin ; lymphome de Hodgkin de l'adulte ; lymphome de Hodgkin de l'enfant ; lymphome de Hodgkin pendant la grossesse ; cancer de l'hypopharynx ; gliome hypothalamique et des voies visuelles de l'enfant ; mélanome intraoculaire ; carcinome à cellules insulaires (pancréas endocrine) ; cancer du système endocrinien ; sarcome de Kaposi ; cancer du rein ; cancer du larynx ; cancer du larynx de l'enfant ; leucémie lymphoblastique aiguë de l'adulte ; leucémie lymphoblastique aiguë de l'enfant ; leucémie myéloïde aiguë de l'adulte ; leucémie myéloïde aiguë de l'enfant ; leucémie lymphoïde chronique ; leucémie myéloïde chronique ; leucémie à tricholeucocytes ; lymphome lymphocytaire ; cancer des lèvres et de la cavité buccale ; cancer du foie de l'adulte (primitif) ; cancer du foie de l'enfant (primitif) ; cancer du poumon ; cancer du poumon non à petites cellules ; cancer du poumon à petites cellules ; leucémie lymphoblastique aiguë de l'adulte ; leucémie lymphoblastique aiguë de l'enfant ; leucémie lymphoïde chronique ; lymphome associé au sida ; lymphome du système nerveux central (primitif) ; lymphome cutané à cellules T ; lymphome de Hodgkin de l'adulte ; lymphome de Hodgkin de l'enfant ; lymphome de Hodgkin pendant la grossesse ; lymphome non hodgkinien de l'adulte ; lymphome non hodgkinien de l'enfant ; lymphome non hodgkinien pendant la grossesse ; lymphome primitif du système nerveux central ; macroglobulinémie de Waldenström ; cancer du sein chez l'homme ; mésothéliome malin de l'adulte ; mésothéliome malin de l'enfant ; thymome malin ; médulloblastome de l'enfant ; mélanome ; mélanome intraoculaire ; carcinome à cellules de Merkel ; mésothéliome malin ; cancer épidermoïde métastatique du cou avec primitif occulte ; syndrome de néoplasie endocrinienne multiple de l'enfant ; myélome multiple/néoplasie à plasmocytes ; mycosis fongoïde ; syndromes myélodysplasiques ; leucémie myéloïde chronique ; leucémie myéloïde aiguë de l'enfant ; myélome multiple ; syndromes myéloprolifératifs chroniques ; cancer des fosses nasales et des sinus paranasaux ; cancer du nasopharynx ; cancer du nasopharynx de l'enfant ; affection cutanée néoplasique ; neuroblastome ; lymphome non hodgkinien de l'adulte ; lymphome non hodgkinien de l'enfant ; lymphome non hodgkinien pendant la grossesse ; cancer du poumon non à petites cellules ; tumeurs du système nerveux central ; cancer à petites cellules ; cancer de la bouche de l'enfant ; cancer de la cavité buccale et des lèvres ; cancer de l'oropharynx ; ostéosarcome/histiocytome fibreux malin des os ; cancer de l'ovaire ; cancer de l'ovaire de l'enfant ; cancer épithélial de l'ovaire ; tumeur germinale de l'ovaire ; tumeur ovarienne à faible potentiel de malignité ; cancer pédiatrique ; cancer du pancréas ; cancer du pancréas de l'enfant ; cancer des cellules insulaires du pancréas ; cancer des sinus paranasaux et des fosses nasales ; cancer de la parathyroïde ; cancer du pénis ; phéochromocytome ; tumeurs neuroectodermiques primitives pinéales et supratentorielles de l'enfant ; tumeur hypophysaire ; néoplasie à plasmocytes/myélome multiple ; blastome pleuropulmonaire ; grossesse et cancer du sein ; grossesse et lymphome de Hodgkin ; grossesse et lymphome non hodgkinien ; lymphome primitif du système nerveux central ; cancer primitif du foie de l'adulte ; cancer primitif du foie de l'enfant ; cancer de la prostate ; leucémie chronique ou aiguë ; tumeurs solides de l'enfant ; hyperéosinophilie ; cancer du rectum ; cancer du rein ; cancer du rein de l'enfant ; cancer à cellules transitionnelles du bassinet rénal et de l'uretère ; rétinoblastome ; rhabdomyosarcome de l'enfant ; cancer des glandes salivaires ; cancer des glandes salivaires de l'enfant ; sarcome de la famille des tumeurs d'Ewing ; sarcome de Kaposi ; sarcome (ostéosarcome)/histiocytome fibreux malin des os ; sarcome, rhabdomyosarcome de l'enfant ; sarcomes des tissus mous ; sarcome des tissus mous de l'adulte ; sarcome des tissus mous de l'enfant ; syndrome de Sézary ; cancer de la peau ; cancer de la peau de l'enfant ; cancer de la peau (mélanome) ; carcinome cutané à cellules de Merkel ; cancer du poumon à petites cellules ; dermatofibrosarcome protubérant ; cancer de l'intestin grêle ; sarcome des tissus mous de l'adulte ; sarcome des tissus mous de l'enfant ; cancer de la tête et du cou ; cancer épidermoïde du cou avec primitif occulte, métastatique ; cancer de l'estomac ; cancer de l'estomac de l'enfant ; tumeurs neuroectodermiques primitives supratentorielles de l'enfant ; lymphome cutané à cellules T ; cancer du testicule ; thymome de l'enfant ; thymome malin ; cancer de la thyroïde ; cancer de la thyroïde de l'enfant ; cancer à cellules transitionnelles du bassinet rénal et de l'uretère ; tumeur trophoblastique gestationnelle ; cancer de l'enfant à site primaire inconnu ; cancers inhabituels de l'enfant ; cancer à cellules transitionnelles de l'uretère et du bassinet rénal ; cancer de l'urètre ; cancer de l'uretère ; cancer du pénis ; tumeurs gynécologiques ; cancer de l'utérus ; sarcome utérin ; carcinome des trompes de Fallope ; carcinome de l'endomètre ; cancer du vagin ; carcinome du vagin ; carcinome de la vulve ; gliome des voies visuelles et de l'hypothalamus de l'enfant ; cancer de la vulve ; macroglobulinémie de Waldenström ; et tumeur de Wilms.

12. Composé, ou sel et/ou solvate pharmaceutiquement acceptables de celui-ci pour une utilisation, ou composition pharmaceutique pour une utilisation selon la revendication 11, dans lequel la maladie inflammatoire ou la maladie associée à une réponse immunitaire indésirable est choisie parmi le groupe constitué des éléments suivants : polyarthrite rhumatoïde, rhumatisme psoriasique, spondylarthrite ankylosante, lupus érythémateux disséminé, sclérose en plaques, psoriasis, maladie inflammatoire de l'intestin, dermatite atopique, fibrose, uvéite, syndromes périodiques associés à la cryopyrine, syndrome de Muckle-Wells, arthrite juvénile idiopathique, bronchopneumopathie chronique obstructive et asthme.

13. Composé ou sel et/ou solvate pharmaceutiquement acceptables de celui-ci pour une utilisation, ou composition pharmaceutique pour une utilisation, selon l'une quelconque des revendications 9 à 12, pour une utilisation en association avec un agent thérapeutique supplémentaire, dans lequel l'agent thérapeutique supplémentaire est choisi parmi le groupe constitué des éléments suivants :
- un corticostéroïde (glucocorticoïde), un rétinoïde, une anthraline, un analogue de la vitamine D, un inhibiteur de la calcineurine, la photothérapie ou la photochimiothérapie ou toute autre forme d'irradiation par ultraviolets, la ciclosporine, une thiopurine, le méthotrexate, un agent anti-TNFα, un inhibiteur de la phosphodiestérase-4 (PDE4), un agent anti-IL-17, un agent anti-IL-12/IL-23, un agent anti-IL-23, un inhibiteur de JAK (Janus Kinase), les échanges plasmatiques, les immunoglobulines intraveineuses (IgIV), le cyclophosphamide, un agent dépléteur des lymphocytes B anti-CD20, un analogue de l'anthracycline, la cladribine, un modulateur du récepteur de la sphingosine-1-phosphate ou un analogue de la sphingosine, une préparation d'interféron bêta, le glatiramère, un traitement anti-CD3, un agent ciblant le CD52, le léflunomide, le tériflunomide, un composé d'or, le laquinimod, un bloqueur des canaux potassiques, l'acide mycophénolique, le mycophénolate mofétil, un analogue de la purine, un inhibiteur de la voie mTOR (cible mécanistique de la rapamycine), une globuline antithymocytaire (ATG), un inhibiteur du récepteur de l'IL-2 (CD25), un agent anti-récepteur de l'IL-6 ou anti-IL-6, un inhibiteur de la tyrosine kinase de Bruton (BTK), un inhibiteur de la tyrosine kinase, l'acide ursodésoxycholique, l'hydroxychloroquine, la chloroquine, un inhibiteur du facteur d'activation des lymphocytes B (BAFF, également connu sous le nom de BlyS, stimulateur des lymphocytes B), une autre thérapie ciblée sur les lymphocytes B, y compris une protéine de fusion ciblant à la fois APRIL (ligand inducteur de prolifération) et BlyS, un inhibiteur de PI3K, y compris un inhibiteur pan-PI3K ou un inhibiteur ciblant les isoformes contenant p110δ et/ou p110γ, un inhibiteur du récepteur de l'interféron α, un bloqueur de la costimulation des lymphocytes T, la thalidomide et ses dérivés, la dapsone, la clofazimine, un antagoniste des leucotriènes, la théophylline, un traitement anti-IgE, un agent anti-IL-5, un agent muscarinique à longue durée d'action, un inhibiteur de la PDE4, le riluzole, un piégeur de radicaux libres, un inhibiteur du protéasome, un inhibiteur de la cascade du complément, dont un dirigé contre C5, l'immunoadsor, la globuline antithymocytaire, les 5-aminosalicylates et leurs dérivés, un agent anti-intégrine, dont un ciblant les intégrines α4β1 et/ou α4β7, un agent anti-CD11-α, un anti-inflammatoire non stéroïdien (AINS), dont un salicylate, un acide propionique, un acide acétique, un oxicam, un fénamate, un inhibiteur sélectif ou relativement sélectif de la COX-2, la colchicine, un inhibiteur du récepteur de l'IL-4, une immunothérapie topique/de contact, un traitement anti-récepteur de l'IL-1, un inhibiteur de l'IL-1β, un traitement neutralisant l'IL-1, le chlorambucil, un antibiotique spécifique doté de propriétés immunomodulatrices et/ou de la capacité de moduler NRF2, un traitement anti-androgénique, la pentoxifylline, l'acide ursodésoxycholique, l'acide obéticholique, un fibrate, un modulateur du régulateur de conductance transmembranaire de la fibrose kystique (CFTR), un inhibiteur du VEGF (facteur de croissance endothélial vasculaire), la pirfénidone et la mizoribine ;
- un traitement palliatif choisi parmi le groupe constitué des éléments suivants : agents antiémétiques, médicaments destinés à soulager la douleur, médicaments utilisés pour diminuer les taux élevés d'acide urique dans le sang, antidépresseurs, sédatifs, anticonvulsivants, laxatifs, antidiarrhéiques et antiacides ;
- un traitement anticancéreux supplémentaire choisi parmi la chimiothérapie, une thérapie ciblée, l'immunothérapie et l'hormonothérapie ; et
- un inhibiteur de la lipase gastrique ou pancréatique ; un agent hypolipémiant ; un antagoniste de CB-1 ; un inhibiteur de la lipoxygénase ; un analogue de la somatostatine ; un composé d'insuline ou un analogue de l'insuline ; un agent sensibilisant à l'insuline tel qu'un agoniste de PPAR-gamma, un agoniste de PPAR-alpha ou un agoniste mixte de PPAR-gamma/alpha ; un sécrétagogue de l'insuline ; un inhibiteur de SGLT2 ; un analogue de l'amyline ; un inhibiteur de DPPIV ; un agoniste de GLP-1 ; un inhibiteur de l'alpha-glucosidase ; un inhibiteur de la phosphodiestérase ; un inhibiteur de la glycogène phosphorylase ; un antagoniste de MCH-1 ; un activateur de la glucokinase ; un antagoniste du glucagon ; un agoniste de la signalisation de l'insuline ; un inhibiteur de PTP1B ; un inhibiteur de la gluconéogenèse ; un inhibiteur de GSK ou un agoniste du récepteur de la galanine.

14. Procédé de préparation d'un composé de formule (Ia) tel que décrit selon l'une quelconque des revendications 1 à 7, ou d'un sel de celui-ci, qui comprend :
(a) la réaction d'un composé de formule (IIa) : 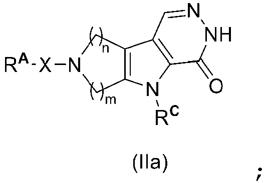
ou d'un sel de celui-ci ;
avec un composé de formule (IIIa) : 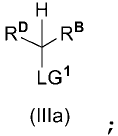
dans lequel R**^{A}**, R**^{B}**, R**^{C}**, R**^{D}**, X, m et n sont définis selon l'une quelconque des revendications 1 à 6, et LG**¹** est un groupe partant ; ou
(b) la réaction d'un composé de formule (IVa) : 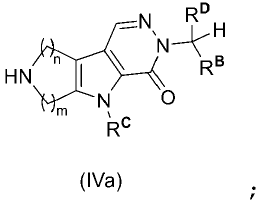
ou d'un sel de celui-ci ;
avec un composé de formule (Va) : 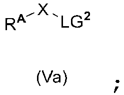
dans lequel R**^{A}**, R**^{B}**, R**^{C}**, R**^{D}**, X, m et n sont définis selon l'une quelconque des revendications 1 à 6, et LG**²** est un groupe partant.

15. Composé choisi parmi le groupe constitué des éléments suivants :
(a) un composé de formule (IIa) : 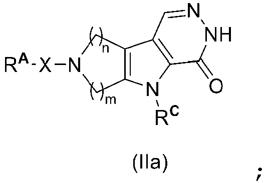
ou un sel de celui-ci ;
dans lequel R**^{A}**, R**^{C}**, X, m et n sont définis selon l'une quelconque des revendications 1 à 3, 5 ou 6 ; et
(b) un composé de formule (IVa) : 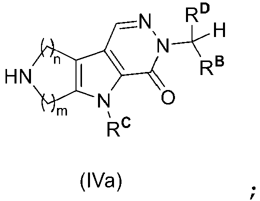
ou un sel de celui-ci ;
dans lequel R**^{B}**, R**^{C}**, R**^{D}**, m et n sont définis selon l'une quelconque des revendications 1 ou 4 à 6.
